(19) Europäisches Patentamt
European Patent Office
Office européen des brevets

(11) EP 4 286 378 A1

(12) EUROPEAN PATENT APPLICATION
published in accordance with Art. 153(4) EPC

(43) Date of publication:
**06.12.2023 Bulletin 2023/49**

(21) Application number: **22745316.4**

(22) Date of filing: **27.01.2022**

(51) International Patent Classification (IPC):
***C07D 403/14*** (2006.01) ***C07D 403/04*** (2006.01)
***C07D 491/052*** (2006.01) ***A61K 31/517*** (2006.01)
***A61K 31/519*** (2006.01) ***A61P 35/00*** (2006.01)
***A61P 35/02*** (2006.01)

(52) Cooperative Patent Classification (CPC):
**A61K 31/517; A61K 31/519; A61P 35/00; A61P 35/02; C07D 403/04; C07D 403/14; C07D 491/052**

(86) International application number:
**PCT/CN2022/074390**

(87) International publication number:
**WO 2022/161443 (04.08.2022 Gazette 2022/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **01.02.2021 CN 202110139674**
**09.03.2021 CN 202110258547**
**24.06.2021 CN 202110706033**
**20.01.2022 CN 202210070174**

(71) Applicant: **Medshine Discovery Inc.**
**Nanjing, Jiangsu 210032 (CN)**

(72) Inventors:
- **ZHANG, Yang**
  **Shanghai 200131 (CN)**
- **WU, Wentao**
  **Shanghai 200131 (CN)**
- **LI, Zhixiang**
  **Shanghai 200131 (CN)**
- **ZHU, Wenyuan**
  **Shanghai 200131 (CN)**
- **YANG, Ping**
  **Shanghai 200131 (CN)**
- **LI, Qiu**
  **Shanghai 200131 (CN)**
- **LI, Jian**
  **Shanghai 200131 (CN)**
- **CHEN, Shuhui**
  **Shanghai 200131 (CN)**

(74) Representative: **D Young & Co LLP**
**120 Holborn**
**London EC1N 2DY (GB)**

(54) **PYRIMIDOPYRAN COMPOUND**

(57) The present application relates to a pyrimidopyran compound, and specifically discloses a compound as represented by formula (III), and a pharmaceutically acceptable salt thereof.

EP 4 286 378 A1

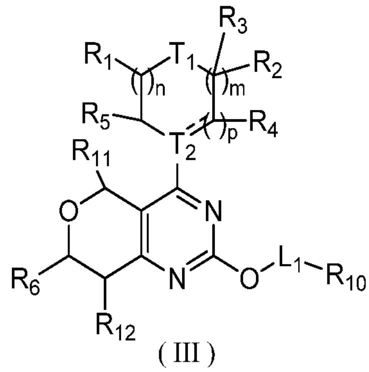

( III )

## Description

**This application claims the priority of:**

CN202110139674.X, filed on February 01, 2021;
CN202110258547.1, filed on March 09, 2021;
CN202110706033.8, filed on June 24, 2021;
CN202210070174.X, filed on January 20, 2022.

## FIELD OF THE INVENTION

The present disclosure relates to a class of pyrimidopyran compounds, specifically to a compound represented by formula (III) and a pharmaceutically acceptable salt thereof.

## BACKGROUND OF THE INVENTION

RAS oncogene mutations are the most common activating mutations in human cancers, occurring in 30% of human tumors. The RAS gene family includes three subtypes (KRAS, HRAS, and NRAS), and 85% of RAS-driven cancers are caused by mutations in KRAS subtypes. KRAS mutations are commonly found in solid tumors, such as lung adenocarcinoma, pancreatic ductal carcinoma and colorectal cancer, etc. In KRAS mutated tumors, 80% of oncogenic mutations occur at codon 12, and the most common mutations include: p.G12D (41%), p.G12V (28%), and p.G12C (14%).

KRAS is a murine sarcoma virus oncogene and is an important member of the RAS protein. KRAS is like a molecular switch, which can control and regulate the path of cell growth when it is normal. After mutation, KRAS gene can independently transmit signals for growth and proliferation to downstream pathways independent of upstream growth factor receptor signals, causing uncontrolled cell growth and tumor progression. Meanwhile, whether KRAS gene has mutations or not is also an important indicator of tumor prognosis.

Currently, small molecules that directly target KRAS mutations are mainly concentrated in the field of $KRAS^{G12C}$, wherein Amgen's AMG510 and Mirati Therapeutics' MRTX849 have shown good therapeutic effects on tumor patients with $KRAS^{G12C}$ mutation in clinical studies. But so far, no small molecule that targets $KRAS^{G12D}$ enters a clinical research stage, and tumor patients with $KRAS^{G12D}$ mutation have not yet benefited from precision medicine.

## SUMMARY OF THE INVENTION

The present disclosure provides a compound represented by formula (III) or a pharmaceutically acceptable salt thereof,

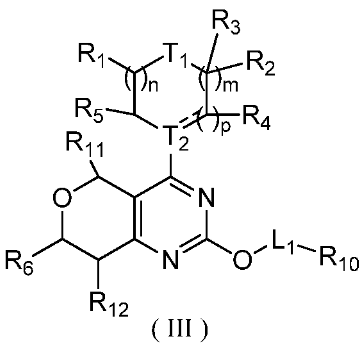

(III)

,

structural moiety

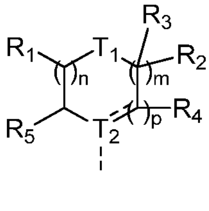

is selected from

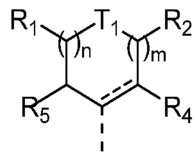, 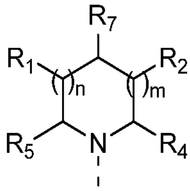,

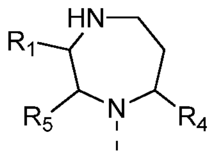, 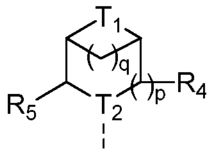, 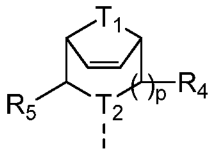, 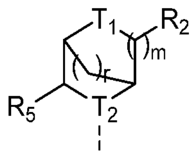, 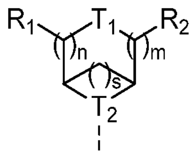,

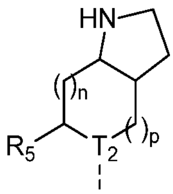, 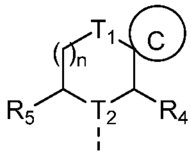 and 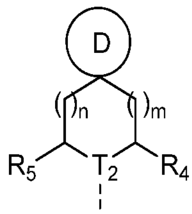;

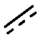 is selected from a single bond and a double bond;

$T_1$ is selected from $CR_7R_8$, $NR_9$ and O;

$T_2$ is selected from CH and N;

Li is selected from $-CH_2-$ and a bond;

$R_1$, $R_2$, $R_3$, $R_4$ and $R_5$ are each independently selected from H and $C_{1-3}$ alkyl, wherein the $C_{1-3}$ alkyl is optionally substituted by 1, 2 or 3 $R_a$;

$R_6$ is selected from $C_{6-10}$ aryl and 5-10 membered heteroaryl, wherein the $C_{6-10}$ aryl and 5-10 membered heteroaryl are optionally substituted by 1, 2, 3, 4 or 5 $R_b$;

$R_7$ and $R_8$ are each independently selected from H, $CH_3$ and $NH_2$;

$R_9$ is selected from H and $CH_3$;

$R_{10}$ is selected from 4-8 membered heterocycloalkyl and

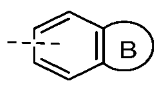,

wherein the 4-8 membered heterocycloalkyl and

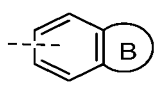

are optionally substituted by 1, 2 or 3 $R_c$;

$R_{11}$ and $R_{12}$ are each independently selected from H, $C_{1-3}$ alkyl and $C_{3-5}$ cycloalkyl, wherein the $C_{1-3}$ alkyl and $C_{3-5}$ cycloalkyl are optionally substituted by 1, 2 or 3 halo;

structural moiety

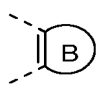

is 5-6 membered heterocycloalkenyl;
structural moiety

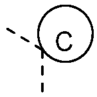

is $C_{3-5}$ cycloalkyl;
structural moiety

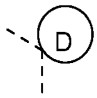

is 4-5 membered heterocycloalkyl;
m is selected from 0, 1 and 2;
n is selected from 0, 1 and 2;
p is selected from 1 and 2;
q is selected from 1, 2 and 3;
r is selected from 1 and 2;
s is selected from 1, 2 and 3;
$R_a$ is each independently selected from F, Cl, Br and I;
$R_b$ is each independently selected from F, Cl, Br, I, OH, $NH_2$, CN, $C_{1-3}$ alkyl, $C_{1-3}$ alkoxy, $C_{2-3}$ alkynyl, $C_{2-3}$ alkenyl, -C(=O)$C_{1-3}$ alkyl and $C_{3-5}$ cycloalkyl, wherein the $C_{1-3}$ alkyl, $C_{1-3}$ alkoxy, $C_{2-3}$ alkynyl, $C_{2-3}$ alkenyl, -C(=O)$C_{1-3}$ alkyl and $C_{3-5}$ cycloalkyl are optionally substituted by 1, 2, 3, 4 or 5 R;
$R_c$ is each independently selected from H, F, Cl, Br, I, OH, CN, $C_{1-3}$ alkyl, $C_{1-3}$ alkoxy and -$C_{1-3}$ alkyl-O-C(=O)-Ci-s alkylamino;
R is each independently selected from F, Cl, Br and I.

In some embodiments of the present disclosure, the $R_1$, $R_2$, $R_3$, $R_4$ and $R_5$ are each independently selected from H, $CH_3$, $CH_2CH_3$ and $CH(CH_3)_2$, wherein the $CH_3$, $CH_2CH_3$ and $CH(CH_3)_2$ are optionally substituted by 1, 2 or 3 $R_a$, and other variables are as defined in the present disclosure.

In some embodiments of the present disclosure, the $R_1$, $R_2$, $R_3$, $R_4$ and $R_5$ are each independently selected from H and $CH_3$, and other variables are as defined in the present disclosure.

In some embodiments of the present disclosure, the structural moiety

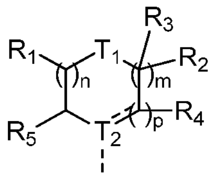

is selected from

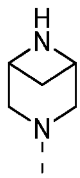 , 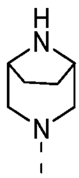 , 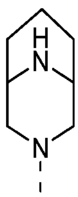 , 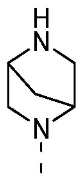 , 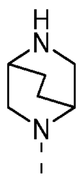 , 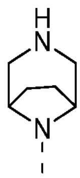 , 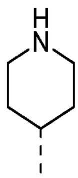 ,

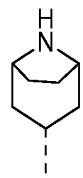 , 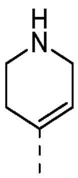 , 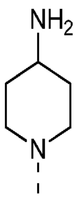 , 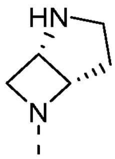 , 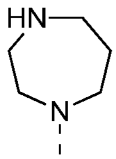 , 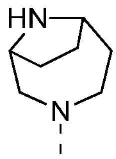 , 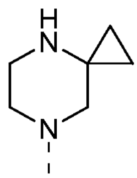 , 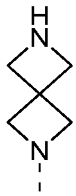 , 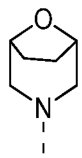

and

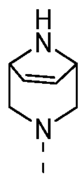 ,

and other variables are as defined in the present disclosure.

In some embodiments of the present disclosure, the structural moiety

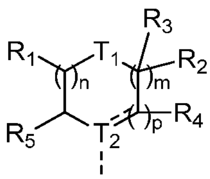

is selected from

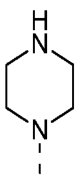 , 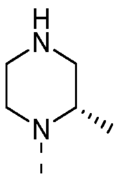 , 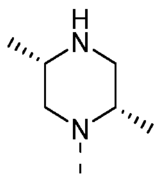 , 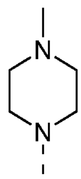 , 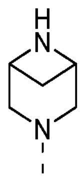 , 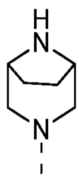 ,

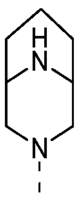 , 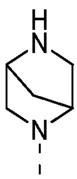 , 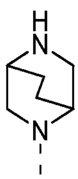 , 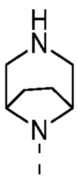 , 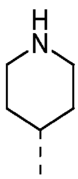 , 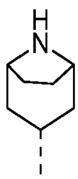 , 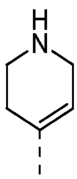 , 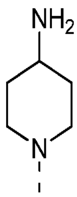 , 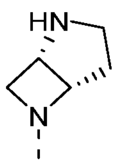 , 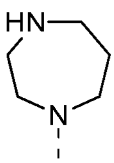 , 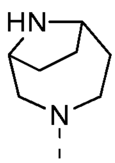 ,

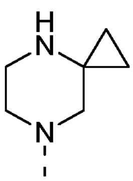 , 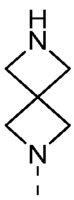 , 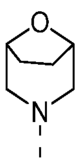 and 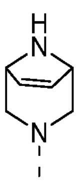 ,

and other variables are as defined in the present disclosure.

In some embodiments of the present disclosure, the $R_b$ is each independently selected from F, Cl, Br, I, OH, $NH_2$, CN, $CH_3$, $CH_2CH_3$, $OCH_3$, $OCH_2CH_3$, $-CH=CH_2$, $-CH_2-CH=CH_2$, $-C\equiv CH$ , $-C(=O)CH_3$ and cyclopropyl, wherein the $CH_3$, $CH_2CH_3$, $OCH_3$, $OCH_2CH_3$, $- CH=CH_2$, $-CH_2-CH=CH_2$, $-C\equiv CH$ , $-C(=O)CH_3$ and cyclopropyl are optionally substituted by 1, 2, 3, 4 or 5 R, and other variables are as defined in the present disclosure.

In some embodiments of the present disclosure, the $R_b$ is each independently selected from F, Cl, Br, I, OH,

$NH_2$, CN, $CH_3$, $CF_3$, $CH_2CH_3$, $CF_2CF_3$, $-CH=CH_2$, $-C\equiv CH$ , $-C(=O)CH_3$ and cyclopropyl, and other variables are as defined in the present disclosure.

In some embodiments of the present disclosure, the $R_6$ is selected from phenyl, pyridyl, naphthyl, indolyl and indazolyl, wherein the phenyl, pyridyl, naphthyl, indolyl and indazolyl are optionally substituted by 1, 2, 3, 4 or 5 $R_b$, and other variables are as defined in the present disclosure.

In some embodiments of the present disclosure, the $R_6$ is selected from

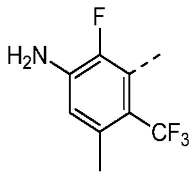 , 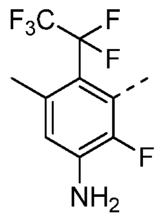 , 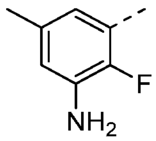 , 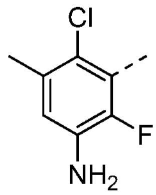 , 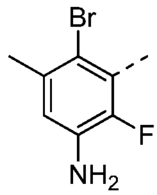 , 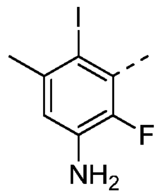 ,

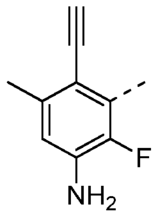 , 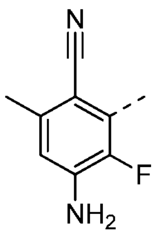 , 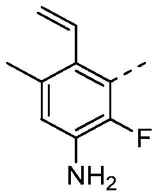 , 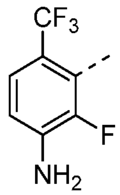 , 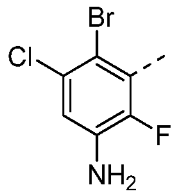 , 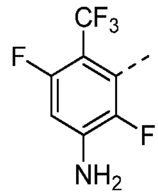 ,

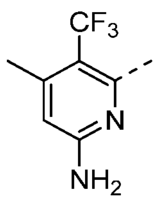 , 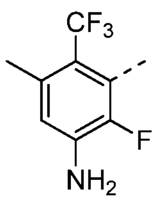 , 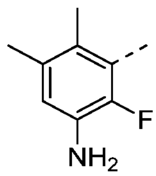 , 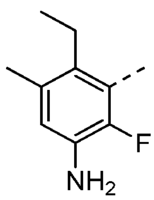 , 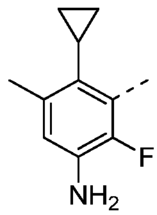 , 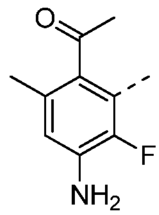 ,

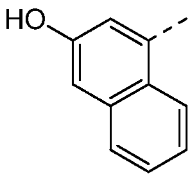 , 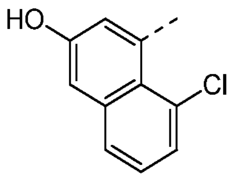 , 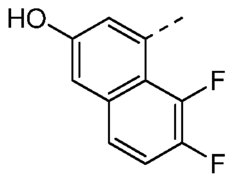 , 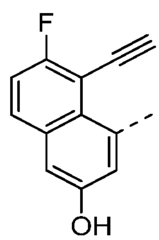 , 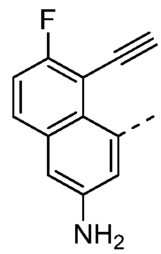

and

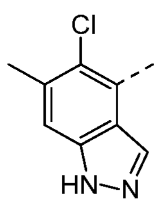 ,

and other variables are as defined in the present disclosure.

In some embodiments of the present disclosure, the $R_c$ is each independently selected from H, F, Cl, Br, OH, CN, $CH_3$, $CH_2CH_3$, $CH_2CF_3$, $OCH_3$, $OCF_3$ and

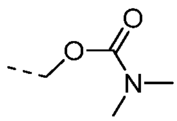

and other variables are as defined in the present disclosure.

In some embodiments of the present disclosure, the $R_{10}$ is selected from tetrahydropyrrolyl, hexahydro-1H-pyrrofizinyl and 1,2,3,4-tetrahydroisoquinolinyl, wherein the tetrahydropyrrolyl, hexahydro-1H-pyrrolizinyl and 1,2,3,4-tetrahydroisoquinolinyl are optionally substituted by 1, 2 or 3 $R_c$, and other variables are as defined in the present disclosure.

In some embodiments of the present disclosure, the $R_{10}$ is selected from

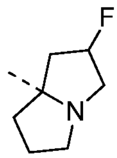

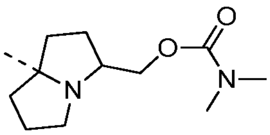

and

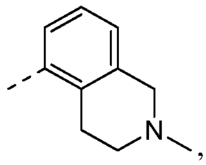

and other variables are as defined in the present disclosure.

In some embodiments of the present disclosure, the $R_{10}$ is

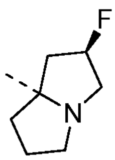

and other variables are as defined in the present disclosure.

In some embodiments of the present disclosure, the $R_{11}$ and $R_{12}$ are each independently selected from H and $CH_3$, and other variables are as defined in the present disclosure.

The present disclosure provides a compound represented by formula (III) or a pharmaceutically acceptable salt thereof,

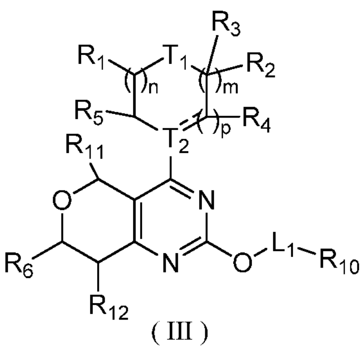

( III ) ,

structural moiety

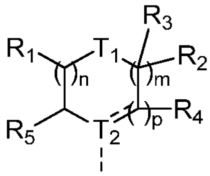

is selected from

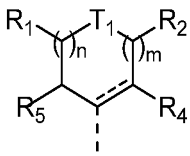,
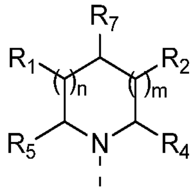,

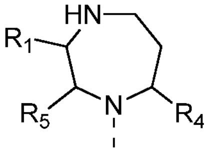,
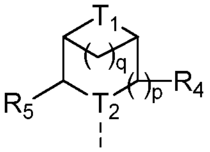,
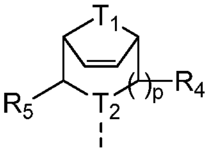,
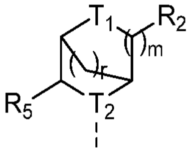,
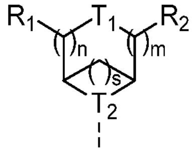,

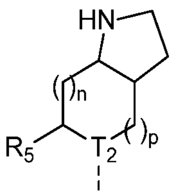,
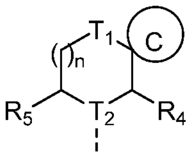 and
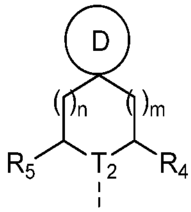;

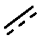 is selected from a single bond and a double bond;
$T_1$ is selected from $CR_7R_8$, $NR_9$ and O;
$T_2$ is selected from CH and N;
Li is selected from $-CH_2-$ and a bond;
$R_1$, $R_2$, $R_3$, $R_4$ and $R_5$ are each independently selected from H and $C_{1-3}$ alkyl, wherein the $C_{1-3}$ alkyl is optionally substituted by 1, 2 or 3 $R_a$;
$R_6$ is selected from $C_{6-10}$ aryl and 5-10 membered heteroaryl, wherein the $C_{6-10}$ aryl and 5-10 membered heteroaryl are optionally substituted by 1, 2, 3, 4 or 5 $R_b$;
$R_7$ and $R_8$ are each independently selected from H, $CH_3$ and $NH_2$;
$R_9$ is selected from H and $CH_3$;
$R_{10}$ is selected from 4-8 membered heterocycloalkyl and

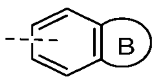,

wherein the 4-8 membered heterocycloalkyl and

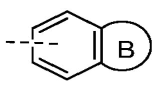

are optionally substituted by 1, 2 or 3 $R_c$;

$R_{11}$ and $R_{12}$ are each independently selected from H, $C_{1-3}$ alkyl and $C_{3-5}$ cycloalkyl, wherein the $C_{1-3}$ alkyl and $C_{3-5}$ cycloalkyl are optionally substituted by 1, 2 or 3 halo;
structural moiety

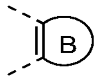

is 5-6 membered heterocycloalkenyl;
structural moiety

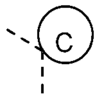

is $C_{3-5}$ cycloalkyl;
structural moiety

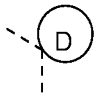

is 4-5 membered heterocycloalkyl;
m is selected from 0, 1 and 2;
n is selected from 0, 1 and 2;
p is selected from 1 and 2;
q is selected from 1, 2 and 3;
r is selected from 1 and 2;
s is selected from 1, 2 and 3;
$R_a$ is each independently selected from F, Cl, Br and I;
$R_b$ is each independently selected from F, Cl, Br, I, OH, $NH_2$, CN, $C_{1-3}$ alkyl, $C_{1-3}$ alkoxy, $C_{2-3}$ alkynyl, $C_{2-3}$ alkenyl and $C_{3-5}$ cycloalkyl, wherein the $C_{1-3}$ alkyl, $C_{1-3}$ alkoxy, $C_{2-3}$ alkynyl, $C_{2-3}$ alkenyl and $C_{3-5}$ cycloalkyl are optionally substituted by 1, 2 or 3 R;
$R_c$ is each independently selected from H, F, Cl, Br, OH, CN, $C_{1-3}$ alkyl, $C_{1-3}$ alkoxy and -$C_{1-3}$ alkyl-O-CO-$C_{1-3}$ alkylamino;
R is each independently selected from F, Cl and Br;

In some embodiments of the present disclosure, the $R_1$, $R_2$, $R_3$, $R_4$ and $R_5$ are each independently selected from H, $CH_3$, $CH_2CH_3$ and $CH(CH_3)_2$, wherein the $CH_3$, $CH_2CH_3$ and $CH(CH_3)_2$ are optionally substituted by 1, 2 or 3 $R_a$, and other variables are as defined in the present disclosure.

In some embodiments of the present disclosure, the $R_1$, $R_2$, $R_3$, $R_4$ and $R_5$ are each independently selected from H and $CH_3$, and other variables are as defined in the present disclosure.

In some embodiments of the present disclosure, the structural moiety

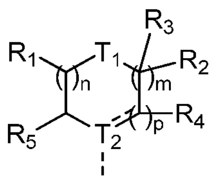

is selected from

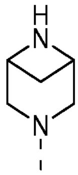, 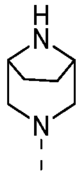, 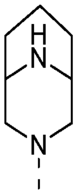, 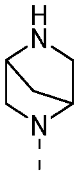, 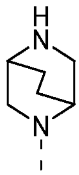, 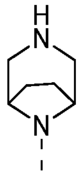, 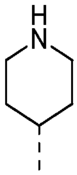,

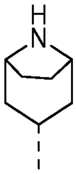, 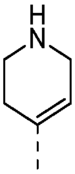, 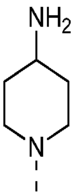, 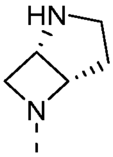, 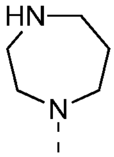, 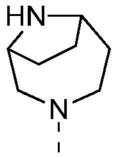, 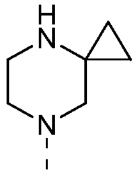, 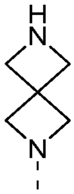, 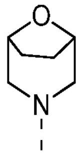

and

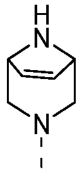,

and other variables are as defined in the present disclosure.

In some embodiments of the present disclosure, the structural moiety

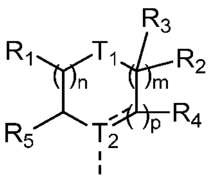

is selected from

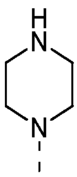, 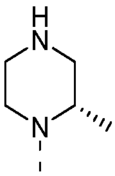, 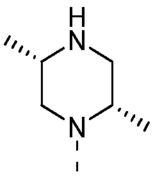, 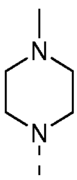, 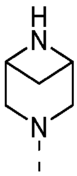, 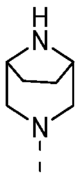,

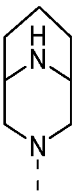, 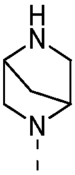, 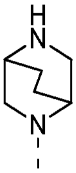, 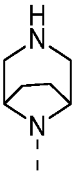, 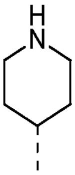, 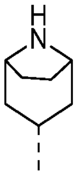, 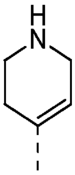, 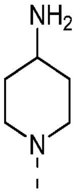, 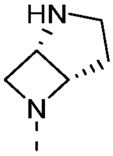, 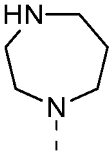, 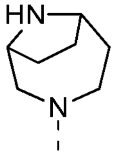,

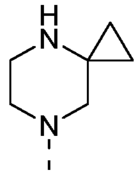, 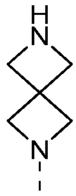, 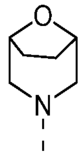 and 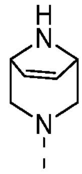,

and other variables are as defined in the present disclosure.

In some embodiments of the present disclosure, the $R_b$ is each independently selected from F, Cl, Br, I, OH, $NH_2$, CN, $CH_3$, $CH_2CH_3$, $OCH_3$, $OCH_2CH_3$, $-CH=CH_2$, $-CH_2-CH=CH_2$ and $-C\equiv CH$ , wherein the $CH_3$, $CH_2CH_3$, $OCH_3$, $OCH_2CH_3$, $-CH=CH_2$, $-CH_2-CH=CH_2$ and $-C\equiv CH$ are optionally substituted by 1, 2 or 3 R, and other variables are as defined in the present disclosure.

In some embodiments of the present disclosure, the $R_b$ is each independently selected from F, OH, $NH_2$, $CH_3$, $CF_3$, $CH_2CH_3$ and $-C\equiv CH$ , and other variables are as defined in the present disclosure.

In some embodiments of the present disclosure, the $R_6$ is selected from phenyl, naphthyl, indolyl and indazolyl, wherein the phenyl, naphthyl, indolyl and indazolyl are optionally substituted by 1, 2, 3, 4 or 5 $R_b$, and other variables are as defined in the present disclosure.

In some embodiments of the present disclosure, the $R_6$ is selected from

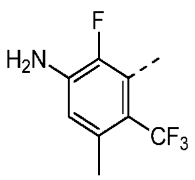, 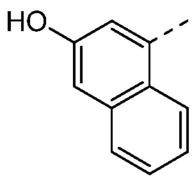, 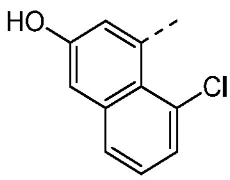, 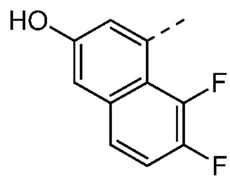 and 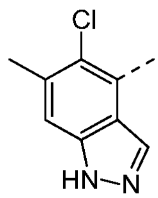,

and other variables are as defined in the present disclosure.

In some embodiments of the present disclosure, the $R_c$ is each independently selected from H, F, Cl, Br, OH, CN, $CH_3$, $CH_2CH_3$, $CH_2CF_3$, $OCH_3$, $OCF_3$ and

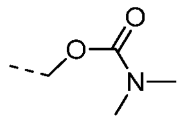,

and other variables are as defined in the present disclosure.

In some embodiments of the present disclosure, the $R_{10}$ is selected from tetrahydropyrrolyl, hexahydro-1H-pyrrofizinyl and 1,2,3,4-tetrahydroisoquinolinyl, wherein the tetrahydropyrrolyl, hexahydro-1H-pyrrolizinyl and 1,2,3,4-tetrahydroisoquinolinyl are optionally substituted by 1, 2 or 3 $R_c$, and other variables are as defined in the present disclosure.

In some embodiments of the present disclosure, the $R_{10}$ is selected from

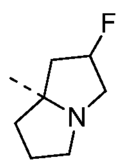,

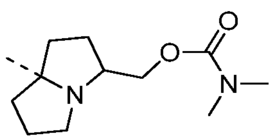 and 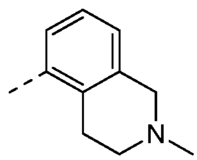,

and other variables are as defined in the present disclosure.

In some embodiments of the present disclosure, the $R_{11}$ and $R_{12}$ are each independently selected from H and $CH_3$, and other variables are as defined in the present disclosure.

The present disclosure provides a compound represented by formula (II) or a pharmaceutically acceptable salt thereof,

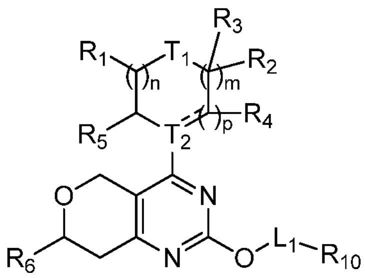

( II ) ,

structural moiety

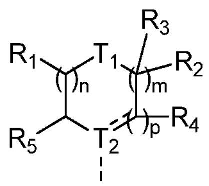

is selected from

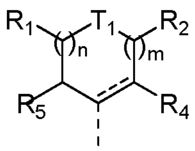 , 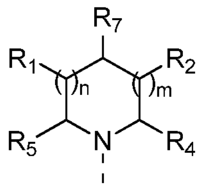 ,

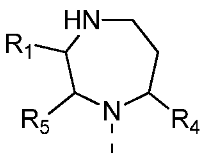 , 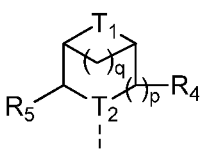 , 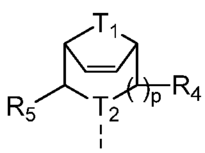 , 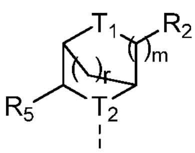 , 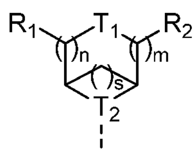 ,

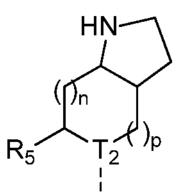 , 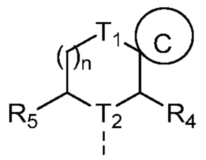 and 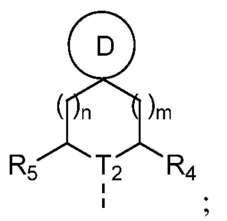 ;

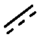 is selected from a single bond and a double bond;

$T_1$ is selected from $CR_7R_8$, $NR_9$ and O;

$T_2$ is selected from CH and N;

$L_1$ is selected from $-CH_2-$ and a bond;
$R_1$, $R_2$, $R_3$, $R_4$ and $R_5$ are each independently selected from H and $C_{1-3}$ alkyl, wherein the $C_{1-3}$ alkyl is optionally substituted by 1, 2 or 3 $R_a$;
$R_6$ is selected from $C_{6-10}$ aryl and 5-10 membered heteroaryl, wherein the $C_{6-10}$ aryl and 5-10 membered heteroaryl are optionally substituted by 1, 2, 3, 4 or 5 $R_b$;
$R_7$ and $R_8$ are each independently selected from H, $CH_3$ and $NH_2$;
$R_9$ is selected from H and $CH_3$;
$R_{10}$ is selected from 4-8 membered heterocycloalkyl and

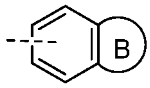,

wherein the 4-8 membered heterocycloalkyl and

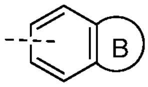

are optionally substituted by 1, 2 or 3 $R_c$;
structural moiety

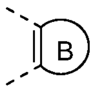

is 5-6 membered heterocycloalkenyl;
structural moiety

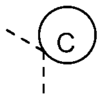

is $C_{3-5}$ membered cycloalkyl;
structural moiety

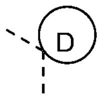

is 4-5 membered heterocycloalkyl;
m is selected from 0, 1 and 2;
n is selected from 0, 1 and 2;
p is selected from 1 and 2;
q is selected from 1, 2 and 3;
r is selected from 1 and 2;
s is selected from 1, 2 and 3;
$R_a$ is each independently selected from F, Cl, Br and I;
$R_b$ is each independently selected from F, Cl, Br, I, OH, $NH_2$, CN, $C_{1-3}$ alkyl, $C_{1-3}$ alkoxy, $C_{2-3}$ alkynyl, $C_{2-3}$ alkenyl and $C_{3-5}$ cycloalkyl, wherein the $C_{1-3}$ alkyl, $C_{1-3}$ alkoxy, $C_{2-3}$ alkynyl, $C_{2-3}$ alkenyl and $C_{3-5}$ cycloalkyl are optionally substituted by 1, 2 or 3 R;
$R_c$ is each independently selected from H, F, Cl, Br, OH, CN, $C_{1-3}$ alkyl, $C_{1-3}$ alkoxy and $-C_{1-3}$ alkyl-O-CO-$C_{1-3}$ alkylamino;
R is each independently selected from F, Cl and Br;
In some embodiments of the present disclosure, the $R_1$, $R_2$, $R_3$, $R_4$ and $R_5$ are each independently selected from

H, $CH_3$, $CH_2CH_3$ and $CH(CH_3)_2$, wherein the $CH_3$, $CH_2CH_3$ and $CH(CH_3)_2$ are optionally substituted by 1, 2 or 3 $R_a$, and other variables are as defined in the present disclosure.

In some embodiments of the present disclosure, the $R_1$, $R_2$, $R_3$, $R_4$ and $R_5$ are each independently selected from H and $CH_3$, and other variables are as defined in the present disclosure.

In some embodiments of the present disclosure, the structural moiety

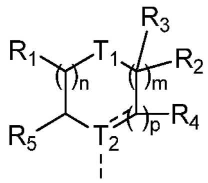

is selected from

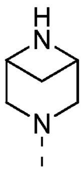, 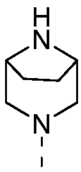, 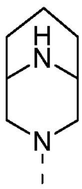, 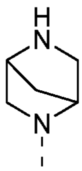, 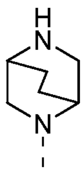, 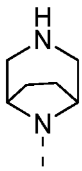, 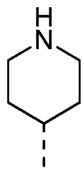, 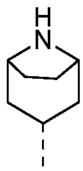, 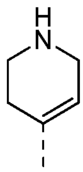,

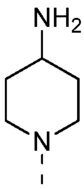, 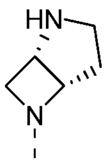, 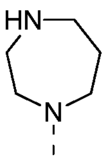, 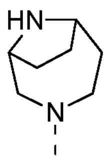, 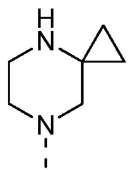, 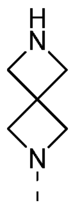, 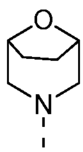 and 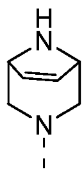,

and other variables are as defined in the present disclosure.

In some embodiments of the present disclosure, the structural moiety

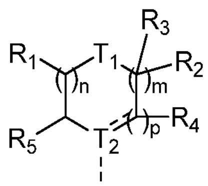

is selected from

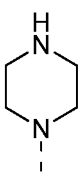, 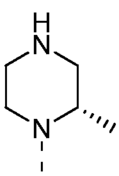, 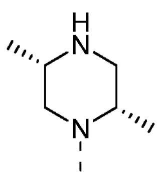, 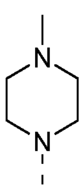, 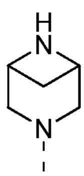, 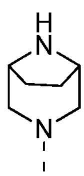, 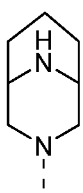, 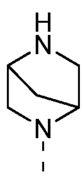,

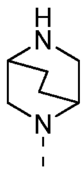, 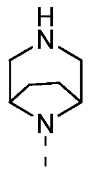, 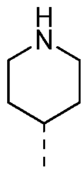, 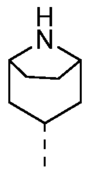, 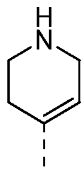, 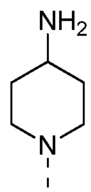, 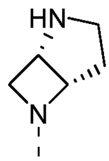, 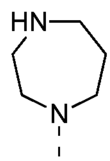, 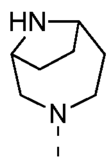, 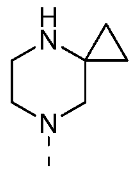,

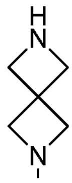, 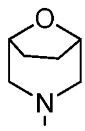 and 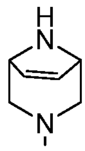,

and other variables are as defined in the present disclosure.

In some embodiments of the present disclosure, the $R_b$ is each independently selected from F, Cl, Br, I, OH, $NH_2$, CN, $CH_3$, $CH_2CH_3$, $OCH_3$, $OCH_2CH_3$, $-CH=CH_2$, $-CH_2-CH=CH_2$ and $-C\equiv CH$, wherein the $CH_3$, $CH_2CH_3$, $OCH_3$, $OCH_2CH_3$, $-CH=CH_2$, $-CH_2-CH=CH_2$ and -C=CH are optionally substituted by 1, 2 or 3 R, and other variables are as defined in the present disclosure.

In some embodiments of the present disclosure, the $R_b$ is each independently selected from F, OH, $NH_2$, $CH_3$, $CF_3$, $CH_2CH_3$ and -C=CH , and other variables are as defined in the present disclosure.

In some embodiments of the present disclosure, the $R_6$ is selected from phenyl, naphthyl, indolyl and indazolyl, wherein the phenyl, naphthyl, indolyl and indazolyl are optionally substituted by 1, 2, 3, 4 or 5 $R_b$, and other variables are as defined in the present disclosure.

In some embodiments of the present disclosure, the $R_6$ is selected from

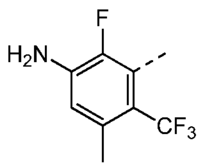, 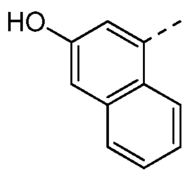, 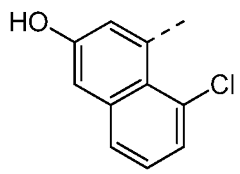, 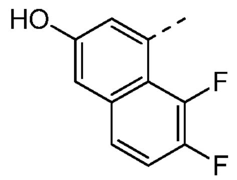 and 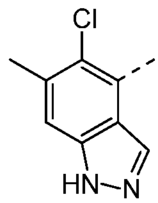,

and other variables are as defined in the present disclosure.

In some embodiments of the present disclosure, the $R_c$ is each independently selected from H, F, Cl, Br, OH, CN, $CH_3$, $CH_2CH_3$, $CH_2CF_3$, $OCH_3$, $OCF_3$ and

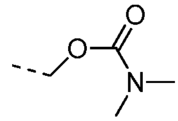,

and other variables are as defined in the present disclosure.

In some embodiments of the present disclosure, the $R_{10}$ is selected from tetrahydropyrrolyl, hexahydro-1H-pyrrofizinyl and 1,2,3,4-tetrahydroisoquinolinyl, wherein the tetrahydropyrrolyl, hexahydro-1H-pyrrolizinyl and 1,2,3,4-tetrahydroisoquinolinyl are optionally substituted by 1, 2 or 3 $R_c$, and other variables are as defined in the present disclosure.

In some embodiments of the present disclosure, the $R_{10}$ is selected from

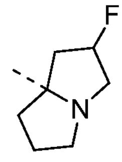

,

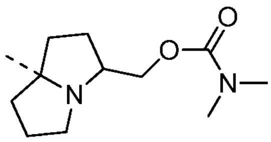 and 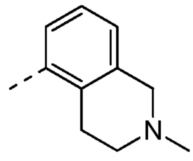,

and other variables are as defined in the present disclosure.

The present disclosure provides a compound represented by formula (I) or a pharmaceutically acceptable salt thereof,

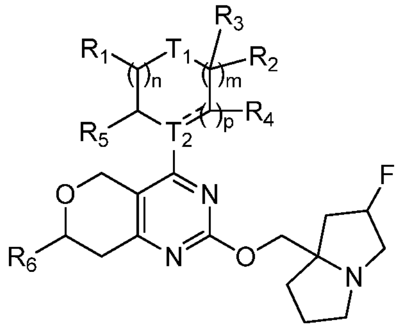

(I)

,

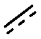 is selected from a single bond and a double bond;

$T_1$ is selected from $CR_7R_8$ and $NR_9$;

when 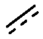 is a single bond, $T_2$ is selected from CH and N;

when S' is a double bond, $T_2$ is C;

$R_1$, $R_2$, $R_3$, $R_4$ and $R_5$ are each independently selected from H and $C_{1-3}$ alkyl, wherein the $C_{1-3}$ alkyl is optionally substituted by 1, 2 or 3 $R_a$;

$R_6$ is selected from phenyl and naphthyl, wherein the phenyl and naphthyl are optionally substituted by 1, 2, 3, 4 or 5 $R_b$;

$R_7$ and $R_8$ are each independently selected from H, $CH_3$ and $NH_2$;

$R_9$ is selected from H and $CH_3$;

or, $R_1$ and $R_2$ form a ring together with the atoms to which they are connected so that the structural moiety

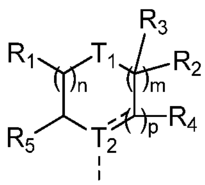

forms

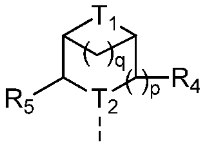

;

or, $R_1$ and $R_4$ form a ring together with the atoms to which they are connected so that the structural moiety

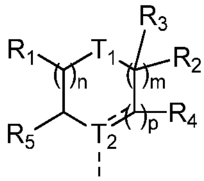

forms

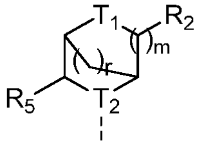

;

or, $R_4$ and $R_5$ form a ring together with the atoms to which they are connected so that the structural moiety

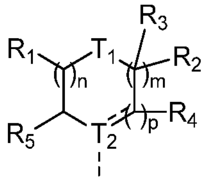

forms

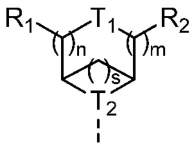

;

or, $R_2$ and $R_7$ form tetrahydropyrrolidinyl together with the atoms to which they are connected;
or, $R_2$ and $R_3$ form $C_{3-5}$ membered cycloalkyl together with the atoms to which they are connected;
or, $R_7$ and $R_8$ form 4-5 membered heterocycloalkyl together with the atoms to which they are connected;
m is selected from 0, 1 and 2;
n is selected from 0, 1 and 2;
p is selected from 1 and 2;
q is selected from 1, 2 and 3;
r is selected from 1 and 2;
s is selected from 1, 2 and 3;
$R_a$ is each independently selected from F, Cl, Br and I;
$R_b$ is each independently selected from F, Cl, Br, I, OH, $NH_2$, CN, $CH_3$, $CF_3$ and $OCH_3$.

In some embodiments of the present disclosure, the $R_1$, $R_2$, $R_3$, $R_4$ and $R_5$ are each independently selected from H, $CH_3$, $CH_2CH_3$ and $CH(CH_3)_2$, wherein the $CH_3$, $CH_2CH_3$ and $CH(CH_3)_2$ are optionally substituted by 1, 2 or 3 $R_a$, and other variables are as defined in the present disclosure.

In some embodiments of the present disclosure, the $R_1$, $R_2$, $R_3$, $R_4$ and $R_5$ are each independently selected from H and $CH_3$, and other variables are as defined in the present disclosure.

In some embodiments of the present disclosure, the structural moiety

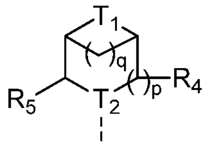

is selected from

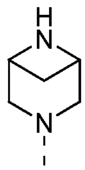 , 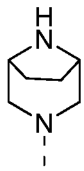 , 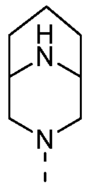 , 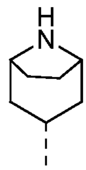 and 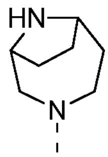 ,

and other variables are as defined in the present disclosure.

In some embodiments of the present disclosure, the structural moiety

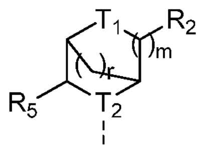

is selected from

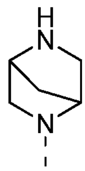 and 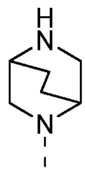 ,

and other variables are as defined in the present disclosure.

In some embodiments of the present disclosure, the structural moiety

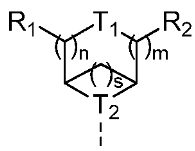

is

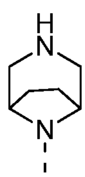 ,

and other variables are as defined in the present disclosure.

In some embodiments of the present disclosure, the $R_2$ and $R_7$ form

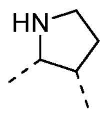

together with the atoms to which they are connected, and other variables are as defined in the present disclosure.

In some embodiments of the present disclosure, the $R_2$ and $R_3$ form

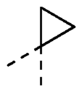

together with the atoms to which they are connected, and other variables are as defined in the present disclosure.

In some embodiments of the present disclosure, the $R_7$ and $R_8$ form

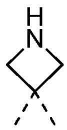

together with the atoms to which they are connected, and other variables are as defined in the present disclosure.

In some embodiments of the present disclosure, the structural moiety

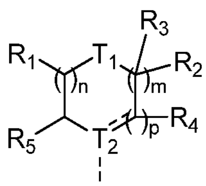

is selected from

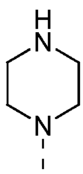 , 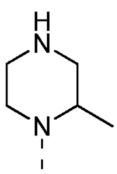 , 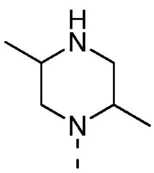 , 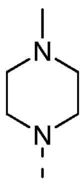 , 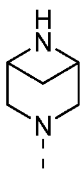 , 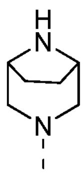 , 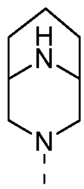 , 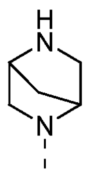 ,

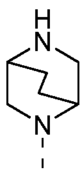 , 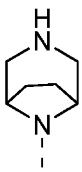 , 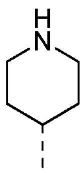 , 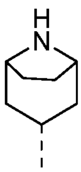 , 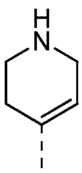 , 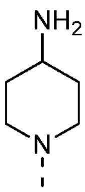 , 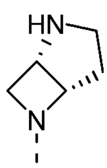 , 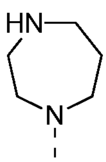 , 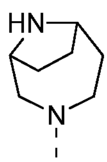 , 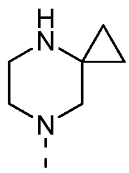 ,

and

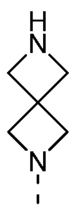 .

In some embodiments of the present disclosure, the structural moiety

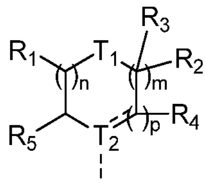

is selected from

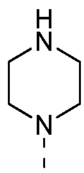, 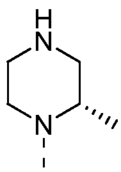, 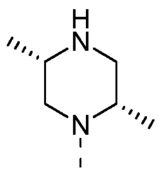, 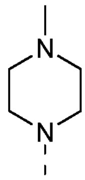, 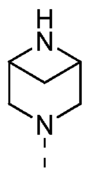, 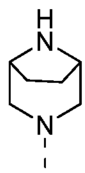, 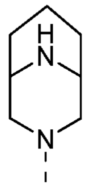, 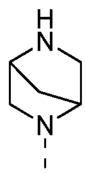,

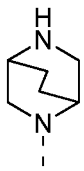, 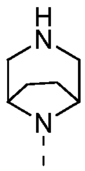, 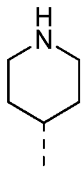, 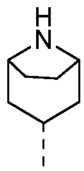, 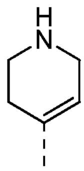, 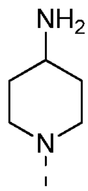, 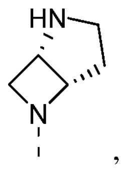, 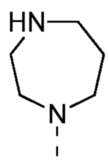, 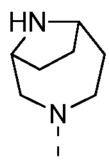, 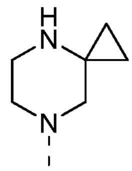,

and

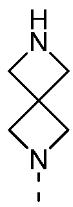,

and other variables are as defined in the present disclosure.

In some embodiments of the present disclosure, the $R_6$ is

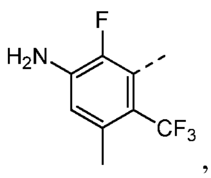,

and other variables are as defined in the present disclosure.

In some embodiments of the present disclosure, provided is a compound or a pharmaceutically acceptable salt thereof, wherein the compound is selected from,

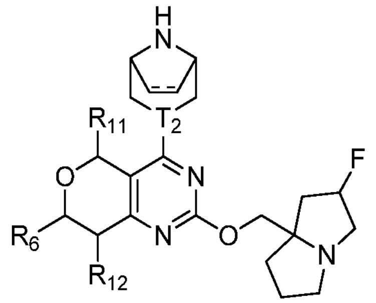

( IV )

wherein

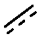 is selected from a single bond and a double bond;
$T_2$, $R_6$, Rn and $R_{12}$ are as defined in the present disclosure.

In some embodiments of the present disclosure, provided is a compound or a pharmaceutically acceptable salt thereof, wherein the compound is selected from,

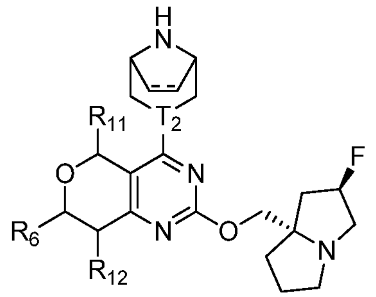

( IV-1 )

wherein

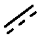 is selected from a single bond and a double bond;
$T_2$, $R_6$, $R_{11}$ and $R_{12}$ are as defined in the present disclosure.

In some embodiments of the present disclosure, provided is a compound or a pharmaceutically acceptable salt thereof, wherein the compound is selected from,

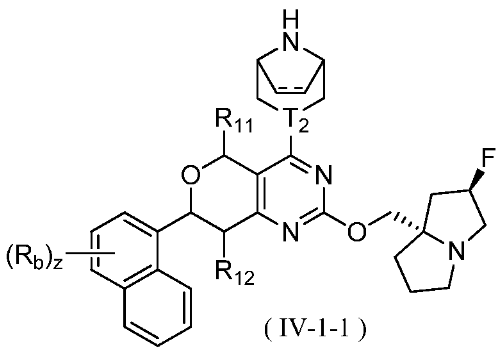

( IV-1-1 )

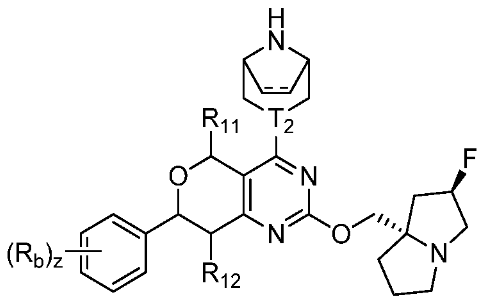

( IV-1-2 )

wherein

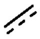 is selected from a single bond and a double bond;

z is selected from 0, 1, 2, 3, 4 and 5;
$T_2$, $R_b$, $R_{11}$ and $R_{12}$ are as defined in the present disclosure.

In some embodiments of the present disclosure, provided is a compound or a pharmaceutically acceptable salt thereof, wherein the compound is selected from,

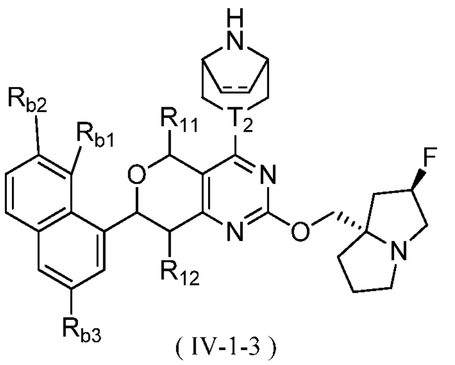
( IV-1-3 )

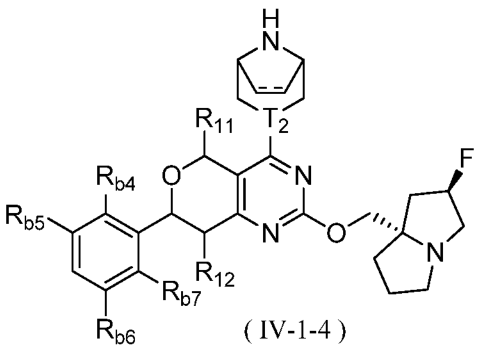
( IV-1-4 )

wherein

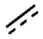 is selected from a single bond and a double bond;
$R_{b1}$, $R_{b2}$, $R_{b3}$, $R_{b4}$, $R_{b5}$, $R_{b6}$ and $R_{b7}$ are each independently selected from H, F, Cl, Br, I, OH, $NH_2$, CN, $C_{1-3}$ alkyl, $C_{1-3}$ alkoxy, $C_{2-3}$ alkynyl, $C_{2-3}$ alkenyl, $-C(=O)C_{1-3}$ alkyl and $C_{3-5}$ cycloalkyl, wherein the $C_{1-3}$ alkyl, $C_{1-3}$ alkoxy, $C_{2-3}$ alkynyl, $C_{2-3}$ alkenyl, $-C(=O)C_{1-3}$ alkyl and $C_{3-5}$ cycloalkyl are optionally substituted by 1, 2, 3, 4 or 5 R;
R is each independently selected from F, Cl, Br and I;
$T_2$, $R_{11}$ and $R_{12}$ are as defined in the present disclosure.

In some embodiments of the present disclosure, the $R_{b1}$, $R_{b2}$, $R_{b3}$, $R_{b4}$, $R_{b5}$, $R_{b6}$ and $R_{b7}$ are each independently selected from H, F, Cl, Br, I, OH, $NH_2$, CN, $CH_3$, $CH_2CH_3$, $OCH_3$, $OCH_2CH_3$, $-CH=CH_2$, $-CH_2-CH=CH_2$, $-C\equiv CH$ , $-C(=O)CH_3$ and cyclopropyl, wherein the $CH_3$, $CH_2CH_3$, $OCH_3$, $OCH_2CH_3$, $-CH=CH_2$, $-CH_2-CH=CH_2$, $-C\equiv CH$ , $C(=O)CH_3$ and cyclopropyl are optionally substituted by 1, 2, 3, 4 or 5 R, and other variables are as defined in the present disclosure.

In some embodiments of the present disclosure, the $R_{b1}$, $R_{b2}$, $R_{b3}$, $R_{b4}$, $R_{b5}$, $R_{b6}$ and $R_{b7}$ are each independently selected from H, F, Cl, Br, I, OH, $NH_2$, CN, $CH_3$, $CHF_2$, $CH_2F$, $CF_3$, $CH_2CH_3$, $CH_2CF_3$, $CF_2CF_3$, $OCH_3$, $OCF_3$, $-CH=CH_2$, $-C\equiv CH$ , $-C(=O)CH_3$, $-C(=O)CF_3$, and cyclopropyl, and other variables are as defined in the present disclosure.

In some embodiments of the present disclosure, the $R_{b1}$, $R_{b2}$, $R_{b3}$, $R_{b4}$, $R_{b5}$, $R_{b6}$ and $R_{b7}$ are each independently selected from H, F, Cl, Br, I, OH, $NH_2$, CN, $CH_3$, $CF_3$, $CH_2CH_3$, $CF_2CF_3$, $-CH=CH_2$, $-C\equiv CH$, $-C(=O)CH_3$ and cyclopropyl, and other variables are as defined in the present disclosure.

In some embodiments of the present disclosure, provided is a compound or a pharmaceutically acceptable salt thereof, wherein the compound is selected from,

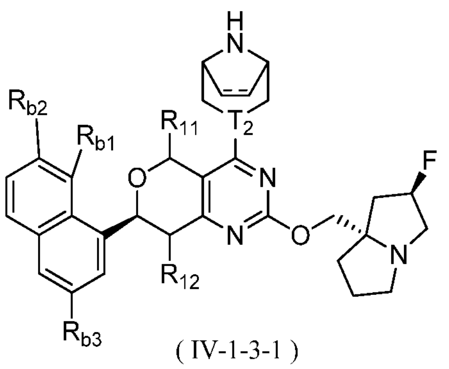
( IV-1-3-1 )

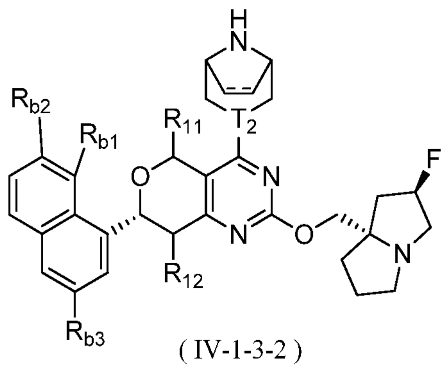
( IV-1-3-2 )

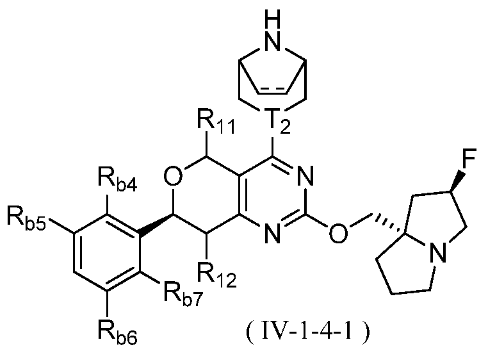

( IV-1-4-1 )

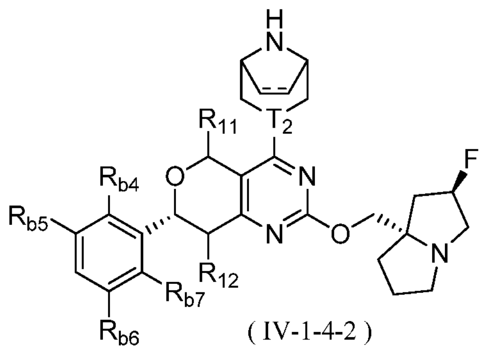

( IV-1-4-2 )

wherein

is selected from a single bond and a double bond;

$T_2$, $R_{b1}$, $R_{b2}$, $R_{b3}$, $R_{b4}$, $R_{b5}$, $R_{b6}$, $R_{b7}$, Rn and $R_{12}$ are as defined in the present disclosure.

The present disclosure also includes some embodiments that are obtained by combining any of the above-mentioned variables.

The present disclosure also provides a compound represented by the following formula or a pharmaceutically acceptable salt thereof,

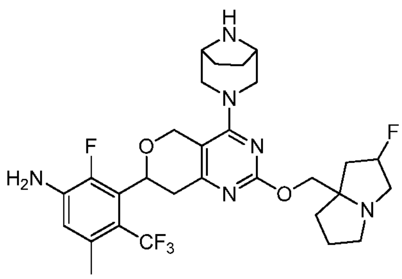

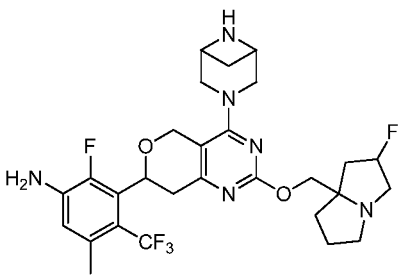

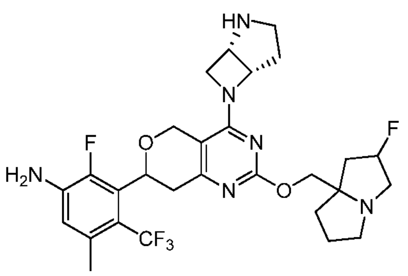

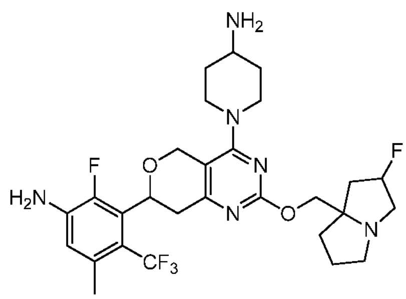

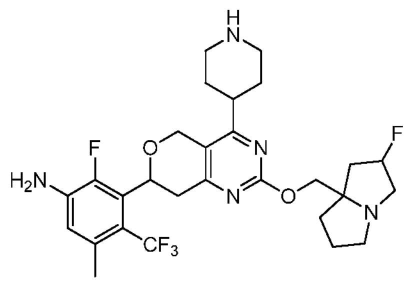

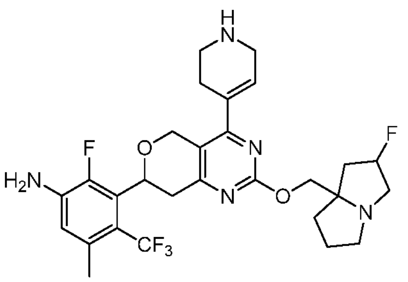

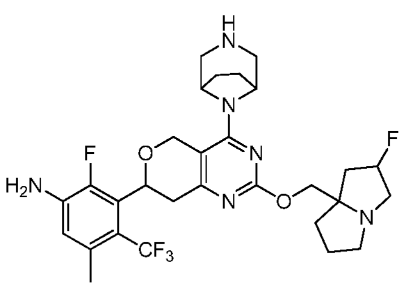

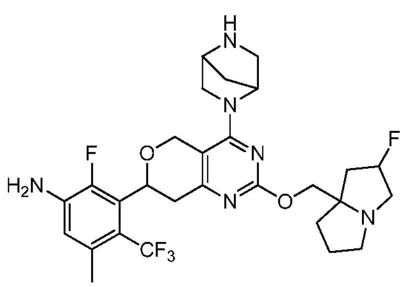

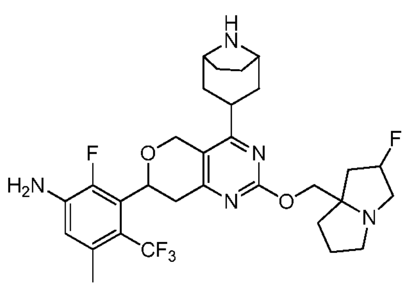

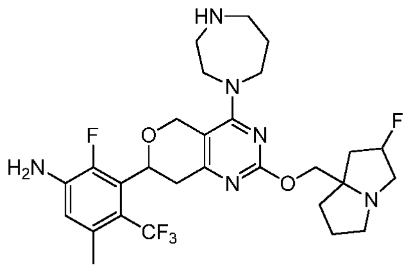

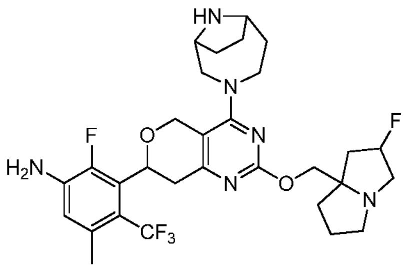

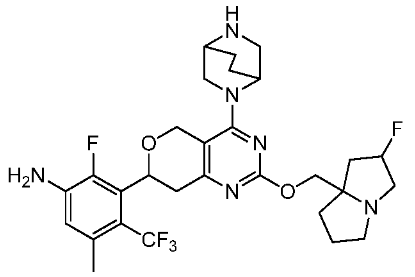

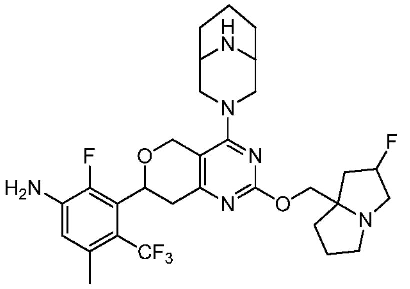

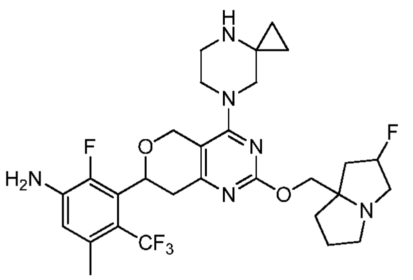

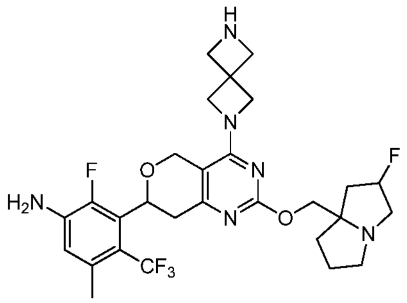

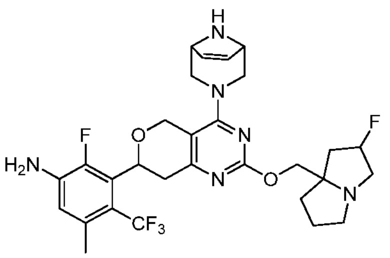

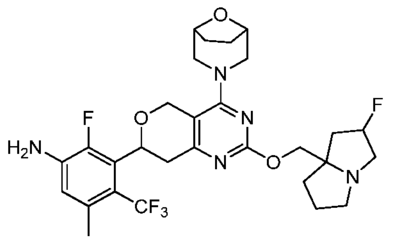

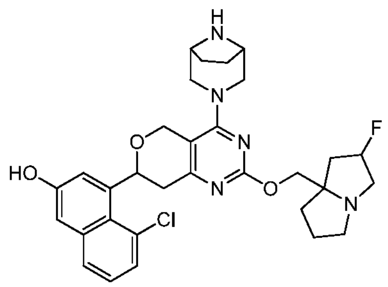

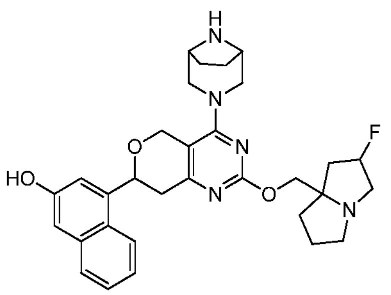

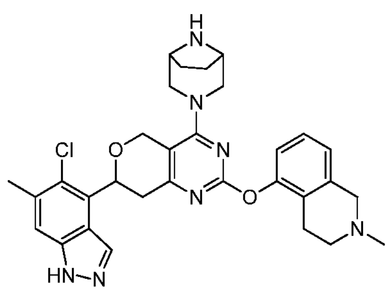

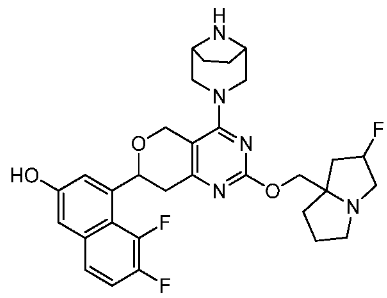

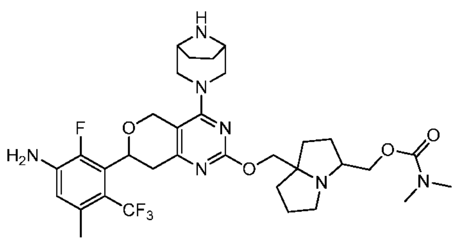

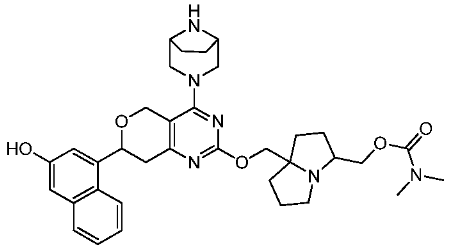

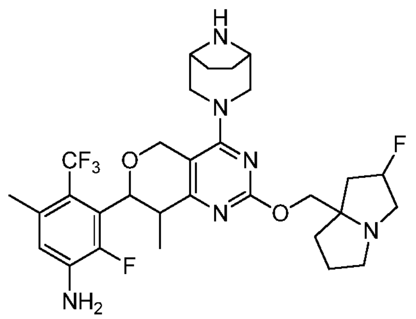
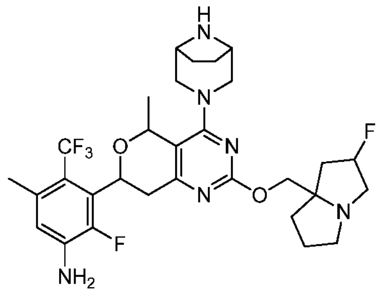
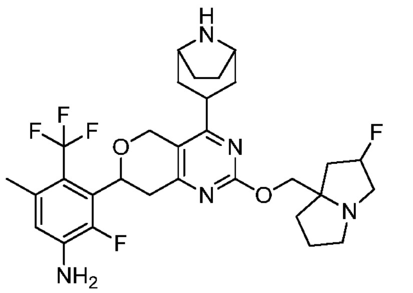

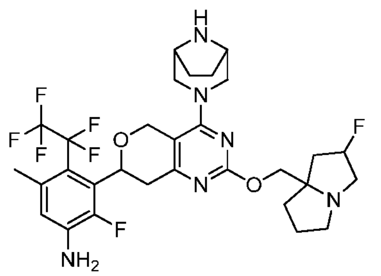
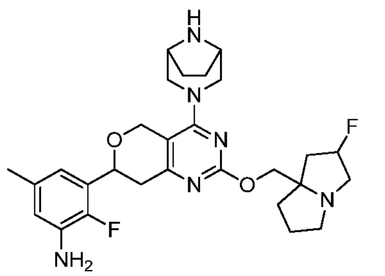
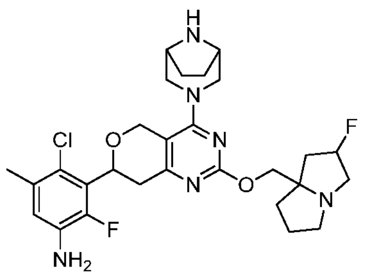

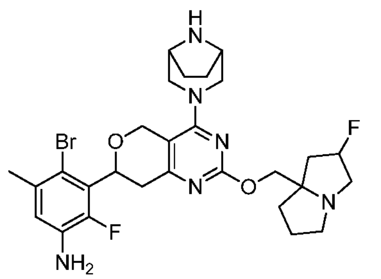
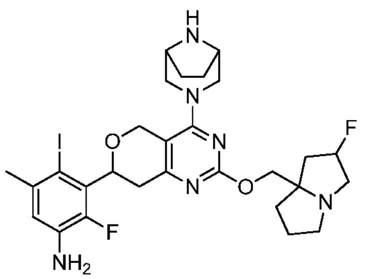
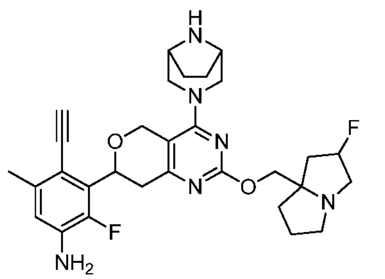

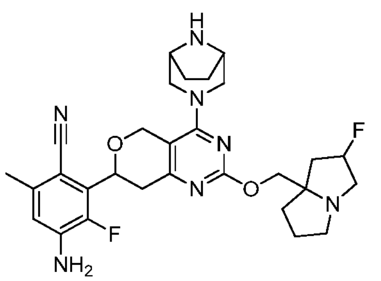
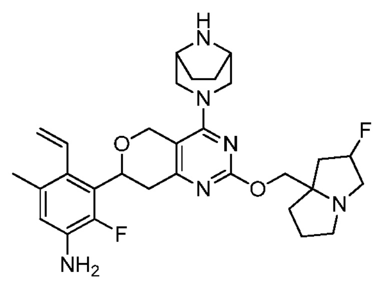
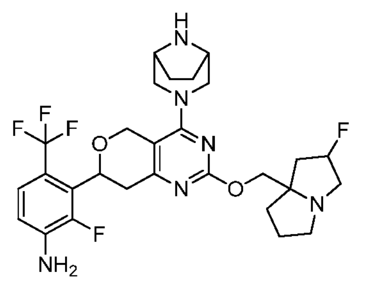

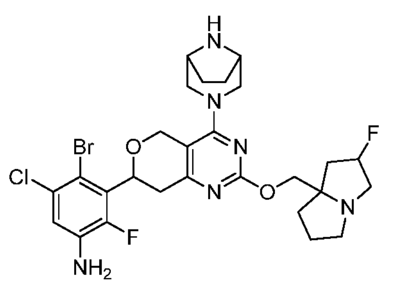
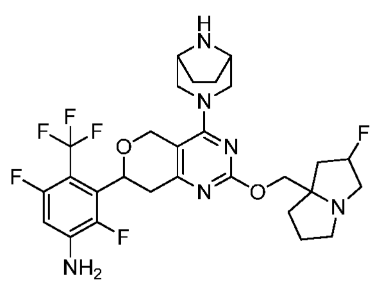
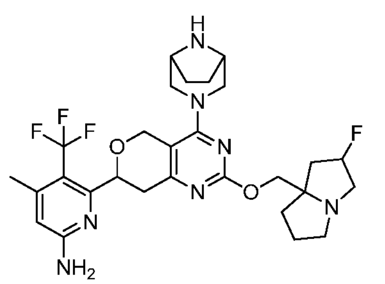

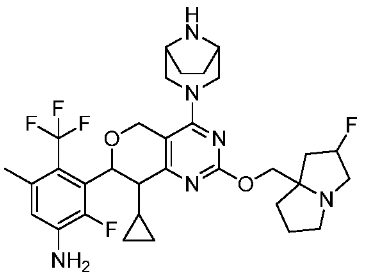
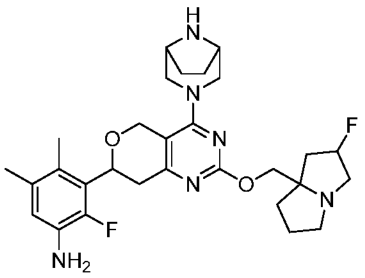
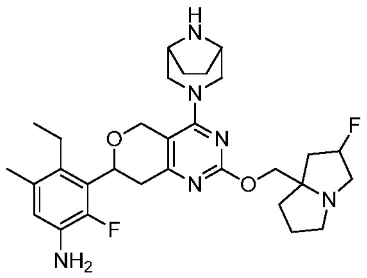
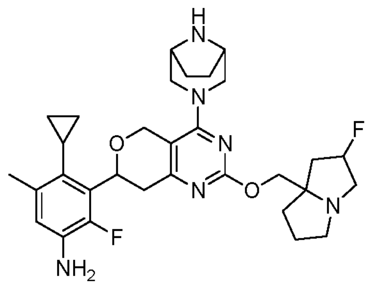
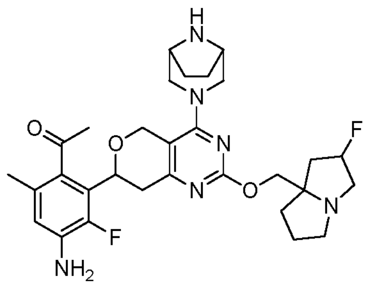
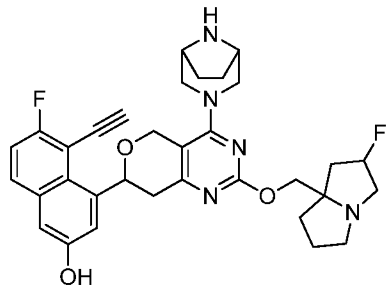

.

In some embodiments of the present disclosure, provided is a compound or a pharmaceutically acceptable salt thereof, wherein the compound is selected from,

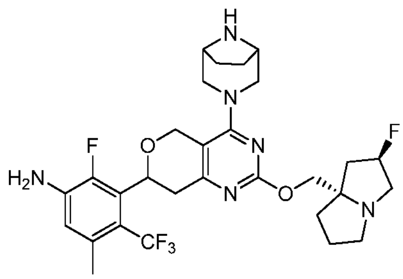
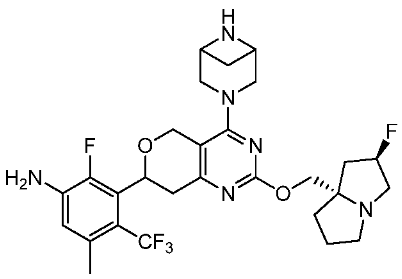
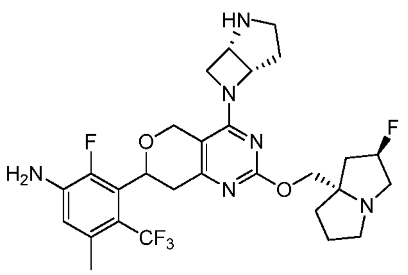
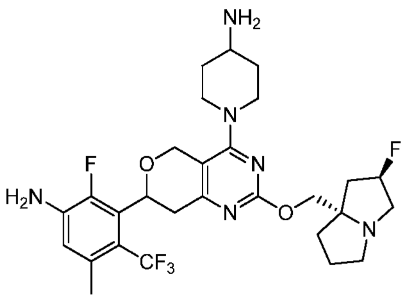
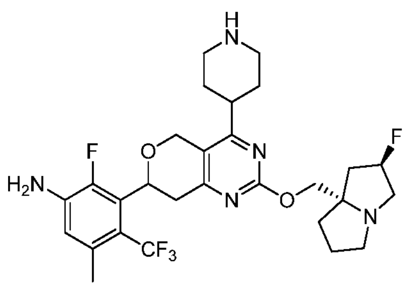
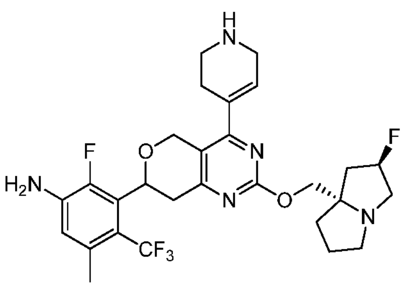
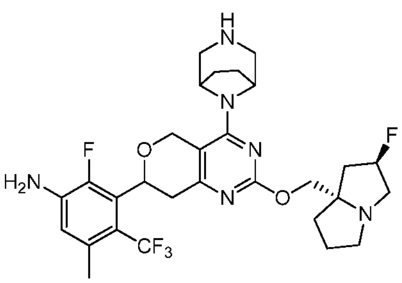
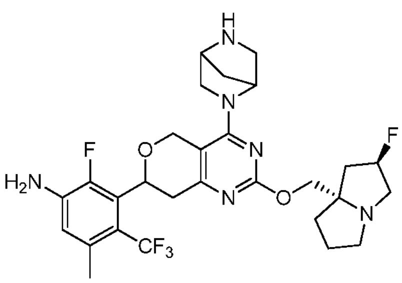
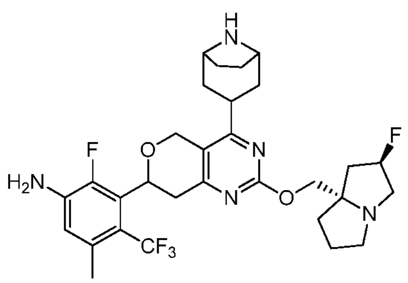

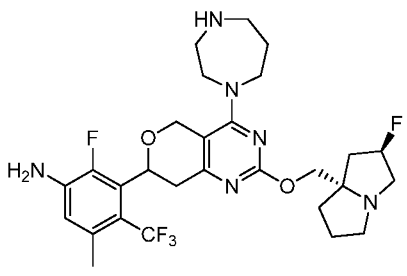

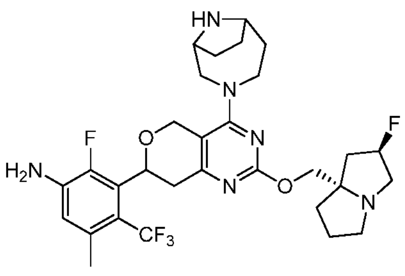

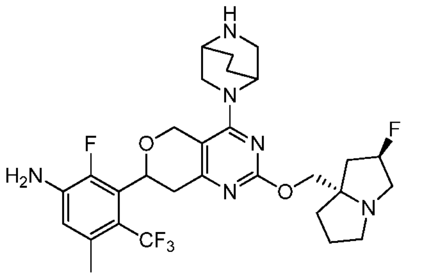

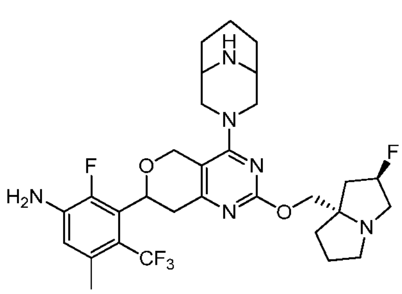

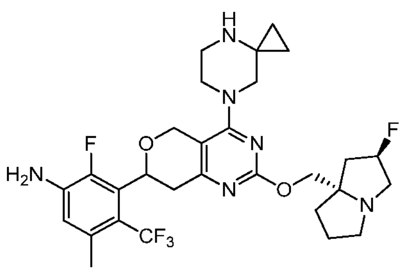

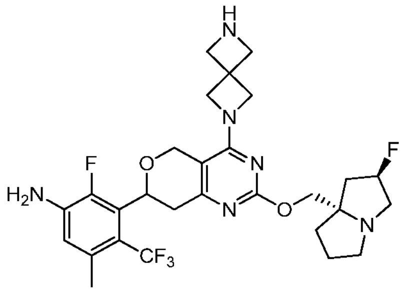

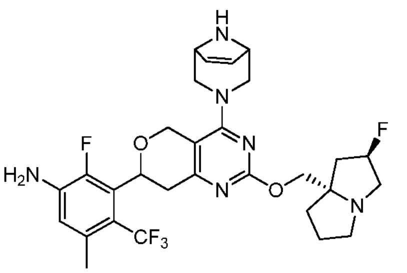

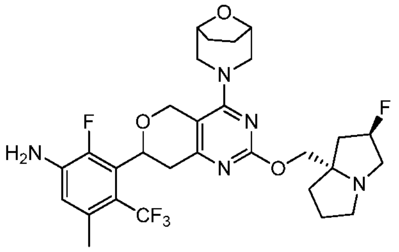

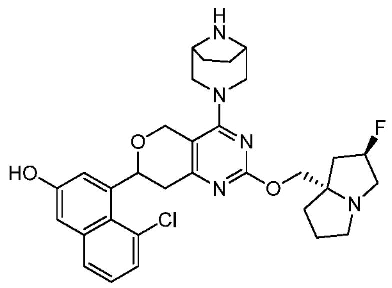

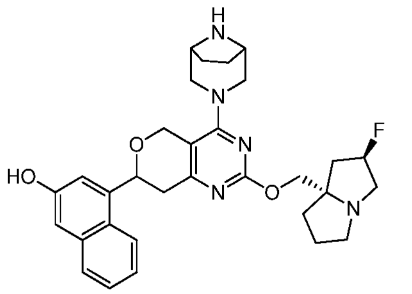

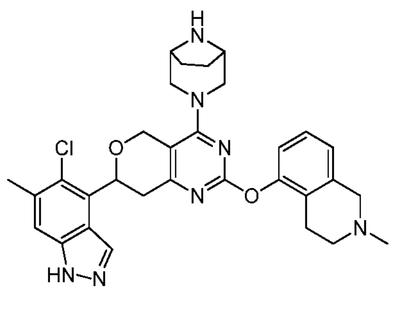

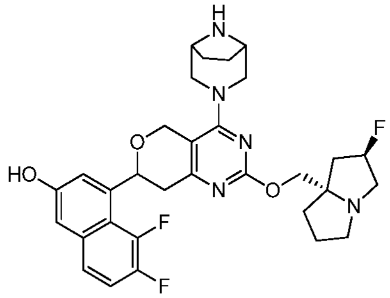

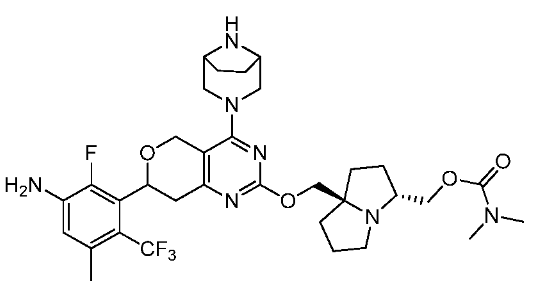

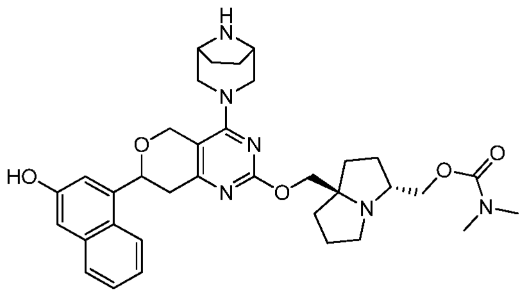

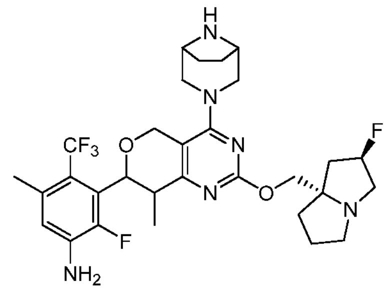
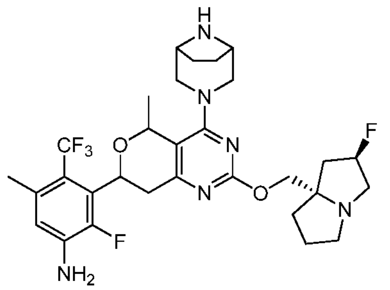
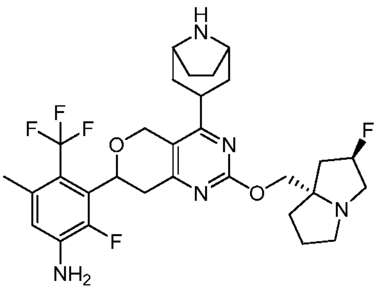

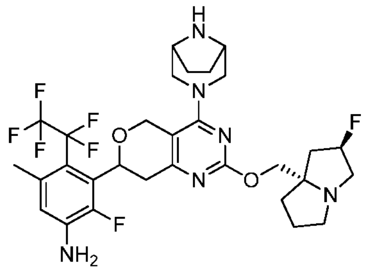
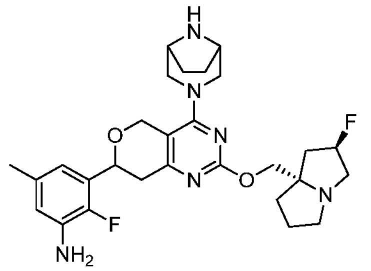
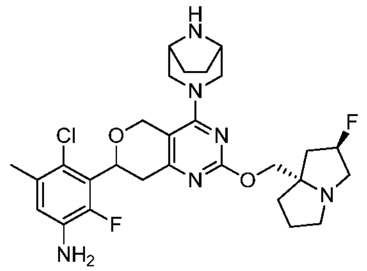

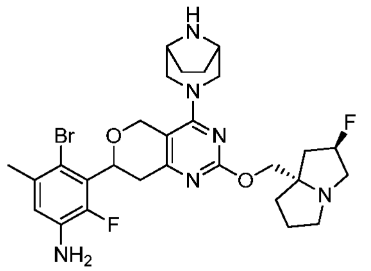
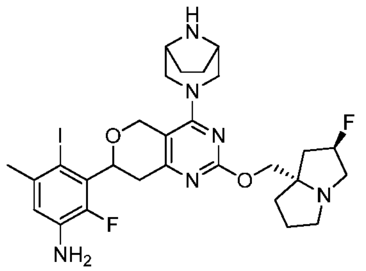
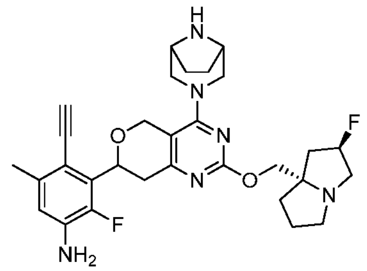

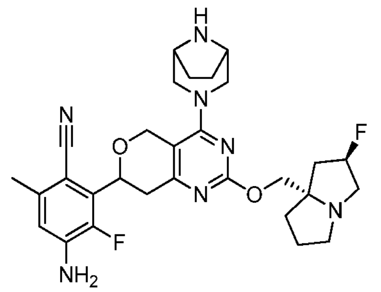
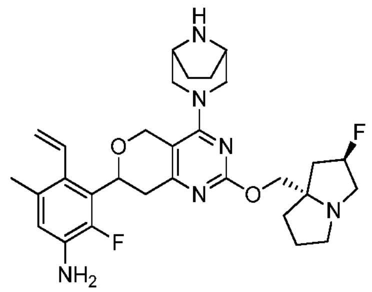
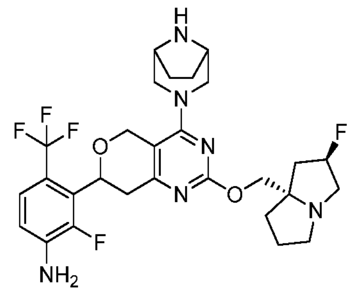

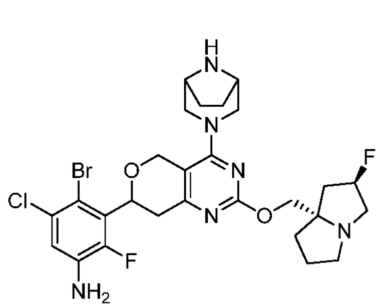
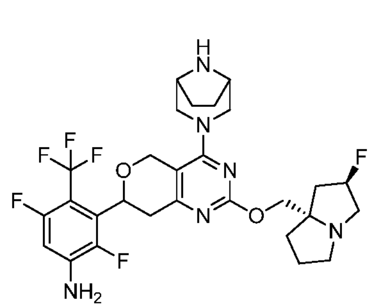
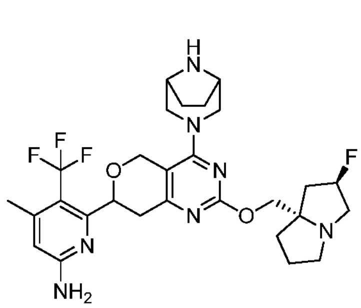

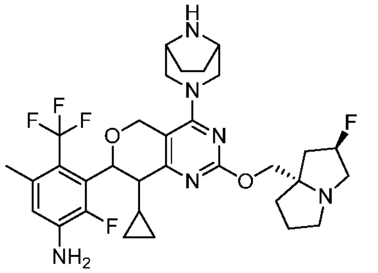
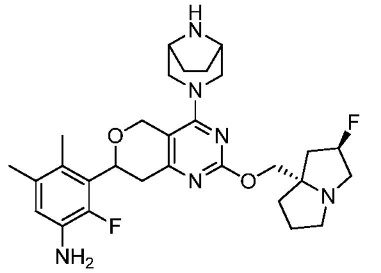
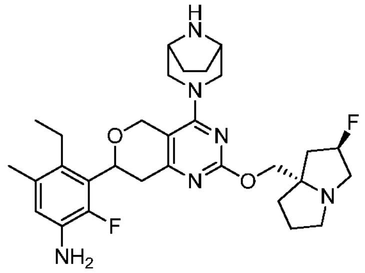
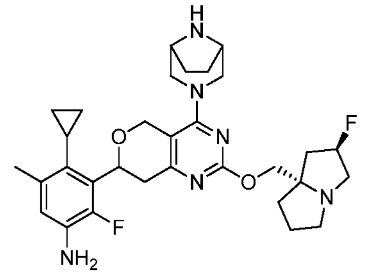
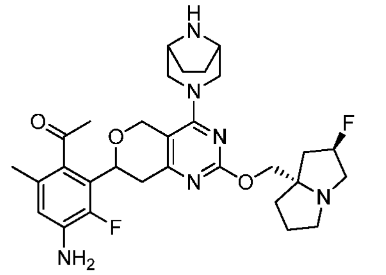
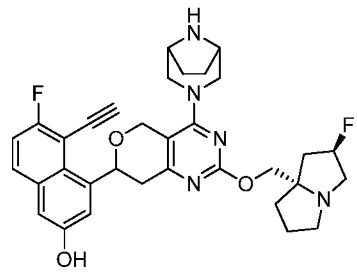
.

In some embodiments of the present disclosure, provided is a compound or a pharmaceutically acceptable salt thereof, wherein the compound is selected from,

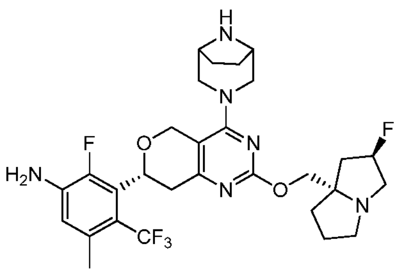
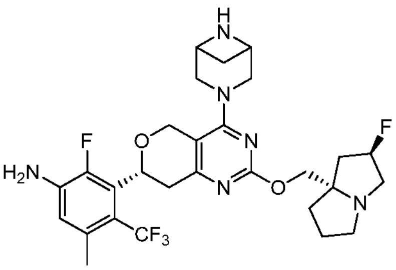
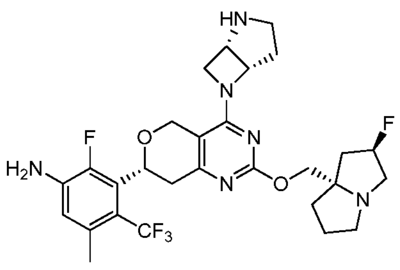
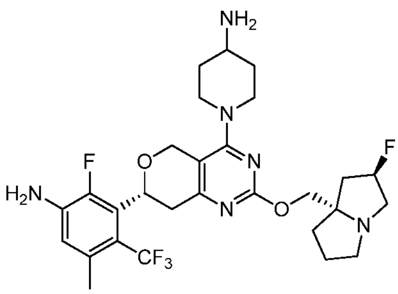
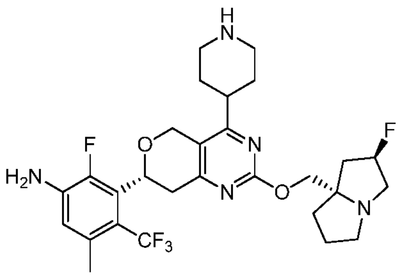
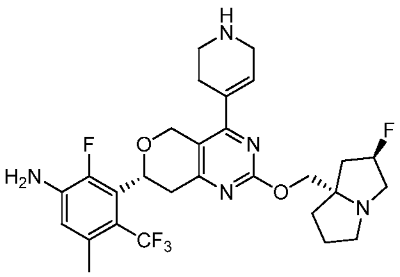
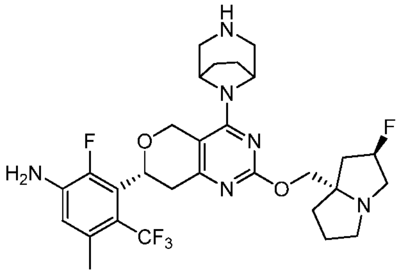
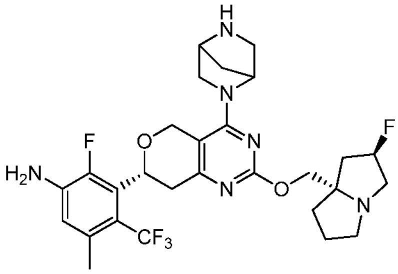
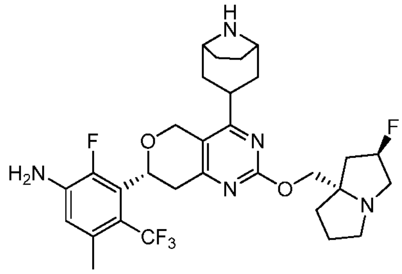

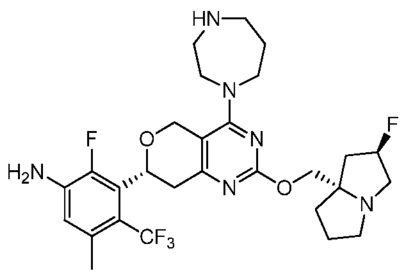
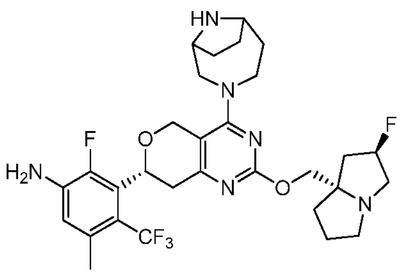
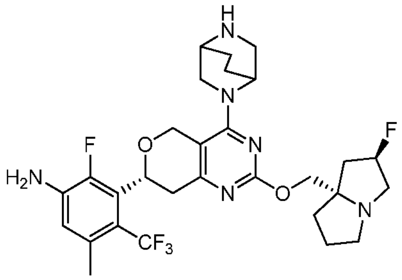
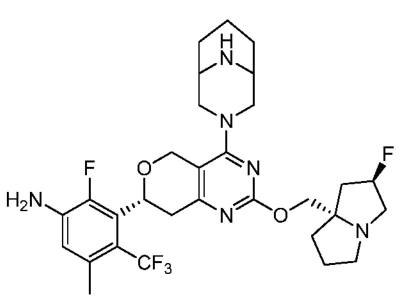
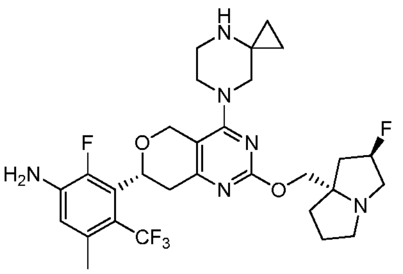
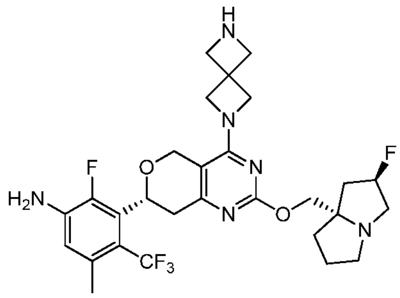
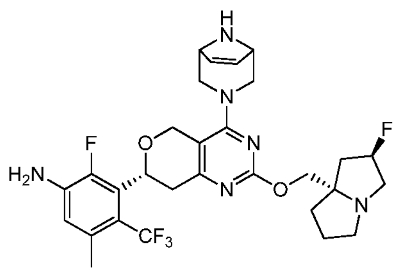
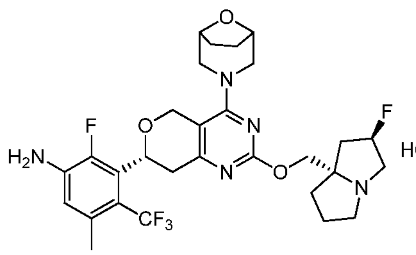
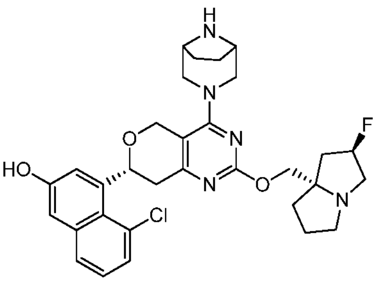
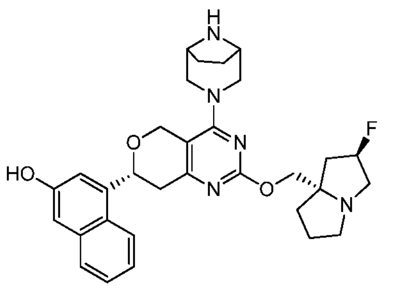
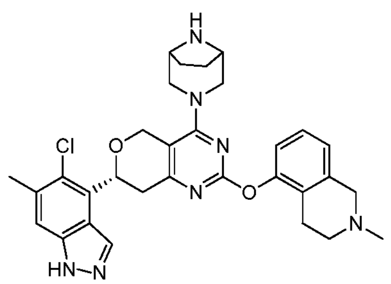
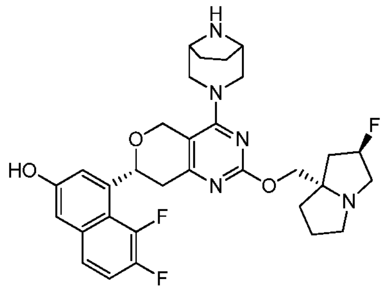
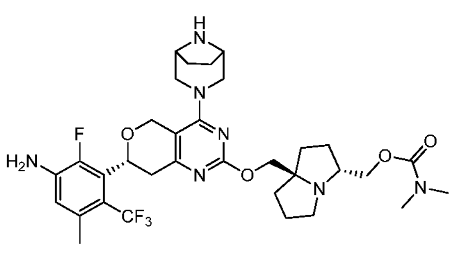
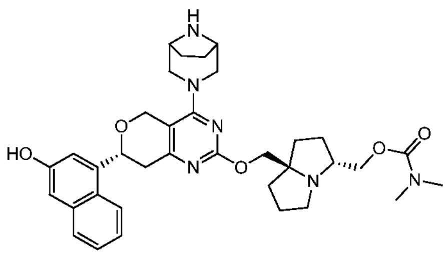

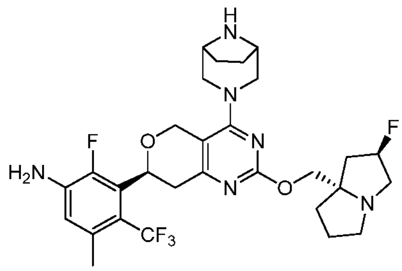
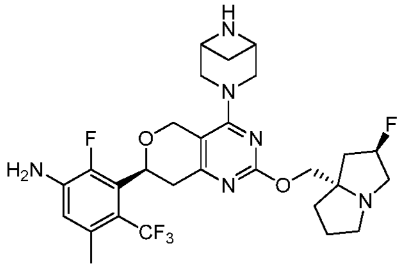
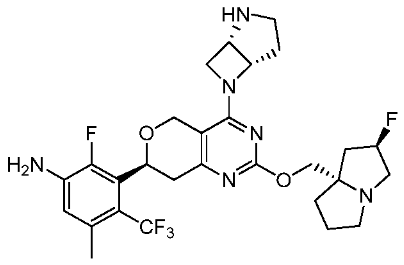

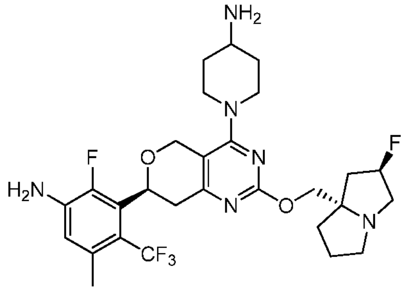
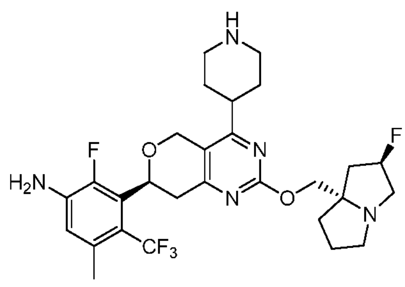
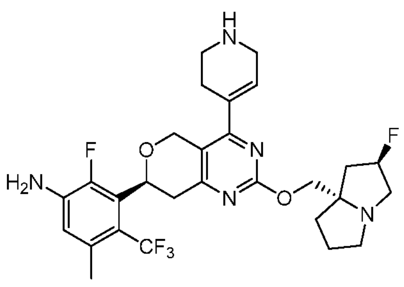

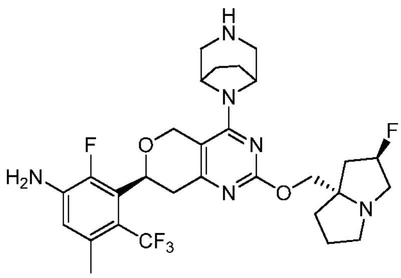
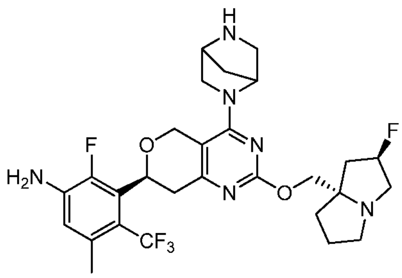
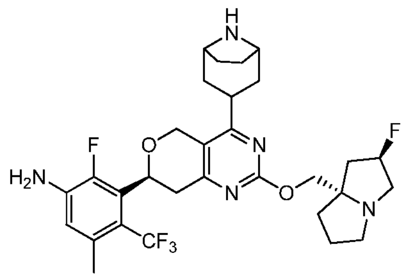

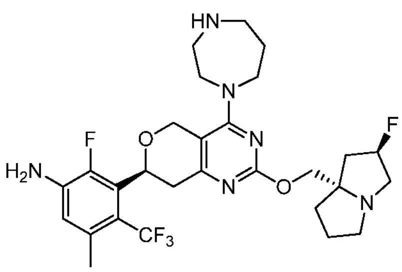
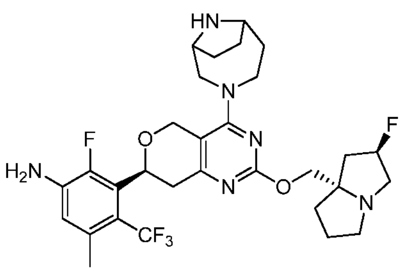
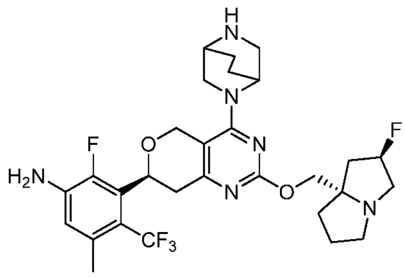

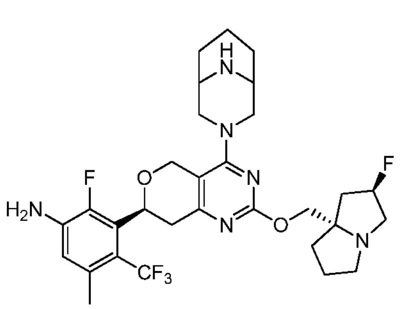
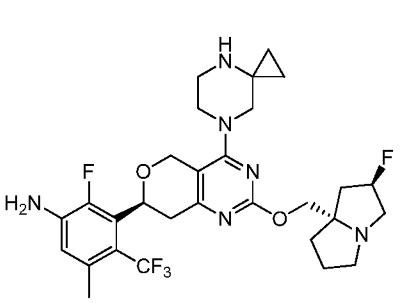
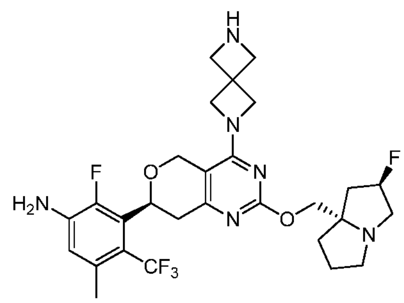

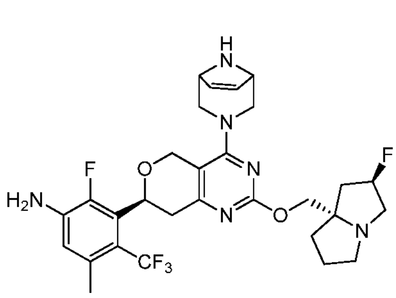
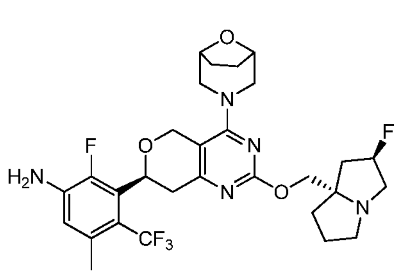
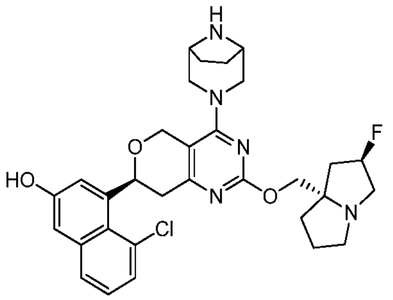
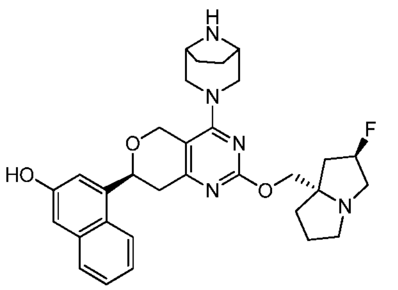
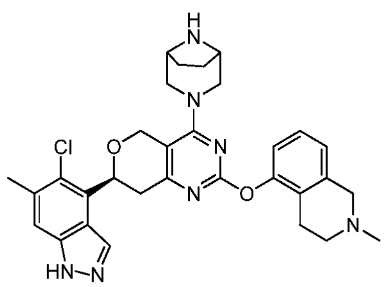
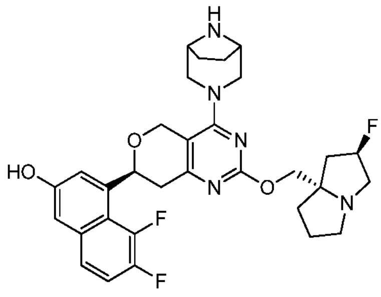
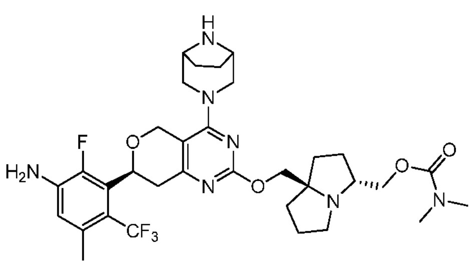
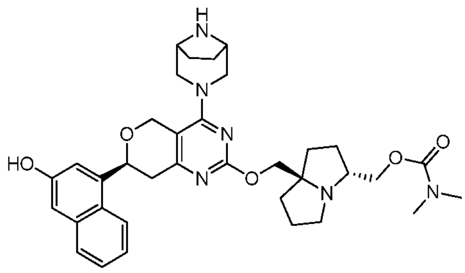
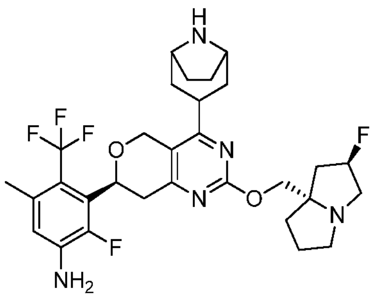
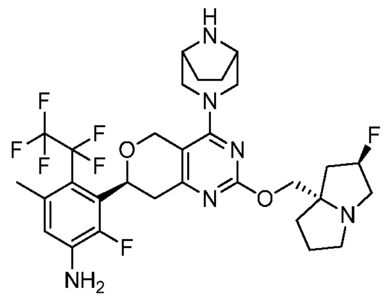
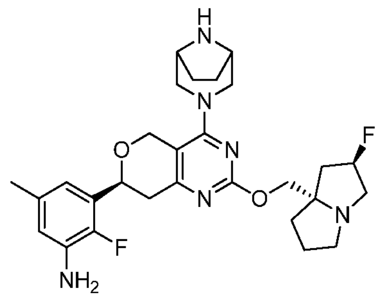
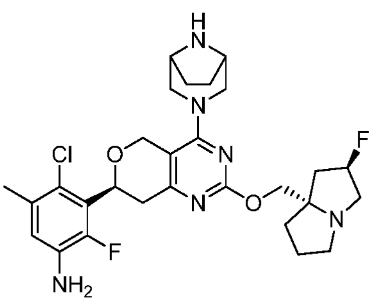
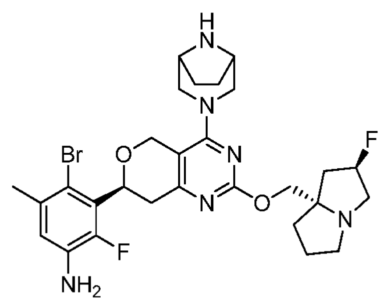
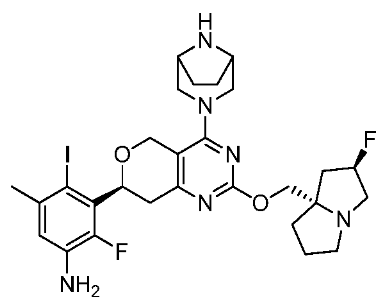

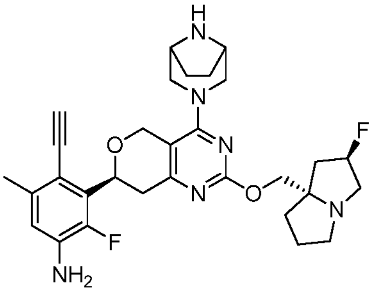
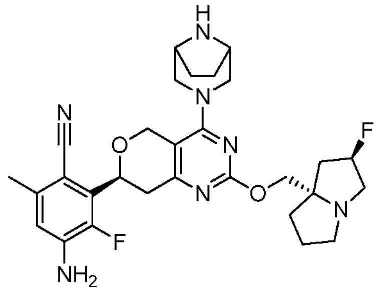
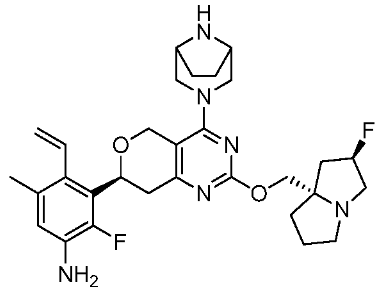
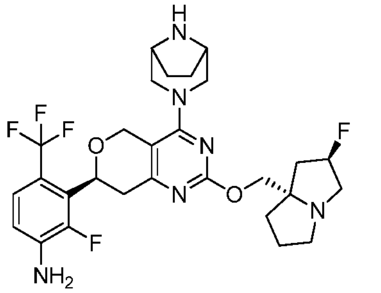
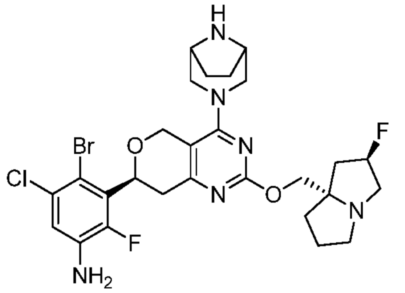
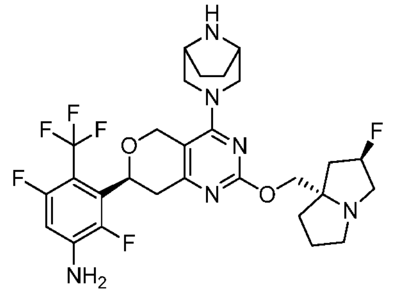
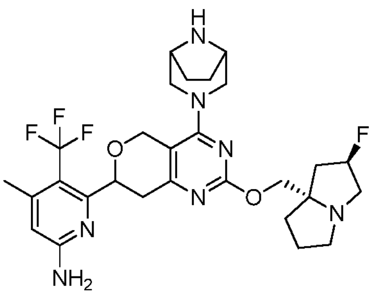
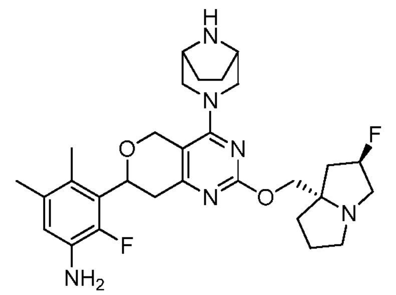
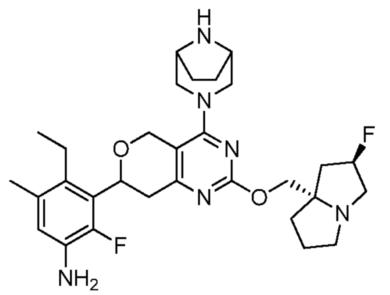
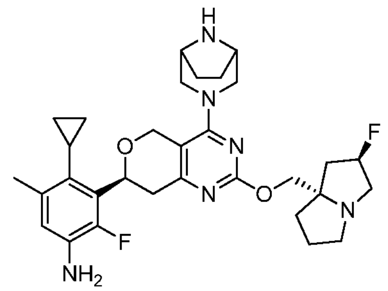
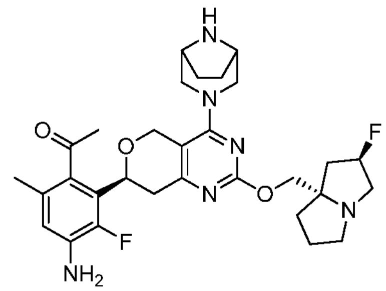
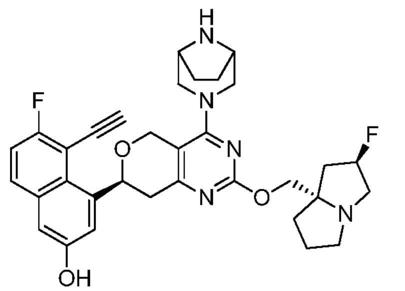
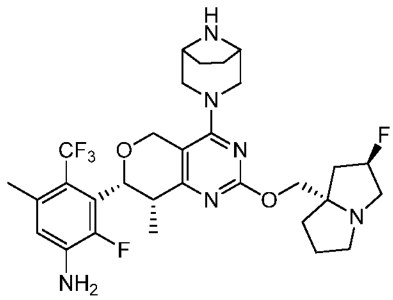
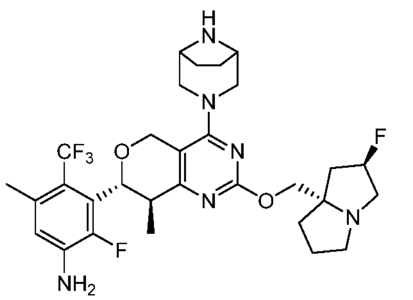
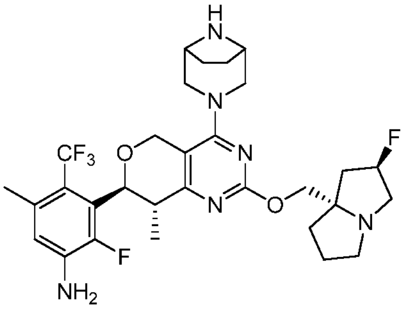

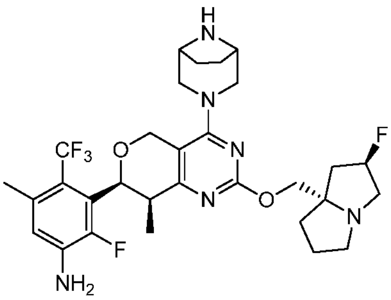 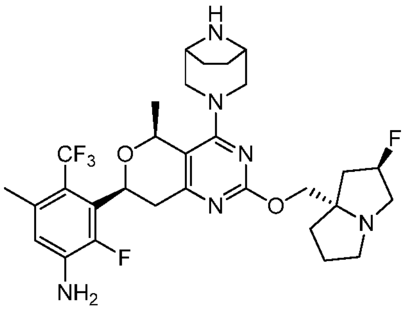 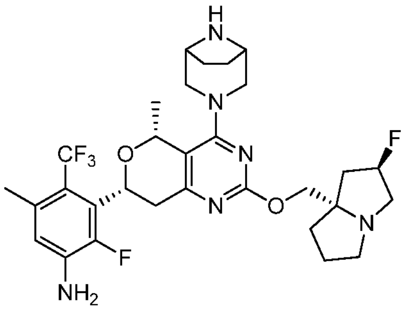

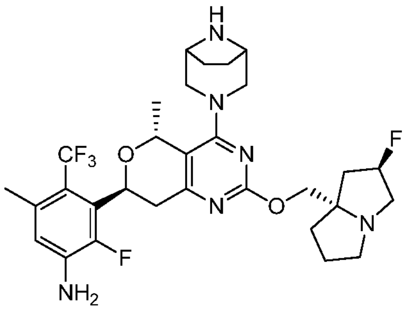 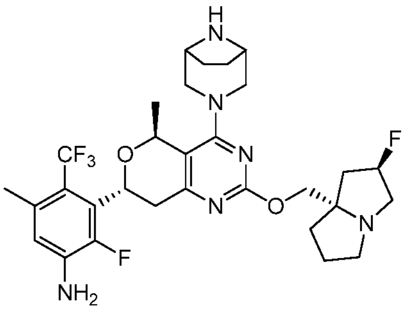 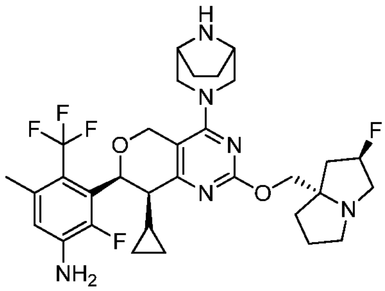

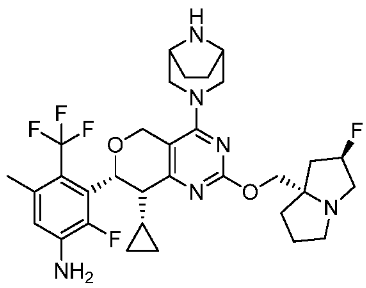 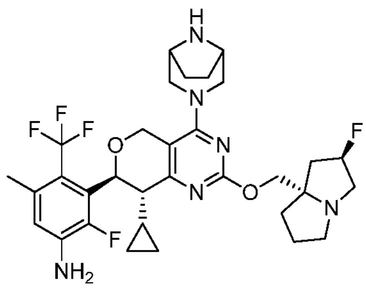 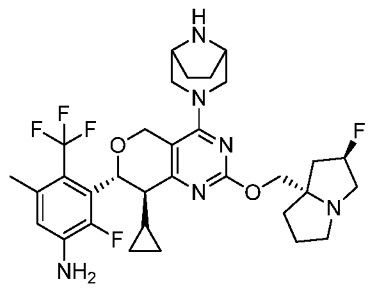 .

The present disclosure also provides use of the above-mentioned compound or a pharmaceutically acceptable salt thereof in the manufacture of a medicament for treating diseases related to $KRAS^{G12D}$ mutation.

The present disclosure also provides use of the above-mentioned compound or a pharmaceutically acceptable salt thereof in the manufacture of a medicament for treating diseases related to tumors.

**Technical effect**

The compounds of the present disclosure have good inhibitory effect on $KRAS^{G12D}$ mutated kinase. The compounds of the present disclosure can effectively inhibit p-ERK, have good cell proliferation inhibitory activity on $KRAS^{G12D}$ mutated cells, can effectively inhibit tumor growth in vivo, and have good drug resistance. The compounds of the present disclosure have moderate to high plasma binding rates and good pharmacokinetic properties.

**Related Definitions**

Unless otherwise specified, the following terms and phrases used herein are intended to have the following meanings. A specific term or phrase should not be considered indefinite or unclear in the absence of a particular definition, but should be understood in the conventional sense. When a trade name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, allergic reaction or other problems or complications, commensurate with a reasonable benefit/risk ratio.

The term "pharmaceutically acceptable salt" means a salt of compounds disclosed herein that is prepared by reacting the compound having a specific substituent disclosed herein with a relatively non-toxic acid or base. When compounds disclosed herein contain a relatively acidic functional group, a base addition salt can be obtained by bringing

the compound into contact with a sufficient amount of base in a pure solution or a suitable inert solvent. The pharmaceutically acceptable base addition salt includes a salt of sodium, potassium, calcium, ammonium, organic amine or magnesium or similar salts. When compounds disclosed herein contain a relatively basic functional group, an acid addition salt can be obtained by bringing the compound into contact with a sufficient amount of acid in a pure solution or a suitable inert solvent. Examples of the pharmaceutically acceptable acid addition salt include an inorganic acid salt, wherein the inorganic acid includes, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid, and the like; and an organic acid salt, wherein the organic acid includes, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid, and the like; and an salt of amino acid (such as arginine and the like), and a salt of an organic acid such as glucuronic acid and the like. Certain specific compounds disclosed herein contain both basic and acidic functional groups and can be converted to any base or acid addition salt.

The pharmaceutically acceptable salt disclosed herein can be prepared from the parent compound that contains an acidic or basic moiety by conventional chemical methods. Generally, such salt can be prepared by reacting the free acid or base form of the compound with a stoichiometric amount of an appropriate base or acid in water or an organic solvent or a mixture thereof.

Compounds disclosed herein may be present in a specific geometric or stereoisomeric form. The present disclosure contemplates all such compounds, including cis and trans isomers, (-)- and (+)-enantiomers, (R)- and (S)-enantiomers, diastereoisomer, (D)-isomer, (L)-isomer, and a racemic mixture and other mixtures, for example, a mixture enriched in enantiomer or diastereoisomer, all of which are encompassed within the scope disclosed herein. The substituent such as alkyl may have an additional asymmetric carbon atom. All these isomers and mixtures thereof are encompassed within the scope disclosed herein.

Compounds disclosed herein may contain an unnatural proportion of atomic isotopes at one or more of the atoms that make up the compounds. For example, a compound may be labeled with a radioisotope such as tritium ($^{3}H$), iodine-125 ($^{125}I$) or C-14($^{14}C$). For another example, hydrogen can be replaced by heavy hydrogen to form a deuterated drug. The bond between deuterium and carbon is stronger than that between ordinary hydrogen and carbon. Compared with undeuterated drugs, deuterated drugs have advantages of reduced toxic side effects, increased drug stability, enhanced efficacy, and prolonged biological half-life of drugs. All changes in the isotopic composition of compounds disclosed herein, regardless of radioactivity, are included within the scope of the present disclosure.

The term "optional" or "optionally" means that the subsequent event or condition may occur but not requisite, that the term includes the instance in which the event or condition occurs and the instance in which the event or condition does not occur.

The term "substituted" means that one or more than one hydrogen atoms on a specific atom are substituted by a substituent, including deuterium and hydrogen variants, as long as the valence of the specific atom is normal and the substituted compound is stable. When the substituent is oxo (i.e., =O), it means two hydrogen atoms are substituted. Positions on an aromatic ring cannot be substituted by oxo. The term "optionally substituted" means an atom can be substituted by a substituent or not, unless otherwise specified, the species and number of the substituent may be arbitrary so long as being chemically achievable.

When any variable (such as R) occurs in the constitution or structure of the compound more than once, the definition of the variable at each occurrence is independent. Thus, for example, if a group is substituted by 0-2 R, the group can be optionally substituted by up to two R, wherein the definition of R at each occurrence is independent. Moreover, a combination of the substituent and/or the variant thereof is allowed only when the combination results in a stable compound.

When the number of a linking group is 0, such as $-(CRR)_0-$, it means that the linking group is a single bond.

When one of variables is a single bond, it means that the two groups linked by the single bond are connected directly. For example, when L in A-L-Z represents a single bond, the structure of A-L-Z is actually A-Z.

When an enumerated linking group does not indicate its linking direction, its linking direction is arbitrary. For example, when the linking group L in

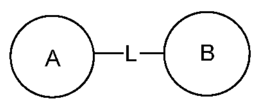

is -M-W-, the -M-W- can be linked to the ring A and the ring B in the same direction as the reading order from left to right to constitute

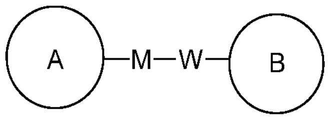,

or can be linked to the ring A and the ring B in the reverse direction as the reading order from left to right to constitute

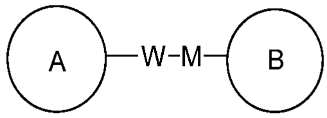.

A combination of the linking groups, substituents and/or variants thereof is allowed only when such combination can result in a stable compound.

Unless otherwise specified, when a group has one or more connectable sites, any one or more sites of the group can be connected to other groups through chemical bonds. Where the connection position of the chemical bond is variable, and there is H atom(s) at a connectable site(s), when the connectable site(s) having H atom(s) is connected to the chemical bond, the number of H atom(s) at this site will correspondingly decrease as the number of the connected chemical bond increases, and the group will become a group of corresponding valence. The chemical bond between the site and other groups can be represented by a straight solid bond

(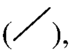),

a straight dashed bond

(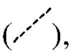),

or a wavy line

(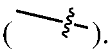).

For example, the straight solid bond in $-OCH_3$ indicates that the group is connected to other groups through the oxygen atom in the group; the straight dashed bond in

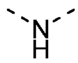

indicates that the group is connected to other groups through two ends of the nitrogen atom in the group; the wavy line in

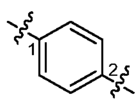

indicates that the group is connected to other groups through the 1- and 2-carbon atoms in the phenyl group;

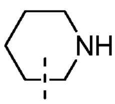

indicates that any connectable site on the piperidinyl group can be connected to other groups through one chemical bond, including at least four connection ways,

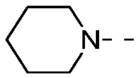,

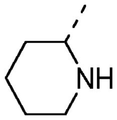, 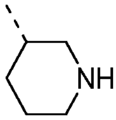 and 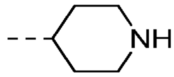;

even if a H atom is drawn on -N-,

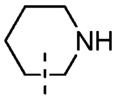

still includes the connection way of

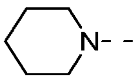;

it's just that when one chemical bond is connected, the H at this site will be reduced by one, and the group will become the corresponding monovalent piperidinyl group.

Unless otherwise specified, a wedged solid bond

(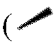)

and a wedged dashed bond

(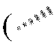)

indicate the absolute configuration of a stereocenter; a straight solid bond

(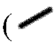)

and a straight dashed bond

(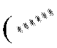)

indicate the relative configuration of a stereocenter; a wavy line

(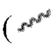)

indicates a wedged solid bond

(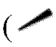)

or a wedged dashed bond

(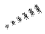);

or a wavy line

(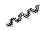)

indicates a straight solid bond

(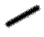)

and a straight dashed bond

(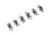).

For example,

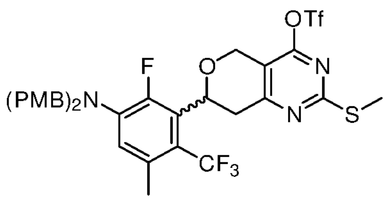

represents

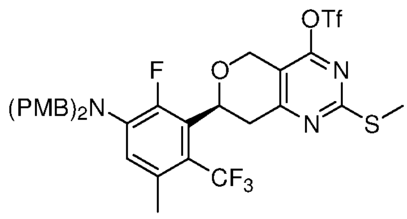

or

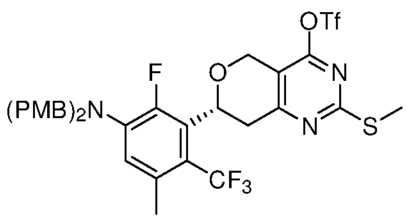

, and

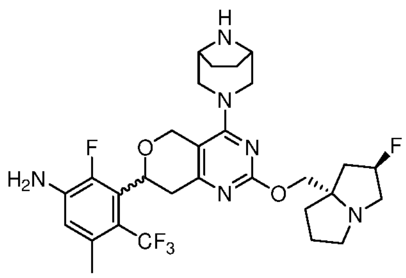

represents

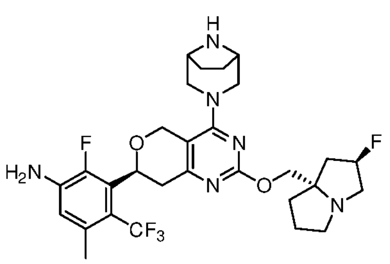

or

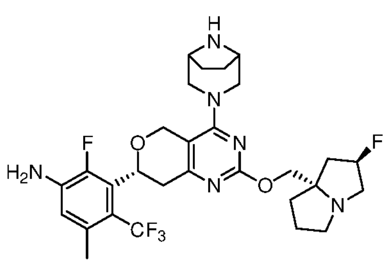

.

Unless otherwise specified, the term "$C_{1-3}$ alkyl" is used to represent a linear or branched saturated hydrocarbon group consisting of 1 to 3 carbon atoms. The $C_{1-3}$ alkyl includes $C_{1-2}$ alkyl, $C_{2-3}$ alkyl, etc. It may be monovalent (such as methyl), divalent (such as methylene) or multivalent (such as methenyl). Examples of the $C_{1-3}$ alkyl include, but are not limited to, methyl (Me), ethyl (Et), propyl (including n-propyl and isopropyl), and the like.

Unless otherwise specified, the term "$C_{1-3}$ alkoxy" means alkyl groups containing 1 to 3 carbon atoms and

attached to the remainder of a molecule by an oxygen atom. The $C_{1-3}$ alkoxy group includes $C_{1-2}$, $C_{2-3}$, $C_3$, and $C_2$ alkoxy groups, and the like. Examples of $C_{1-3}$ alkoxy groups include, but are not limited to, methoxy, ethoxy, propoxy (including n-propoxy and isopropoxy), and the like.

Unless otherwise specified, the term "$C_{1-3}$ alkylamino" means alkyl groups containing 1 to 3 carbon atoms and attached to the remainder of a molecule by an amino group. The $C_{1-3}$ alkylamino group includes $C_{1-2}$, $C_3$ and $C_2$ alkylamino groups and the like. Examples of $C_{1-3}$ alkylamino groups include, but are not limited to $-NHCH_3$, $-N(CH_3)_2$, $-NHCH_2CH_3$, $-N(CH_3)CH_2CH_3$, $-NHCH_2CH_2CH_3$, $-NHCH_2(CH_3)_2$, and the like.

Unless otherwise specified, "$C_{2-3}$ alkenyl" is used to represent a linear or branched hydrocarbon group consisting of 2 to 3 carbon atoms containing at least one carbon-carbon double bond, wherein the carbon-carbon double bond can be located at any position of the group. The $C_{2-3}$ alkenyl includes $C_3$ and $C_2$ alkenyl. The $C_{2-3}$ alkenyl may be monovalent, divalent or multivalent. Examples of the $C_{2-3}$ alkenyl include, but are not limited to, vinyl, propenyl, and the like.

Unless otherwise specified, "$C_{2-3}$ alkynyl" is used to represent a linear or branched hydrocarbon group consisting of 2 to 3 carbon atoms containing at least one carbon-carbon triple bond, wherein the carbon-carbon triple bond can be located at any position of the group. The $C_{2-3}$ alkynyl may be monovalent, divalent or multivalent. The $C_{2-3}$ alkynyl includes $C_3$ and $C_2$ alkynyl. Examples of the $C_{2-3}$ alkynyl include, but are not limited to, ethynyl, propynyl, and the like.

Unless otherwise specified, the term "4-5 membered heterocycloalkyl" alone or in combination with other terms respectively represents a saturated monocyclic group consisting of 4 to 5 ring atoms, in which 1, 2, 3 or 4 ring atoms are heteroatoms independently selected from O, S and N, and the remainder is carbon atoms, wherein the nitrogen atom is optionally quaternized and the nitrogen and sulfur heteroatoms are optionally oxidized (i.e., NO and $S(O)_p$, p is 1 or 2). In addition, with respect to the "4-5 membered heterocycloalkyl", the heteroatom may be present on the position of attachment of the heterocycloalkyl group to the remainder of a molecule. The 4-5 membered heterocycloalkyl includes 4 membered and 5 membered heterocycloalkyl. Examples of the 4-5 membered heterocycloalkyl include, but are not limited to, azetidinyl, oxetanyl, thietanyl, pyrrolidinyl, pyrazolidinyl, imidazolidinyl, tetrahydrothienyl (including tetrahydrothien-2-yl and tetrahydrothien-3-yl and the like), tetrahydrofuranyl (including tetrahydrofuran-2-yl and the like), or the like.

Unless otherwise specified, "$C_{3-5}$ cycloalkyl" refers to a saturated cyclic hydrocarbon group consisting of 3 to 5 carbon atoms, which is a monocyclic system. The $C_{3-5}$ cycloalkyl includes $C_{3-4}$ and $C_{4-5}$ cycloalkyl, etc., and may be monovalent, divalent, or polyvalent. Examples of $C_{3-5}$ cycloalkyl include, but are not limited to, cyclopropyl, cyclobutyl, cyclopentyl, etc.

Unless otherwise specified, the terms "$C_{6-10}$ aromatic ring" and "$C_{6-10}$ aryl" may be used interchangeably in this disclosure. The term"$C_{6-10}$ aromatic ring" or "$C_{6-10}$ aryl" means a cyclic hydrocarbon group having a conjugated pi electron system and consisting of 6 to 10 carbon atoms. It may be a monocyclic, fused bicyclic or fused tricyclic ring system, wherein each ring is aromatic. It may be monovalent, divalent or multivalent. The $C_{6-10}$ aryl includes $C_{6-9}$, $C_9$, $C_{10}$ and $C_6$ aryl, etc. Examples of $C_{6-10}$ aryl include, but are not limited to, phenyl, naphthyl (including 1-naphthyl and 2-naphthyl, etc.).

Unless otherwise specified, the terms "5-10 membered heteroaromatic ring" and "5-10 membered heteroaryl" may be used interchangeably. The term "5-10 membered heteroaryl" means a cyclic group having a conjugated pi electron system and consisting of 5 to 10 ring atoms, in which 1, 2, 3 or 4 ring atoms are heteroatoms independently selected from O, S and N, and the remainder is carbon atoms. It may be a monocyclic, fused bicyclic or fused tricyclic ring system, wherein each ring is aromatic, and wherein the nitrogen atom is optionally quaternized and the nitrogen and sulfur heteroatoms are optionally oxidized (i.e., NO and $S(O)_p$, p is 1 or 2). A 5-10 membered heteroaryl can be attached to the remainder of a molecule through a heteroatom or a carbon atom. The 5-10 membered heteroaryl group includes 10-membered, 9-membered, 9- to 10-membered, 5-8 membered, 5-7 membered, 5-6 membered, 5-membered and 6-membered heteroaryl groups. Examples of the 5-10 membered heteroaryl include, but are not limited to, pyrrolyl (including N-pyrrolyl, 2-pyrrolyl, 3-pyrrolyl, and the like), pyrazolyl (including 2-pyrazolyl and 3-pyrazolyl, and the like), imidazolyl (including N-imidazolyl, 2-imidazolyl, 4-imidazolyl, and 5-imidazolyl, and the like), oxazolyl (including 2-oxazolyl, 4-oxazolyl, and 5-oxazolyl, and the like), triazolyl (1H-1,2,3-triazolyl, 2H-1,2,3-triazolyl, 1H-1,2,4-triazolyl and 4H-1,2,4-triazolyl, and the like), tetrazolyl, isoxazolyl (3-isoxazolyl, 4-isoxazolyl and 5-isoxazolyl, and the like), thiazolyl (including 2-thiazolyl, 4-thiazolyl and 5-thiazolyl, and the like), furyl (including 2-furyl and 3-furyl, and the like), thienyl (including 2-thienyl and 3-thienyl, and the like), pyridyl (including 2-pyridyl, 3-pyridyl and 4-pyridyl, and the like), pyrazinyl or pyrimidinyl (including 2-pyrimidinyl and 4-pyrimidinyl, and the like), benzothiazolyl (including 5-benzothiazolyl, and the like), purinyl, benzimidazolyl (including 2-benzimidazolyl, and the like), benzoxazolyl, indolyl (including 5-indolyl, and the like), isoquinolyl (including 1-isoquinolyl, 5-isoquinolyl, and the like), quinoxalinyl (including 2-quinoxalinyl, 5-quinoxalinyl, and the like) or quinolyl (including 3-quinolyl, 6-quinolyl, and the like).

Unless otherwise specified, the term "4-8 membered heterocycloalkyl" alone or in combination with other terms respectively represents a saturated cyclic group consisting of 4 to 8 ring atoms, in which 1, 2, 3 or 4 ring atoms are heteroatoms independently selected from O, S and N, and the remainder is carbon atoms, wherein the nitrogen atom is optionally quaternized, and the nitrogen and sulfur heteroatoms are optionally oxidized (i.e., NO and $S(O)_p$, p is 1 or

2). The ring comprises monocyclic and bicyclic ring systems, wherein the bicyclic ring systems comprise spiro, fused, and bridged cyclic rings. In addition, with respect to the "4-8 membered heterocycloalkyl", the heteroatom may be present on the position of attachment of the heterocycloalkyl group to the remainder of a molecule. The 4-8 membered heterocycloalkyl includes 4-6 membered, 5-6 membered, 4-membered, 5-membered, and 6-membered heterocycloalkyl, etc. Examples of the 4-8 membered heterocycloalkyl include, but are not limited to, azetidinyl, oxetanyl, thietanyl, pyrrolidinyl, pyrazolidinyl, imidazolidinyl, tetrahydrothienyl (including tetrahydrothien-2-yl and tetrahydrothien-3-yl and the like), tetrahydrofuranyl (including tetrahydrofuran-2-yl and the like), tetrahydropyranyl, piperidinyl (including 1-piperidinyl, 2-piperidinyl and 3-piperidinyl and the like), piperazinyl (including 1-piperazinyl and 2-piperazinyl and the like), morpholinyl (including 3-morpholinyl and 4-morpholinyl and the like), dioxanyl, dithianyl, isoxazolidinyl, isothiazolidinyl, 1,2-oxazinyl, 1,2-thiazinyl, hexahydropyridazinyl, homopiperazinyl, homopiperidinyl or dioxepanyl, and the like.

Unless otherwise specified, the term "5-6 membered heterocycloalkenyl" alone or in combination with other terms respectively represents a partially unsaturated cyclic group containing at least one carbon-carbon double bond and consisting of 5 to 6 ring atoms, of which 1, 2, 3 or 4 ring atoms are heteroatoms independently selected from O, S and N, and the remainder atoms are carbon atoms, wherein the nitrogen atom is optionally quaternized and the nitrogen and sulfur heteroatoms are optionally oxidized (i.e., NO and $S(O)_p$, wherein p is 1 or 2). The 5-6 membered heterocycloalkenyl includes monocyclic and bicyclic systems, wherein the bicyclic system includes spiro-, fused- and bridged-rings, and any ring of the system is nonaromatic. In addition, with respect to the "5-6 membered heterocycloalkenyl", a heteroatom may be present at the position of attachment of the heterocycloalkenyl group to the remainder of a molecule. The 5-6 membered heterocycloalkenyl group includes 5-membered and 6-membered heterocycloalkenyl groups, etc. Examples of 5-6 membered heterocycloalkenyl groups include, but are not limited to,

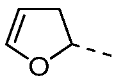, 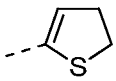, 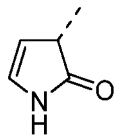, 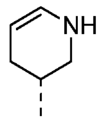, 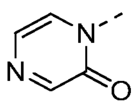,

or

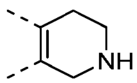.

Unless otherwise specified, $C_{n-n+m}$ or $C_n$-$C_{n+m}$ includes any specific case of n to n+m carbons, for example, $C_{1-12}$ includes $C_1$, $C_2$, $C_3$, $C_4$, $C_5$, $C_6$, $C_7$, $C_8$, $C_9$, $C_{10}$, $C_{11}$ and $C_{12}$, also includes any range from n to n+m, for example, $C_{1-12}$ includes $C_{1-3}$, $C_{1-6}$, $C_{1-9}$, $C_{3-6}$, $C_{3-9}$, $C_{3-12}$, $C_{6-9}$, $C_{6-12}$ and $C_{9-12}$, etc.; similarly, n membered to n+m membered indicates that the number of atoms on a ring is n to n+m, for example, 3-12 membered ring includes 3-membered ring, 4-membered ring, 5-membered ring, 6-membered ring, 7-membered ring, 8-membered ring, 9-membered ring, 10-membered ring, 11-membered ring, and 12-membered ring, also includes any range from n to n+m, for example, 3-12 membered ring includes 3-6 membered ring, 3-9 membered ring, 5-6 membered ring, 5-7 membered ring, 6-7 membered ring, 6-8 membered ring, and 6-10 membered ring, and the like.

Compounds disclosed herein can be prepared by a variety of synthetic methods well known to those skilled in the art, including the following enumerated embodiment, the embodiment formed by the following enumerated embodiment in combination with other chemical synthesis methods, and equivalent replacement well known to those skilled in the art. Alternative embodiments include, but are not limited to the embodiment disclosed herein.

The structures of compounds disclosed herein can be confirmed by conventional methods well known to those skilled in the art. If the present disclosure relates to an absolute configuration of a compound, the absolute configuration can be confirmed by conventional techniques in the art, such as single crystal X-Ray diffraction (SXRD). In the single crystal X-Ray diffraction (SXRD), the diffraction intensity data of the cultivated single crystal is collected using a Bruker D8 venture diffractometer with a light source of CuKα radiation in a scanning mode of φ/ω scan; after collecting the relevant data, the crystal structure is further analyzed by the direct method (Shelxs97) to confirm the absolute configuration.

Solvents used in the present disclosure are commercially available. The following abbreviations are used in the present disclosure: hr represents hour; LDA represents lithium diisopropylamide; $B_2Pin_2$ represents diboron pinacol ester; $Pd(dppf)Cl_2 \cdot CH_2Cl_2$ represents [1,1'-bis(diphenylphosphino)ferrocene]palladium dichloride dichloromethane complex; DIPEA represents N,N-diisopropylethylamine; NBS represents N-bromosuccinimide; NIS represents N-iodosuccinimide; $PdCl_2(PPh_3)_2$ represents bis(triphenylphosphine)palladium dichloride; CuI represents cuprous iodide; EtsN represents triethylamine; $K_4FeCN_6$ represents potassium hexacyanoferrate; n-BuLi represents n-butyllithium; $PhNTf_2$

represents N-phenyl-bis(trifluoromethanesulfonimide); $Pd(dppf)Cl_2$ represents [1,1'-bis(diphenylphosphino)ferrocene] palladium dichloride.

Compounds are named according to general naming principles in the art or by ChemDraw® software, and commercially available compounds are named with their vendor directory names.

## DETAILED DESCRIPTION OF THE INVENTION

The present disclosure is described in detail below by means of examples. However, it is not intended that these examples have any disadvantageous limitations to the present disclosure. The present disclosure has been described in detail herein, and embodiments are also disclosed herein. It will be apparent to those skilled in the art that various changes and modifications may be made to the embodiments disclosed herein without departing from the spirit and scope disclosed herein.

### Reference example 1

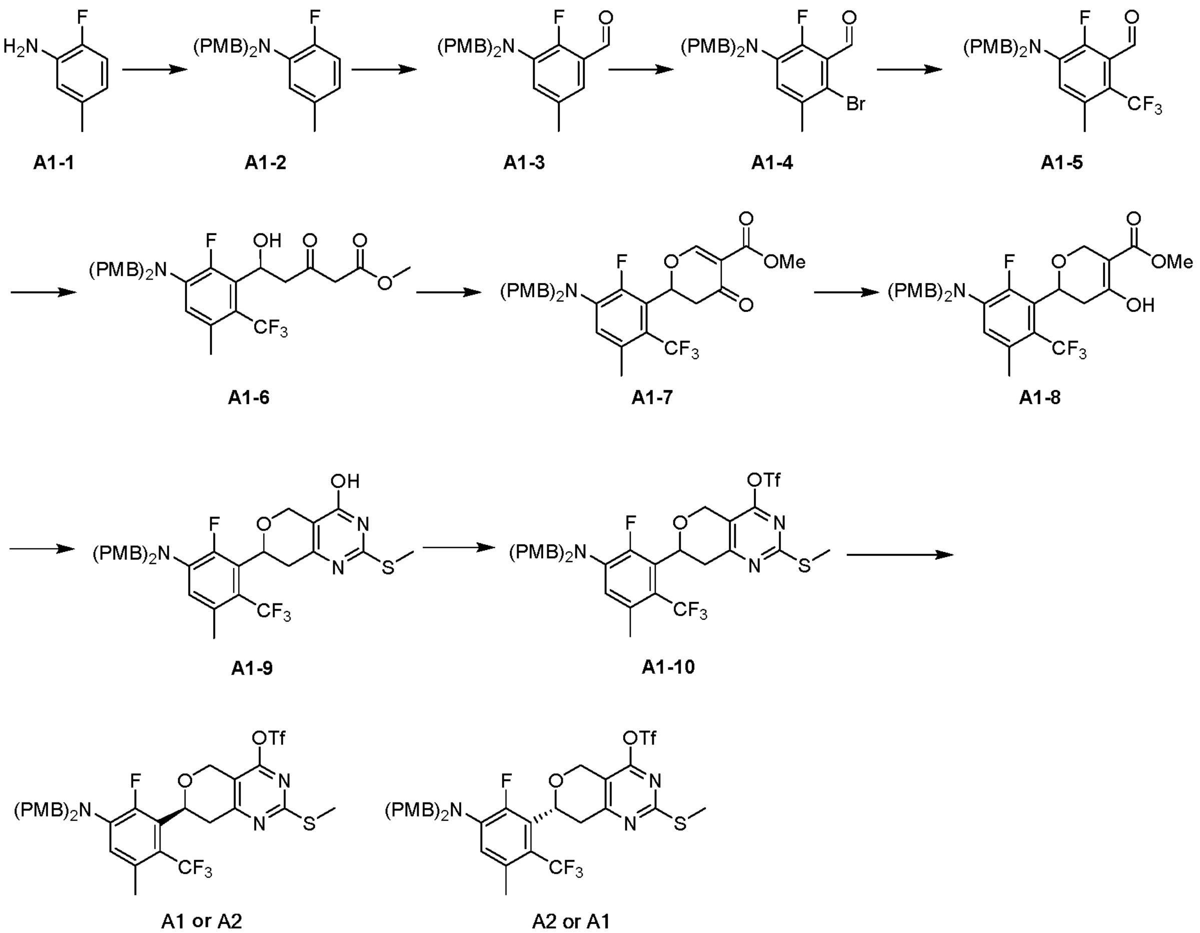

A1-1 A1-2 A1-3 A1-4 A1-5

A1-6 A1-7 A1-8

A1-9 A1-10

A1 or A2 A2 or A1

### Step 1: Synthesis of compound A1-2

In a dry 2 L three-necked flask, sodium hydride (39.12 g, 978.08 mmol, 60%) was added to N,N-dimethylformamide (510 mL). The reaction system was heterogeneous gray and cooled down to 0 °C. A solution of compound **A1-1** (51 g, 407.53 mmol) in N,N-dimethylformamide (200 mL) was added dropwise under nitrogen. The mixture was reacted at 0 °C for another 0.5 hours, and p-methoxybenzyl chloride (140.41 g, 896.57 mmol, 122.10 mL) was added. The mixture was slowly warmed to 20 °C and stirred for another 7.5 hours under nitrogen. The reaction solution was slowly added to 200 mL of saturated ammonium chloride. The mixture was extracted with methyl tert-butyl ether (200 mL * 2). The combined organic phase was washed with 200 mL of saturated brine, dried over anhydrous sodium sulfate, and filtered. The organic solvent was removed under reduced pressure. The resulting crude product was purified by silica

gel column chromatography (eluent: petroleum ether:ethyl acetate = 100:0-10:1) to give compound **A1-2**. $^1$H NMR (400 MHz, $CDCl_3$) δ: 7.23 - 7.18 (m, 4H), 6.91 - 6.87(m, 1H), 6.82 - 6.76 (m, 4H), 6.65 -6.59 (m, 2H), 4.20 (s, 4H), 3.79 (s, 6H), 2.19 (s, 3H). MS m/z: 366.1 $[M+H]^+$.

**Step 2: Synthesis of compound A1-3**

2,2,6,6-Tetramethylpiperidine (31.31 g, 221.65 mmol, 37.63 mL) was added to anhydrous tetrahydrofuran (300 mL). The mixture was cooled down to -5 °C, and n-butyl lithium (2.5 M, 94.57 mL) was added dropwise. The mixture was reacted at -5-0 °C for 15 minutes and cooled down to -60 °C. A solution of compound **A1-2** (27 g, 73.88 mmol) in THF (60 mL) was added to the reaction solution. The mixture was reacted at -60 °C for 0.5 hours and N,N-dimethylformamide (108.00 g, 1.48 mol, 113.69 mL) was added quickly. The reaction solution was stirred at -60 °C for another 10 minutes. To the reaction solution was added 400 mL of saturated ammonium chloride. The mixture was extracted with methyl tert-butyl ether (200 mL * 2). The combined organic phase was washed with 200 mL of saturated brine, dried over anhydrous sodium sulfate, and filtered. The organic solvent was removed under reduced pressure. The resulting crude product was slurried with a solvent mixture (petroleum ether:methyl tert-butyl ether = 5:1, 70 mL) for 0.5 hours, and filtered. The filter cake was dried. The filtrate was concentrated, and then the residue was purified by silica gel column chromatography (eluent: petroleum ether:ethyl acetate = 100:0-10:1). The filter cake and the product of column chromatography were combined to give compound **A1-3.** $^1$H NMR (400MHz, $CDCl_3$) δ: 10.43 - 10.35 (m, 1H), 7.21 - 7.18 (m, 5H), 6.92 - 6.81 (m, 5H), 4.25 (s, 4H), 3.80 (s, 6H), 2.23 (s, 3H). MS *m*/*z*: 394.2 $[M+H]^+$.

**Step 3: Synthesis of compound A1-4**

Compound **A1-3** (17.8 g, 45.24 mmol) was added to N,N-dimethylformamide (170 mL), and bromosuccinimide (8.05 g, 45.24 mmol) was added. The reaction solution was stirred at 20 °C for another 20 minutes. The reaction solution was added to 300 mL of water. The mixture was extracted with methyl tert-butyl ether (150 mL * 2). The combined organic phase was washed with saturated brine (100 mL * 2), dried over anhydrous sodium sulfate, and filtered. The organic solvent was removed under reduced pressure. The resulting crude product was slurried with a solvent mixture (ethyl acetate:methyl tert-butyl ether=1:1, 50 mL) for 0.5 hours, and filtered. The filter cake was dried to give compound **A1-4**. $^1$H NMR (400MHz, $CDCl_3$) δ: 10.39 (s, 1H), 7.17 (d, *J*= 8.8 Hz, 4H), 6.89 (d, *J*= 8.8 Hz, 1H), 6.85 - 6.82 (m, 4H), 4.22 (s, 4H), 3.79 (s, 6H), 2.28 (s, 3H). MS *m*/*z*: 472.1$[M+H]^+$, 474.1$[M+3H]^+$.

**Step 4: Synthesis of compound A1-5**

Compound **A1-4** (19.3 g, 40.86 mmol) was added to N,N-dimethylformamide (190 mL). To the reaction solution were added cuprous iodide (15.56 g, 81.72 mmol) and methyl fluorosulfonyldifluoroacetate (39.25 g, 204.30 mmol, 25.99 mL). The reaction solution was heated to 100 °C and stirred for another 1 hour. The mixture was cooled down. The reaction solution was filtered through a pad of Celite, and the filtrate was added to 300 mL of water. The mixture was extracted with methyl tert-butyl ether (150 mL * 2). The combined organic phase was washed with saturated brine (200 mL * 2), dried over anhydrous sodium sulfate, and filtered. The organic solvent was removed under reduced pressure. The resulting crude product was purified by silica gel column chromatography (eluent: petroleum ether:ethyl acetate = 100:0-10:1) to give compound **A1-5**. $^1$H NMR (400MHz, $CDCl_3$) δ: 10.37 (q, *J* = 4.0 Hz, 1H), 7.18 - 7.11 (m, 4H), 6.89 - 6.82 (m, 4H), 6.73 (d, *J* = 8.8 Hz, 1H), 4.36 (s, 4H), 3.81 (s, 6H), 2.37 - 2.29 (m, 3H). MS m/z: 484.0$[M+Na]^+$.

**Step 5: Synthesis of compound A1-6**

Anhydrous tetrahydrofuran (50 mL) and sodium hydride (1.17 g, 29.26 mmol, 60%) were added to a dry three-necked flask. The mixture was cooled down to 0 °C. Ethyl acetoacetate (3.40 g, 29.26 mmol, 3.15 mL) was added dropwise under nitrogen. The reaction solution was stirred at 0 °C for another 0.5 hours, and n-butyl lithium (2.5 M, 11.70 mL) was added dropwise. The reaction solution was stirred under this condition for another 0.5 hours and cooled to -60 °C. A solution of compound **A1-5** (4.5 g, 9.75 mmol) in tetrahydrofuran (20 mL) was added dropwise. The reaction solution was stirred at -60 °C for another 0.5 hours. To the reaction solution was added 100 mL of saturated ammonium chloride solution. The mixture was extracted with 30 mL of ethyl acetate. The organic phase was washed with 80 mL of saturated brine, dried over anhydrous sodium sulfate, and filtered. The organic solvent was removed under reduced pressure. The resulting crude product was purified by silica gel column chromatography (eluent: petroleum ether:ethyl acetate = 100:0-3:1) to give compound **A1-6**. $^1$H NMR (400MHz, $CDCl_3$) δ: 7.18 - 7.15 (m, 4H), 6.90 - 6.78 (m, 4H), 6.61 (d, *J* = 8.8 Hz, 1H), 5.72 - 5.57 (m, 1H), 4.31 (m, 4H), 3.81(s, 6H), 3.76(s, 3H), 3.56 (s, 2H), 3.50 - 3.38 (m, 1H), 2.98 - 2.93 (m, 1H), 2.38 - 2.26 (m, 3H). MS *m*/*z*: 578.1$[M+H]^+$.

**Step 6: Synthesis of compound A1-7**

Compound **A1-6** (3 g, 5.19 mmol) was added to anhydrous dichloromethane (30 mL), and N,N-dimethylformamide dimethyl acetal (742.74 mg, 6.23 mmol, 828.02 μL) was added. The reaction solution was stirred at 20 °C for 16 hours. Boron trifluoride diethyl ether (884.66 mg, 6.23 mmol, 769.27 μL) was added. The mixture was stirred at 20 °C for another 1 hour. The reaction solution was added to 20 mL of saturated sodium bicarbonate solution. The layers were separated. The aqueous phase was extracted with 20 mL of dichloromethane. The combined organic phase was dried over anhydrous sodium sulfate, and filtered. The organic solvent was removed under reduced pressure. The resulting crude product was purified by silica gel column chromatography (eluent: petroleum ether:ethyl acetate = 100:0-3:1) to give compound **A1-7**. $^1H$ NMR (400MHz, $CDCl_3$) δ: 8.43 (d, *J* = 0.8 Hz, 1H), 7.21 - 7.10 (m, 4H), 6.91 - 6.81 (m, 4H), 6.70 (d, *J* = 8.8 Hz, 1H), 5.93 (dd, *J* = 3.2, 14.8 Hz, 1H), 4.35 (s, 4H), 3.8(s, 3H), 3.81 (s, 6H), 3.38 - 3.29 (m, 1H), 2.68 (dd, *J* = 3.6, 16.8 Hz, 1H), 2.39 - 2.24 (m, 3H). MS *m/z*: 588.2$[M+H]^+$.

**Step 7: Synthesis of compound A1-8**

Compound **A1-7** (2.1 g, 3.57 mmol) was added to anhydrous tetrahydrofuran (21 mL). The mixture was cooled down to -60 °C. Tri-sec-butyl lithium borohydride (1 M, 4.29 mL) was added under nitrogen. The reaction solution was stirred at -60 °C for another 0.5 hours. The reaction solution was added to 30 mL of saturated ammonium chloride. The layers were separated. The aqueous layer was extracted with ethyl acetate (30 mL*2). The combined organic phase was washed with 20 mL of saturated brine, dried over anhydrous sodium sulfate, and filtered. The organic solvent was removed under reduced pressure. The resulting crude product was purified by silica gel column chromatography (eluent: petroleum ether:ethyl acetate = 100:0 = 3:1) to give compound **A1-8**. $^1H$ NMR (400MHz, $CDCl_3$) δ: 7.167 - 7.14(m, 4H), 6.87 - 6.83 (m, 4H), 6.63 (d, *J* = 8.8 Hz, 1H), 5.05 - 5.00 (m, 1H), 4.61 - 4.58 (m, 1H), 4.42 - 4.24 (m, 5H), 3.85 - 3.73 (m, 10H), 3.13 - 3.05 (m, 1H), 2.47 - 2.38 (m, 1H), 2.35 - 2.31 (m, 3H). MS *m/z*: 590.1$[M+H]^+$.

**Step 8: Synthesis of compound A1-9**

Compound **A1-8** (1.27 g, 2.15 mmol) was added to ethanol (15 mL) and water (3 mL), and sodium bicarbonate (3.62 g, 43.08 mmol, 1.68 mL) and methylisothiourea sulfate (4.05 g, 21.54 mmol) were added. The reaction solution was stirred at 50 °C for another 4 hours. The reaction solution was added to 40 mL of water. The mixture was extracted with ethyl acetate (20 mL * 2). The combined organic phase was washed with saturated brine (20 mL * 2), dried over anhydrous sodium sulfate, and filtered. The organic solvent was removed under reduced pressure. The resulting crude product was purified by silica gel column chromatography (eluent: petroleum ether:ethyl acetate = 100:0-1:1) to give compound **A1-9**. $^1H$ NMR (400MHz, $CDCl_3$) δ: 7.22 - 7.14 (m, 4H), 6.91 - 6.82 (m, 4H), 6.65 (dd, J = 8.4 Hz 1H), 5.12 - 5.08 (m, 1H), 4.97 - 4.91 (m, 1H), 4.67 - 4.57 (m, 1H), 4.45 - 4.22 (m, 4H), 3.88 - 3.74 (m, 6H), 3.43 - 3.35 (m, 1H), 2.77 - 2.72 (m, 1H), 2.59 (m, 3H), 2.40 - 2.31 (m, 3H). MS m/z: 630.2$[M+H]^+$.

**Step 9: Synthesis of compounds A1 and A2**

Compound **A1-9** (51 g, 81.00 mmol) was dissolved in dichloromethane (500 mL), and N,N-diisopropylethylamine (31.40 g, 242.99 mmol, 42.32 mL) was added. The mixture was cooled down to 0~10 °C, and trifluoromethanesulfonic anhydride (34.28 g, 121.49 mmol, 20.05 mL) was slowly added to the reaction solution. The mixture was reacted at this temperature for 15 minutes. The reaction solution was poured into saturated aqueous ammonium chloride solution (400 mL). The layers were separated. The aqueous phase was extracted with dichloromethane (50 mL * 2). The combined organic phase was dried over anhydrous sodium sulfate, and filtered. The organic solvent was removed under reduced pressure. The resulting crude product was slurried with a solvent mixture (petroleum ether:methyl tert-butyl ether = 20:1, 100 mL), and filtered. The filter cake was dried to give **A1-10**. 20 g of **A1-10** was taken to be purified by supercritical liquid chromatography (SFC) (column: DAICEL CHIRALPAK IG (250mm*50mm, 10μm); mobile phase: A ($CO_2$) and B (0.1%$NH_3H_2O$ EtOH); gradient: EtOH%: 11%-11%, 8min). **A1** (column: Chiralpak IG-3, 3 μm, 0.46 cm id × 5cm L; mobile phase: A ($CO_2$) and B (EtOH, containing 0.1% isopropylamine); gradient: B%=5-50%, 3 min; flow rate: 3.4 mL/min; wavelength: 220nm; pressure: 1800psi, Rt=0.924 min, MS: *m/z* (ESI): 762.0 $[M+H]^+$, optical rotation: $[\alpha]_D^{20}=16.82$, concentration: 0.1682 g/100 mL.) and **A2** (column: Chiralpak IG-3, 3 μm, 0.46 cm id × 5cm L; mobile phase: A ($CO_2$) and B (EtOH, containing 0.1% isopropylamine); gradient: B%=5-50%, 3 min; flow rate: 3.4 mL/min; wavelength: 220nm; pressure: 1800psi, Rt=1.073min, chiral purity: 99.99%, MS: *m/z* (ESI): 762.0 $[M+H]^+$, optical rotation: $[\alpha]_D^{20}$=-18.41, concentration: 0.3476 g/100 mL) were obtained. $^1H$ NMR (400 MHz, $CDCl_3$) δ: 7.03 - 7.14 (m, 4 H), 6.73 - 6.82 (m, 4

H), 6.57(d, *J*=8.4,1 H) 5.08 (d, *J*=9.6,1 H), 4.92 (d, *J*=15.6,1 H), 4.67 (d, *J*=15.6,1 H), 4.24 (q, *J*=10,4 H), 3.719 (s, 6 H) 3.42 - 3.59 (m, 1 H), 2.87 - 3.04 (m, 1 H), 2.47 (s, 3 H), 2.19 - 2.35 (m, 3 H).

**Example 1**

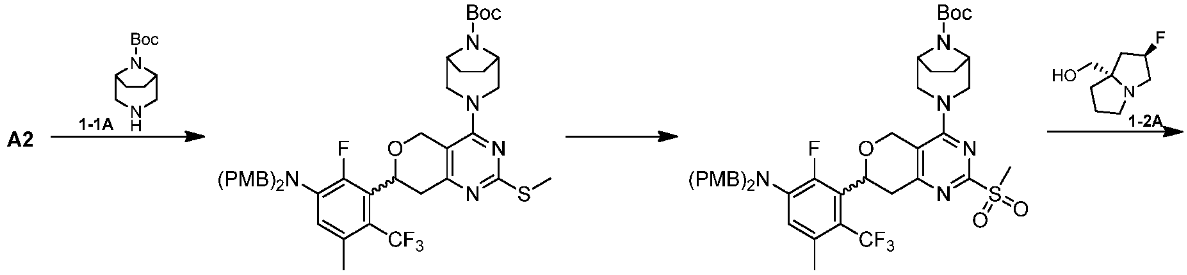


**1-1** **1-2**

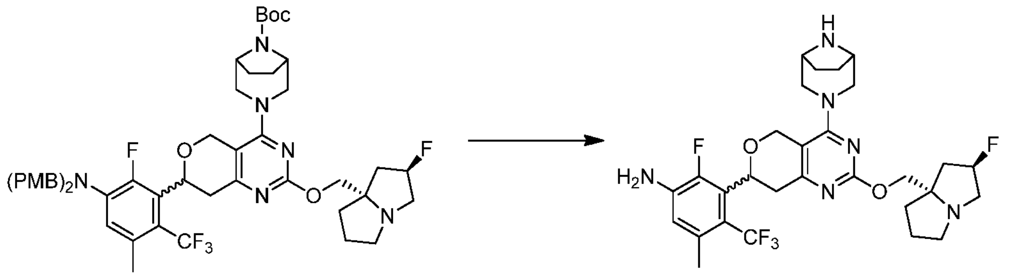

**1-3** **1**

**Step 1: Preparation of intermediate 1-1**

Compound **A2** (80 mg, 105.02 μmol) and compound **1-A1** (26.75 mg, 126.03 μmol) were dissolved in N,N-dimethylformamide (2 mL), and diisopropylethylamine (40.72 mg, 315.07 μmol, 54.88 μL) was added. The reaction solution was heated to 100 °C and stirred for another 1 hour. The mixture was cooled down. The organic solvent was removed under reduced pressure. The resulting crude product was purified by preparative thin layer chromatography (developer: petroleum ether:ethyl acetate = 2:1) to give compound **1-1**. MS m/z =824.3 $[M+H]^+$.

**Step 2: Preparation of intermediate 1-2**

Compound **1-1** (70 mg, 84.96 μmol) was dissolved in dichloromethane (2 mL), and m-chloroperoxybenzoic acid (34.50 mg, 169.92 μmol, 85% content) was added. The reaction solution was stirred at 20 °C for another 3 hours. The organic solvent was removed under reduced pressure, and the resulting crude product was purified by preparative thin layer chromatography (developer: dichloromethane: methanol = 10:1) to give compound **1-2**. MS m/z =856.2 $[M+H]^+$.

**Step 3: Preparation of intermediate 1-3**

Compound **1-2A** (12.09 mg, 75.94 μmol) was dissolved in anhydrous toluene (1 mL) under the condition of an ice-water bath. Sodium tert-butoxide (7.30 mg, 75.94 μmol) was added, and the reaction solution was stirred for another 30 minutes. A solution of compound **1-2** (50 mg, 58.42 μmol) in toluene (1 mL) was added, and the reaction solution was stirred for another 2 hours under an ice-water bath. The organic solvent was removed under reduced pressure, and the resulting crude product was purified by preparative thin layer chromatography (developer: dichloromethane: methanol = 10:1) to give compound **1-3.** MS m/z =935.3 $[M+H]^+$.

**Step 4: Preparation of compound 1 hydrochloride**

Compound **1-3** (40 mg, 42.78 μmol) was dissolved in anhydrous dichloromethane (2 mL), and trifluoroacetic acid (1 mL) was added. The reaction solution was stirred at 20 °C for another 2 hours. The solvent was removed under

reduced pressure. The resulting crude product was purified by high-performance liquid chromatography (column: Phenomenex Synergi C18 150*30mm*4μm; mobile phase: [water (0.05% hydrochloric acid)-acetonitrile]; (acetonitrile)%: 15%-45%, 9 minutes) to give compound **1** hydrochloride. $^1H$ NMR (400MHz, $CD_3OD$) δ: 6.86 - 6.84 (d, *J*=8.0 Hz, 1H), 5.66 - 5.53 (m, 1H), 5.27 - 5.25 (m, 1H), 5.01 - 4.98 (m, 1H), 4.84 - 4.77 (m, 2H), 4.30 - 4.17 (m, 2H), 4.12 - 3.82 (m, 5H), 3.61 - 3.58 (m, 1H), 3.51 - 3.38 (m, 2H), 3.08 - 3.03 (m, 1H), 2.80 - 2.47 (m, 7H), 2.44 - 1.98 (m, 10H). MS m/z =595.6 $[M+H]^+$.

**Example 2**

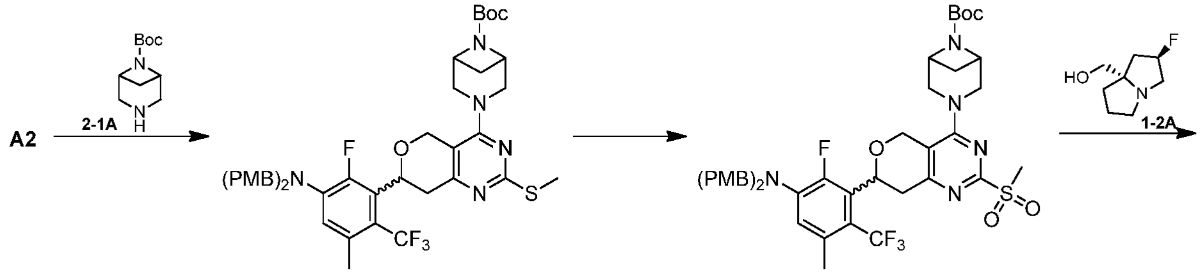


2-1 2-2

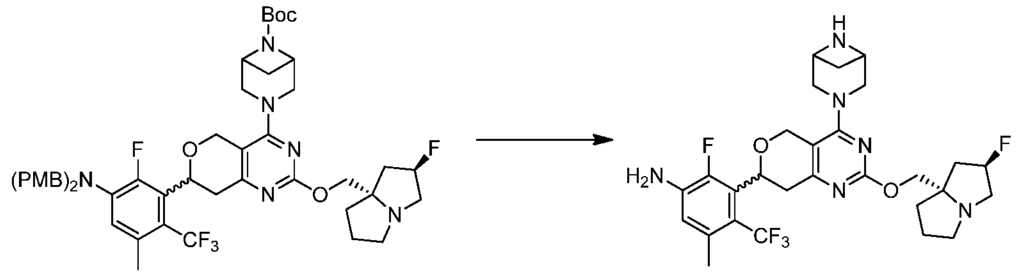

2-3 2

**Step 1: Preparation of intermediate 2-1**

Compound **A2** (80 mg, 105.02 μmol) and compound **2-A1** (27.07 mg, 136.53 μmol) were dissolved in N,N-dimethylformamide (1.2 mL), and diisopropylethylamine (33.93 mg, 262.56 μmol, 45.73 μL) was added. The reaction solution was heated to 100 °C and stirred for another 1 hour. The mixture was cooled down. The organic solvent was removed under reduced pressure. The resulting crude product was purified by preparative thin layer chromatography (developer: petroleum ether:ethyl acetate = 3:1) to give compound **2-1.** MS m/z =810.1 $[M+H]^+$.

**Step 2: Preparation of intermediate 2-2**

Compound **2-1** (73 mg, 90.13 μmol) was dissolved in dichloromethane (1 mL), and m-chloroperoxybenzoic acid (36.60 mg, 180.27 μmol, 85% content) was added. The reaction solution was stirred at 20 °C for another 15 hours. The organic solvent was removed under reduced pressure, and the resulting crude product was purified by preparative thin layer chromatography (developer: petroleum ether:ethyl acetate = 1:1) to give compound **2-2.** MS m/z =842.0 $[M+H]^+$.

**Step 3: Preparation of intermediate 2-3**

Compound **1-2A** (20.80 mg, 130.66 μmol) was dissolved in anhydrous tetrahydrofuran (1 mL) under the condition of 15 °C, and sodium tert-butoxide (12.56 mg, 130.66 μmol) was added. The reaction solution was stirred for another 30 minutes. Compound **2-2** (55 mg, 65.33 μmol) was added, and the reaction solution was stirred at this temperature for another 1 hour. The organic solvent was removed under reduced pressure, and the resulting crude product was purified by preparative thin layer chromatography (developer: dichloromethane: methanol = 10:1) to give compound **2-3.** MS m/z =921.4 $[M+H]^+$.

**Step 4: Preparation of compound 2 hydrochloride**

Compound **2-3** (42 mg, 45.60 μmol) was dissolved in anhydrous dichloromethane (0.5 mL), and trifluoroacetic acid (0.25 mL) was added. The reaction solution was stirred at 15 °C for another 2 hours. The solvent was removed under reduced pressure. The resulting crude product was purified by high-performance liquid chromatography (column: Phenomenex Synergi C18 150*30mm*4μm; mobile phase: [water (0.05% hydrochloric acid)-acetonitrile]; (acetonitrile)%: 12%-42%, 9 minutes) to give compound **2** hydrochloride. MS m/z =581.6 $[M+H]^+$.

**Example 3**

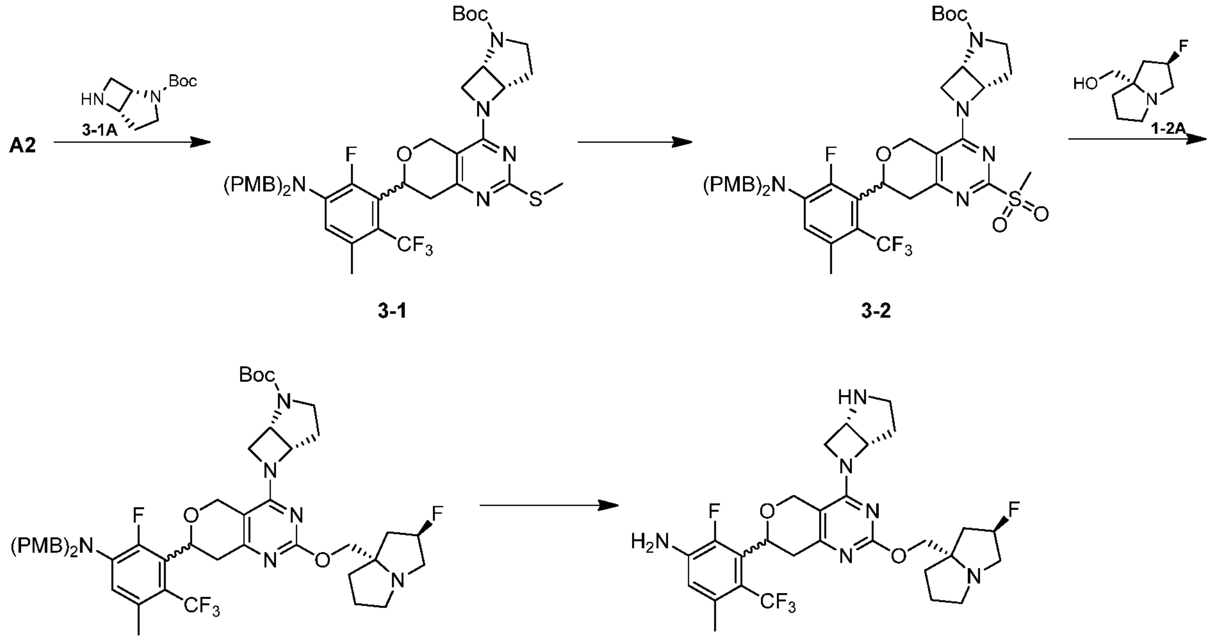


3-1 3-2 3-3 3

**Step 1: Preparation of intermediate 3-1**

Compound **A2** (80 mg, 105.02 μmol) and compound **3-1A** (24.99 mg, 126.03 μmol) were dissolved in N,N-dimethylformamide (1.2 mL), and diisopropylethylamine (40.72 mg, 315.07 μmol, 54.88 μL) was added. The reaction solution was heated to 100 °C and stirred for another 1 hour. The mixture was cooled down. The organic solvent was removed under reduced pressure. The resulting crude product was purified by preparative thin layer chromatography (developer: petroleum ether:ethyl acetate = 2:1) to give compound **3-1.** MS m/z =810.2 $[M+H]^+$.

**Step 2: Preparation of intermediate 3-2**

Compound **3-1** (67 mg, 82.73 μmol) was dissolved in dichloromethane (1 mL), and m-chloroperoxybenzoic acid (33.59 mg, 165.45 μmol, 85% content) was added. The reaction solution was stirred at 20 °C for another 5 hours. The organic solvent was removed under reduced pressure. The resulting crude product was purified by preparative thin layer chromatography (developer: petroleum ether:ethyl acetate = 1:1) to give compound **3-2.** MS m/z =842.4 $[M+H]^+$.

**Step 3: Preparation of intermediate 3-3**

Compound **1-2A** (15.13 mg, 95.02 μmol) was dissolved in anhydrous tetrahydrofuran (1 mL) under the condition of 15 °C, and sodium tert-butoxide (9.13 mg, 95.02 μmol) was added. The reaction solution was stirred for another 30 minutes. Compound **3-2** (40 mg, 47.51 μmol) was added, and the reaction solution was stirred at this temperature for another 1 hour. The organic solvent was removed under reduced pressure. The resulting crude product was purified by preparative thin layer chromatography (developer: dichloromethane: methanol = 10:1) to give compound **3-3.** MS m/z =921.4 $[M+H]^+$.

**Step 4: Preparation of compound 3 hydrochloride**

Compound **3-3** (20 mg, 21.72 μmol) was dissolved in anhydrous dichloromethane (0.5 mL), and trifluoroacetic acid (0.25 mL) was added. The reaction solution was stirred at 15 °C for another 2 hours. The solvent was removed under reduced pressure. The resulting crude product was purified by high-performance liquid chromatography (column: Phenomenex Synergi C18 150*30mm*4μm; mobile phase: [water (0.05% hydrochloric acid)-acetonitrile]; (acetonitrile)%: 12%-42%, 9 minutes) to give compound **3** hydrochloride. MS m/z =581.6 $[M+H]^+$.

**Example 5**

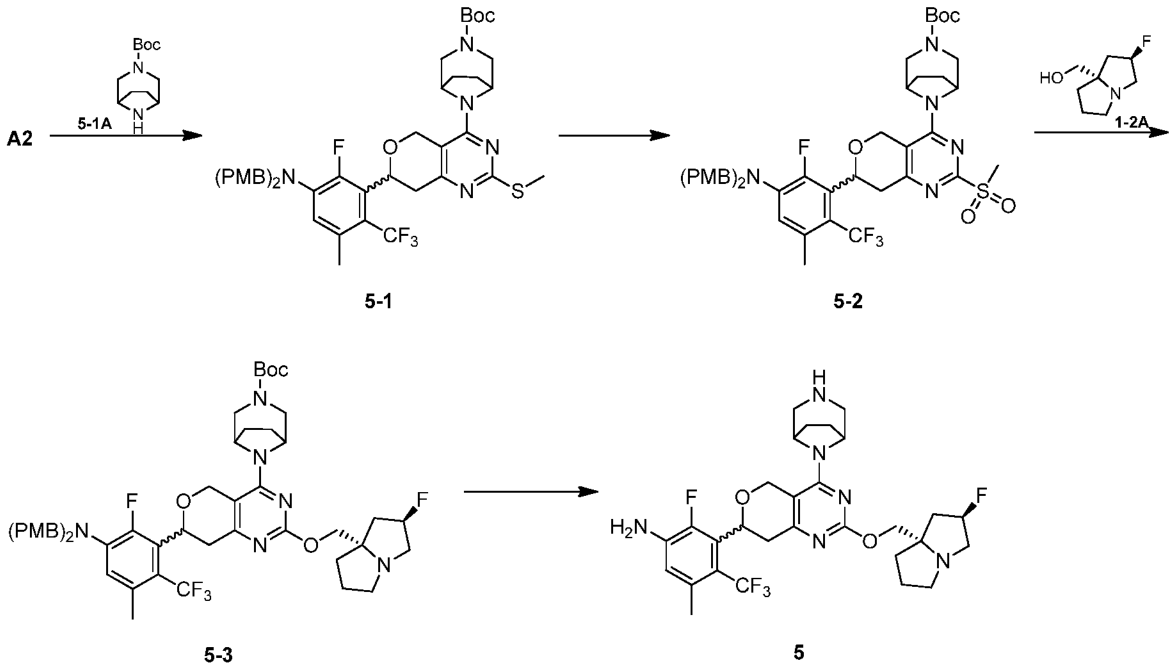


5-1 5-2 5-3 5

**Step 1: Preparation of intermediate 5-1**

Compound **A2** (80 mg, 105.02 μmol) and compound **5-1A** (22.30 mg, 105.02 μmol) were dissolved in N,N-dimethylformamide (1 mL), and diisopropylethylamine (40.72 mg, 315.07 μmol, 54.88 μL) was added. The reaction solution was heated to 100 °C and stirred for another 1 hour. The mixture was cooled down. The organic solvent was removed under reduced pressure. The resulting crude product was purified by preparative thin layer chromatography (developer: petroleum ether:ethyl acetate = 5:1) to give compound **5-1.** MS m/z =824.3 $[M+H]^+$.

**Step 2: Preparation of intermediate 5-2**

Compound **5-1** (60 mg, 72.82 μmol) was dissolved in dichloromethane (2 mL), and m-chloroperoxybenzoic acid (29.57 mg, 145.64 μmol, 85% content) was added. The reaction solution was stirred at 20 °C for another 16 hours. The organic solvent was removed under reduced pressure. The resulting crude product was purified by preparative thin layer chromatography (developer: petroleum ether:ethyl acetate = 1:1) to give compound **5-2.** MS m/z =856.3 $[M+H]^+$.

**Step 3: Preparation of intermediate 5-3**

Compound **1-2A** (12.09 mg, 75.94 μmol) was dissolved in anhydrous tetrahydrofuran (1 mL), and sodium tert-butoxide (7.30 mg, 75.94 μmol) was added. The reaction solution was stirred at 20 °C for another 30 minutes. Compound **5-2** (50 mg, 58.42 μmol) was added, and the reaction solution was stirred at this temperature for another 1 hour. The organic solvent was removed under reduced pressure. The resulting crude product was purified by preparative thin layer chromatography (developer: dichloromethane: methanol = 10:1) to give compound **5-3.** MS m/z =935.3 $[M+H]^+$.

**Step 4: Preparation of compound 5 hydrochloride**

Compound **5-3** (22 mg, 23.53 μmol) was dissolved in anhydrous dichloromethane (1.4 mL), and trifluoroacetic acid (0.7 mL) was added. The reaction solution was stirred at 20 °C for another 1 hour. The solvent was removed under reduced pressure. The resulting crude product was purified by high-performance liquid chromatography (column: Phenomenex Synergi C18 150*30mm*4μm; mobile phase: [water (0.05% hydrochloric acid)-acetonitrile]; (acetonitrile)%: 20%-50%, 9 minutes) to give compound **5** hydrochloride. $^{1}H$ NMR (400MHz, $CD_3OD$) δ: 6.86 - 6.84 (d, *J*=8.4 Hz, 1H), 5.69 - 5.51 (m, 1H), 5.27 - 5.25 (m, 1H), 5.09 - 5.04 (m, 2H), 5.00 - 4.94 (m, 4H), 4.81 - 4.78 (m, 1H), 3.97 - 3.88 (m, 3H), 3.72 - 3.68 (m, 1H), 3.53 - 3.38 (m, 5H), 3.05 - 3.01 (m, 1H), 2.66 - 2.63 (m, 2H), 2.52 - 2.45 (m, 1H), 2.42 - 2.15 (m, 10H). MS m/z: 595.1 $[M+H]^+$.

**Example 6**

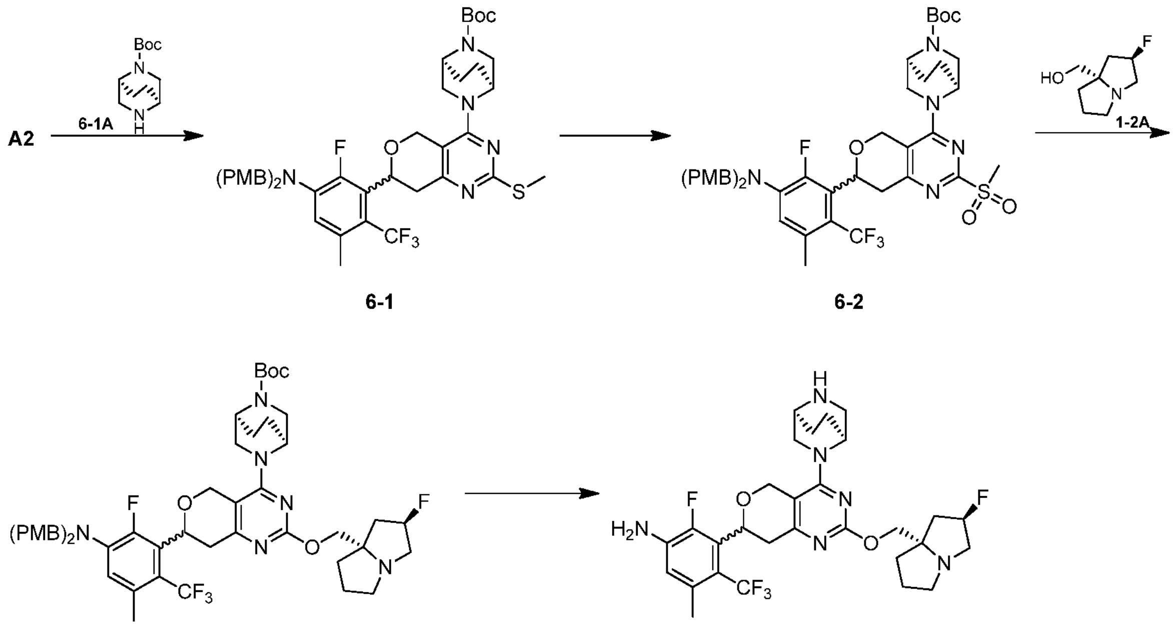


6-1

6-2

6-3

6

**Step 1: Preparation of intermediate 6-1**

Compound **A2** (80 mg, 105.02 μmol) and compound **6-1A** (22.30 mg, 105.02 μmol) were dissolved in N,N-dimethylformamide (1 mL), and diisopropylethylamine (40.72 mg, 315.07 μmol, 54.88 μL) was added. The reaction solution was heated to 100 °C and stirred for another 1 hour. The mixture was cooled down. The organic solvent was removed under reduced pressure. The resulting crude product was purified by preparative thin layer chromatography (developer: petroleum ether:ethyl acetate = 5:1) to give compound **6-1.** MS m/z =824.5 $[M+H]^+$.

**Step 2: Preparation of intermediate 6-2**

Compound **6-1** (70 mg, 84.96 μmol) was dissolved in dichloromethane (1.5 mL), and m-chloroperoxybenzoic acid (34.50 mg, 169.92 μmol, 85% content) was added. The reaction solution was stirred at 15 °C for another 6 hours. The organic solvent was removed under reduced pressure. The resulting crude product was purified by preparative thin layer chromatography (developer: petroleum ether:ethyl acetate = 1:1) to give compound **6-2.** MS m/z =856.4 $[M+H]^+$.

**Step 3: Preparation of intermediate 6-3**

Compound **1-2A** (12.09 mg, 75.94 μmol) was dissolved in anhydrous tetrahydrofuran (1 mL), and sodium tert-butoxide (7.30 mg, 75.94 μmol) was added. The reaction solution was stirred at 15 °C for another 30 minutes. A solution of compound **6-2** (50 mg, 58.42 μmol) in anhydrous tetrahydrofuran (0.2 mL) was added, and the reaction solution was

stirred at this temperature for another 1.5 hours. The organic solvent was removed under reduced pressure. The resulting crude product was purified by preparative thin layer chromatography (developer: petroleum ether:ethyl acetate = 1:1) to give compound **6-3.** MS m/z =935.6 $[M+H]^+$.

**Step 4: Preparation of compound 6 hydrochloride**

Compound **6-3** (36 mg, 38.50 μmol) was dissolved in anhydrous dichloromethane (1.0 mL), and trifluoroacetic acid (0.5 mL) was added. The reaction solution was stirred at 15 °C for another 2 hours. The solvent was removed under reduced pressure. The resulting crude product was purified by high-performance liquid chromatography (column: Phenomenex Synergi C18 150*30mm*4μm; mobile phase: [water (0.05% hydrochloric acid)-acetonitrile]; (acetonitrile)%: 15%-45%, 9 minutes) to give compound **6** hydrochloride. MS m/z: 595.6 $[M+H]^+$.

**Example 7**

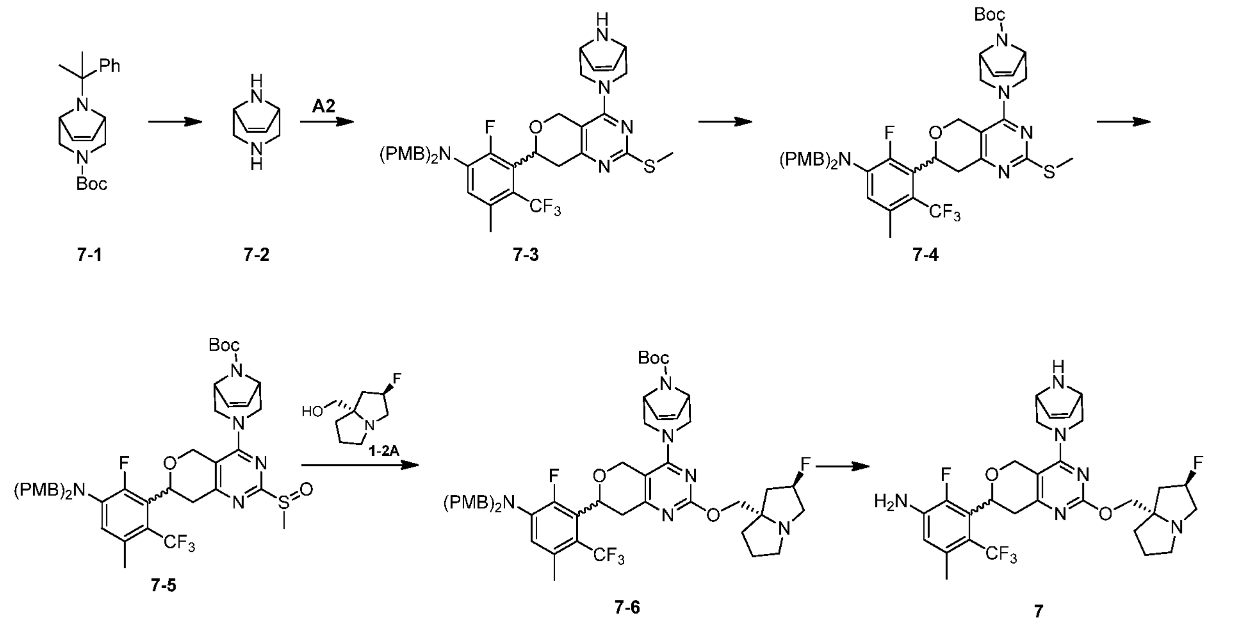


7-1 7-2 7-3 7-4

7-5 7-6 7

**Step 1: Preparation of intermediate 7-2**

Compound **7-1** (160 mg, 487.14 μmol) was dissolved in dichloromethane (2 mL) at 20 °C, and trifluoroacetic acid (2 mL) was added. The reaction solution was stirred at this temperature for another 18 hours. The organic solvent was removed under reduced pressure to give a crude product 7-2, which was directly used in the next step without further purification.

**Step 2: Preparation of intermediate 7-3**

Compound **A2** (350.00 mg, 459.48 μmol) and compound **7-2** (50.62 mg, 459.48 μmol) were dissolved in N,N-dimethylformamide (2 mL), and diisopropylethylamine (178.15 mg, 1.38 mmol, 240.10 μL) was added. The reaction solution was heated to 100 °C and stirred for another 1 hour. The mixture was cooled down to give a solution of compound **7-3,** which was directly used in the next step without further purification. MS m/z: 722.1 $[M+H]^+$.

**Step 3: Preparation of intermediate 7-4**

The solution of intermediate **7-3** obtained in step 2 was dissolved in dichloromethane (10 mL) at 20 °C, and diisopropylethylamine (177.26 mg, 1.37 mmol, 238.90 μL) and di-tert-butyl dicarbonate (149.67 mg, 685.78 μmol, 157.55 μL) were added. The reaction solution was stirred at this temperature for another 18 hours. The organic solvent was removed under reduced pressure. The resulting crude product was purified by silica gel column chromatography (eluent: ethyl acetate/petroleum ether=0~15%) to give compound **7-4.** $^1H$ NMR (400MHz, $CD_3OD$) δ: 7.17 - 7.14 (d, *J*=8.8 Hz,

4H), 6.86 - 6.84 (d, $J$=8.8 Hz, 4H), 6.64 - 6.62 (d, $J$=8.0 Hz, 1H), 6.26 - 6.20 (m, 2H), 5.20 - 5.16 (m, 1H), 4.73 - 4.61 (m, 4H), 4.35 - 4.29 (m, 4H), 3.81 (s, 6H), 3.35 - 2.81 (m, 6H), 2.51 (s, 3H), 2.35 (s, 3H), 1.52 (s, 9H). MS m/z: 822.3 $[M+H]^+$.

**Step 4: Preparation of intermediate 7-5**

Compound **7-4** (50.06 mg, 60.91 μmol) was dissolved in methanol (5 mL) at 20 °C, and potassium hydrogenperoxomonosulphate (37.44 mg, 60.91 μmol) was added. The reaction solution was stirred at this temperature for another 1 hour. The organic solvent was removed under reduced pressure. The resulting crude product was purified by preparative thin layer chromatography (developer: petroleum ether:ethyl acetate = 1:1) to give compound **7-5.** MS m/z: 838.3 $[M+H]^+$.

**Step 5: Preparation of intermediate 7-6**

Compound **1-2A** (7.42 mg, 46.60 μmol) was dissolved in anhydrous tetrahydrofuran (1 mL) at 20 °C, and sodium tert-butoxide (4.48 mg, 46.60 μmol) was added. The reaction solution was stirred at this temperature for another 30 minutes. Compound **7-5** (30.04 mg, 35.85 μmol) was added, and the reaction solution was stirred at this temperature for another 1 hour. The organic solvent was removed under reduced pressure. The resulting crude product was purified by preparative thin layer chromatography (developer: dichloromethane: methanol = 10:1) to give compound **7-6.** MS m/z =933.5 $[M+H]^+$.

**Step 6: Preparation of compound 7 hydrochloride**

Compound **7-6** (25 mg, 26.79 μmol) was dissolved in anhydrous dichloromethane (1.0 mL) at 20 °C, and trifluoroacetic acid (1 mL) was added. The reaction solution was stirred at this temperature for another 1 hour. The solvent was removed under reduced pressure. The resulting crude product was purified by high-performance liquid chromatography (column: Xtimate C18 150*40mm*5μm; mobile phase: [water (0.05% hydrochloric acid)-acetonitrile]; (acetonitrile)%: 1%-30%, 10 minutes) to give compound **7** hydrochloride. MS m/z: 593.5 $[M+H]^+$.

**Example 8**

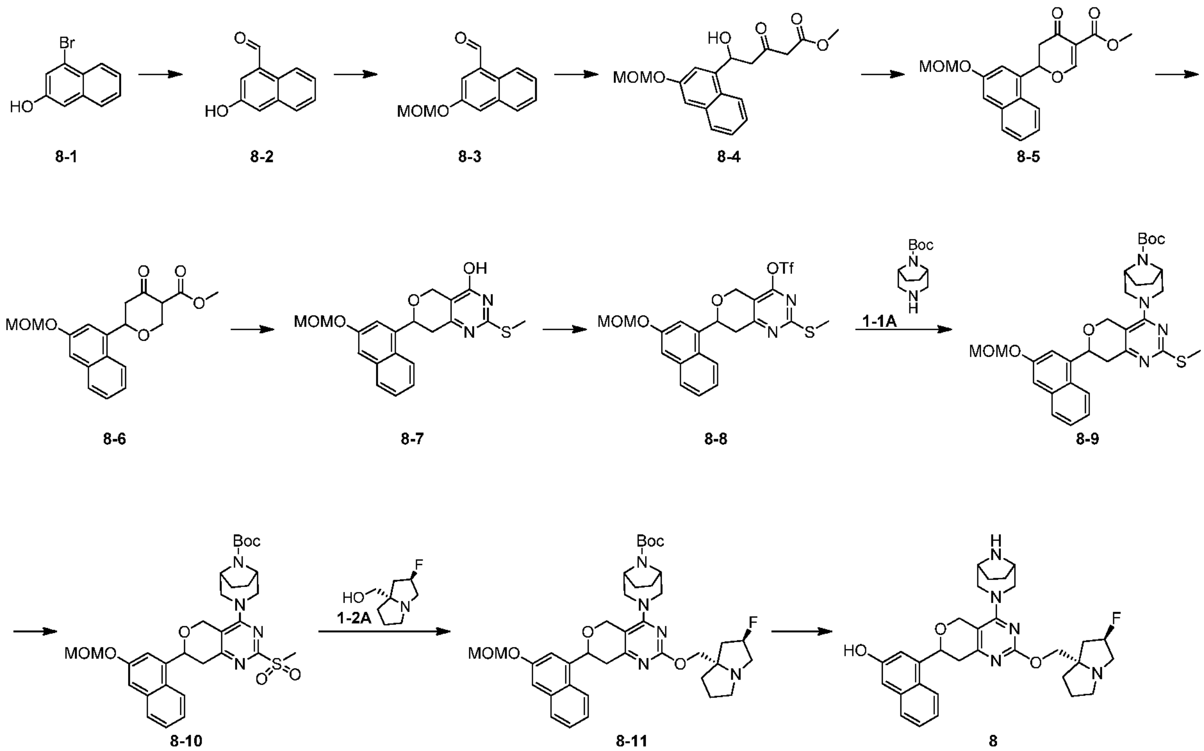

8-1 8-2 8-3 8-4 8-5
8-6 8-7 8-8 8-9
8-10 8-11 8

**Step 1: Preparation of intermediate 8-2**

Sodium hydride (2.33 g, 58.28 mmol, 60% content) was suspended in anhydrous tetrahydrofuran (120 mL) under the condition of an ice-water bath, and compound **8-1** (10 g, 44.83 mmol) was added. The reaction solution was stirred at this temperature for another 1 hour, and then cooled down to -78 °C. n-Butyl lithium (2.5 M, 30.48 mL) was added dropwise, and the reaction solution was stirred for another 1 hour. N,N-dimethylformamide (16.38 g, 224.15 mmol, 17.25 mL) was added finally. The resulting reaction solution was stirred for 0.5 hours. The reaction was quenched with 2 M aqueous hydrochloric acid solution (10 mL). Then the mixture was extracted with ethyl acetate (50 mL*3). The organic phases were combined. The organic solvent was removed under reduced pressure. The resulting crude product was purified by silica gel column chromatography (eluent: ethyl acetate/petroleum ether=0~30%) to give compound **8-2.** $^1$H NMR (400MHz, $CDCl_3$) δ: 10.42 (s, 1H), 9.10 - 9.06 (m, 1H), 7.76 - 7.75 (m, 1H), 7.67 - 7.66 (m, 1H), 7.59 - 7.57 (m, 2H), 7.46 - 7.45 (m, 1H), 5.64 (brs,1H).

**Step 2: Preparation of intermediate 8-3**

Compound **8-2** (3.0 g, 17.42 mmol) was dissolved in anhydrous dichloromethane (50 mL) under the condition of an ice-water bath, and diisopropylethylamine (6.76 g, 52.27 mmol, 9.10 mL) and chloromethyl methyl ether (2.10 g, 26.14 mmol, 1.99 mL) were added. The resulting reaction solution was stirred for another 2 hours. The organic solvent was removed under reduced pressure. The residue was dissolved in ethyl acetate (100 mL). The mixture was washed with water (10 mL*3) and saturated brine (10 mL). The organic solvent was removed under reduced pressure. The resulting crude product was purified by silica gel column chromatography (eluent: ethyl acetate/petroleum ether=0~10%) to give compound **8-3.** $^1$H NMR (400MHz, $CDCl_3$) δ: 10.40 (s, 1H), 9.14 - 9.11 (m, 1H), 7.82 - 7.80 (m, 1H), 7.78 - 7.77 (m, 1H), 7.69 - 7.67 (m, 1H), 7.57 - 7.55 (m, 2H), 5.36 (s, 2H), 3.55 (s, 3H).

**Step 3: Preparation of intermediate 8-4**

Sodium hydride (414.37 mg, 10.36 mmol, 60% content) was suspended in anhydrous tetrahydrofuran (8 mL) under nitrogen, and the mixture was cooled down to 0 °C. Methyl acetoacetate (1.20 g, 10.36 mmol, 1.11 mL) was added, and the reaction solution was stirred for another 30 minutes. n-Butyl lithium (2.5 M, 4.14 mL) was added dropwise, and the mixture was reacted with stirring for another 30 minutes. The reaction solution was cooled down to - 78 °C, and a solution of compound **8-3** (1.12 g, 5.18 mmol) in anhydrous tetrahydrofuran (2 mL) was added dropwise. The reaction solution was stirred at this temperature for another 1 hour. The reaction solution was quenched with water (20 mL). The mixture was extracted with ethyl acetate (80 mL *3). The combined organic phase was dried over anhydrous sodium sulfate. The organic solvent was removed under reduced pressure. The resulting crude product was purified by silica gel column chromatography (eluent: ethyl acetate/petroleum ether=0~75%) to give compound **8-4.** $^1$H NMR (400MHz, $CDCl_3$) δ: 7.90 - 7.88 (m, 1H), 7.79 - 7.77 (m, 1H), 7.49 - 7.35 (m, 4H), 5.98 - 5.95 (m, 1H), 5.32 (s, 2H), 3.78 (s, 3H), 3.56 (s, 2H), 3.53 (s, 3H), 3.15 - 3.01 (m, 3H). MS m/z: 350.2 $[M+H_2O]^+$.

**Step 4: Preparation of intermediate 8-5**

Compound **8-4** (2.43 g, 7.31 mmol) was dissolved in dichloromethane (15 mL) at 18 °C, and N,N-dimethylformamide dimethyl acetal (871.27 mg, 7.31 mmol, 971.31 μL) was added. The reaction solution was stirred at this temperature for another 2 hours, and then the reaction solution was cooled down to 0 °C. Boron trifluoride etherate (1.04 g, 7.31 mmol, 902.37 μL) was added, and the reaction solution was stirred for another 1 hour. The organic solvent was removed under reduced pressure. To the resulting crude product was added ethyl acetate (100 mL). The mixture was washed with water (20 mL), saturated aqueous sodium bicarbonate solution (20 mL) and saturated brine (10 mL). The organic solvent was removed under reduced pressure. The resulting crude product was purified by silica gel column chromatography (eluent: ethyl acetate/petroleum ether = 0-35%) to give compound **8-5.** $^1$H NMR (400MHz, $CDCl_3$) δ: 8.54 (s, 1H), 7.79 - 7.77 (m, 2H), 7.49 - 7.35 (m, 4H), 6.30 - 6.26 (m, 1H), 5.32 (s, 2H), 3.87 (s, 3H), 3.56 (s, 3H), 3.15 - 3.01 (m, 2H). MS m/z: 343.2 $[M+H]^+$.

**Step 5: Preparation of intermediate 8-6**

Compound **8-5** (1.42 g, 4.15 mmol) was dissolved in anhydrous tetrahydrofuran (15 mL) under nitrogen, and the reaction solution was cooled down to -78 °C. Tri-sec-butyl lithium borohydride (1 M, 4.15 mL) was added dropwise, and the reaction solution was stirred at this temperature for another 1 hour. The reaction solution was quenched with water (1 mL). The mixture was diluted with ethyl acetate (80 mL). The organic phase was washed with water (20 mL) and saturated brine (20 mL), and dried over anhydrous sodium sulfate. The organic solvent was removed under reduced

pressure. The resulting crude product was purified by silica gel column chromatography (eluent: ethyl acetate/petroleum ether=0~15%) to give compound **8-6.** MS m/z: 367.1 $[M+Na]^+$.

**Step 6: Preparation of intermediate 8-7**

Compound **8-6** (1.14 g, 3.31 mmol) and 2-methylthiourea (1.87 g, 9.93 mmol) were added to ethanol (20 mL) under nitrogen, and sodium carbonate (1.05 g, 9.93 mmol) was added. The reaction solution was heated to 60 °C and stirred for another 15 hours. The organic solvent was removed under reduced pressure. Water (15 mL) and ethyl acetate (100 mL) were added to the residue. The mixture was adjusted to a pH of 5-6 with 6M aqueous hydrochloric acid solution. The layers were separated. The organic phase was washed with saturated brine (20 mL). The organic solvent was removed under reduced pressure to give crude compound **8-7,** which was directly used in the next step without further purification. MS m/z: 385.1 $[M+H]^+$.

**Step 7: Preparation of intermediate 8-8**

Compound **8-7** (1.34 g, 3.49 mmol) was dissolved in N,N-dimethylformamide (20 mL) at 16 °C, and N,N-diisopropylethylamine (1.35 g, 10.47 mmol, 1.82 μL) and N-phenylbis(trifluoromethanesulfonimide) (1.87 g, 5.24 mmol) were added. The reaction solution was stirred at this temperature for another 3 hours. The reaction solution was diluted with ethyl acetate (100 mL). The mixture was washed with water (20 mL *2) and saturated brine (20 mL), dried over anhydrous sodium sulfate, and filtered. The organic solvent was removed under reduced pressure. The resulting crude product was purified by silica gel column chromatography (eluent: ethyl acetate/petroleum ether=0~10%) to give compound **8-8.** $^1H$ NMR (400MHz, $CDCl_3$) δ: 7.92 - 7.90 (m, 1H), 7.82 - 7.80 (m, 1H), 7.49 - 7.46 (m, 1H), 7.43 - 7.35 (m, 3H), 5.50 - 5.47 (m,1H), 5.32 (s, 2H), 5.06 - 5.02 (m, 1H), 4.94 - 4.90 (m, 1H), 3.54 (s, 3H), 3.40 - 3.22 (m, 2H), 2.55 (s, 3H). MS m/z: 517.0 $[M+H]^+$.

**Step 8: Preparation of intermediate 8-9**

Compound **8-8** (300 mg, 580.82 μmol) and compound **1-1A** (160.29 mg, 755.07 μmol) were dissolved in N,N-dimethylformamide (3 mL), and diisopropylethylamine (225.20 mg, 1.74 mmol, 303.51 μL) was added. The reaction solution was heated to 100 °C and stirred for another 1 hour. The mixture was cooled down. The organic solvent was removed under reduced pressure. The resulting crude product was purified by silica gel column chromatography (eluent: ethyl acetate/petroleum ether=0~25%) to give compound **8-9.** $^1H$ NMR (400MHz, $CDCl_3$) δ: 7.98 - 7.96 (m, 1H), 7.80 - 7.78 (m, 1H), 7.49 - 7.36 (m, 4H), 5.50 - 5.47 (m, 1H), 5.31 (s, 2H), 4.92 - 4.89 (m, 1H), 4.78 - 4.75 (m, 1H), 4.36 - 4.30 (m, 2H), 3.97 - 3.78 (m, 1H), 3.58 - 3.30 (m, 6H), 3.19 - 3.12 (m, 2H), 2.53 (s, 3H), 1.78 - 1.46 (m, 13H). MS m/z: 579.8 $[M+H]^+$.

**Step 9: Preparation of intermediate 8-10**

Compound **8-9** (270 mg, 466.55 μmol) was dissolved in dichloromethane (2.5 mL) at 15 °C, and m-chloroperoxybenzoic acid (189.44 mg, 933.09 μmol, 85% content) was added. The reaction solution was stirred at this temperature for another 18 hours. The organic solvent was removed under reduced pressure. The resulting crude product was purified by silica gel column chromatography (eluent: ethyl acetate/petroleum ether=0~60%) to give compound **8-10.** MS m/z = 611.2 $[M+H]^+$.

**Step 10: Preparation of intermediate 8-11**

Compound **1-2A** (125.13 mg, 785.96 μmol) was dissolved in anhydrous tetrahydrofuran (2 mL) at 15 °C, and sodium tert-butoxide (75.53 mg, 785.96 μmol) was added. The reaction solution was stirred at this temperature for another 1 hour. Compound **8-10** (240 mg, 392.98 μmol) was added. The reaction solution was stirred at this temperature for another 0.5 hours. The organic solvent was removed under reduced pressure. The resulting crude product was purified by silica gel column chromatography (eluent: methanol/dichloromethane=0~4%) to give compound **8-11.** MS m/z =690.3 $[M+H]^+$.

**Step 11: Preparation of compound 8 hydrochloride**

Compound **8-11** (53 mg, 76.83 μmol) was dissolved in a solution of hydrogen chloride in dioxane (2 mL, 4 M) at 18 °C. The reaction solution was stirred at this temperature for another 30 minutes. The solvent was removed under reduced pressure. The resulting crude product was purified by high-performance liquid chromatography (column: Phenomenex Synergi C18 150*30mm*4μm; mobile phase: [water (0.05% hydrochloric acid)-acetonitrile]; (acetonitrile)%: 9%-39%, 9 minutes) to give compound **8** hydrochloride. $^1$H NMR (400MHz, $D_2O$) δ: 7.95 - 7.93 (m, 1H), 7.76 - 7.74 (m, 1H), 7.48 - 7.36 (m, 2H), 7.22 - 7.20 (m, 2H), 5.95 - 5.47 (m, 2H), 4.96 - 4.93 (m, 1H), 4.66 - 4.53 (m, 4H), 4.20 - 4.17 (m, 2H), 3.87 - 3.68 (m, 6H), 3.48 - 3.38 (m, 2H), 3.17 - 3.15 (m, 2H), 2.60 - 2.37 (m, 2H), 2.28 - 2.23 (m, 3H), 2.08 - 2.03 (m, 4H), 1.88 - 1.85 (m, 1H). MS m/z =546.3 $[M+H]^+$.

**Example 9**

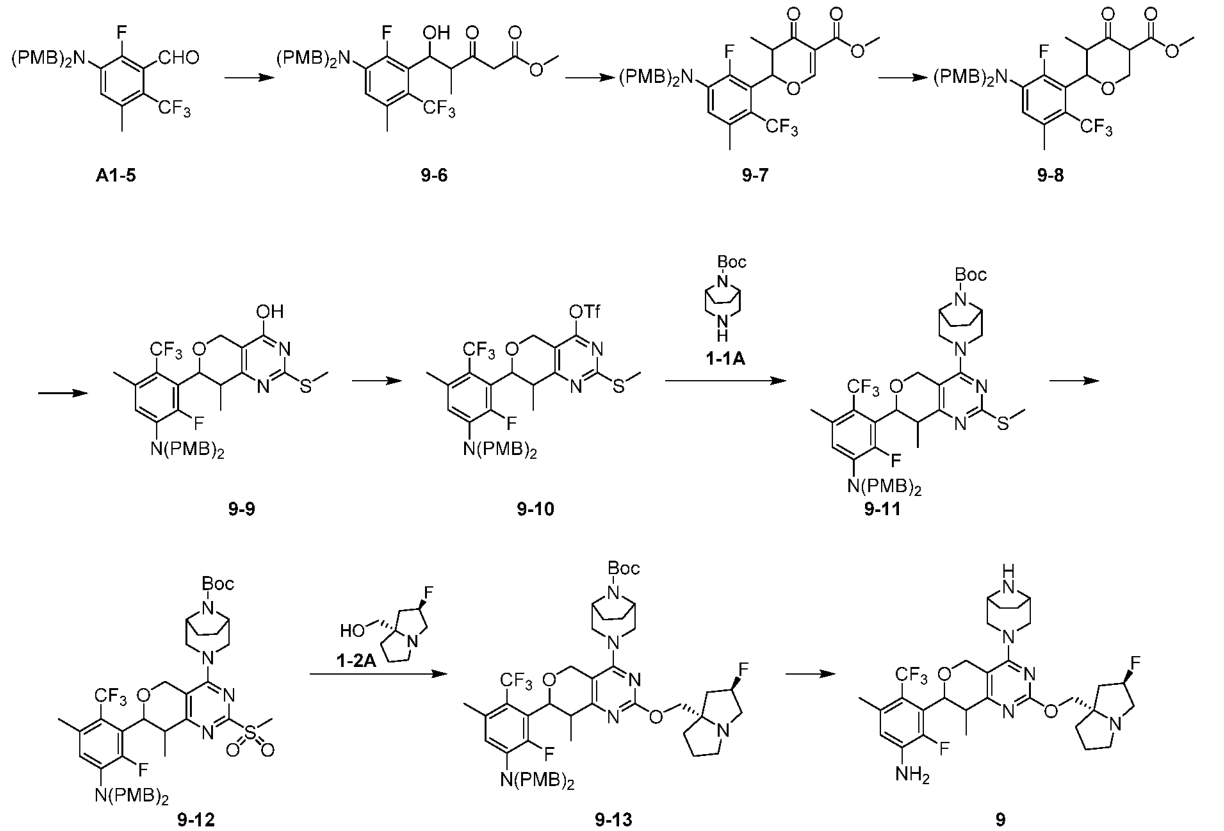


A1-5 9-6 9-7 9-8

9-9 9-10 9-11

9-12 9-13 9

**Step 1: Preparation of intermediate 9-6**

Sodium hydride (346.70 mg, 8.67 mmol, 60% content) was suspended in anhydrous tetrahydrofuran (10 mL) under nitrogen, and the mixture was cooled down to 0 °C. Methyl propionoylacetate (1.13 g, 8.67 mmol, 1.07 mL) was added, and the reaction solution was stirred for another 30 minutes. n-Butyl lithium (2.5 M, 3.47 mL) was added dropwise, and the mixture was reacted with stirring for another 30 minutes. The reaction solution was cooled down to -78 °C, and a soluiton of compound **A1-5** (2.0 g, 4.33 mmol) in anhydrous tetrahydrofuran (10 mL) was added dropwise. The reaction solution was stirred at this temperature for another 1.5 hours. The reaction solution was quenched with 0.5 M aqueous hydrochloric acid solution (20 mL). The layers were separated. The aqueous phase was extracted with ethyl acetate (50 mL * 2). The combined organic phase was washed with saturated brine (20 mL), and dried over anhydrous sodium sulfate. The organic solvent was removed under reduced pressure. The resulting crude product was purified by silica gel column chromatography (eluent: ethyl acetate/petroleum ether=0~35%) to give compound **9-6.** MS m/z: 614.5 $[M+Na]^+$.

**Step 2: Preparation of intermediate 9-7**

Compound **9-6** (2.0 g, 3.38 mmol) was dissolved in dichloromethane (10 mL) at 20 °C, and N,N-dimethylformamide dimethyl acetal (1.21 g, 10.14 mmol, 1.35 mL) was added. The reaction solution was stirred at this temperature for another 18 hours. The organic solvent was removed under reduced pressure. The resulting crude product was purified by silica gel column chromatography (eluent: ethyl acetate/petroleum ether=0~35%) to give compound **9-7.** MS m/z:

602.2 [M+H]+.

**Step 3: Preparation of intermediate 9-8**

Compound **9-7** (750 mg, 1.25 mmol) was dissolved in anhydrous tetrahydrofuran (20 mL) under nitrogen. The reaction solution was cooled down to -78 °C, and tri-sec-butyl lithium borohydride (1 M, 1.25 mL) was added dropwise. The reaction solution was stirred at this temperature for another 1 hour. The reaction solution was quenched with 0.5 M aqueous hydrochloric acid solution (5 mL). The mixture was extracted with ethyl acetate (100 mL). The organic phase was washed with saturated brine (20 mL), and dried over anhydrous sodium sulfate. The organic solvent was removed under reduced pressure. The resulting crude product was purified by silica gel column chromatography (eluent: ethyl acetate/petroleum ether=0~20%) to give compound **9-8.** MS m/z: 604.2 [M+H]+.

**Step 4: Preparation of intermediate 9-9**

Compound **9-8** (750 mg, 1.24 mmol) and 2-methylthiourea (701.63 mg, 3.73 mmol) were added to ethanol (10 mL) under nitrogen, and sodium carbonate (263.39 mg, 2.49 mmol) was added. The reaction solution was heated to 60 °C and stirred for another 15 hours. The organic solvent was removed under reduced pressure. Water (10 mL) was added to the residue. The mixture was adjusted to a pH of 5-6 with 2M aqueous hydrochloric acid solution, and extracted with ethyl acetate (30 mL*3). The combined organic phase was washed with saturated brine (10 mL). The organic solvent was removed under reduced pressure to give crude compound **9-9,** which was directly used in the next step without further purification. MS m/z: 666.4 [M+Na]+.

**Step 5: Preparation of intermediate 9-10**

Compound **9-9** (855 mg, 1.33 mmol) was dissolved in N,N-dimethylformamide (10 mL) at 20 °C, and N,N-diisopropylethylamine (515.68 mg, 3.99 mmol, 694.99 μL) and N-phenylbis(trifluoromethanesulfonimide) (570.17 mg, 1.60 mmol) were added. The reaction solution was stirred at this temperature for another 3 hours. The reaction solution was diluted with ethyl acetate (50 mL). The mixture was washed with water (15 mL *4), dried over anhydrous sodium sulfate, and filtered. The organic solvent was removed under reduced pressure. The resulting crude product was purified by silica gel column chromatography (eluent: ethyl acetate/petroleum ether=0~15%) to give compound **9-10.** MS m/z: 776.1 [M+H]+.

**Step 6: Preparation of intermediate 9-11**

Compound **9-10** (240 mg, 309.38 μmol) and compound **1-1A** (78.81 mg, 371.25 μmol) were dissolved in N,N-dimethylformamide (2 mL), and diisopropylethylamine (119.95 mg, 928.13 μmol, 161.66 μL) was added. The reaction solution was heated to 100 °C and stirred for another 1 hour. The mixture was cooled down. The organic solvent was removed under reduced pressure. The resulting crude product was purified by silica gel column chromatography (eluent: ethyl acetate/petroleum ether=0~45%) to give compound **9-11.** MS m/z: 838.5 [M+H]+.

**Step 7: Preparation of intermediate 9-12**

Compound **9-11** (260 mg, 310.28 μmol) was dissolved in dichloromethane (2 mL) at 20 °C, and m-chloroperoxybenzoic acid (3 125.98 mg, 620.55 μmol, 85% content) was added. The reaction solution was stirred at this temperature for another 15 hours. The organic solvent was removed under reduced pressure. The resulting crude product was purified by silica gel column chromatography (eluent: ethyl acetate/petroleum ether=0~25%) to give compound **9-12.** MS m/z =870.3 [M+H]+.

**Step 8: Preparation of intermediate 9-13**

Compound **1-2A** (101.56 mg, 637.96 μmol) was dissolved in anhydrous tetrahydrofuran (2 mL) at 20 °C, and sodium tert-butoxide (40.87 mg, 425.31 μmol) was added. The reaction solution was stirred at this temperature for another 1 hour, and compound **9-12** (185 mg, 212.65 μmol) was added. The reaction solution was stirred at this temperature for another 1 hour. The organic solvent was removed under reduced pressure. The resulting crude product was purified by silica gel column chromatography (eluent: ethyl acetate/petroleum ether=0~65%) to give compound **9-13.** MS m/z =949.3 [M+H]+.

**Step 9: Preparation of compound 9**

Compound **9-13** (151 mg, 159.11 μmol) was dissolved in trifluoroacetic acid (1.2 mL) at 20 °C. The reaction solution was stirred at this temperature for another 1 hour. The solvent was removed under reduced pressure. The resulting crude product was purified by high-performance liquid chromatography (column: Phenomenex C18 80*40mm*3μm; mobile phase: [water (0.5% ammonia water)-acetonitrile]; (acetonitrile)%: 47%-77%, 8 minutes) to give compound **9.** $^1$H NMR (400MHz, CDsOD) δ: 6.74 - 6.72 (d, *J*=8.8 Hz, 1H), 5.38 - 5.24 (m, 1H), 4.64 - 4.60 (m, 2H), 4.20 - 4.10 (m, 2H), 3.59 - 3.37 (m,6H), 3.26 - 3.03 (m, 5H), 2.40 - 2.37 (m, 3H), 2.28 - 1.68 (m, 11H), 1.21 - 1.17 (m, 3H). MS m/z =609.3 [M+H]$^+$.

**Example 10**

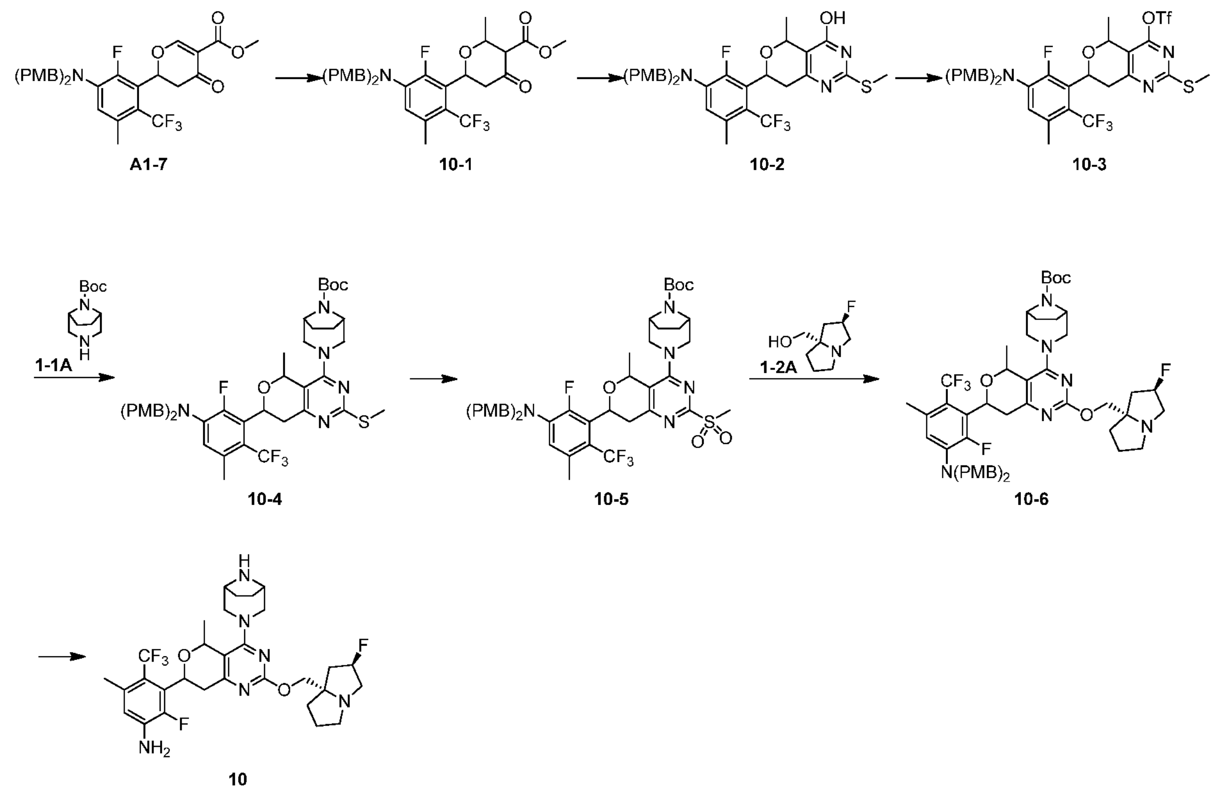


**Step 1: Preparation of intermediate 10-1**

Compound **A1-7** (518 mg, 881.62 μmol) was dissolved in anhydrous tetrahydrofuran (2 mL) under nitrogen. The reaction solution was cooled down to -78 °C, and dimethyl copper lithium (0.5 M, 5.29 mL) was added dropwise. The reaction solution was stirred at this temperature for another 0.5 hours. The reaction solution was added to water (10 mL) and ethyl acetate (50 mL). The mixture was filtered. The layers were separated. The aqueous phase was extracted with ethyl acetate (20 mL*3). The organic phases were combined. The organic solvent was removed under reduced pressure. The resulting crude product was purified by silica gel column chromatography (eluent: ethyl acetate/petroleum ether=0~20%) to give compound **10-1.** $^1$H NMR (400MHz, $CDCl_3$) δ: 7.17 - 7.14 (m, 4H), 6.87 - 6.83 (m, 4H), 6.63 - 6.61 (d, *J* = 7.2 Hz, 1H), 5.42 - 5.39 (m, 1H), 4.86 - 4.84 (m, 1H), 4.38 - 4.24 (m, 5H), 3.80 - 3.73 (m, 9H), 3.13 - 3.05 (m, 1H), 2.41 - 2.38 (m, 4H), 1.48 - 1.37 (m, 3H). MS m/z: 604.2 [M+H]$^+$.

**Step 2: Preparation of intermediate 10-2**

Compound **10-1** (488 mg, 808.48 μmol) and 2-methylthiourea (456.53 mg, 2.43 mmol) were added to ethanol (5 mL) under nitrogen, and sodium carbonate (171.38 mg, 1.62 mmol) was added. The reaction solution was heated to 60 °C and stirred for another 32 hours. The organic solvent was removed under reduced pressure. Water (20 mL) was added to the residue. The mixture was adjusted to a pH of 5-6 with 2M aqueous hydrochloric acid solution, and extracted

with ethyl acetate (100 mL*3). The combined organic phase was dried over anhydrous sodium sulfate. The organic solvent was removed under reduced pressure to give crude compound **10-2,** which was directly used in the next step without further purification. MS m/z: 644.3 $[M+H]^+$.

**Step 3: Preparation of intermediate 10-3**

Compound **10-2** (502 mg, 779.88 μmol) was dissolved in N,N-dimethylformamide (5 mL) at 20 °C, and N,N-diisopropylethylamine (302.38 mg, 2.34 mmol, 407.52 μL) and N-phenylbis(trifluoromethanesulfonimide) (417.92 mg, 1.17 mmol) were added. The reaction solution was stirred at this temperature for another 2 hours. The organic solvent was removed under reduced pressure. The resulting crude product was purified by silica gel column chromatography (eluent: ethyl acetate/petroleum ether=0~20%) to give compound **10-3.** MS m/z: 776.1 $[M+H]^+$.

**Step 4: Preparation of intermediate 10-4**

Compound **10-3** (185 mg, 238.48 μmol) and compound **1-1A** (65.81 mg, 310.02 μmol) were dissolved in N,N-dimethylformamide (1.5 mL), and diisopropylethylamine (92.46 mg, 715.43 μmol, 124.62 μL) was added. The reaction solution was heated to 100 °C and stirred for another 1 hour. The mixture was cooled down. The organic solvent was removed under reduced pressure. The resulting crude product was purified by silica gel column chromatography (eluent: ethyl acetate/petroleum ether=0~30%) to give compound **10-4.** MS m/z: 838.8 $[M+H]^+$.

**Step 5: Preparation of intermediate 10-5**

Compound **10-4** (105.00 mg, 125.30 μmol) was dissolved in dichloromethane (2 mL) at 20 °C, and m-chloroperoxybenzoic acid (50.88 mg, 250.61 μmol, 85% content) was added. The reaction solution was stirred at this temperature for another 1.5 hours. The organic solvent was removed under reduced pressure. The resulting crude product was purified by silica gel column chromatography (eluent: ethyl acetate/petroleum ether=0~35%) to give compound **10-5.** MS m/z =870.3 $[M+H]^+$.

**Step 6: Preparation of intermediate 10-6**

Compound **1-2A** (47.21 mg, 296.56 μmol) was dissolved in anhydrous tetrahydrofuran (1 mL) at 20 °C, and sodium tert-butoxide (19.00 mg, 197.71 μmol) was added. The reaction solution was stirred at this temperature for another 1 hour, and compound **10-5** (86.00 mg, 98.85) was added. The reaction solution was stirred at this temperature for another 1 hour. The organic solvent was removed under reduced pressure. To the resulting crude product were added saturated brine (1 mL) and ethyl acetate (5 mL). The layers were separated. The organic solvent was removed under reduced pressure. The crude product was purified by silica gel column chromatography (eluent: methanol/dichloromethane=0~4%) to give compound **10-6.** MS m/z =949.5 $[M+H]^+$.

**Step 7: Preparation of compound 10 formate**

Compound **10-6** (75.00 mg, 79.03 μmol) was dissolved in trifluoroacetic acid (1.5 mL) at 20 °C. The reaction solution was stirred at this temperature for another 30 minutes. The solvent was removed under reduced pressure. The resulting crude product was purified by high-performance liquid chromatography (column: Phenomenex C18 150*40mm*5μm; mobile phase: [water (0.025% formic acid)-acetonitrile]; (acetonitrile)%: 5%-35%, 10 minutes) to give compound **10** formate. $^1H$ NMR (400MHz, CDsOD) δ: 8.51 (s, 1H), 6.72 - 6.70 (d, *J*=8.4 Hz, 1H), 5.51 - 5.31 (m, 3H), 4.36 - 4.19 (m, 4H), 4.10 - 4.07 (m, 2H), 3.71 - 3.40 (m, 4H), 3.21-3.18 (m, 2H), 2.82 - 2.70 (m, 1H), 2.51 - 1.98 (m, 15H), 1.51 - 1.47 (m, 3H). MS m/z =609.6 $[M+H]^+$.

**Example 11**

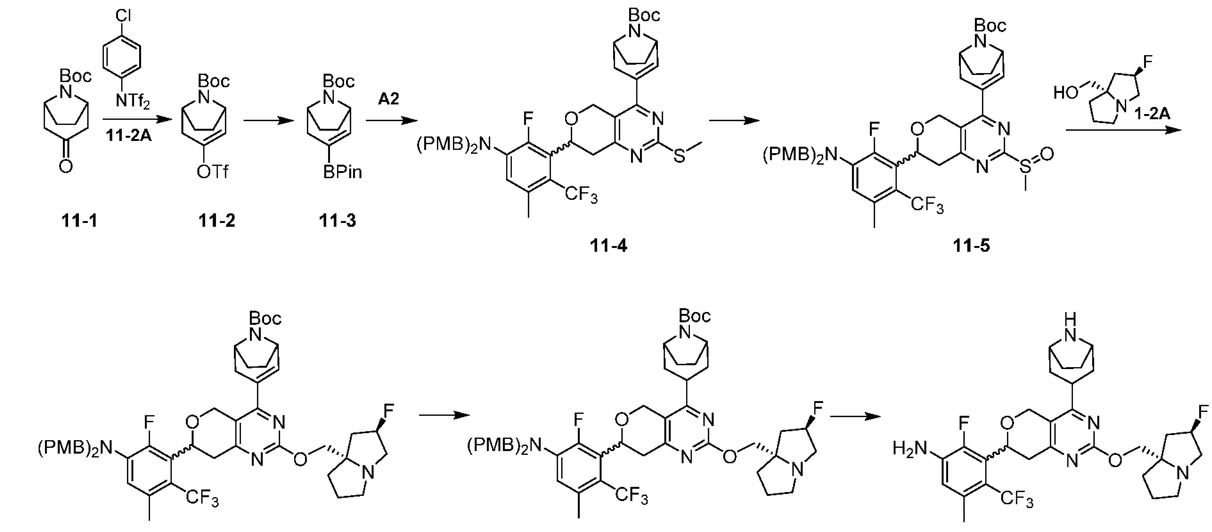


11-1 11-2 11-3 11-4 11-5

11-6 11-7 11

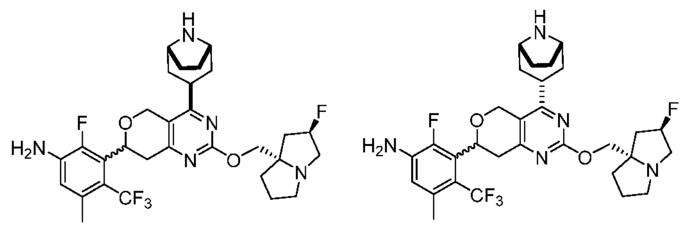

11A or 11B 11B or 11A

**Step 1: Preparation of intermediate 11-2**

**11-1** (10 g, 44.39 mmol, 1 eq) was dissolved in THF (100 mL). LDA (2 M, 24.41 mL, 1.1 eq) was added dropwise at -78 °C. After completion of the addition, the mixture was stirred for another 0.5 hr, and then a solution of **11-2A** (18.30 g, 46.61 mmol, 1.05 eq) in THF (50 mL) was added dropwise. The mixture was stirred for 0.5 hr, and then stirred at room temperature for 0.5 hr. The reaction was quenched by adding saturated ammonium chloride solution. The mixture was extracted with ethyl acetate (500mL*2). After extraction, the organic phases were combined, washed with 1L of saturated brine, dried over anhydrous sodium sulfate, and filtered. The filtrate was rotary evaporated to dryness. The residue was separated by column chromatography (eluent: 10% ethyl acetate/petroleum ether) to give **11-2.**

**Step 2: Preparation of intermediate 11-3**

**11-2** (15.2 g, 42.54 mmol, 1 eq), $B_2Pin_2$ (12.96 g, 51.04 mmol, 1.2 eq), $Pd(dppf)Cl_2 \cdot CH_2Cl_2$ (3.47 g, 4.25 mmol, 0.1 eq) and KOAc (12.52 g, 127.61 mmol, 3 eq) were dissolved in 1,4-dioxane (130 mL). The mixture was reacted at 90 °C for 16 hr under nitrogen. The mixture was cooled down to room temperature, filtered through a pad of celite, and separated by column chromatography (eluent: 10% ethyl acetate/petroleum ether) to give **11-3.**

**Step 3: Preparation of intermediate 11-4**

To a solution mixture of **A2** (3.3 g, 4.33 mmol) and **11-3** (2.18 g, 6.50 mmol) in 1,4-dioxane (30mL) and water (1 mL) were added sodium carbonate (1.38 g, 13.00 mmol) and 1,1-bis(diphenylphosphino)ferrocenepalladium chloride (530.68 mg, 649.84μmol). The atmosphere was replaced with nitrogen three times. The system was stirred and heated at 90 °C for 12 hours. The mixture was filtered. The filtrate was rotary evaporated to dryness. The residue was separated by column chromatography (eluent: 10-20% ethyl acetate/petroleum ether) to give **11-4.** MS m/z: 821.4 $[M+H]^+$.

**Step 4: Preparation of intermediate 11-5**

To a solution of **11-4** (530 mg, 645.61 μmol) in dichloromethane (50 mL) was added m-chloroperoxybenzoic acid (131.07 mg, 645.61 μmol), and the system was stirred at 20 °C for 0.5 hours. The reaction solution was washed

sequentially with 30 mL of saturated sodium bicarbonate solution and 30 mL of saturated brine, dried over anhydrous sodium sulfate, and filtered. The filtrate was rotary evaporated to dryness. The residue was separated by column chromatography (eluent: 20-60% ethyl acetate/petroleum ether) to give **11-5.** MS m/z: 859.3 $[M+Na]^+$.

**Step 5: Preparation of intermediate 11-6**

To a solution of **1-2A** (164.35 mg, 1.03 mmol) in tetrahydrofuran (15 mL) was added sodium tert-butoxide (99.21 mg, 1.03 mmol) at 20 °C. The system was stirred at this temperature for 0.5 hours, and then **11-5** (720.00 mg, 860.29 μmol) was added. The mixture was stirred for another 0.5 hours. The reaction solution was diluted with 80 mL of ethyl acetate. The mixture was washed with 30 mL of saturated brine. The organic phase was dried over anhydrous sodium sulfate, and filtered. The filtrate was rotary evaporated to dryness. The residue was separated by column chromatography (eluent: 0-5% methanol/dichloromethane) to give **11-6.** MS m/z: 932.4 $[M+H]^+$.

**Step 6: Preparation of intermediate 11-7**

Palladium hydroxide (467.12 mg, 3.33 mmol) was added to a solution of compound **11-6** (620 mg, 665.22 μmol) in ethanol (40 mL). The system was reacted at 50 °C for 15 hours under a hydrogen pressure of 50 psi, and filtered. The filtrate was rotary evaporated to dryness to give **11-7.** MS m/z: 934.4 $[M+H]^+$.

**Step 7: Preparation of compound 11A hydrochloride and compound 11B hydrochloride**

To a soluton of **11-7** (500 mg, 535.31 μmol, 1 eq) in dichloromethane (5 mL) was added trifluoroacetic acid (5 mL). The system was stirred at 20 °C for 1 hours. The reaction solution was rotary evaporated to dryness. The rusidue was separated by preparative HPLC (column: Xtimate C18 150*40mm*5μm; mobile phase: [water (0.05% hydrochloric acid)-acetonitrile]; (acetonitrile)%: 10%-30%, 10min) to give compound **11A** hydrochloride and compound **11B** hydrochloride, MS m/z =594.1 $[M+H]^+$. **11A** hydrochloride: $^1H$ NMR (400MHz, CDsOD) δ = 7.02 (d, *J* =8.0 Hz, 1H), 5.71 - 5.52 (m, 1H), 5.26 - 5.16 (m, 2H), 4.98 - 4.92 (m, 1H), 4.78 - 4.67 (m, 2H), 4.18 (br s, 2H), 4.05 - 3.87 (m, 3H), 3.58 - 3.44 (m, 2H), 3.30 - 3.23 (m, 1H), 3.02 - 2.93 (m, 1H), 2.83 - 2.58 (m, 2H), 2.54 - 2.38 (m, 8H), 2.36 - 2.17 (m, 5H), 2.04 - 1.86 (m, 2H). **11B** hydrochloride: $^1H$ NMR (400MHz, CDsOD) δ = 6.95 (d, *J* =8.4 Hz, 1H), 5.73 - 5.50 (m, 1H), 5.31 - 5.17 (m, 2H), 5.05 - 4.95 (m, 1H), 4.80 - 4.60 (m, 2H), 4.23 - 3.85 (m, 5H), 3.59 - 3.35 (m, 3H), 3.05 - 2.69 (m, 2H), 2.66 - 2.35 (m, 10H), 2.33 - 1.91 (m, 6H).

**Example 12**

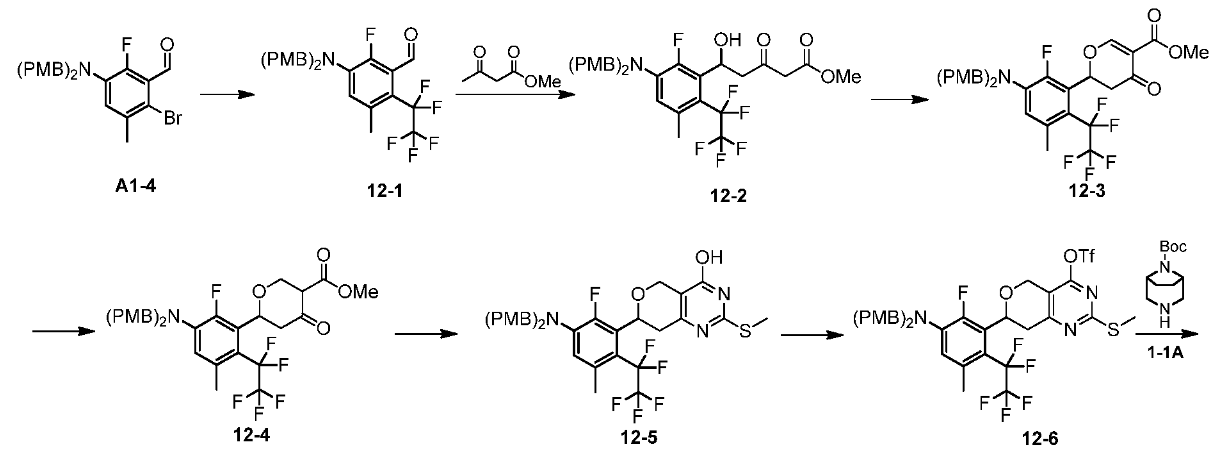


A1-4 12-1 12-2 12-3

12-4 12-5 12-6

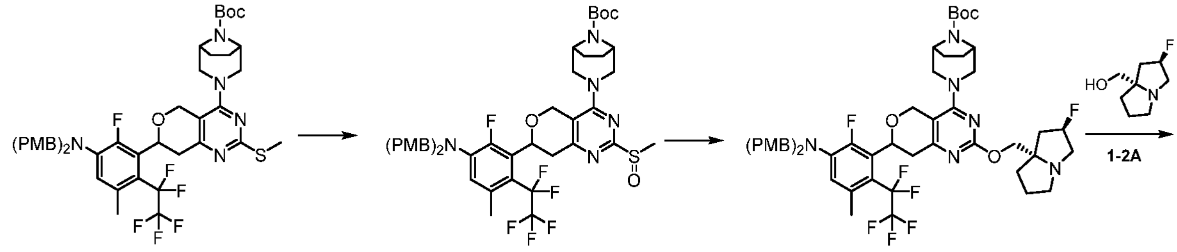


12-7 12-8 12-9

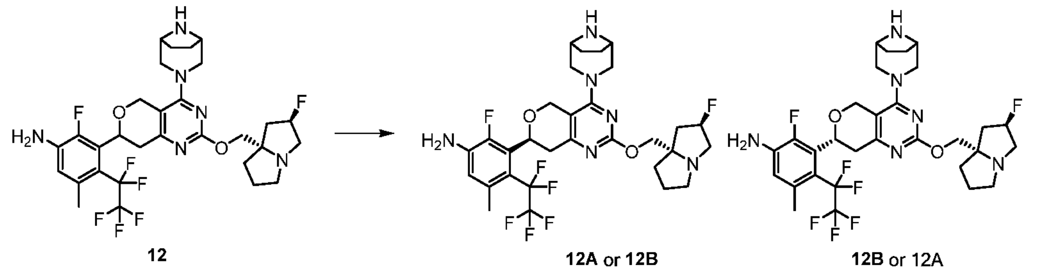

12 12A or 12B 12B or 12A

**Step 1: Preparation of intermediate 12-1**

To a 100 milliliters of stuffy jar were added the raw material **A1-4** (5 g, 10.59 mmol) and copper powder (3.36 g, 52.93 mmol) under nitrogen, and then DMSO (40 mL) and pentafluoroiodoethane (5.21 g, 21.17 mmol) were added. After sealing, the mixture was heated to 120 °C and stirred for 12 hours. To the reaction solution were added 50 milliliters of saturated brine and 200 milliliters of methyl tert-butyl ether. The mixture was stirred for 10 minutes, and filtered. The mixture was left to stand and the layers were separated to remove the aqueous phase. The organic phase was concentrated under reduced pressure. The residue is purified by chromatography purification system (eluent: 5 % ethyl acetate/petroleum ether) to give compound **12-1.** MS m/z =512.1[M+H]$^+$.

**Step 2: Preparation of intermediate 12-2**

Sodium hydride (1.56 g, 39.10 mmol, 60% content) was added to tetrahydrofuran (50 mL) at 0 °C (in an ice-water bath) under nitrogen. After the mixture was stirred for 15 minutes, ethyl acetoacetate (4.54 g, 39.10 mmol) was added dropwise. After the mixture was stirred for another 15 minutes, n-butyl lithium (2.5 M, 15.64 mL) was added dropwise. The mixture was stirred for 30 minutes, and a solution of the raw material **12-1** (4 g, 7.82 mmol) in tetrahydrofuran (10 mL) was added dropwise. The obtained mixture was naturally warmed to room temperature and stirred at 25 °C for 1 hour. The reaction was quenched by slowly adding 50 milliliters of saturated aqueous ammonium chloride solution to the reaction solution. To the mixture was added 100 milliliters of methyl tert-butyl ether and the mixture was stirred for 5 minutes. The aqueous phase was removed. The organic phase was concentrated under reduced pressure. The residue was purified by chromatography purification system (eluent: 10-20% ethyl acetate/petroleum ether) to give compound **12-2.** MS m/z =628.2[M+H]$^+$.

**Step 3: Preparation of intermediate 12-3**

To a solution of the raw material **12-2** (1.6 g, 2.55 mmol) in dichloromethane (20 mL) was added dropwise DMF-DMA (486.09 mg, 4.08 mmol) at room temperature (25 °C) under nitrogen. The mixture was stirred for 1 hour, and then the reaction bottle was cooled down to 0 °C in an ice-water bath. Boron trifluoride etherate (542.77 mg, 3.82 mmol) was added. The mixture was stirred for 1 hour. To the reaction solution were added 20 milliliters of saturated aqueous sodium bicarbonate solution and 30 milliliters of dichloromethane. The mixture was stirred for 5 minutes. The aqueous phase was removed. The organic phase was concentrated under reduced pressure. The residue was purified by chromatography purification system (eluent: 10-30% ethyl acetate/petroleum ether) to give compound **12-3.** MS m/z =638.1[M+H]$^+$.

**Step 4: Preparation of intermediate 12-4**

To a solution of the raw material **12-3** in tetrahydrofuran (15 mL) was added dropwise tri-sec-butyl lithium borohydride (1 M, 1.73 mL) at -60 °C (in a dry ice-ethyl acetate bath) under nitrogen. The obtained mixture was stirred

for 60 minutes. To the reaction solution were added 2 milliliters of 0.5M aqueous HCl solution, 20 milliliters of saturated brine and 50 milliliters of ethyl acetate. The mixture was stirred for 10 minutes. The aqueous phase was removed. The organic phase was concentrated under reduced pressure. The residue was purified by chromatography purification system (eluent: 30 % ethyl acetate/petroleum ether) to give compound **12-4.** MS m/z =640.2[M+H]$^+$.

**Step 5: Preparation of intermediate 12-5**

To a solution of the raw material **12-4** (1 g, 1.56 mmol) and S-methylisothiourea sulfate (1.31 g, 4.69 mmol) in ethanol (15 mL) was added the sodium carbonate (331.43 mg, 3.13 mmol). The obtained mixture was heated to 45 °C and stirred for 12 hours. The reaction solution was concentrated under reduced pressure to remove most of the ethanol. To the residue were added 10 milliliters of 0.5M dilute hydrochloric acid and 30 milliliters of 2-methyltetrahydrofuran. The mixture was stirred for 10 minutes. The aqueous phase was removed. The organic phase was concentrated under reduced pressure. The residue was purified by chromatography purification system (eluent: 10-30% ethyl acetate/petroleum ether) to give compound **12-5.** MS m/z =680.1[M+H]$^+$.

**Step 6: Preparation of intermediate 12-6**

To a solution of the raw material **12-5** (0.6 g, 882.78 μmol) in dichloromethane (10 mL) was added DIPEA (228.19 mg, 1.77 mmol) at 0 °C (in an ice-water bath) under nitrogen, and then trifluoromethanesulfonic anhydride (373.60 mg, 1.32 mmol, 218.48 μL) was added. The mixture was stirred for 1 hour. The reaction solution was diluted by adding 20 milliliters of dichloromethane, and then 20 milliliters of saturated aqueous ammonium chloride solution was added. The mixture was stirred for 10 minutes. The aqueous phase was removed. The organic phase was concentrated under reduced pressure to give compound **12-6.** MS m/z =812.0[M+H]$^+$.

**Step 7: Preparation of intermediate 12-7**

To a solution of the raw material **12-6** (0.75 g, 923.95 μmol) in DMF (10 mL) were added DIPEA (358.24 mg, 2.77 mmol) and **1-1A** (235.37 mg, 1.11 mmol) at room temperature (25 °C) under nitrogen. The mixture was heated to 50 °C and stirred for 30 minutes. To the reaction solution were added 20 milliliters of water and 30 milliliters of ethyl acetate. The mixture was stirred for 10 minutes. The aqueous phase was removed. The organic phase was concentrated under reduced pressure. The residue was purified by chromatography purification system (eluent: 10-20% ethyl acetate/petroleum ether) to give compound **12-7.** MS m/z =874.2[M+H]$^+$.

**Step 8: Preparation of intermediate 12-8**

To a solution of the raw material **12-7** (0.32 g, 366.16 μmol) in dichloromethane (5 mL) was added m-chloroperoxybenzoic acid (81.77 mg, 402.77 μmol, 85% content) at 0 °C (in an ice-water bath) under nitrogen. The mixture was stirred for 2 hours. The reaction solution was diluted by adding 20 milliliters of dichloromethane, and then 10 milliliters of saturated aqueous sodium bicarbonate solution and 10 milliliters of saturated $Na_2SO_3$ solution were added. The mixture was stirred for 10 minutes (detected by starch potassium iodide paper). The aqueous phase was removed. The organic phase was concentrated under reduced pressure. The residue was purified by chromatography purification system (eluent: 10-30% ethyl acetate/petroleum ether) to give compound **12-8.** MS m/z =890.2[M+H]$^+$.

**Step 9: Preparation of intermediate 12-9**

To a solution of the raw material **1-2A** (85.87 mg, 539.36 μmol) in tetrahydrofuran (5 mL) was added sodium tert-butoxide (69.11 mg, 719.15 μmol) at 0 °C (in an ice-water bath) under nitrogen. After the mixture was stirred for 30 minutes, the raw material **12-8** (0.32 g, 359.57 μmol) was added. The obtained mixture was stirred for 1 hour. To the reaction solution were added 10 milliliters of saturated aqueous ammonium chloride solution and 20 milliliters of ethyl acetate. The mixture was stirred for 10 minutes. The aqueous phase was removed. The organic phase was concentrated under reduced pressure. The residue was purified by chromatography purification system (eluent: 10% methanol/dichloromethane) to give compound **12-9.** MS m/z =985.3[M+H]$^+$.

**Step 10: Preparation of compound 12 formate, compound 12A and compound 12B**

To a solution of the raw material **12-9** (0.1 g, 101.52 μmol) in dichloromethane (1.5 mL) was added trifluoroacetic acid (0.5 mL) at room temperature (25 °C). The mixture was stirred for 4 hours. The reaction solution was directly concentrated under reduced pressure. The residue was purified by preparative HPLC (column: Phenomenex Luna C18

75*30mm*3μm; mobile phase: [water (0.025% formic acid)-acetonitrile]; (acetonitrile)%: 1%-35%, 8min) to give **12** formate. Then **12** formate was subjected to chiral separation (column: DAICEL CHIRALPAK AD (250mm*30mm, 10μm); mobile phase: [0.1%$NH_3H_2O$ MeOH]; (methanol) %: 40%-40%, 10min) to give compound **12A** (Rt=3.473 min) and compound **12B** (Rt=4.102 min).

Compound **12A:** $^1H$ NMR (400 MHz, CDsOD) δ = 6.74 (d, *J* = 8.6 Hz, 1H), 5.40-5.20 (m, 1H), 5.16 - 5.06 (m, 1H), 4.76 - 4.54 (m, 3H), 4.20 - 4.05 (m, 3H), 3.65 (br s, 2H), 3.59 - 3.51 (m, 1H), 3.49 - 3.39 (m, 1H), 3.27 - 3.16 (m, 3H), 3.12 - 2.97 (m, 2H), 2.84 (br dd, *J* = 3.2, 17.7 Hz, 1H), 2.36 (br dd, *J* = 2.7, 6.9 Hz, 5H), 2.16 - 1.68 (m, 8H). MS m/z =645.3$[M+H]^+$.

Compound **12B:** $^1H$ NMR (400 MHz, CDsOD) δ = 6.74 (d, *J* = 8.6 Hz, 1H), 5.40-5.18 (m, 1H), 5.10 (br dd, *J* = 3.9, 10.8 Hz, 1H), 4.74 - 4.49 (m, 3H), 4.21 - 4.08 (m, 2H), 4.04 (d, *J* = 10.3 Hz, 1H), 3.56 - 3.37 (m, 4H), 3.29 - 3.13 (m, 4H), 3.07 - 2.93 (m, 2H), 2.83 (br dd, *J* = 3.1, 16.9 Hz, 1H), 2.40 - 2.15 (m, 5H), 2.14 - 1.63 (m, 9H). MS m/z =645.3$[M+H]^+$.

**Example 13**

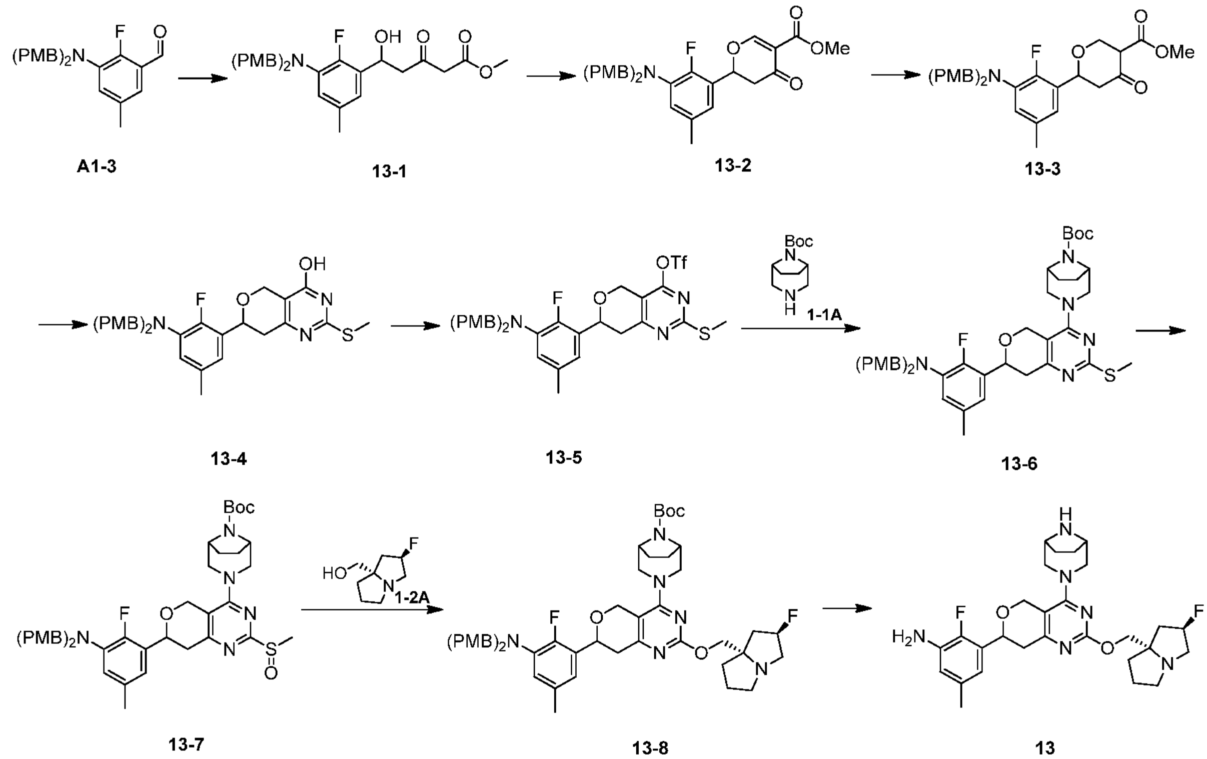


**Step 1: Preparation of intermediate 13-1**

Sodium hydride (10.17 g, 254.16 mmol, 60% content) was slowly added to tetrahydrofuran (500 mL) in portions at -5 °C. The atmosphere was replaced with nitrogen three times, and then methyl acetoacetate (29.51 g, 254.16 mmol) was added slowly. The system was reacted at this temperature for 10 min, and then n-butyl lithium (2.5 M, 101.66 mL) was added dropwise. After completion of the addition, the mixture was stirred for another 10 min, and cooled down to -10 °C. A solution of **A1-3** (50 g, 127.08 mmol) in tetrahydrofuran (100 mL) was then added dropwise. After completion of the addition, the mixture was reacted for another 10 min. The reaction was quenched by adding 400 mL of saturated ammonium chloride solution. The mixture was extracted with ethyl acetate (500 mL*2). After extraction, the organic phases were combined, washed with 1 L of saturated brine, dried over anhydrous sodium sulfate, and filtered. The filtrate was rotary evaporated to dryness. The residue was separated by column chromatography (eluent: 15-40 % ethyl acetate/petroleum ether) to give **13-1.**

**Step 2: Preparation of intermediate 13-2**

To a solution of **13-1** (58.5 g, 114.80 mmol) in dichloromethane (350 mL) was added dimethylformamide dimethyl acetal (21.89 g, 183.69 mmol), and the system was reacted at 25°C for 1 hour. The system was cooled down to 0 °C. Boron trifluoride etherate (24.44 g, 172.21 mmol) was added dropwise slowly, and the system was reacted for another 15 min. To the system was added 350 mL of saturated sodium bicarbonate solution. The mixture was extracted with dichloromethane (300 mL*2). After extraction, the organic phases were combined, washed with 500mL of saturated brine, dried over anhydrous sodium sulfate, and filtered. The filtrate was rotary evaporated to dryness. The residue was separated by column chromatography (eluent: 15-40% ethyl acetate/petroleum ether) to give **13-2.** MS m/z: 520.3 $[M+H]^+$.

**Step 3: Preparation of intermediate 13-3**

To a solution of **13-2** (42.5 g, 81.80 mmol) in tetrahydrofuran (500 mL) was added dropwise tri-sec-butyl lithium borohydride (1 M, 89.98 mL) at -60 °C. The system was reacted at this temperature for 10 min. The reaction solution was poured into 1 L of 1N hydrochloric acid solution. The mixture was extracted with ethyl acetate (1 L*2). After extraction, the organic phases were combined, washed with saturated brine (1.5 L), dried over anhydrous sodium sulfate, and filtered. The filtrate was rotary evaporated to dryness. The residue was separated by column chromatography (eluent: 0-10% ethyl acetate/petroleum ether) to give **13-3.** MS m/z: 522.3 $[M+H]^+$.

**Step 4: Preparation of intermediate 13-4**

To a solution of **13-3** (28.5 g, 54.64 mmol) and methylisothiourea sulfate (45.63 g, 163.93 mmol) in ethanol (400 mL) was added sodium carbonate (11.58 g, 109.28 mmol, 2 eq). The system was heated with stirring at 50 °C for 18 hours. The reaction solution was concentrated under reduced pressure to remove most of the ethanol. To the system were added 400 mL of water and 400 mL of ethyl acetate. The mixture was adjusted to a pH of 4 with 1N hydrochloric acid. The organic phase was separated out. The aqueous phase was extracted with ethyl acetate (400 mL). After extraction, the organic phases were combined, and washed with 500mL of saturated brine. There were a lot of insoluble solids in the organic phase. The solids were filtered out and the filtrate was evaporated to dryness to give **13-4.** $^1$H NMR (400MHz, DMSO-$d_6$) $\delta$ = 7.20 (d, *J* =8.4 Hz, 4H), 6.88 - 6.80 (m, 5H), 6.73 (br d, *J*=6.4 Hz, 1H), 4.90 (dd, *J* =4.0, 10.0 Hz, 1H), 4.68 - 4.41 (m, 2H), 4.15 (s, 4H), 3.71 (s, 6H), 2.80 - 2.64 (m, 2H), 2.47 (s, 3H), 2.14 (s, 3H). MS m/z: 562.2 $[M+H]^+$.

**Step 5: Preparation of intermediate 13-5**

To a solution of **13-4** (24.5 g, 43.62 mmol, 1 eq) in N,N-dimethylformamide (300 mL) was added diisopropylethylamine (16.91 g, 130.86 mmol), and then N-phenyltrifluoromethanesulfonimide (18.70 g, 52.34 mmol) was added. The system was stirred at 20 °C for 0.5 hours. The reaction solution was diluted with 1.5L of ethyl acetate. The mixture was then washed sequentially with water (800mL*2) and saturated brine (1L), dried over anhydrous sodium sulfate, and filtered. The filtrate was rotary evaporated to dryness. The residue was separated by column chromatography (eluent: 0-10% ethyl acetate/petroleum ether) to give **13-5.** MS m/z: 694.1 $[M+H]^+$.

**Step 6: Preparation of intermediate 13-6**

To a solution of **13-5** (21.5 g, 30.99 mmol) in N,N-dimethylformamide (150 mL) was added **1-1A** (7.24 g, 34.09 mmol). The system was heated at 90 °C for 1 hour. The reaction solution was rotary evaporated to dryness. The residue was separated by column chromatography (eluent: 5-20% ethyl acetate/petroleum ether) to give **13-6.** $^1$H NMR (400MHz, $CDCl_3$) $\delta$ 7.19 (d, *J* =8.8 Hz, 4H), 6.88 - 6.79 (m, 5H), 6.63 (d, *J* =8.0 Hz, 1H), 5.12 (dd, *J* =4.0, 10.8 Hz, 1H), 4.88 - 4.70 (m, 2H), 4.42 - 4.25 (m, 2H), 4.20 (s, 4H), 3.81 (s, 6H), 3.48 -3.42 (m, 2H), 3.18 - 2.92 (m, 4H), 2.53 (s, 3H), 2.21 (s, 3H), 2.03 - 1.89 (m, 3H), 1.75-1.65 (m, 1H), 1.52 (s, 9H). MS m/z: 756.4 $[M+H]^+$.

**Step 7: Preparation of intermediate 13-7**

**13-6** (1 g, 1.32 mmol, 1 eq) was weighed, and DCM (30 mL) and m-CPBA (268.57 mg, 1.32 mmol, 85% content, 1 eq) were added. The mixture was reacted at 25 °C for 1 hr. The reaction was quenched by adding sodium bicarbonate. The mixture was extracted with DCM. The organic phase was dried over anhydrous sodium sulfate, and rotary evaporated to dryness to give **13-7,** which was directly used in the next step.

**Step 8: Preparation of intermediate 13-8**

To a solution of **1-2A** (404.09 mg, 2.54 mmol, 2 eq) in toluene (50 mL) was added sodium tert-butoxide (10 mg, 0.103 mmol) at 20 °C, and then **13-7** (979.69 mg, 1.27 mmol, 1 eq) was added. The mixture was reacted at 120 °C for 15hr. The rection solution was cooled down to room temperature. The reaction was quenched by adding water. The mixture was extracted with ethyl acetate. The organic phase was dried over anhydrous sodium sulfate, and rotary evaporated to dryness. The residue was separated by column chromatography (eluent: 0~5% methanol/dichloromethane) to give **13-8.** MS m/z: 867.3 [M+H]$^+$.

**Step 9: Preparation of compound 13 hydrochloride**

To **13-8** (0.6 g, 692.02 μmol, 1 eq) was added trifluoroacetic acid (5 mL). The system was stirred at 55 °C for 5 hours. The reaction solution was rotary evaporated to dryness. The residue was purified by preparative HPLC (column: Phenomenex C18 150*40mm*5μm; mobile phase: [water (0.05% hydrochloric acid)-acetonitrile]; (acetonitrile)%: 1%-30%, 10min) to give compound **13** hydrochloride. $^1$H NMR (400MHz, CDsOD) δ 7.57 - 7.50 (m, 1 H), 7.40 - 7.30 (m, 1 H), 5.74 - 5.55 (m, 1 H), 5.33 - 5.23 (m, 1 H), 5.21 - 5.11 (m, 1 H), 4.84 - 4.76 (m, 2 H), 4.34 - 4.22 (m, 2 H), 4.17 - 3.83 (m, 5 H), 3.71 - 3.61 (m, 1 H), 3.53 - 3.42 (m, 1 H), 3.36 - 3.22 (m, 3 H), 3.17 - 3.04 (m, 1 H), 2.85 - 2.47 (m, 3 H), 2.01 - 2.43 (m, 10H). MS m/z: 527.2 [M+H]$^+$.

**Example 14**

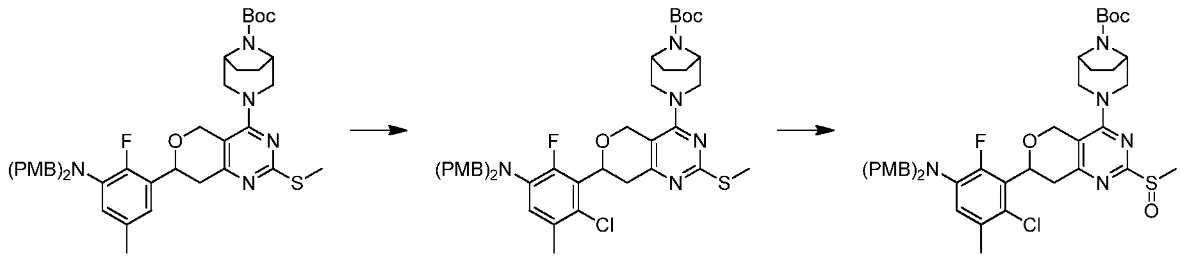

**13-6** **14-1** **14-2**

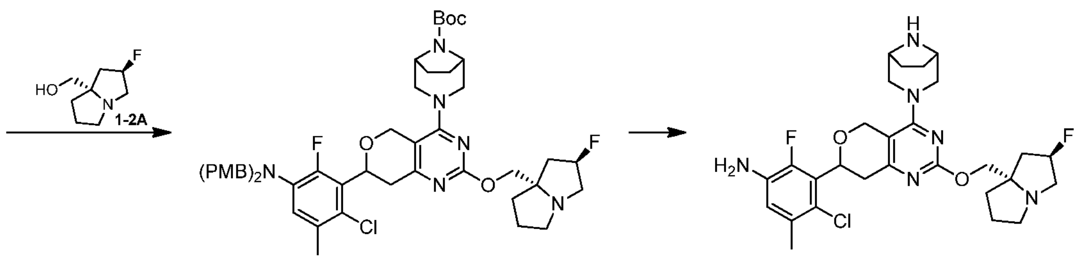


**14-3** **14**

**Step 1: Synthesis of intermediate 14-1**

Compound **13-6** (200 mg, 264.57 μmol, 1 eq) was dissolved in N,N-dimethylformamide (3 mL), and then N-chlorosuccinimide (45.93 mg, 343.94 μmol, 1.3 eq) was added. The resulting reaction solution was reacted with stirring at 25 °C for 15 hours. The reaction solution is directly separated by high-performance liquid chromatography with separation conditions of: (column: Welch Xtimate C18 100*40mm*3μm; mobile phase: [water (0.025% trifluoroacetic acid)-acetonitrile]; acetonitrile%: 50%-80%, 8min) to give compound **14-1** trifluoroacetate. MS m/z =790.4 [M+H]$^+$.

**Step 2: Synthesis of intermediate 14-2**

Compound **14-1** trifluoroacetate (123 mg) was dissolved in anhydrous dichloromethane (2 mL), and then m-chloroperoxybenzoic acid (31.59 mg) was added. The resulting reaction solution was reacted with stirring at 15 °C for

15 hours. The reaction solution was rotary evaporated to dryness to give a crude product. The crude product was purified by column chromatography (methanol/dichloromethane=0~3%) to give **14-2.** MS m/z =806.2[M+H]$^+$.

**Step 3: Synthesis of intermediate 14-3**

Compound **1-2A** (37.96 mg, 238.47 μmol, 3eq), sodium tert-butoxide (15.28 mg, 158.98 μmol, 2eq) and compound **14-2** (64.1mg, 79.49 μmol, 1eq) were added to toluene (2 mL). The mixture was reacted with stirring at 15 °C for 4 hours. The reaction solution was diluted with 30 mL of ethyl acetate. The mixture was washed with 5 mL of water and 5 mL of saturated brine. The organic phase was rotary evaporated to dryness to give a crude product. The crude product was purified by column chromatography (methanol/dichloromethane=0~5%) to give compound **14-3.** MS m/z=901.3[M+H]$^+$.

**Step 4: Synthesis of compound 14 hydrochloride**

Compound **14-3** (67 mg, 74.32 μmol, 1 eq) was added to trifluoroacetic acid (2 mL), and the mixture was reacted with stirring at 25 °C for 4 hours. The reaction solution was rotary evaporated to dryness. To the residue were added 300 mg of sodium carbonate and 5 mL of ethyl acetate. The mixture was stirred for 20 minutes, and filtered. The solvent was removed from the filtrate under reduced pressure to give a crude product. The crude product was separated by preparative high-performance liquid chromatography (separation conditions: column: Xtimate C18 150*40mm*5μm; mobile phase: [water (0.05% hydrochloric acid)-acetonitrile]; (acetonitrile)%: 1%-30%, 10min) to give compound **14** hydrochloride, MS m/z=561.2[M+H]$^+$.

**Example 15**

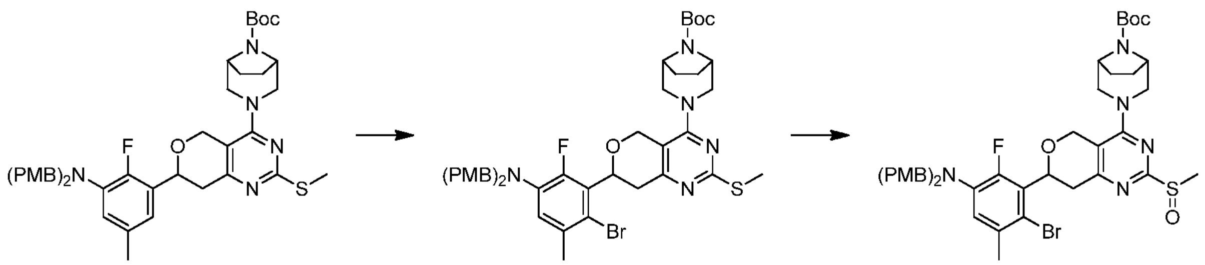

**13-6** **15-1** **15-2**

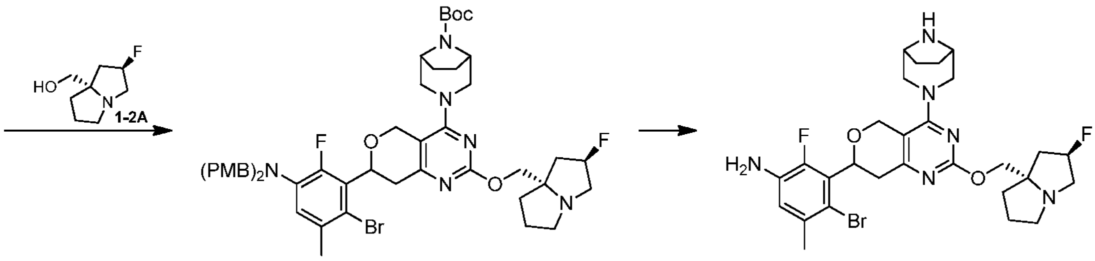


**15-3** **15**

**Step 1: Preparation of intermediate 15-1**

**13-6** (2.00 g, 2.64 mmol, 1 eq) was weighed, and DMF (50 mL) and NBS (940.54 mg, 5.28 mmol, 2 eq) were added. After completion of the addition, the mixture was reacted at 25 °C for 4 hr. The reaction was quenched by adding sodium bicarbonate. The mixture was extracted with ethyl acetate. The organic phase was dried over anhydrous sodium sulfate, and rotary evaporated to dryness. The residue was separated by column chromatography (eluent: 5-20% ethyl acetate/petroleum ether) to give **15-1.** MS m/z: 834.2 [M+H]$^+$.

**Step 2: Preparation of intermediate 15-2**

**15-1** (1 g, 1.32 mmol, 1 eq) was weighed, and DCM (30 mL) and m-CPBA (268.57 mg, 1.32 mmol, 85% content, 1 eq) were added. The mixture was reacted at 25 °C for 1 hr. The reaction was quenched by adding sodium bicarbonate. The mixture was extracted with DCM. The organic phase was dried over anhydrous sodium sulfate, and rotary evaporated to dryness to give **15-2,** which was directly used in the next step.

**Step 3: Preparation of intermediate 15-3**

To a solution of **1-2A** (16 mg, 0.103 mmol) in tetrahydrofuran (15 mL) was added sodium tert-butoxide (10 mg, 0.103 mmol) at 20 °C. The system was stirred at this temperature for 0.5 hours, and then **15-2** (73 mg, 86 μmol) was added. The mixture was stirred for another 0.5 hours. The reaction solution was diluted with 10 mL of ethyl acetate. The mixture was washed with 10 mL of saturated brine, dried over anhydrous sodium sulfate, and filtered. The filtrate was rotary evaporated to dryness. The residue was separated by column chromatography (eluent: 0-5% methanol/dichloromethane) to give **15-3.** MS m/z: 946.8 $[M+H]^+$.

**Step 4: Preparation of compound 15 hydrochloride**

To **15-3** (40mg, 1 eq) was added trifluoroacetic acid (1 mL), and the system was stirred at 55 °C for 2 hours. The reaction solution was rotary evaporated to dryness. The residue was purified by preparative HPLC (column: Xtimate C18 150*40mm*5μm; mobile phase: [water (0.05% hydrochloric acid)-acetonitrile]; (acetonitrile)%: 1%-30%, 10min) to give compound 15 hydrochloride. MS m/z: 605.0 $[M+H]^+$.

**Example 16**

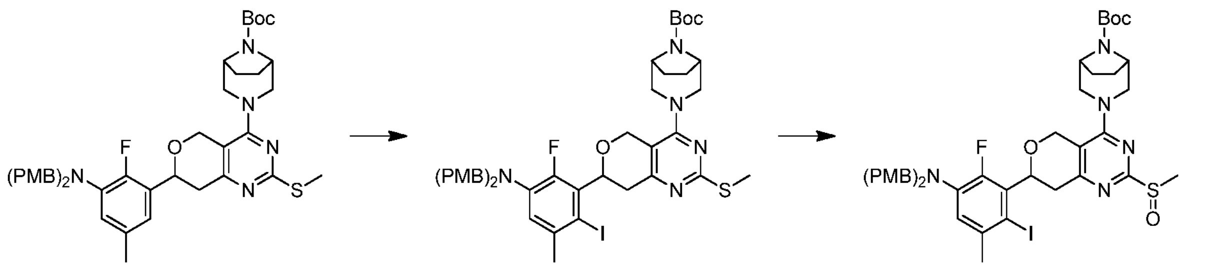

13-6 16-1 16-2

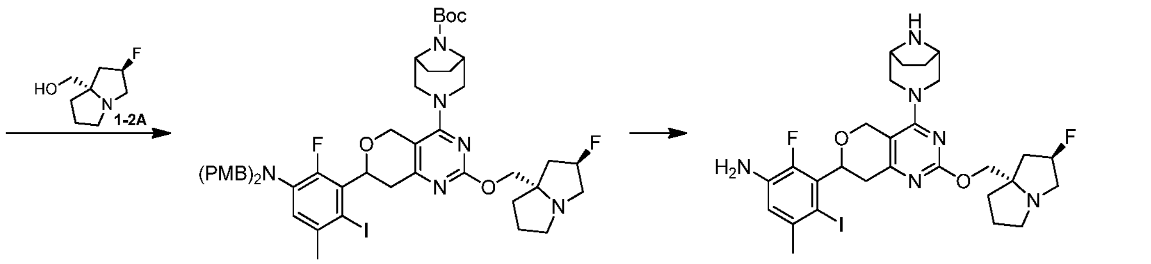


16-3 16

**Step 1: Preparation of intermediate 16-1**

**13-6** (1 g, 1.32 mmol, 1 eq) was weighed, and DMF (25 mL) and NIS (892.85 mg, 3.97 mmol, 3 eq) were added. The mixture was reacted at 25 °C for 5 hr. The reaction was quenched by adding water. The mixture was extracted with ethyl acetate. The organic phase was washed with water and dried. The residue was separated by column chromatography (eluent: 5~20% ethyl acetate/petroleum ether) to give **16-1.** MS m/z: 882.2 $[M+H]^+$.

**Step 2: Preparation of intermediate 16-2**

**16-1** (0.6 g, 680.40 μmol, 1 eq) was weighed, and DCM (30 mL) and m-CPBA (138.14 mg, 680.40 μmol, 85% content, 1 eq) were added. The mixture was reacted at 25 °C for 1 hr. The reaction was quenched by adding sodium bicarbonate. The mixture was extracted with DCM. The organic phase was dried over anhydrous sodium sulfate, and rotary evaporated to dryness to give **16-2,** which was directly used in the next step.

**Step 3: Preparation of intermediate 16-3**

To a solution of **1-2A** (132.99 mg, 835.34 μmol, 1.5 eq) in tetrahydrofuran (50 mL) was added sodium tert-butoxide (80.28 mg, 835.34 μmol, 1.5 eq) at 20 °C, and then **16-2** (0.5 g, 556.90 μmol, 1 eq) was added. After completion of the addition, the mixture was reacted at 25 °C for 15 hr. The reaction was quenched by adding water. The mixture was extracted with ethyl acetate. The organic phase was dried over anhydrous sodium sulfate, and rotary evaporated to dryness. The residue was separated by column chromatography (eluent: 0-5% methanol/dichloromethane) to give **16-3.** MS m/z: 993.2 $[M+H]^+$.

**Step 4: Preparation of compound 16 hydrochloride**

To **16-3** (0.3 g, 302.14 μmol, 1 eq) was added trifluoroacetic acid (5 mL). The system was stirred at 55 °C for 5 hours. The reaction solution was rotary evaporated to dryness. The residue was purified by preparative HPLC (column: Xtimate C18 150*40mm*5μm; mobile phase: [water (0.05% hydrochloric acid)-acetonitrile]; (acetonitrile)%: 1%-30%, 10min) to give compound **16** hydrochloride. MS m/z: 653.3 $[M+H]^+$.

**Example 17**

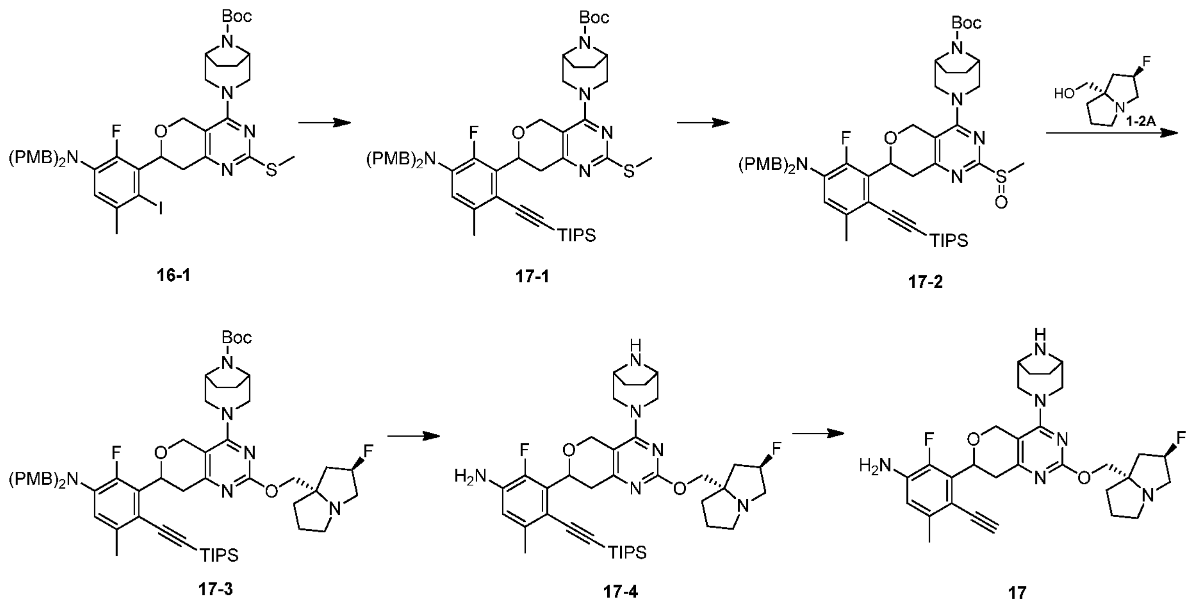


16-1 17-1 17-2

17-3 17-4 17

**Step 1: Preparation of intermediate 17-1**

**16-1** (0.8 g, 907.20 μmol, 1 eq) was weighed, and $PdCl_2(PPh_3)_2$ (127.35 mg, 181.44 μmol, 0.2 eq), CuI (51.83 mg, 272.16 μmol, 0.3 eq), EtOH (20 mL), EtsN (229.50 mg, 2.27 mmol, 315.68 μL, 2.5 eq) and trimethylsilylacetylene (330.91 mg, 1.81 mmol, 407.07 μL, 2 eq) were added. The atmosphere was replaced with nitrogen 3 times. The mixture was reacted at 80 °C for 5 hours. The mixture was filtered through a pad of celite. The filtrate was rotary evaporated to dryness. The residue was separated by column chromatography (eluent: 5-20% ethyl acetate/petroleum ether) to give **17-1.** MS m/z: 936.4 $[M+H]^+$.

**Step 2: Preparation of intermediate 17-2**

**17-1** (0.4 g, 427.21 μmol, 1 eq) was weighed, and DCM (10 mL) and m-CPBA (86.73 mg, 427.21 μmol, 85% content, 1 eq) were added. After completion of the addition, the mixture was reacted at 25 °C for 1 hr. The reaction was quenched by adding sodium bicarbonate. The mixture was extracted with DCM. The organic phase was dried over anhydrous sodium sulfate, and rotary evaporated to dryness to give **17-2,** which was directly used in the next step. MS m/z: 952.4 $[M+H]^+$.

**Step 3: Preparation of intermediate 17-3**

To a solution of **1-2A** (100.31 mg, 630.05 μmol, 1.5 eq) in tetrahydrofuran (5 mL) was added sodium tert-butoxide (60.55 mg, 630.05 μmol, 1.5 eq) at 20 °C, and then **17-2** (0.4 g, 420.04 μmol, 1 eq) was added. After completion of the addition, the mixture was reacted at 25 °C for 15 hr. The reaction was quenched by adding water. The mixture was extracted with ethyl acetate. The organic phase was dried over anhydrous sodium sulfate, and rotary evaporated to dryness to give **17-3.** MS m/z: 1047.5 $[M+H]^+$.

**Step 4: Preparation of intermediate 17-4 formate**

To **17-3** (0.3 g, 302.14 μmol, 1 eq) was added trifluoroacetic acid (6 mL), and the system was stirred at 25 °C for 5 hours. The reaction solution was rotary evaporated to dryness. The residue was purified by preparative HPLC (column: Xtimate C18 150*40mm*5μm; mobile phase: [water (0.025% formic acid)-acetonitrile]; (acetonitrile)%: 17%-57%, 8min) to give compound **17-4** formate. MS m/z: 707.4 $[M+H]^+$.

**Step 5: Preparation of compound 17**

**17-4** formate (30 mg) was weighed, and THF (2 mL) and tetramethylammonium fluoride (11.86 mg, 127.30 μmol, 3 eq) were added. The mixture was reacted at 60 °C for 4 hr. The reaction solution was directly rotary evaporated to dryness. The residue was purified by preparative HPLC (column: Phenomenex C18 80*40mm*3μm; mobile phase: [water (0.5% ammonia water)-acetonitrile]; (acetonitrile)%: 43%-73%, 8min) to give compound **17.** MS m/z: 551.2 $[M+H]^+$.

**Example 18**

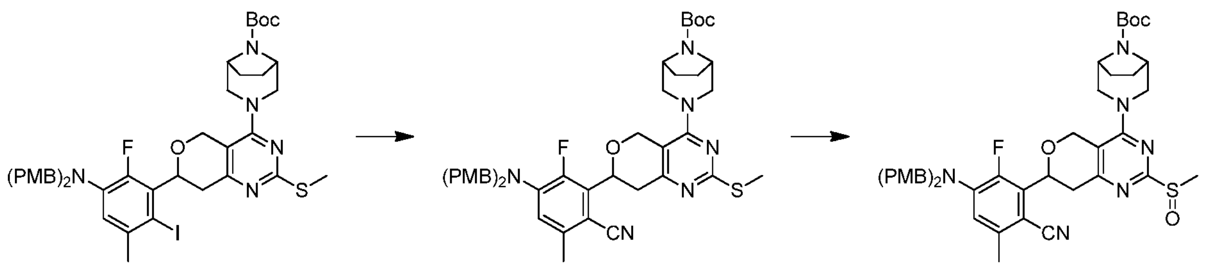

**16-1** **18-1** **18-2**

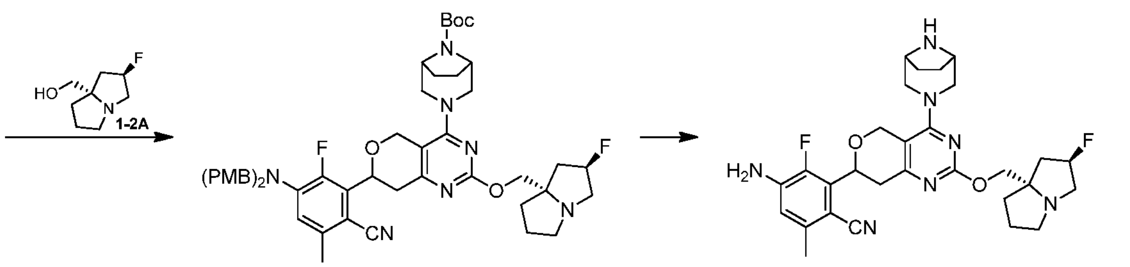


**18-3** **18**

**Step 1: Preparation of intermediate 18-1**

**16-1** (0.4 g, 453.60 μmol, 1 eq) was weighed, and $K_4FeCN_6$ (36.76 mg, 99.79 μmol, 0.22 eq), $Na_2CO_3$ (48.08 mg, 453.60 μmol, 1 eq), $Pd(OAc)_2$ (20.37 mg, 90.72 μmol, 0.2 eq) and DMAc (5 mL) were added. The atmosphere was replaced with nitrogen 3 times. The mixture was reacted at 120 °C for 15 hours. The reaction was quenched by adding water. The mixture was extracted with ethyl acetate. The organic phase was dried, and rotary evaporated to dryness. The residue was separated by column chromatography (eluent: 5~20% ethyl acetate/petroleum ether) to give **18-1.** MS m/z: 781.2 $[M+H]^+$.

**Step 2: Preparation of intermediate 18-2**

**18-1** (100 mg, 128.05 μmol, 1 eq) was weighed, and DCM (10 mL) and m-CPBA (26.00 mg, 128.05 μmol, 85% content, 1 eq) were added. After completion of the addition, the mixture was reacted at 25 °C for 1 hr. The reaction was quenched by adding sodium bicarbonate. The mixture was extracted with DCM. The organic phase was dried over anhydrous sodium sulfate, and rotary evaporated to dryness to give **18-2,** which was directly used in the next step. MS m/z: 797.2 $[M+H]^+$.

**Step 3: Preparation of intermediate 18-3**

To a solution of **1-2A** (27.97 mg, 175.67 μmol, 2 eq) in toluene (5 mL) at 20 °C was added sodium tert-butoxide (10.97 mg, 114.19 μmol, 1.3 eq), and then **18-2** (70 mg, 87.84 μmol, 1 eq) was added. After completion of the addition, the mixture was reacted at 120 °C for 5 hr. The reaction was quenched by adding water. The mixture was extracted with ethyl acetate. The organic phase was dried over anhydrous sodium sulfate, and rotary evaporated to dryness to give **18-3.** MS m/z: 892.4 $[M+H]^+$.

**Step 4: Preparation of compound 18**

To **18-3** (60 mg, 108.77 μmol, 1 eq) was added trifluoroacetic acid (5 mL). The system was stirred at 50 °C for 2 hours. The reaction solution was rotary evaporated to dryness. The residue was separated sequentially by acidic HPLC (column: Xtimate C18 150*40mm*5μm; mobile phase: [water (0.05% hydrochloric acid)-acetonitrile]; (acetonitrile)%: 1%-30%, 10min) and basic HPLC (column: Phenomenex C18 80*40mm*3μm; mobile phase: [water (0.5% ammonia water)-acetonitrile]; (acetonitrile)%: 40%-70%, 8min) to give compound **18.** MS m/z: 552.3 $[M+H]^+$.

**Example 19**

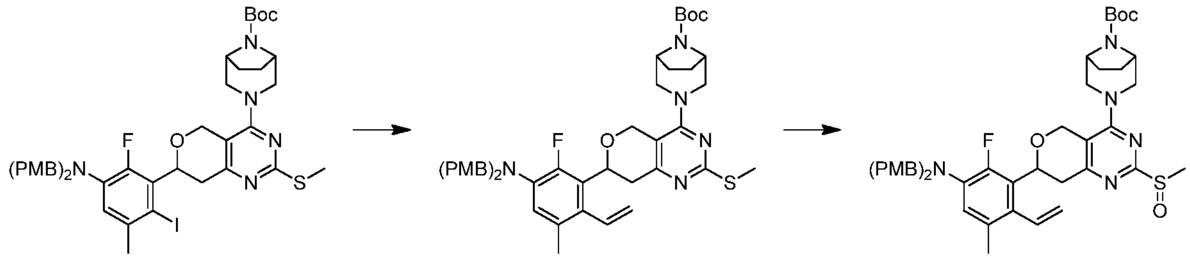

16-1 19-1 19-2

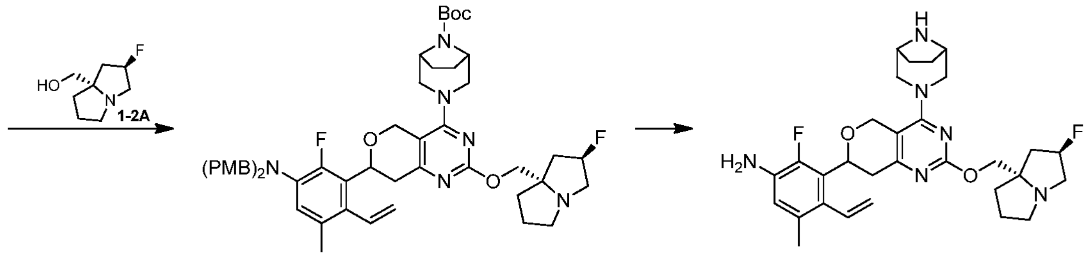


19-3 19

**Step 1: Preparation of intermediate 19-1**

**16-1** (0.1 g, 113.40 μmol, 1 eq) was weighed, and Pd(dppf)Ch (16.60 mg, 22.68 μmol, 0.2 eq), 1,4-dioxane (5 mL), $H_2O$ (1 mL), vinylboronic acid pinacol ester (26.20 mg, 170.10 μmol, 28.85 μL, 1.5 eq) and $K_2CO_3$ (23.51 mg, 170.10 μmol, 1.5 eq) were added. The atmosphere was replaced with nitroge 3 times. The mixture was reacted at 95 °C for 15 hours. The reaction solution was directly rotary evaporated to dryness. The residue was separated by column chromatography (eluent: 5~20% ethyl acetate/petroleum ether) to give **19-1.** MS m/z: 782.3 $[M+H]^+$.

**Step 2: Preparation of intermediate 19-2**

**19-1** (0.07 g, 89.52 μmol, 1 eq) was weighed, and DCM (10 mL) and m-CPBA (18.17 mg, 89.52 μmol, 85% content, 1 eq) were added. After completion of the addition, the mixture was reacted at 25 °C for 1 hr. The reaction was quenched by adding sodium bicarbonate. The mixture was extracted with DCM. The organic phase was dried over anhydrous sodium sulfate, and rotary evaporated to dryness to give **19-2,** which was directly used in the next step. MS m/z: 798.3 $[M+H]^+$.

**Step 3: Preparation of intermediate 19-3**

To a solution of **1-2A** (19.95 mg, 125.32 μmol, 2 eq) in toluene (5 mL) was added sodium tert-butoxide (9.03 mg, 93.99 μmol, 1.5 eq) at 20 °C, and then **19-2** (0.05 g, 62.66 μmol, 1 eq) was added. After completion of the addition, the mixture was reacted at 120 °C for 5 hr. The reaction was quenched by adding water. The mixture was extracted with ethyl acetate. The organic phase was dried over anhydrous sodium sulfate, and rotary evaporated to dryness to give **19-3.** MS m/z: 893.3 $[M+H]^+$.

**Step 4: Preparation of compound 19 hydrochloride**

To **19-3** (50 mg, 55.99 μmol, 1 eq) was added trifluoroacetic acid (3 mL). The system was stirred at 55 °C for 5 hours. The reaction solution was rotary evaporated to dryness. The residue was separated by acidic HPLC (chromatographic column: Xtimate C18 150*40mm*5μm; mobile phase: [water (0.05% hydrochloric acid)-acetonitrile]; (acetonitrile)%: 1%-30%, 10min) to give compound **19** hydrochloride. MS m/z: 553.3 $[M+H]^+$.

**Example 20**

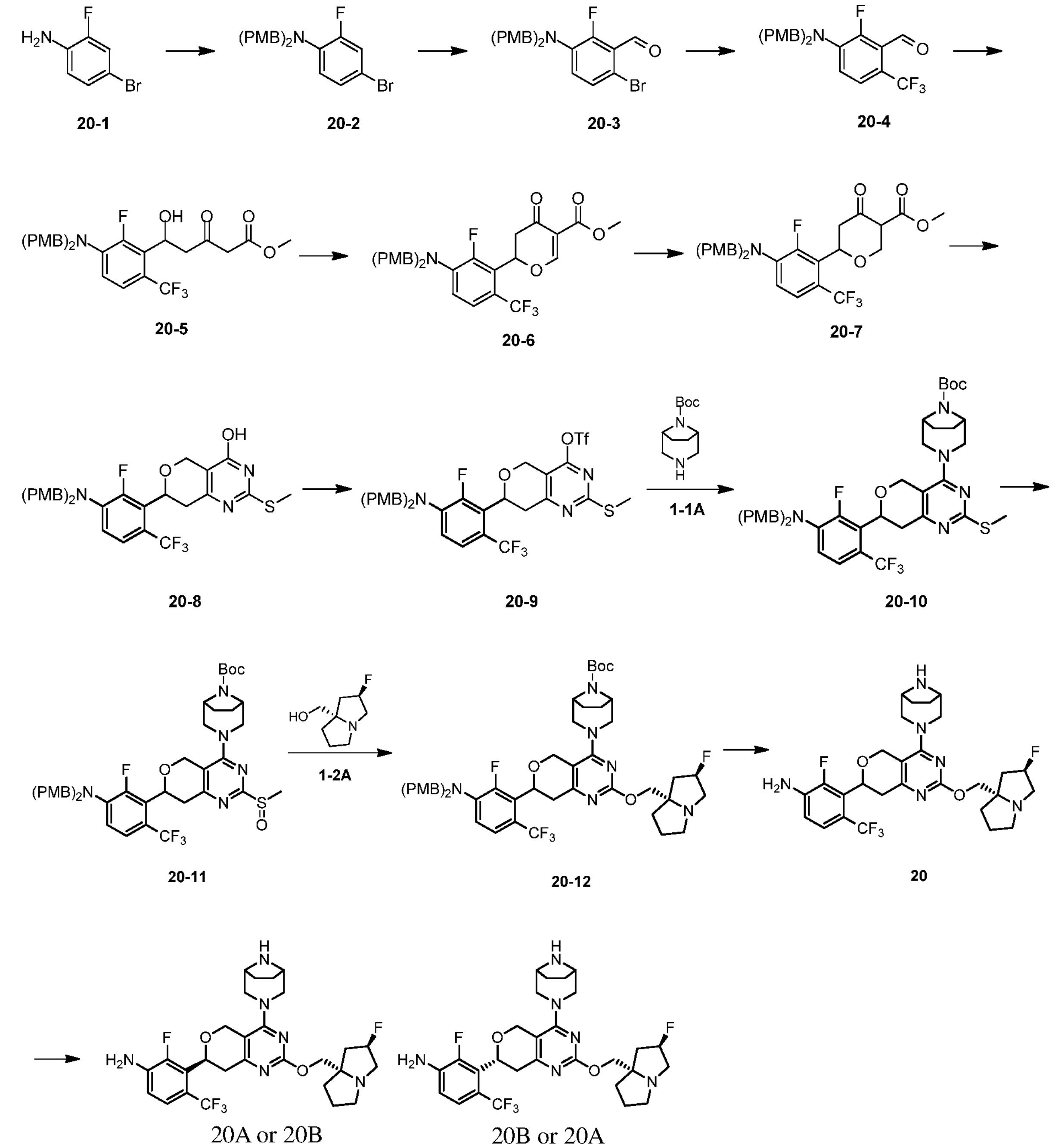


20-1 20-2 20-3 20-4

20-5 20-6 20-7

20-8 20-9 20-10

20-11 20-12 20

20A or 20B 20B or 20A

**Step 1: Synthesis of intermediate 20-2**

Compound **20-1** (85 g, 447.34 mmol, 1 eq), potassium carbonate (154.57 g, 1.12 mol, 2.5 eq) and potassium iodide (74.26 g, 447.34 mmol, 1 eq) were added to N-methylpyrrolidone (850 mL), and p-methoxybenzyl chloride (143.62 g, 917.04 mmol, 124.89 mL, 2.05 eq) was added dropwise slowly. The system slowly released heat to 30 °C. Gas was obviously produced. The mixture was reacted for 1 hour. The reaction solution was poured into 1 L of water, and then 500 mL of methyl tert-butyl ether was added. The mixture was stirred. The layers were separated and the organic phases were collected. The aqueous phase was extracted with methyl tert-butyl ether (500 mL * 2). The organic phases were combined, washed with saturated brine (1 L × 2), dried over anhydrous sodium sulfate, and concentrated under reduced pressure. To the crude product was added 400 mL of petroleum ether. The mixture was slurried for 2 hours, and filtered. The filter cake was rinsed with petroleum ether (100 mL*2). The filtrate was rotary evaporated to dryness to give compound **20-2.** MS m/z =430.0$[M+H]^+$.

**Step 2: Synthesis of intermediate 20-3**

2,2,6,6-Tetramethylpiperidine (39.39 g, 278.87 mmol, 47.34 mL, 3 eq) was added to anhydrous tetrahydrofuran (400 mL). The mixture was cooled down to -10 °C, and the atmosphere was replaced with nitrogen three times. n-Butyl lithium (2.5 M, 111.55 mL, 3 eq) was added dropwise under nitrogen. The mixture was reacted at -10 °C for 10 minutes, and cooled down to -60 °C. A solution of compound **20-2** (40 g, 92.96 mmol, 1 eq) in anhydrous tetrahydrofuran (100mL) was added dropwise. After the mixture was reacted for 0.5 hours, N,N-dimethylformamide (67.94 g, 929.56 mmol, 71.52 mL, 10 eq) was added quickly. The mixture was reacted for 10 minutes. The reaction solution was added to 500 mL of saturated ammonium chloride. The mixture was extracted with ethyl acetate (200 mL * 2). The organic phases were combined, washed with 500 mL of saturated brine, dried over anhydrous sodium sulfate, and filtered. The filtrate was concentrated. The crude product was slurried with 100 mL of solvent mixture (petroleum ether:methyl tert-butyl ether = 5:1) for 16 hours. The mixture was filtered. The filter cake was rinsed (petroleum ether:methyl tert-butyl ether = 5:1, 50 mL * 2), and then rotary evaporated to dryness to give compound **20-3.** $^1$H NMR (400 MHz, $CDCl_3$) $\delta$ = 10.35 (s, 1H), 7.24 - 7.13 (m, 5H), 6.90 - 6.77 (m, 5H), 4.24 (s, 4H), 3.79 (s, 6H). MS m/z =458.0[M+H]$^+$.

**Step 3: Synthesis of intermediate 20-4**

Compound **20-3** (42 g, 91.64 mmol, 1 eq) was added to N,N-dimethylformamide (210 mL). The atmosphere was replaced with nitrogen three times. Copper iodide (3.49 g, 18.33 mmol, 0.2 eq) was added under nitrogen. The mixture was heated to 80°C. Methyl fluorosulfonyldifluoroacetate (52.82 g, 274.92 mmol, 34.98 mL, 3 eq) was added dropwise. The mixture was heated to 100 °C and reacted for 1 hour. The reaction solution was filtered through a pad of Celite. After the filter cake was rinsed with methyl tert-butyl ether (300 mL * 4), the filtrate was washed sequentially with 1 L of water and 1 L of saturated brine, dried over anhydrous sodium sulfate, and filtered. The filtrate was concentrated to give compound **20-4.** $^1$H NMR (400 MHz, $CDCl_3$) $\delta$ = 10.44 (s, 1H), 7.34 (d, *J* = 8.4 Hz, 1H), 7.17 (d, *J* = 8.0 Hz, 4H), 6.97 (t, *J* = 8.4 Hz, 1H), 6.86 (d, *J* = 8.4 Hz, 4H), 4.39 (s, 4H), 3.80 (s, 6H), MS m/z =448.0[M+H]$^+$.

**Step 4: Synthesis of intermediate 20-5**

Sodium hydride (1.27 g, 31.85 mmol, 60% content, 2.5 eq) was dissolved in tetrahydrofuran (60 mL). The atmosphere was replaced with nitrogen twice, and then the mixture was cooled down to 0 °C. Methyl acetoacetate (3.70 g, 31.85 mmol, 3.42 mL, 2.5 eq) was added. The mixture was stirred for 10 min, and then n-butyl lithium (2.5 M, 12.74 mL, 2.5 eq) was added. The mixture was stirred for another 10 min, and cooled down to -15 °C. A solution of compound **20-4** (5.7 g, 12.74 mmol, 1 eq) in tetrahydrofuran (5 mL) was added. The mixture was stirred for another 30 min. To the reaction solution was added 100 mL of saturated ammonium chloride. The mixture was extracted with ethyl acetate (100 mL*2). The organic phases were combined, dried over anhydrous sodium sulfate, and filtered. The filtrate was concentrated. The residue was purified by column chromatography (petroleum ether:ethyl acetate = 10:1) to give compound **20-5.** MS m/z =586.2[M+Na]$^+$.

**Step 5: Synthesis of intermediate 20-6**

Compound **20-5** (6 g, 10.39 mmol, 1 eq) was dissolved in dichloromethane (60 mL), and then N,N-dimethylformamide dimethyl acetal (2.48 g, 20.78 mmol, 2.76 mL, 2 eq) was added. The mixture was stirred 25 °C for 12 hr, and then cooled down to 0 °C. Boron trifluoride etherate complex (2.65 g, 18.70 mmol, 2.31 mL, 1.8 eq) was added, and the mixture was stirred 25°C for 1 hr. The filtrate was slowly poured into 50 mL of saturated ammonium chloride solution. The mixture was extracted with ethyl acetate (50 mL*2). The organic phase was washed with saturated brine (30 mL), dried over anhydrous sodium sulfate, and filtered. The filtrate was concentrated under reduced pressure. The residue was purified by column (petroleum ether:ethyl acetate = 10:1) to give compound **20-6.** MS m/z =596.1[M+Na]$^+$.

**Step 6: Synthesis of intermediate 20-7**

Compound **20-6** (4.2 g, 7.32 mmol, 1 eq) was dissolved in tetrahydrofuran (40 mL). The mixture was cooled down to -65 °C, and tri-sec-butyl lithium borohydride (1 M, 8.79 mL, 1.2 eq) was added. The mixture was stirred for another 0.5 hr. The reaction was quenched by adding water. The reaction system was slowly poured into 10 mL of saturated ammonium chloride solution. The mixture was extracted with ethyl acetate (10mL*2). The organic phase was washed with saturated brine (20mL), dried over anhydrous sodium sulfate, and filtered. The filtrate was concentrated under reduced pressure to give compound **20-7.** MS m/z =576.2[M+H]$^+$.

**Step 7: Synthesis of intermediate 20-8**

Compound **20-7** (2 g, 3.47 mmol, 1 eq) and S-methylisothiourea sulfate (4.84 g, 17.37 mmol, 5 eq) were dissolved in ethanol (40 mL) and water (5 mL), and then sodium carbonate (1.29 g, 12.16 mmol, 3.5 eq) was added. The mixture was stirred at 50 °C for another 16 hr. To the reaction solution was added 50 mL of water. The mixture was extracted with ethyl acetate (40 mL*2). The organic phase was washed with saturated brine (20 mL), dried over anhydrous sodium sulfate, and filtered. The filtrate was concentrated under reduced pressure to give compound **20-8.** MS m/z =616.1[M+H]$^+$.

**Step 8: Synthesis of intermediate 20-9**

Compound **20-8** (2.25 g, 3.65 mmol, 1 eq) and N,N-diisopropylethylamine (2.83 g, 21.93 mmol, 3.82 mL, 6 eq) were dissolved in dichloromethane (20 mL). The mixture was cooled down to 0 °C, and then trifluoromethanesulfonic anhydride (4.64 g, 16.45 mmol, 2.71 mL, 4.5 eq) was added. The mixture was stirred at 0 °C for another 0.5 hr. The reaction solution was washed sequentially with saturated ammonium chloride (20 mL) and saturated brine (20 mL). The organic phase was dried over anhydrous sodium sulfate, and filtered. The filtrate was concentrated under reduced pressure. The residue was purified by column (petroleum ether:ethyl acetate = 10:1) to give compound **20-9.** MS m/z =748.1[M+H]$^+$.

**Step 9: Synthesis of intermediate 20-10**

Compound **20-9** (0.35 g, 468.10 μmol, 1 eq) and N,N-diisopropylethylamine (302.50 mg, 2.34 mmol, 407.68 μL, 5 eq) were dissolved in N,N-dimethylformamide (3 mL), and then **1-1A** (248.43 mg, 1.17 mmol, 2.5 eq) was added. The mixture was stirred at 50 °C for another 0.5 hr. To the reaction solution was added 25 mL of ethyl acetate. The mixture was washed sequentially with saturated ammonium chloride (25 mL) and saturated brine (25 mL*2). The organic phase was dried over anhydrous sodium sulfate, and filtered. The filtrate was concentrated. The residue was purified by column (petroleum ether:ethyl acetate = 10:1) to give compound **20-10.** $^1$H NMR (400 MHz, $CDCl_3$) δ 7.24-7.27 (m, 1H),7.14 -7.17(m, 4H), 6.81-6.84 (m, 5H), 5.13-5.16 (m, 1H), 4.37-4.26 (m, 6H), 3.89-3.79 (m, 8H), 3.46-3.39 (m, 3H), 2.97-3.07 (m, 2H), 2.52 (s, 3H), 1.93-1.99 (m, 3H), 1.64-1.68 (m, 2H), 1.50 (s, 9H). MS m/z =810.3[M+H]$^+$.

**Step 10: Synthesis of intermediate 20-11**

Compound **20-10** (300 mg, 370.41 μmol, 1 eq) was dissolved in dichloromethane (3 mL), and then m-chloroperoxybenzoic acid (97.76 mg, 481.54 μmol, 85% content, 1.3 eq) was added. The mixture was stirred at 15°C for another 0.5 hr. The reaction solution was diluted with 20 mL of dichloromethane. The mixture was washed sequentially with 5% sodium thiosulfate (10 mL), saturated sodium bicarbonate (10 mL) and saturated brine (10 mL), dried over anhydrous sodium sulfate, and filtered. The filtrate was concentrated under reduced pressure. The residue was purified by column (petroleum ether:ethyl acetate = 5:1) to give compound **20-11.** MS m/z =826.3[M+H]$^+$.

**Step 11: Synthesis of intermediate 20-12**

**1-2A** (77.10 mg, 484.31 μmol, 2.5 eq) was dissolved in tetrahydrofuran (3 mL). The mixture was cooled down to -15 °C, and then sodium tert-butoxide (37.24 mg, 387.45 μmol, 2 eq) was added. The mixture was stirred at -15 °C for another 0.5 hr, and then a solution of compound **20-11** (160 mg, 193.73 μmol, 1 eq) in tetrahydrofuran (1 mL) was added. The mixture was stirred for another 1 hr. The reaction solution was slowly poured into 20 mL of saturated ammonium chloride solution. The mixture was extracted with ethyl acetate (15 mL*2). The organic phase was washed with saturated brine (20 mL), dried over anhydrous sodium sulfate, and filtered. The filtrate was concentrated under reduced pressure. The residue was purified by column (dichloromethane:methanol=50:1) to give compound **20-12.** MS m/z =921.4[M+H]$^+$.

**Step 12: Synthesis of compound 20 hydrochloride, compound 20A and compound 20B**

Compound **20-12** (0.11 g, 119.43 μmol, 1 eq) was dissolved in dichloromethane (4 mL), and then trifluoroacetic acid (1.36 g, 11.94 mmol, 884.31 μL, 100 eq) was added. The mixture was stirred at 15 °C for 5 hr. The reaction solution was slowly poured into 10 mL of water. The layers were separated. The aqueous phase was adjusted to a pH of 9 with saturated sodium bicarbonate, and extracted with ethyl acetate (15 mL*2). The organic phases were combined, dried over anhydrous sodium sulfate, and filtered. The filtrate was concentrated under reduced pressure. The residue was separated by pre-HPLC (column: Phenomenex Luna 80*30mm*3μm; mobile phase: [water (0.05% hydrochloric acid)-acetonitrile]; (acetonitrile)%: 5%-25%, 8min) to give compound **20** hydrochloride. Then compound **20** hydrochloride was subjected to chiral separation (column: DAICEL CHIRALCEL OD (250mm*30mm, 10μm); mobile phase: [0.1% ammonia water ethanol]; (ethanol)%: 40%-40%, 12min) to give compound **20A** (Rt = 3.920) and compound **20B** (Rt = 4.275).

Compound **20A:** $^1$H NMR (400 MHz, $CDCl_3$) δ 7.27-7.29 (m, 2H), 6.75-6.79(m, 1H), 5.12-5.33 (m, 2H), 4.75-4.79 (m, 2H), 3.88-4.10 (m, 5H), 2.96-3.58 (m, 12H), 2.15-2.28 (m, 3H), 1.87-1.96(m, 5H). MS m/z =581.2[M+H]$^+$.

Compound **20B:** $^1$H NMR (400 MHz, $CDCl_3$) δ = 7.30 - 7.27 (m, 1H), 6.77 (t, *J* = 8.4 Hz, 1H), 5.34 - 5.17 (m, 1H), 5.15 - 5.07 (m, 1H), 4.80 - 4.71 (m, 2H), 4.17 - 4.06 (m, 3H), 3.97 - 3.86 (m, 2H), 3.59 (s, 2H), 3.49 - 3.08 (m, 6H), 3.06 - 2.89 (m, 3H), 2.31- 2.10 (m, 3H), 2.06 - 1.65 (m, 7H), MS m/z =581.2[M+H]$^+$.

**Example 21**

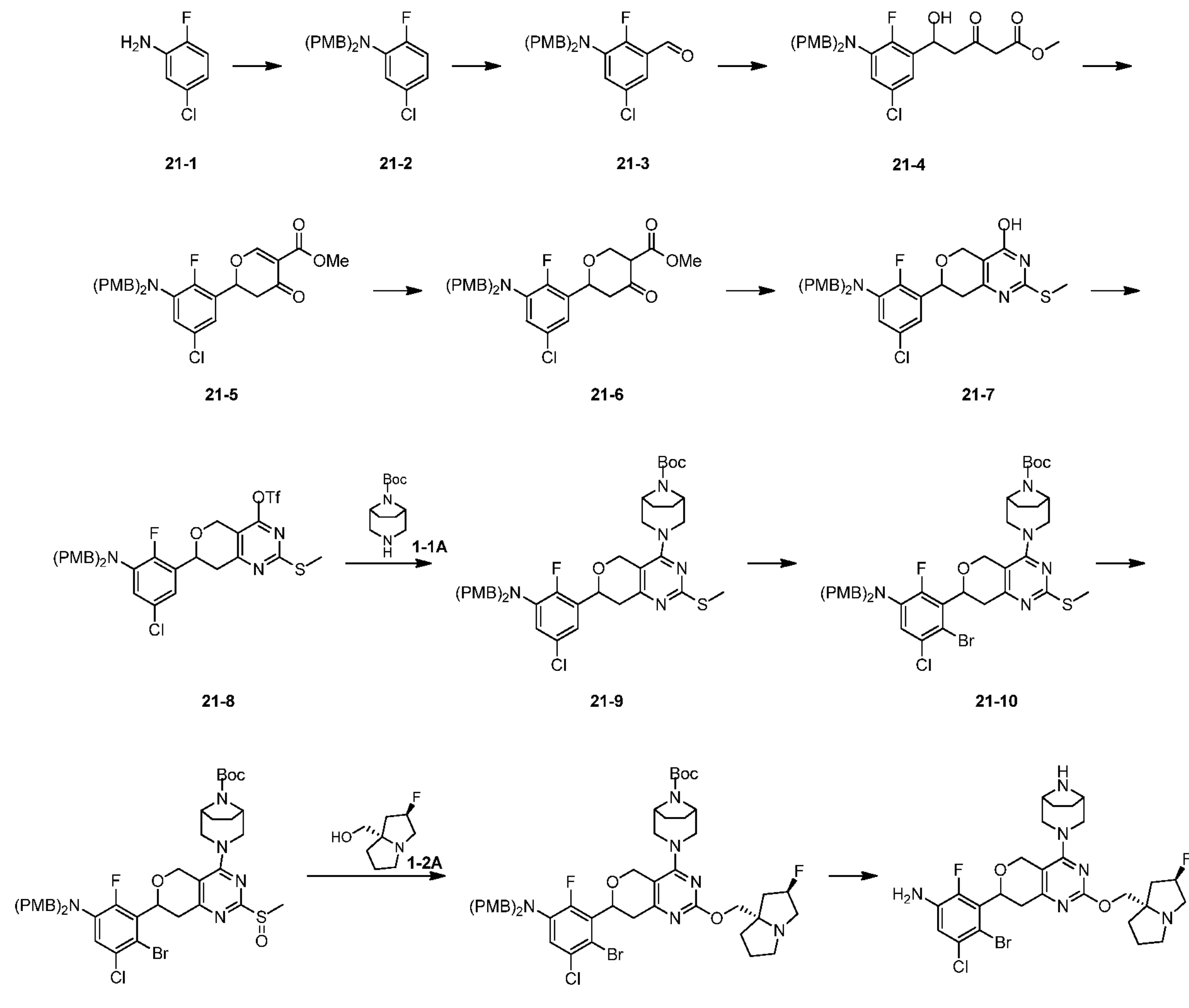


21-1 21-2 21-3 21-4

21-5 21-6 21-7

21-8 21-9 21-10

21-11 21-12 21

**Step 1: Synthesis of intermediate 21-2**

To a pre-dried reaction flask were added compound **21-1** (20 g, 137.40 mmol, 1 eq), N,N-dimethylformamide (200 mL), potassium iodide (22.81 g, 137.40mmoL, 1 eq) and anhydrous potassium carbonate (47.47 g, 343.50 mmol, 2.5 eq). p-Methoxybenzyl chloride (44.11 g, 281.67 mmol, 38.36 mL, 2.05 eq) was added under stirring, and then the mixture was heated to 65 °C and stirred for 4 hr. The reaction solution was cooled down to room temperature, and then filtered through a pad of Celite. The filter cake was rinsed with 200 mL of methyl tert-butyl ether, and then the filtrare was added to 200 mL of water for extraction. The aqueous phase was extracted with ethyl acetate (100 mL*2). The organic phases were combined, washed with saturated brine (200 mL*3), dried over anhydrous sodium sulfate, and

filtered. The filtrare was concentrated to give a crude product. The crude product was slurried with 50 mL of petroleum ether for 48 hr. The mixture was filtered. The filter cake was collected, and dried to give compound **21-2,** which was directly used in the next step. $^1$H NMR (400 MHz, DMSO-$d_6$) δ = 7.18 - 7.17 (m, 5H), 6.85 - 6.82 (m, 6H), 4.24 (s, 4H), 3.74 - 3.71 (m, 6H). MS m/z =386.1[M+H]$^+$.

**Step 2: Synthesis of intermediate 21-3**

2,2,6,6-Tetramethylpiperidine (43.93 g, 311.00 mmol, 52.80 mL, 4 eq) was dissolved in tetrahydrofuran (300 mL), and then the mixture was cooled down to -5 °C. n-Butyl lithium (2.5 M, 124.40 mL, 4 eq) was added. The mixture was stirred for 0.5 hr, and then cooled down to -60 °C. A solution of compound **21-2** (30 g, 77.75 mmol, 1 eq) in tetrahydrofuran (30 mL) was added. The mixture was stirred for 0.5 hr, and then N,N-dimethylformamide (113.66 g, 1.55 moL, 119.64 mL, 20 eq) was added. The mixture was stirred for another 0.5 hr. To the reaction solution was added 200 mL of saturated ammonium chloride. The mixture was extracted with ethyl acetate (200 mL). The layers were separated. The organic phase was washed with 100 mL of saturated brine, dried over anhydrous sodium sulfate, and filtered. The filtrare was concentrated. The residue was purified by column (petroleum ether:ethyl acetate = 10:1) to give compound **21-3.**

**Step 3: Synthesis of intermediate 21-4**

Sodium hydride (6.38 g, 159.47 mmol, 60% content, 2.2 eq) was dissolved in tetrahydrofuran (300 mL). The atmosphere was replaced with nitrogen twice, and then the mixture was cooled down to 0 °C. Methyl acetoacetate (18.52 g, 159.47 mmol, 17.15 mL, 2.2 eq) was added. The mixture was stirred for 10 min, and then n-butyl lithium (2.5 M, 63.79 mL, 2.2 eq) was added. The mixture was stirred for another 10 min, and cooled down to -15 °C. A solution of compound **21-3** (30 g, 72.49 mmol, 1 eq) in tetrahydrofuran (50 mL) was added. The mixture was stirred for another 30 min. To the reaction solution was added 100 mL of saturated ammonium chloride. The mixture was extracted with ethyl acetate (100 mL*2). The organic phases were combined, dried over anhydrous sodium sulfate, and filtered. The filtrate was concentrated. The residue was purified by column (petroleum ether:ethyl acetate = 10:1) to give compound **21-4.** MS m/z =530.2[M+H]$^+$.

**Step 4: Synthesis of intermediate 21-5**

Compound **21-4** (20 g, 37.74 mmol, 1 eq) was dissolved in anhydrous dichloro (50 mL), and N,N-dimethylformamide dimethyl acetal (5.40 g, 45.28 mmoL, 6.02 mL, 1.2 eq) was added under nitrogen. The mixture was reacted at 25 °C for 16 hr. Boron trifluoride etherate (6.43 g, 45.28 mmol, 5.59 mL, 1.2 eq) was added, and the mixture was reacted at 20 °C for 1 hr. The reaction solution was added to 50 mL of saturated sodium bicarbonate solution. The mixture was extracted with dichloromethane (20 mL*2), and the layers were separated. The organic phases were combined, washed with 30 mL of saturated brine, dried over anhydrous sodium sulfate, and filtered. The filtrate was concentrated. The crude product was purified by column (petroleum ether:ethyl acetate=10:1-1:1) to give compound **21-5.** $^1$H NMR (400 MHz, $CDCl_3$) δ = 8.45 (s, 1H), 7.14 (m, 4H), 7.00 - 6.69 (m, 6H), 5.80 (m, 1H), 4.27 - 4.10 (m, 5H), 3.79 (m, 1H), 3.94 - 3.66 (m, 8H), 2.98 - 2.73 (m, 1H). MS m/z =540.2[M+H]$^+$.

**Step 5: Synthesis of intermediate 21-6**

Compound **21-5** (12 g, 22.22 mmol, 1 eq) was added to anhydrous tetrahydrofuran (30 mL), and tri-sec-butyl lithium borohydride (11.83 g, 62.22 mmol, 13.60 mL, 2.8 eq) was added under nitrogen. The mixture was reacted at -60 °C for 1 hr. The reaction solution was added to 40 mL of water. The mixture was extracted with ethyl acetate (20 mL*2). The organic phases were combined, washed with 20 mL of saturated brine, dried over anhydrous sodium sulfate, and concentrated. The crude product was purified by column (petroleum ether:ethyl acetate=100:0=3:1) to give compound **21-6.** MS m/z =542.2[M+H]$^+$.

**Step 6: Synthesis of intermediate 21-7**

Compound **21-6** (5.5 g, 10.15 mmol, 1 eq) was added to ethanol (15 mL) and water (3 mL), and sodium bicarbonate (2.15 g, 20.30 mmol, 2 eq) and methylisothiourea sulfate (2.74 g, 30.44 mmol, 3 eq) were added. The mixture was reacted at 45-50 °C for 16 hr. The reaction solution was extracted with water (20 mL) and ethyl acetate (20 mL*2). The organic phases were combined, washed with saturated brine (20 mL*2), dried over anhydrous sodium sulfate, and concentrated to give a crude product of compound **21-7.** The crude product was directly used for the next step. MS m/z =582.2[M+H]$^+$.

**Step 7: Synthesis of intermediate 21-8**

Compound **21-7** (6 g, 8.04 mmol, 1 eq) was added to anhydrous dichloromethane (20 mL), and N,N-diisopropylethylamine (3.12 g, 24.12 mmol, 4.20 mL, 3 eq) was added at 0°C. The mixture was cooled down to 0-10 °C. Trifluoromethanesulfonic anhydride (4.08 g, 14.47 mmol, 2.39 mL, 1.8 eq) was added dropwise slowly. The mixture was reacted at 0 °C for 0.5 hr. The reaction solution was added to 20 mL of saturated ammonium chloride. The mixture was extracted with anhydrous dichloromethane (10 mL*2). The organic phases were combined, washed with 20 mL of saturated brine, dried over anhydrous sodium sulfate, and filtered. The filtrate was concentrated. The crude product was purified by column (petroleum ether:ethyl acetate=1:0-0:1) to give compound **21-8.** MS m/z =714.1[M+H]$^+$.

**Step 8: Synthesis of intermediate 21-9 hydrochloride**

Compound **21-8** (0.8 g, 1.12 mmoL, 1 eq), compound **1-1A** (475.62 mg, 2.24 mmol, 2 eq), and DIPEA (434.33 mg, 3.36 mmoL, 585.35 μL, 3 eq) were added to N,N-dimethylformamide (5 mL). The mixture was reacted at 50 °C for 1 hr. The reaction solution was added to saturated ammonium chloride (20mL). The mixture was extracted with ethyl acetate (20mL*2). The organic phases were combined, washed with 20mL of saturated brine, dried over anhydrous sodium sulfate, and concentrated. The crude product was purified by column (petroleum ether:ethyl acetate=3:1), and then the product was separated by preparative HPLC (column: Phenomenex Luna C18 250*50mm*10 μm; mobile phase: [water (0.05% hydrochloric acid)-acetonitrile]; (acetonitrile)%: 65%-95%, 10min) to give compound **21-9** hydrochloride. MS m/z =776.3[M+H]$^+$.

**Step 9: Synthesis of intermediate 21-10**

Compound **21-9** hydrochloride (1.2 g) was dissolved in N,N-dimethylformamide (5 mL). The mixture was cooled down to 0 °C, and then N-bromosuccinimide (330.13 mg, 1.85 mmol, 1.2 eq) was added. The mixture was stirred at 20 °C for 1 hr. To the reaction solution was added 30 mL of water. The mixture was extracted with ethyl acetate (30 mL*2). The organic phases were combined, washed with saturated brine (20 mL*2), dried over anhydrous sodium sulfate, and filtered. The filtrate was concentrated. The residue was purified by column (petroleum ether:ethyl acetate = 3:1-1:1) to give compound **21-10.** MS m/z =856.1[M+H]$^+$.

**Step 10: Synthesis of intermediate 21-11**

Dichloromethane (10 mL) was added to a dry reaction flask, and then compound **21-10** (0.3 g, 350.8 μmol, 1 eq) was added and stirred. Then m-chloroperoxybenzoic acid ((213.6 mg, 1052.3 μmoL, 85% content, 3 eq) was added, and the reaction system was stirred at 25 °C for 1 hour. The reaction solution was diluted with 10 mL of dichloromethane. The mixture was washed twice with 5mL of 5% sodium thiosulfate solution and 10mL of saturated brine, dried over anhydrous sodium sulfate, and filtered. The filtrate was concentrated under reduced pressure to give a crude product. The crude product was separated by preparative HPLC (column: Phenomenex Luna 80*30mm*3μm; mobile phase: [water (0.05% hydrochloric acid)-acetonitrile]; (acetonitrile)%: 50%-80%, 8min) give compound **21-11.** MS m/z =872.1[M+H]$^+$.

**Step 11: Synthesis of intermediate 21-12 hydrochloride**

Compound **1-2A** (274.09 mg, 1.72 mmol, 5 eq) was added to anhydrous tetrahydrofuran (10 mL), and sodium tert-butoxide (148.91 mg, 1.55 mmol, 4.5 eq) was added. The mixture was reacted at -15 °C for 30 min. Compound **21-11** (0.3 g, 344.33 μmol, 1 eq) was added, and the mixture was reacted at -15 °C for 1 hr. To the reaction solution was added 5 mL of saturated ammonium chloride solution. Ethyl acetate (5 mL*2) was added for extraction. The organic phases were combined, washed with saturated brine (5 mL*2), dried over anhydrous sodium sulfate, and concentrated. The residue was purified by preparative HPLC (column: Phenomenex Luna C18 80*40mm*3 μm; mobile phase: [water (0.05% hydrochloric acid)-acetonitrile]; (acetonitrile)%: 46%-66%, 7min) to give compound **21-12** hydrochloride. MS m/z =967.3[M+H]$^+$.

**Step 12: Synthesis of compound 21 hydrochloride**

Compound **21-12** hydrochloride (90.00 mg) was added to a slolution of trifluoroacetic acid (2.12 g, 18.63 mmol, 1.38 mL, 200 eq) in anhydrous dichloro (7 mL). The mixture was reacted at -10-0 °C for 1 hr. The product was poured into 10 mL of water. The mixture was extracted with ethyl acetate (5 mL*2). The organic phases were combined, washed with saturated brine (5 mL*2), dried over anhydrous sodium sulfate, and concentrated. The crude product was separated

by preparative HPLC (column: Phenomenex Luna 80*30mm*3μm; mobile phase: [water (0.05% hydrochloric acid)-acetonitrile]; (acetonitrile)%: 15%-35%, 8min) to give compound **21** hydrochloride. MS m/z =627.1[M+H]$^+$.

**Example 22**

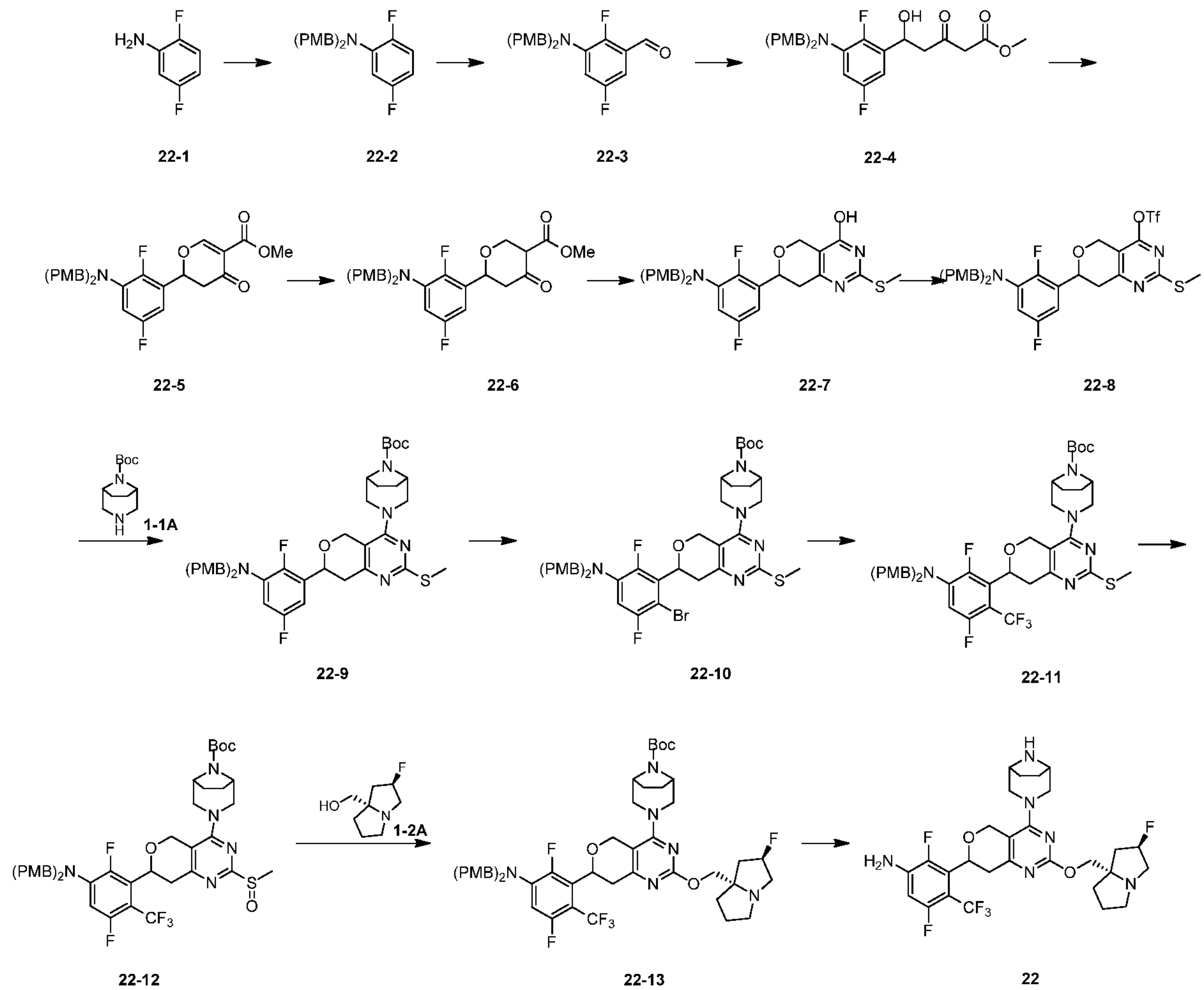


22-1, 22-2, 22-3, 22-4, 22-5, 22-6, 22-7, 22-8, 22-9, 22-10, 22-11, 22-12, 22-13, 22

**Step 1: Synthesis of intermediate 22-2**

Compound **22-1** (50 g, 387.28 mmol, 1 eq), potassium iodide (64.29 g, 387.28 mmol, 1 eq) and anhydrous potassium carbonate (133.81 g, 968.19 mmol, 2.5 eq) were added to N,N-dimethylformamide (500 mL). p-Methoxybenzyl chloride (121.30 g, 774.55 mmol, 105.48 mL, 2 eq) was added dropwise under stirring. The mixture was reacted at 60 °C for 5 hr. The reaction solution was added to 300 mL of water. The mixture was extracted with ethyl acetate (200 mL*2). The organic phases were combined, washed with 100 mL of saturated brine, dried over anhydrous sodium sulfate, and filtered. The filtrate was concentrated. The crude product was purified by column (petroleum ether:ethyl acetate=1:0-0:1) to give compound **22-2.** MS m/z =370.0[M+H]$^+$.

**Step 2: Synthesis of intermediate 22-3**

2,2,6,6-Tetramethylpiperidine (81.22 g, 574.98 mmol, 97.62 mL, 4 eq) was added to anhydrous tetrahydrofuran (500 mL). The mixture was cooled down to -5 °C, and n-butyl lithium (2.5 M, 229.99 mL, 4 eq) was added dropwise. The mixture was reacted at -5-0 °C for 15 minutes, and cooled down to -60 °C. A solution of compound **22-2** (59 g, 143.75 mmol, 1 eq) in tetrahydrofuran (50 mL) was added, and the mixture was reacted at -60 °C for 0.5 hours. N,N-dimethylformamide (210.13 g, 2.87 mol, 221.19 mL, 20 eq) was added quickly, and the mixture was reacted at -60 °C for 10min. The reaction solution was poured into 300mL of saturated ammonium chloride. The mixture was extracted with methyl

tert-butyl ether (100mL*2). The organic phases were combined, washed with 100mL of saturated brine, dried over anhydrous sodium sulfate, and filtered. The filtrate was concentrated. The crude product was purified by column (petroleum ether:ethyl acetate =10:1) to give compound **22-3.** MS m/z =398. 1[M+H]$^+$.

**Step 3: Synthesis of intermediate 22-4**

NaH (5.31 g, 132.86 mmol, 60% content, 2.2 eq) and anhydrous tetrahydrofuran (150 mL) were reacted at 0 °C for 0.5 hr under nitrogen. Methyl acetoacetate (15.43 g, 132.86 mmol, 14.28 mL, 2.2 eq) was added dropwise, and the mixture was reacted at 0 °C for 0.5 hr. n-BuLi (2.5 M, 53.14 mL, 2.2 eq) was added dropwise. The mixture was reacted at 0 °C for 0.5 hr, and cooled down to -50 °C. A solution of compound **22-3** (24 g, 60.39 mmol, 1 eq) in anhydrous tetrahydrofuran (50 mL) was added dropwise, and the mixture was reacted at -50 °C for 0.5 hr. To the reaction solution was added 80mL of saturated ammonium chloride solution. The mixture was extracted with ethyl acetate (50mL*2). The organic phases were combined, washed with 100mL of saturated brine, dried over anhydrous sodium sulfate, and concentrated. The crude product was purified by column (petroleum ether:ethyl acetate = 1:0-3:1) to give compound **22-4.** $^1$H NMR (400 MHz, $CDCl_3$) $\delta$ = 7.18 - 7.16 (m, 4H), 6.84 - 6.82 (m, 4H), 6.76 (m, 1H), 6.48 (m 1H), 5.52 - 5.44 (m, 1H), 4.21 (s, 4H), 3.88 - 3.67 (m, 9H), 3.53 (s, 2H), 3.30 (d, *J* = 4Hz, 1H), 3.01 - 2.88 (m, 2H). MS m/z =514.2[M+H]$^+$.

**Step 4: Synthesis of intermediate 22-5**

Compound **22-4** (12 g, 23.37 mmol, 1 eq) was dissolved in anhydrous dichloro (50 mL), and N,N-dimethylformamide dimethyl acetal (4.18 g, 35.05 mmol, 4.66 mL, 1.5 eq) was added under nitrogen. The mixture was reacted at 25 °C for 16 hr. Boron trifluoride etherate (6.63 g, 46.74 mmol, 5.77 mL, 2 eq) was added, and the mixture was reacted at 20 °C for 1 hr. The reaction solution was added to 20 mL of saturated sodium bicarbonate solution. The layers were separated. The aqueous phase was further extracted with 20 mL of dichloromethane. The organic phases were combined, washed with 20 mL of saturated brine, dried over anhydrous sodium sulfate, and filtered. The filtrate was concentrated. The crude product was purified by column (petroleum ether:ethyl acetate = 1:0-3:1) to give compound **22-5.** MS m/z =524.2[M+H]$^+$.

**Step 5: Synthesis of intermediate 22-6**

Compound **22-5** (10.5 g, 20.06 mmol, 1 eq) was added to anhydrous tetrahydrofuran (30 mL), and tri-sec-butyl lithium borohydride (1 M, 22.06 mL, 1.1 eq) was added under nitrogen. The mixture was reacted at -60 °C for 1 hr. The reaction mixture was added to 30 mL of dilute hydrochloric acid, and the mixture was extracted with ethyl acetate (10 mL*2). The layers were separated. The organic phases were combined, washed with 20 mL of saturated brine, dried over anhydrous sodium sulfate, and concentrated. The crude product was purified by column (petroleum ether:ethyl acetate=1:0-3:1) to give compound **22-6.** MS m/z =526.2[M+H]$^+$.

**Step 6: Synthesis of intermediate 22-7**

Compound **22-6** (4 g, 7.61 mmol, 1 eq) was added to ethanol (16 mL) and water (4 mL), and sodium bicarbonate (1.61 g, 15.22 mmol, 2 eq) and methylisothiourea sulfate (2.06 g, 22.83 mmol, 3 eq) were added. The mixture was reacted at 45-50 °C for 16 hr. The reaction mixture was added to 10 mL of water. The mixture was extracted with ethyl acetate (10 mL*2). The organic phases were combined, washed with saturated brine (10 mL*2), dried over anhydrous sodium sulfate, and concentrated to give a crude product of compound **22-7.** The crude product was directly used in the next step. MS m/z =566.2[M+H]$^+$.

**Step 7: Synthesis of intermediate 22-8**

Compound **22-7** (4.8 g, 4.67 mmol, 1 eq) was added to anhydrous dichloromethane (20 mL), and trifluoromethanesulfonic anhydride (1.98 g, 7.00 mmol, 1.16 mL, 1.5 eq) was added. The mixture was cooled down to 0-10 °C. N,N-Diisopropylethylamine (1.81 g, 14.00 mmol, 2.44 mL, 3 eq) was added dropwise slowly. The mixture was reacted at 0 °C for 0.5 hr. The reaction solution was added to 20mL of saturated ammonium chloride. The layers were separated. The organic phase was washed with saturated brine (5mL*2), dried over anhydrous sodium sulfate, and filtered. The filtrate was concentrated. The crude product was purified by column (petroleum ether:ethyl acetate = 1:0-5:1) to give compound **22-8.** $^1$H NMR (400 MHz, $CDCl_3$) $\delta$ = 7.17 - 7.15 (m, 4H), 6.86 - 6.83 (m, 4H), 6.75 - 6.72 (m, 1H), 6.54 - 6.50 (m, 1H), 5.09 - 5.00 (m, 2H), 4.83 - 4.79 (m, 1H), 4.23 - 4.20 (m, 5H), 3.82 - 3.80 (m, 7H), 2.56 (s, 3H). MS m/z =698.1[M+H]$^+$.

**Step 8: Synthesis of intermediate 22-9**

Compound **22-8** (3.7 g, 5.30 mmol, 1 eq), compound **1-1A** (2.25 g, 10.61 mmol, 2 eq) and N,N-diisopropylethylamine (2.06 g, 15.91 mmol, 2.77 mL, 3 eq) were added to N,N-dimethylformamide (20mL). The mixture was reacted at 50 °C for 1 hr. The reaction solution was added to 20 mL of saturated ammonium chloride. The mixture was extracted with ethyl acetate (20 mL*2). The organic phases were combined, washed with 20 mL of saturated brine, dried over anhydrous sodium sulfate, and concentrated. The crude product was purified by column (petroleum ether:ethyl acetate=3:1) to give compound **22-9.** $^1H$ NMR (400 MHz, $CDCl_3$) $\delta$ = 7.17-7.15 (m, 4H), 6.85-6.83 (m, 4H), 6.72-6.70 (m, 1H), 6.50-6.46 (m, 1H), 5.11-5.07 (m, 1H), 4.83 - 4.71 (m, 2H), 4.32 - 4.23 (m, 6H), 3.80 (s, 6H), 3.43 - 2.84 (m, 5H), 2.51 (s, 3H), 2.01 - 1.93 (m, 3H), 1.69 - 1.66 (m, 2H), 1.50 (s, 9H). MS m/z =760.3$[M+H]^+$.

**Step 9: Synthesis of intermediate 22-10**

Compound **22-9** (0.6 g, 789.58 μmol, 1 eq) was dissolved in N,N-dimethylformamide (10 mL), and N-bromosuccinimide (98.37 mg, 552.70 μmol, 0.7 eq) was added. The mixture was reacted at 0-10 °C for 1 hr. 20 mL of water was added to the reaction solution. The mixture was extracted with ethyl acetate (10 mL*2). The organic phases were combined, washed with saturated brine (10 mL*2), dried over anhydrous sodium sulfate, and concentrated. The residue was separated by preparative thin layer chromatography (petroleum ether:ethyl acetate=1:1) to give compound **22-10.** MS m/z =838.2$[M+H]^+$.

**Step 10: Synthesis of intermediate 22-11**

Compound **22-10** (0.3 g, 357.65 μmol, 1 eq), methyl fluorosulfonyldifluoroacetate (343.55 mg, 1.79 mmol, 227.52 μL, 5 eq) and cuprous iodide (136.23 mg, 715.31 μmol, 2 eq) were dissolved in N,N-dimethylformamide (10 mL). The atmosphere was replaced with nitrogen three times, and reacted at 100 °C for 2 hours under nitrogen. The reaction solution was poured into 10 mL of water. The mixture was extracted with methyl tert-butyl ether (10 mL*2). The organic phases were combined, washed with 10 mL of saturated brine, dried over anhydrous sodium sulfate, and concentrated. The residue was purified by preparative HPLC (column: Phenomenex luna C18 (250*70mm, 15 μm); mobile phase: [water (0.05% hydrochloric acid)-acetonitrile]; (acetonitrile)%: 50%-98%, 20min). The separated solution was adjusted to a pH of 7-8 with saturated sodium bicarbonate solution. Then the mixture was extracted with ethyl acetate, washed with saturated brine, dried over anhydrous sodium sulfate, and filtered. The filtrate was concentrated to give compound **22-11.** MS m/z =828.3$[M+H]^+$.

**Step 11: Synthesis of intermediate 22-12**

Dichloromethane (10 mL) was added to a dry reaction flask, and then compound **22-11** (130 mg, 157.02 μmol, 1 eq) was added and stirred. Then m-chloroperoxybenzoic acid (22.32 mg, 109.92 μmol, 85% content, 0.7 eq) was added, and the reaction system was stirred at 25 °C for 1 hour. The reaction solution was diluted with 10 mL of dichloromethane. The mixture was washed twice with 5 mL of 5% sodium thiosulfate solution and 10 mL of saturated brine, dried over anhydrous sodium sulfate, and filtered. The filtrate was concentrated under reduced pressure to give a crude product. The crude product was separated by preparative thin layer chromatography (dichloromethane:methanol=10:1) to give compound **22-12.** $^1H$ NMR (400 MHz, $CDCl_3$) $\delta$ = 7.17 - 7.10 (m, 4H), 6.87 - 6.83 (m, 4H), 6.58-6.53 (m, 1H), 5.21 - 5.10 (m, 1H), 4.87 - 4.75(m, 2H), 4.44 - 4.22 (m, 6H), 3.80 (s, 6H), 3.58 - 3.44 (m, 3H), 3.20 - 3.14 (m, 2H), 2.97 (m, 4H), 2.05 - 1.92 (m, 4H), 1.49 (s, 9H), MS m/z =844.3$[M+H]^+$.

**Step 12: Synthesis of intermediate 22-13**

Compound **1-2A** (226.38 mg, 1.42 mmol, 20 eq) was added to anhydrous tetrahydrofuran (10 mL), and sodium tert-butoxide (109.32 mg, 1.14 mmol, 16 eq) was added. The mixture was reacted at -15 °C for 15 min. Compound **22-12** (60 mg, 71.10 μmol, 1 eq) was added, and the mixture was reacted at -15 °C for 1 hr. 5mL of saturated ammonium chloride was added to the reaction solution. After the reaction solutions were combined, the mixture was extracted with ethyl acetate (10mL*3). The organic phases were combined, washed with saturated brine (20mL*2), dried over anhydrous sodium sulfate, and filtered. The filtrate was concentrated. The residue was separated by preparative thin layer chromatography (dichloromethane:methanol=10:1) to give compound **22-13.** MS m/z =939.5$[M+H]^+$.

**Step 13: Synthesis of compound 22 hydrochloride**

Compound **22-13** (60.00 mg, 63.90 μmol, 1 eq) was added to a solution of trifluoroacetic acid (1.46 g, 12.78

mmol, 946.19 μL, 200 eq) in anhydrous dichloro (5 mL). The mixture was reacted at -10-0 °C for 1 hr. The product was poured into 10 mL of water. The aqueous phase was extracted with ethyl acetate (5 mL*2). The organic phases were combined, washed with saturated brine (5 mL*2), dried over anhydrous sodium sulfate, and concentrated. The residue was purified by preparative HPLC (column: Phenomenex luna C18 80*40mm*3 μm; mobile phase: [water (0.05% hydrochloric acid)-acetonitrile]; (acetonitrile)%: 1%-30%, 7min) to give compound **22** hydrochloride. $^1$H NMR (400 MHz, CDsOD) δ = 6.65-6.60 (m, 1H), 6.74 - 6.53 (m, 1H), 5.66 - 5.53 (m, 1H), 5.22 - 5.19 (m, 1H), 4.99-4.96 (m, 3H), 4.25-4.18 (m, 2H), 4.04 - 3.87 (m, 5H), 3.55 - 3.38 (m, 3H), 3.08 - 2.73 (m, 1H), 2.67 - 1.87 (m, 11H). MS m/z =599.2[M+H]$^+$.

**Example 23**

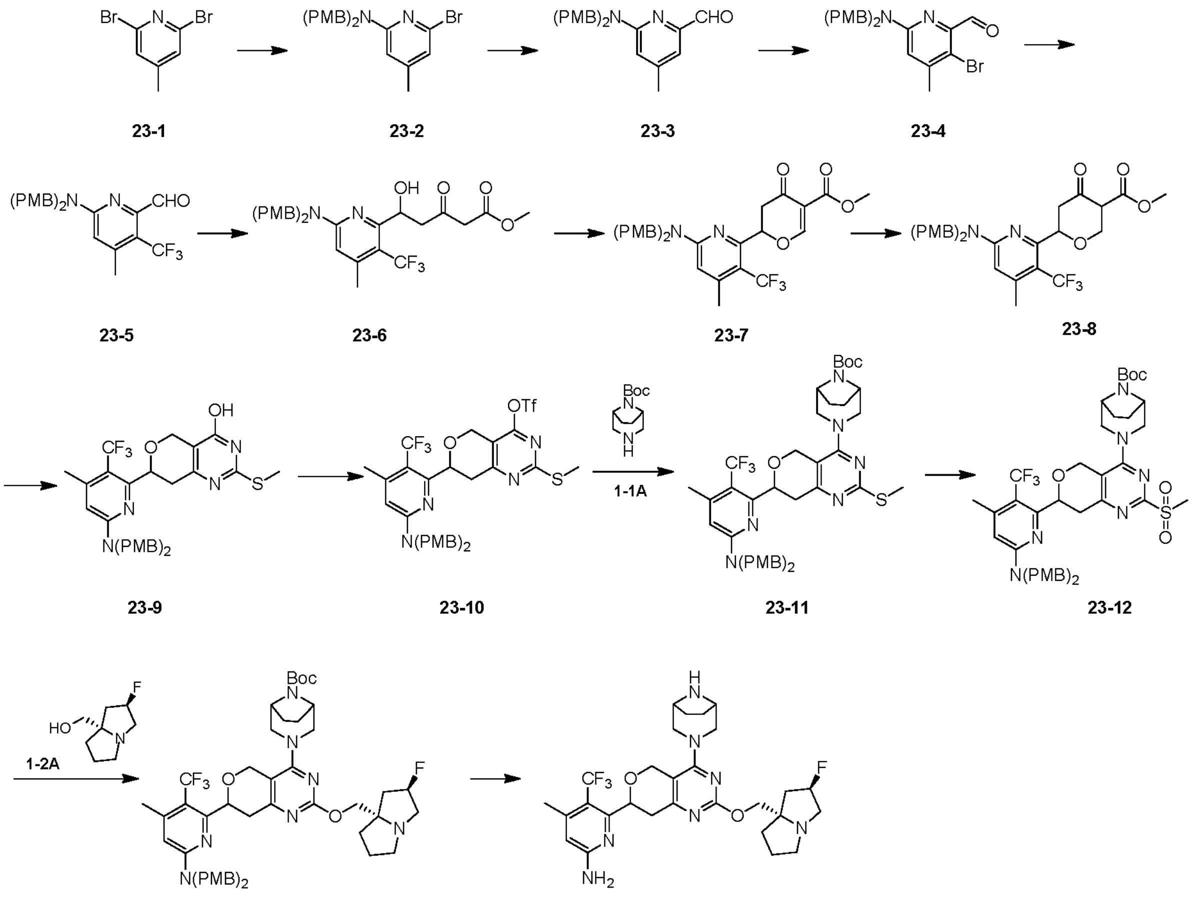


23-1, 23-2, 23-3, 23-4, 23-5, 23-6, 23-7, 23-8, 23-9, 23-10, 23-11, 23-12, 23-13, 23

**Step 1: Synthesis of intermediate 23-2**

Compound **23-1** (1.2g, 4.78mmol, 1eq) and bis-(4-methoxybenzyl)-amine (2.46g, 9.56mmol, 2eq) were added to N-methylpyrrolidone (30mL). The mixture was reacted with microwave at 200 °C for 1 hour. The reaction solution was diluted with 250 mL of ethyl acetate, and then the mixture was washed with water (20 mL×3) and 20 mL of saturated brine. The organic phase was dried and filtered to remove the desiccant. The solvent was removed from the filtrate under reduced pressure to give a crude product. The crude product was purified by column (ethyl acetate/petroleum ether=0~35%) to give compound **23-2.** MS m/z=428.6 [M+H] $^+$.

**Step 2: Synthesis of intermediate 23-3**

Compound **23-2** (4g, 9.36mmol, 1eq) was dissolved in anhydrous tetrahydrofuran (20mL). The mixture was cooled down to -78 °C under nitrogen, and then n-butyl lithium (2.5M, 6.74mL, 1.1 eq) was added dropwise. After completion of the addition, the mixture was reacted with stirring for 1 hour, and then N,N-dimethylformamide (2.05 g, 28.08 mmol, 3eq) was added dropwise. After completion of the addition, the mixture was reacted with stirring for 0.5

hours. The reaction was quenched with 10 mL of saturated ammonium chloride and 20 mL of water, and then the mixture was extracted with ethyl acetate (50 mL×2). The organic phases were combined, and rotary evaporated to dryness to give a crude product. The crude product was purified by column (ethyl acetate/petroleum ether=0-15%) to give compound **23-3.** $^1$H-NMR (400MHz, $CDCl_3$) δ: 9.93 (s, 1H), 7.23 (s, 1H), 7.15 (d, *J* = 8.8Hz, 4H), 6.86 (d, *J* = 8.8Hz, 4H), 6.49 (s, 1H), 4.77 (s, 4H), 3.81 (s, 6H), 2.26 (s, 3H).

**Step 3: Synthesis of intermediate 23-4**

Compound **23-3** (2.09 g, 2.66 mmol, 1 eq) was dissolved in DMF (20 mL), and then NBS (988.15 mg, 5.55 mmol, 1 eq) was added. The resulting reaction solution was reacted with stirring at room temperature (20 °C) for 2 hours under nitrogen. The reaction solution was diluted with 60 mL of ethyl acetate, and then the mixture was washed with water (20 mL×3) and 20 mL of saturated brine. The organic phase was rotary evaporated to dryness to give a crude product. The crude product was purified by column (ethyl acetate/petroleum ether=0~25%) to give compound **23-4.**

**Step 4: Synthesis of intermediate 23-5**

Compound **23-4** (1.98 g, 4.34 mmol, 1 eq) and methyl fluorosulfonyldifluoroacetate (4.17 g, 2.17 mmol, 5 eq) were added to DMF (20 mL), and then CuI (205 mg, 1.08mol, 1 eq) was added. The resulting reaction solution was purged with nitrogen, placed in an oil bath (100 °C), and reacted with stirring for 8 hours. The reaction solution was rotary evaporated to dryness to give a crude product. The crude product was purified by column (ethyl acetate/petroleum ether=0-25%) to give compound **23-5.** MS m/z=445.1[M+H]$^+$.

**Step 5: Synthesis of intermediate 23-6**

Sodium hydride (899.91 mg, 22.50 mmol, 60% content, 2 eq) was suspended in anhydrous tetrahydrofuran (50 mL). The mixture was cooled down to 0 °C under nitrogen, and then methyl acetoacetate (2.61 g, 22.50 mmol, 2.42 mL, 2.0 eq) was slowly added dropwise. After completion of the addition, the mixture was stirred for 30 minutes, and then n-butyl lithium (2.5 M, 9.0 mL, 2eq) was added dropwise. After completion of the addition, the mixture was stirred for 30 minutes. The ice bath was removed. The mixture was cooled down to -78 °C. Finally, to the mixture was added dropwise a solution of compound **23-5** (5 g, 11.25 mmol, 1 eq) in anhydrous tetrahydrofuran (10 mL). After completion of the addition, the mixture was reacted with stirring for 1 hour. The reaction was quenched by adding 20 mL of water to the reaction solution, and then the mixture was extracted with ethyl acetate (50 mL×3). The organic phases were combined, and rotary evaporated to dryness to give a crude product. The crude product was purified by column (ethyl acetate/petroleum ether=0~45%) to give compound **23-6.** MS m/z=561.2 [M+H]$^+$.

**Step 6: Synthesis of intermediate 23-7**

Compound **23-6** (2.9 g, 5.17 mmol, 1 eq) and N,N-dimethylformamide dimethyl acetal (1.85 g, 15.52 mmol, 2.06 mL, 3 eq) were added to anhydrous dichloromethane (20 mL). The mixture was reacted with stirring at room temperature (20 °C) for 24 hours, and then cooled down to 0 °C. Finally, to the mixture was added boron trifluoride etherate (734.25 mg, 5.17 mmol, 636.26 μL, 1 eq). After completion of the addition, the mixture was reacted with stirring at 20 °C for 1 hour. The reaction solution was rotary evaporated to dryness to give a crude product. The crude product was purified by column (ethyl acetate/petroleum ether=0~40%) to give compound **23-7.** MS m/z=593.1 [M+Na]$^+$.

**Step 7: Synthesis of intermediate 23-8**

Compound **23-7** (635 mg, 1.11 mmol, 1 eq) was dissolved in anhydrous tetrahydrofuran (5 mL). The mixture was cooled down to -78 °C under nitrogen, and then tri-sec-butyl lithium borohydride (1 M, 1.11 mL, 1eq) was added dropwise. After completion of the addition, the mixture was reacted with stirring for 1 hour. The reacticon was quenched with 1 mL of 1 M hydrochloric acid, and then 20 mL of saturated brine and 50 mL of ethyl acetate were added. The mixture was stirred for 5 minutes. The organic phase was separated out, and rotary evaporated to dryness to give a crude product. The crude product was purified by column (ethyl acetate/petroleum ether=0~25%) to give compound **23-8.** MS m/z=573.2 [M+H]$^+$.

**Step 8: Synthesis of intermediate 23-9**

Compound **23-8** (630 mg, 1.1 mmol, 1eq) and 2-methylthiourea monosulfate (621.31 mg, 3.30mmol, 3eq) were added to anhydrous ethanol (10 mL), and then sodium carbonate (233.24 mg, 2.2mmol, 2eq) was added. The resulting

reaction solution was placed in an oil bath (60 °C) and reacted with stirring for 18 hours. The reaction solution was rotary evaporated to dryness. To the residue were added 5 mL of water and 50 mL of ethyl acetate. The mixture was adjusted to a pH of ~6-7 with 2M hydrochloric acid. The organic phase was separated. The aqueous phase was extracted with 30 mL of ethyl acetate. The organic phases were combined, dried over anhydrous sodium sulfate, and filtered to remove the desiccant. The solvent was removed from the filtrate under reduced pressure to give compound **23-9.** MS m/z=613.2$[M+H]^+$.

**Step 9: Synthesis of intermediate 23-10**

Compound **23-9** (541 mg, 883.63 mmol, 1eq) was dissolved in N,N-dimethylformamide (4 mL), and then $PhNTf_2$ (473.19 mg, 1.32 mmol, 1.5eq) and N,N-diisopropylethylamine (342.38 mg, 2.65 mmol, 461.43 μL, 3 eq) were added. The resulting reaction solution was reacted with stirring at room temperature (20°C) for 1.5 hours. The reaction solution was diluted with 100 mL of ethyl acetate, and then the mixture was washed with water (10 mL×3) and 10 mL of saturated brine. The organic phase was rotary evaporated to dryness to give a crude product. The crude product was purified by column chromatography (ethyl acetate/petroleum ether=0~10%) to give compound **23-10.** MS m/z=745.3 $[M+H]^+$.

**Step 10: Synthesis of intermediate 23-11**

Compound **23-10** (240mg, 322.27 μmol, 1eq) and compound **1-1A** (82.1 mg, 386.72 μmol, 1.3eq) were added to N,N-dimethylformamide (3 mL), and then N,N-diisopropylethylamine (124.95 mg, 966.80 μmol, 168.40 μL, 3 eq) were added. The resulting reaction solution was placed in an oil bath (100 °C) and reacted with stirring for 1 hour. The reaction solution was rotary evaporated to dryness to give a crude product. The crude product was purified by column (ethyl acetate/petroleum ether=0-35%) to give compound **23-11.** MS m/z=807.4$[M+H]^+$.

**Step 11: Synthesis of intermediate 23-12**

Compound **23-11** (210 mg, 260.27 μmol, 1eq) was dissolved in anhydrous dichloromethane (2 mL), and then m-chloroperoxybenzoic acid (105.67 mg, 520.49 μmol, 85% content, 2eq) was added. The resulting reaction solution was reacted with stirring at 20 °C for 2 hours. The reaction solution was rotary evaporated to dryness to give a crude product. The crude product was purified by column (ethyl acetate/petroleum ether=0-45%) to give compound **23-12.** MS m/z=839.3$[M+H]^+$.

**Step 12: Synthesis of intermediate 23-13**

Compound **1-2A** (121.83 mg, 765.26 μmol, 3eq) and sodium tert-butoxide (49.03 mg, 510.17 μmol, 2eq) were added to tetrahydrofuran (2 mL). The mixture was reacted with stirring for 1 hour, and then a solution of compound **23-12** (214 mg, 255.09 μmol, 1eq) in tetrahydrofuran (1 mL) was added. The reaction solution was reacted with stirring at 20 °C for 1 hour. The reaction solution was rotary evaporated to dryness. To the residue were added 30 mL of ethyl acetate and 5 mL of saturated brine. The mixture was stirred to clear. The organic phase was separated out, and rotary evaporated to dryness to give a crude product. The crude product was purified by column (methanol/dichloromethane=0-10%) to give compound **23-13.** MS m/z=918.2$[M+H]^+$.

**Step 13: Synthesis of compound 23 formate**

Compound **23-13** (194 mg, 221.32 μmol, 1 eq) was added to trifluoroacetic acid (2 mL), and the mixture was reacted with stirring at 55 °C for 15 hours. The reaction solution was rotary evaporated to dryness. To the residue were added 300 mg of sodium carbonate and 5 mL of ethyl acetate. The mixture was stirred for 20 minutes, and filtered. The solvent was removed from the filtrate under reduced pressure to give a crude product. The crude product was separated by preparative high-performance liquid chromatography: (separation conditions: column: Phenomenex C18 150*40mm*5μm; mobile phase: [water (0.025% formic acid)-acetonitrile]; (acetonitrile)% : 1%-30%, 10min) to give compound **23** formate. MS m/z=578.4$[M+H]^+$.

**Example 24**

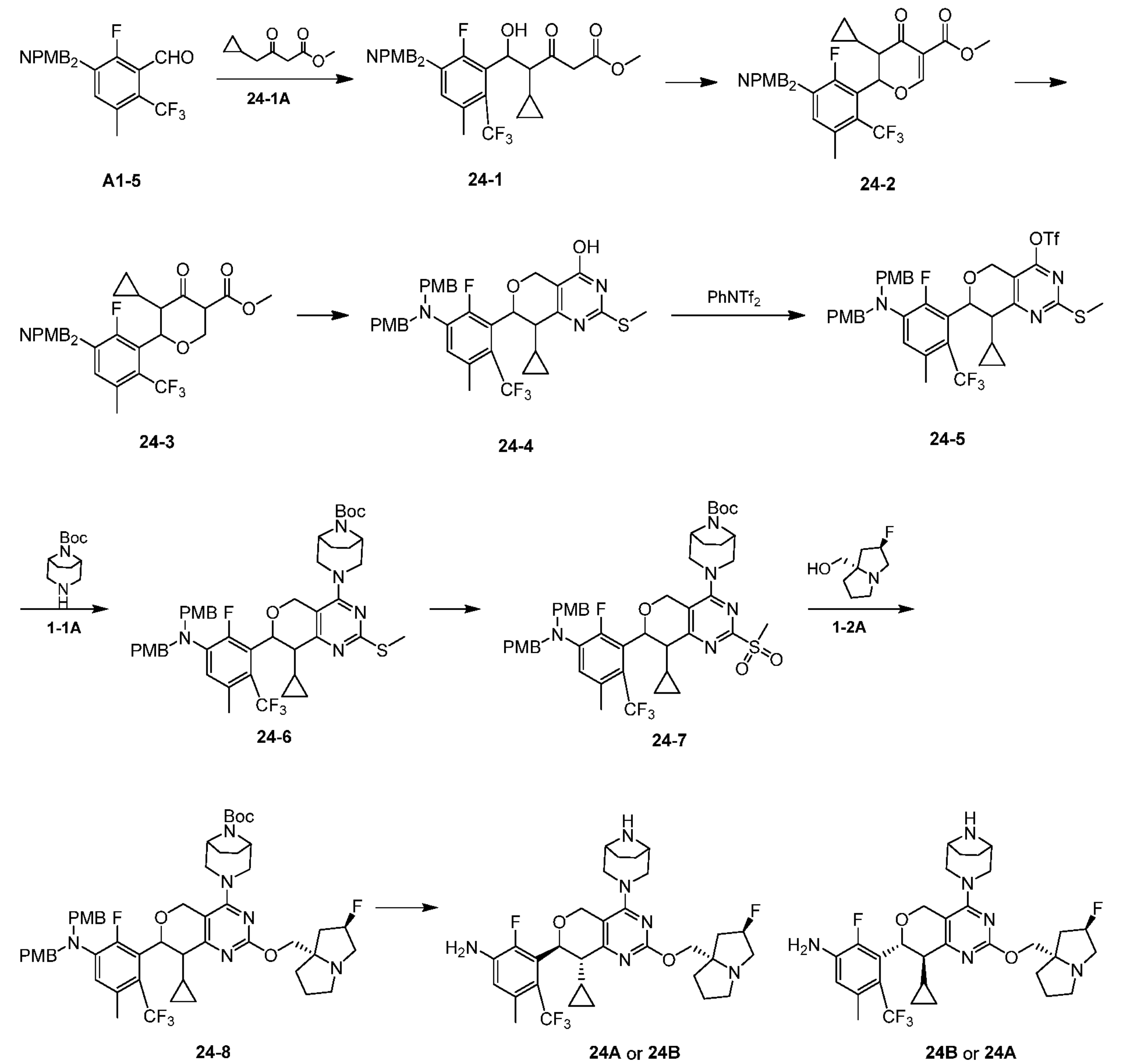


A1-5 24-1 24-2 24-3 24-4 24-5 24-6 24-7 24-8

24A or 24B    24B or 24A

**Step 1: Synthesis of intermediate 24-1**

Sodium hydride (866.75 mg, 21.67 mmol, 60% content, 2eq) was suspended in anhydrous tetrahydrofuran (50 mL). The mixture was cooled down to 0 °C under nitrogen, and then compound **24-1A** (3.38 g, 21.67 mmol, 2eq) was added dropwise slowly. After completion of the addition, the mixture was stirred for 30 minutes, and then n-butyl lithium (2.5 M, 8.67 mL, 2eq) was added dropwise. After completion of the addition, the mixture was stirred for 30 minutes. The ice bath was removed. The mixture was cooled down to -78 °C. Finally, to the mixture was added dropwise a solution of compound **A1-5** (5 g, 10.84 mmol, 1 eq) in anhydrous tetrahydrofuran (10 mL). After completion of the addition, the mixture was reacted with stirring for 1 hour. The reaction was quenched by adding 30 mL of water to the reaction solution, and then the mixture was extracted with ethyl acetate (50 mL×3). The organic phases were combined, and rotary evaporated to dryness to give a crude product. The crude product was purified by column (ethyl acetate/petroleum ether=0~45%) to give compound **24-1.** MS m/z=640.1 $[M+Na]^+$.

**Step 2: Synthesis of intermediate 24-2**

Compound **24-1** (6.50 g, 10.52 mmol, 1 eq) and N,N-dimethylformamide dimethyl acetal (3.76 g, 31.57 mmol, 4.19 mL, 3 eq) were added to anhydrous dichloromethane (20 mL). The mixture was reacted with stirring at room temperature (20 °C) for 24 hours, and then cooled down to 0°C. Finally, to the mixture was added boron trifluoride etherate (1.49 g, 10.52 mmol, 1.30 mL, 1 eq). After completion of the addition, the mixture was reacted with stirring at

20 °C for 4 hours. The reaction was quenched by adding 20 mL of saturated sodium bicarbonate solution to the reaction solution, and then the mixture was extracted with ethyl acetate (50 mL×3). The organic phases were combined, dried over anhydrous sodium sulfate, and filtered. The filtrate was concentrated under reduced pressure to give a crude product. The crude product was purified by column (ethyl acetate/petroleum ether=0~45%) to give compound **24-2.** MS m/z=628.2 $[M+H]^+$.

**Step 3: Synthesis of intermediate 24-3**

Compound **24-2** (5.0 g, 7.97 mmol, 1 eq) was dissolved in anhydrous tetrahydrofuran (30 mL). The mixture was cooled down to -78 °C under nitrogen, and then tri-sec-butyl lithium borohydride (1 M, 7.97 mL, 1eq) was added dropwise. After completion of the addition, the mixture was reacted with stirring for 1 hour. The reaction was quenched with 10 mL of 1M hydrochloric acid, and then the mixture was extracted with ethyl acetate (3×50 mL). The organic phases were combined, and concentrated to give a crude product. The crude product was purified by column (ethyl acetate/petroleum ether=0~25%) to give compound **24-3.** MS m/z=630.2 $[M+H]^+$.

**Step 4: Synthesis of intermediate 24-4**

Compound **24-3** (2.93 g, 4.65 mmol, 1 eq) and 2-methylthiourea monosulfate (2.63 g, 13.96 mmol, 3 eq) were added to anhydrous ethanol (10 mL), and then sodium carbonate (986.43 mg, 9.31 mmol, 2eq) was added. The resulting reaction solution was placed in an oil bath (60 °C) and reacted with stirring for 18 hours. The reaction solution was rotary evaporated to dryness. To the residue were added 15 mL of water and 80 mL of ethyl acetate. The mixture was adjusted to a pH of ~6-7 with 2M hydrochloric acid. The organic phase was separated. The aqueous phase was extracted with ethyl acetate (50 mL×3). The organic phases were combined, dried over anhydrous sodium sulfate, and filtered to remove the desiccant. The filtrate was concentrated to give a crude product of compound **24-4.** MS m/z=670.2 $[M+H]^+$.

**Step 5: Synthesis of intermediate 24-5**

Compound **24-4** (3.15 g, 4.70 mmol, 1eq) was dissolved in N,N-dimethylformamide (30 mL), and then compound $PhNTf_2$ (2.52 g, 7.06 mmol, 1.5eq) and N,N-diisopropylethylamine (1.82 g, 14.11 mmol, 2.46 mL, 3eq) were added. The resulting final reaction solution was reacted with stirring at room temperature (20 °C) for 2 hours. The reaction solution was rotary evaporated to dryness to give a crude product. The crude product was purified by column (ethyl acetate/petroleum ether=0~15%) to give compound **24-5.** MS m/z=802.2 $[M+H]^+$.

**Step 6: Synthesis of intermediate 24-6**

Compound **24-5** (3.15 g, 4.70 mmol, 1 eq) was dissolved in N,N-dimethylformamide (30 mL), and then compound **1-1A** (2.52 g, 7.06 mmol, 1.5 eq) and N,N-diisopropylethylamine (1.82 g, 14.11 mmol, 2.46 mL, 3eq) were added. The resulting final reaction solution was reacted with stirring at room temperature (20 °C) for 2 hours. The reaction solution was rotary evaporated to dryness to give a crude product. The crude product was purified by column (ethyl acetate/petroleum ether=0~15%) to give compound **24-6.** MS m/z=864.3$[M+H]^+$.

**Step 7: Synthesis of intermediate 24-7**

Compound **24-6** (160 mg, 185.19 μmol, 1eq) was dissolved in anhydrous dichloromethane (2 mL), and then m-chloroperoxybenzoic acid (75.19 mg, 370.37 μmol, 85% content, 2eq) was added. The resulting reaction solution was reacted with stirring at 20 °C for 15 hours. The reaction solution was rotary evaporated to dryness to give a crude product. The crude product was purified by column (ethyl acetate/petroleum ether=0~25%) to give compound **24-7.** MS m/z=896.3$[M+H]^+$.

**Step 8: Synthesis of intermediate 24-8**

Compound **1-2A** (35.89 mg, 225.45 μmol, 2eq) and sodium tert-butoxide (21.67 mg, 225.45 μmol, 2eq) were added to tetrahydrofuran (1 mL). The mixture was reacted with stirring for 1 hour, and then a solution of compound **24-7** (101 mg, 112.72 μmol, 1eq) in tetrahydrofuran (1 mL) was added. The resulting final reaction solution was reacted with stirring at 25 °C for 1 hour. The reaction solution was concentrated to give a crude product. The crude product was purified by column (ethyl acetate/petroleum ether=0~65%) to give compound **24-8.** MS m/z=975.4$[M+H]^+$.

**Step 9: Synthesis of compound 24A hydrochloride and compound 24B**

Compound **24-8** (94 mg, 96.40 μmol, 1 eq) was added to trifluoroacetic acid (2 mL). The mixture was reacted with stirring at 25 °C for 1 hour. The mixture was concentrated to give a crude product. The crude product was separated by preparative high-performance liquid chromatography: (separation conditions: Phenomenex C18 150*40mm*5μm; mobile phase [water (0.025% formic acid)-acetonitrile]; (acetonitrile)%: 5%-35%, 10min), and then subjected to chiral separation with separation conditions of: (column: DAICEL CHIRALPAK IG (250mm*30mm, 10μm); mobile phase: [0.1% ammonia water ethanol]; (ethanol)%: 45%-45%) retention time Rt=2.034min, to give compound **24B.** MS m/z=635.9[M+H]$^+$. Additional isomer (retention time Rt=2.469min) was further separated by preparative high-performance liquid chromatography with separation conditions of: (column: Welch Xtimate C18 100*40mm*3μm; mobile phase: [water (0.025% formic acid)-acetonitrile]; (acetonitrile)%: 10%-40%, 8min). 0.5mL of HCl/1,4-dioxane was added to give compound **24A** hydrochloride. MS m/z=635.8[M+H]$^+$.

**Example 25**

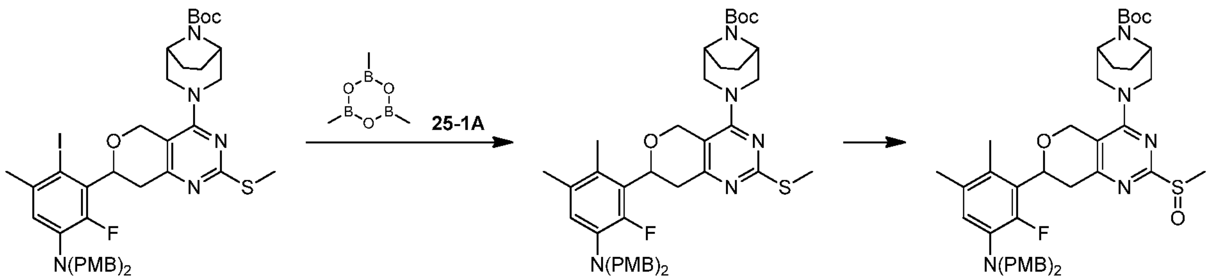


16-1 25-1 25-2

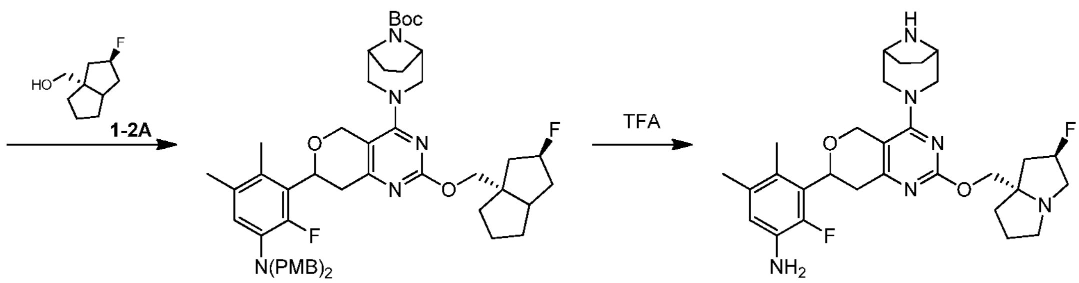


25-3 25

**Step 1: Synthesis of intermediate 25-1**

Compound **16-1** (00 mg, 113.40 μmol, 1 eq), compound **25-1A** (42.71mg, 170.10 μmol, 47.56 μL, 50% THF solution, 1.5 eq) and potassium carbonate (31.35 mg, 226.80 μmol, 2 eq) were added to a solvent mixture of dioxane (2 mL)/water (0.4 mL), and then Pd(dppf)$Cl_2$ (16.60 mg, 22.68 μmol, 0.2 eq) was added. The resulting reaction solution was purged with nitrogen, heated to 95 °C and reacted with stirring for 15 hours. The reaction solution was concentrated to give a crude product. The crude product was purified by column (ethyl acetate/petroleum ether=0~30%) to give compound **25-1.**

**Step 2: Synthesis of intermediate 25-2**

Compound **25-1** (72mg, 93.51 μmol, 1eq) was dissolved in anhydrous dichloromethane (1 mL), and then m-chloroperoxybenzoic acid (18.98mg, 93.51 μmol, 85% content, 1eq) was added. The resulting reaction solution was reacted with stirring at 15 °C for 1 hour. The reaction solution was concentrated to give compound **25-2.** MS m/z=786.3[M+H]$^+$.

**Step 3: Synthesis of intermediate 25-3**

Compound **1-2A** (45.57 mg, 286.27 μmol, 3eq), sodium tert-butoxide (18.34 mg, 190.85 μmol, 2eq) and compound **25-2** (75mg, 95.42 μmol, 1eq) were added to toluene (2 mL). The mixture was reacted with stirring at 15 °C for 2 hours. The reaction solution was diluted with 30 mL of ethyl acetate, and then the mixture was washed with 5 mL of water and 5 mL of saturated brine. The organic phase was concentrated to give a crude product. The crude product was purified by column (methanol/dichloromethane=0-5%) to give compound **25-3.** MS m/z=881.9[M+H]$^+$.

**Step 4: Synthesis of intermediate 25 hydrochloride**

Compound **25-3** (71 mg, 80.58 μmol, 1 eq) was added to trifluoroacetic acid (2 mL), and the mixture was reacted with stirring at 50 °C for 5 hours. The reaction solution was concentrated to give a crude product. The crude product was separated by preparative high-performance liquid chromatography (separation conditions: column: Xtimate C18 150*40mm*5μm; mobile phase: [water (0.05% hydrochloric acid)-acetonitrile]; (acetonitrile)%: 1%-30%, 10min) to give compound **25** hydrochloride, MS m/z=541.3[M+H]$^+$.

**Example 26**

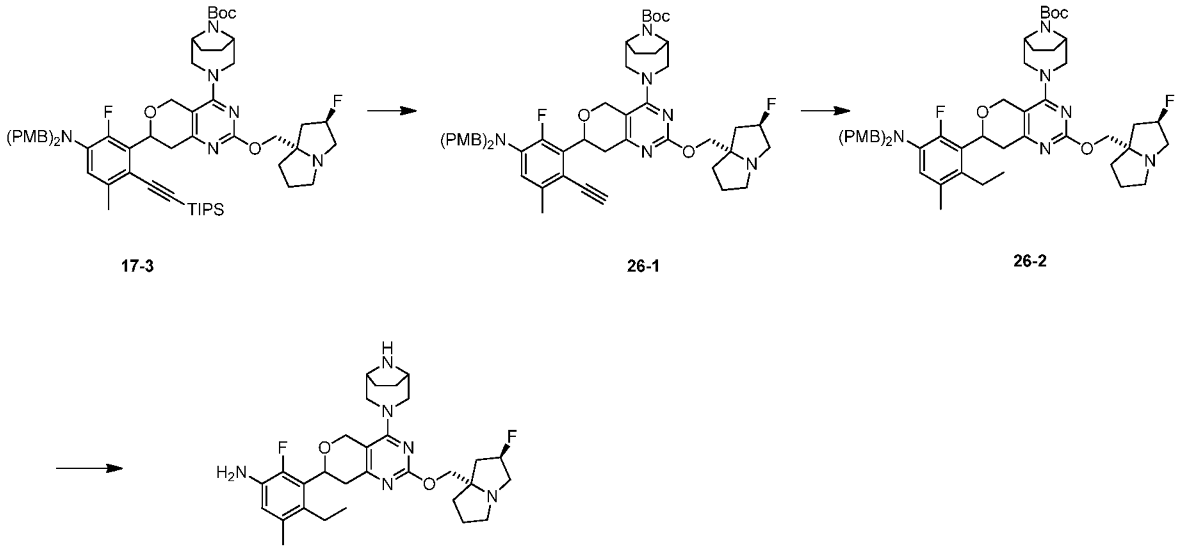

17-3 26-1 26-2

26

**Step 1: Preparation of intermediate 26-1**

To a solution of **17-3** (420 mg, 0.40 mmol) in tetrahydrofuran (10 mL) was added tetramethylammonium fluoride (150 mg, 1.61 mmol). The system was heated at 60 °C for 16 hours, and then diluted with 50 mL of ethyl acetate. Then the mixture was washed with 30 mL of saturated brine, dried over anhydrous sodium sulfate, and filtered. The filtrate was concentrated to give **26-1.** MS m/z: 891.6 [M+1]$^+$.

**Step 2: Preparation of intermediate 26-2**

To a solution of **26-1** (250 mg, 280.57 μmol, 1 eq) in methanol (10 mL) was added palladium/carbon (20 mg, 10% content). The system was stirred at 20 °C for 1 hour under a hydrogen pressure of 15 psi. The mixture was filtered. The filtrate was concentrated to give **26-2.** MS m/z: 895.6 [M+1]$^+$.

**Step 3: Preparation of compound 26 hydrochloride**

To a solution of **26-2** (220 mg, 245.79 μmol, 1 eq) in dichloromethane (3 mL) was added trifluoroacetic acid (3 mL). The system was stirred at 45 °C for 20 hours. The reaction solution was rotary evaporated to dryness. The residue was purified by preparative HPLC (column: Phenomenex C18 150*40mm*5μm; mobile phase: [water (0.05% hydrochloric

acid)-acetonitrile]; (acetonitrile)%: 1%-30%, 10min) to give compound **26** hydrochloride. $^1H$ NMR (400MHz, CDsOD) δ = 6.71 (d, *J*=9.0 Hz, 1H), 5.48 - 5.19 (m, 2H), 4.74 - 4.59 (m, 3H), 4.31 - 4.14 (m, 3H), 3.97 - 3.52 (m, 4H), 3.48 - 3.37 (m, 2H), 3.30 - 3.06 (m, 3H), 2.92 - 2.63 (m, 3H), 2.45 - 2.27 (m, 2H), 2.24 (s, 3H), 2.22 - 1.84 (m, 8H), 1.12 (t, J =7.4 Hz, 3H). MS m/z: 555.2 $[M+1]^+$.

**Example 27**

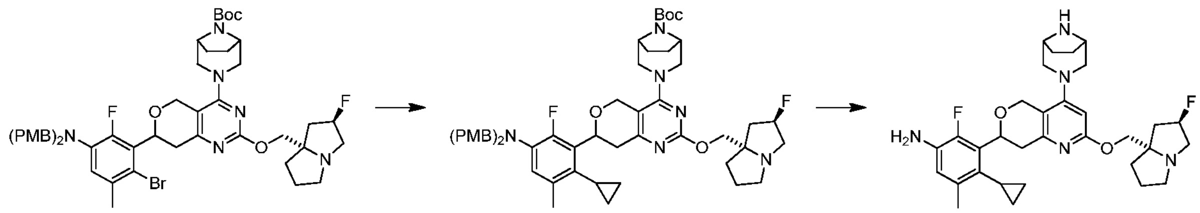

15-3 27-1 27

**Step 1: Preparation of intermediate 27-1**

**15-3** (200 mg, 0.212 mmol) and cyclopropylboronic acid (92 mg, 1.059 mmol) were added to a solution mixture of 1,4-dioxane (10 mL) and water (1 mL), and then [1,1'-bis(diphenylphosphino)ferrocene]palladium dichloride (20 mg) and sodium carbonate (45 mg, 0.425 mmol) were added. The atmosphere was replaced with nitrogen three times. The system was reacted at 90 °C for 15 hours, and filtered. The filtrate was rotary evaporated to dryness. The residue was separated by preparative thin layer chromatography (developer: dichloromethane/methanol = 10:1) to give **27-1.** MS m/z: 907.6 $[M+H]^+$.

**Step 2: Preparation of compound 27 hydrochloride**

To a solution of **27-1** (60 mg, 0.066 mmol) in dichloromethane (2 mL) was added trifluoroacetic acid (2 mL). The system was stirred at 20 °C for 1 hour, and concentrated. The residue was purified by preparative HPLC (column: Phenomenex C18 150*40mm*5μm; mobile phase: [water (0.05% hydrochloric acid)-acetonitrile]; (acetonitrile)%: 1%-30%, 10min) to give compound 27 hydrochloride. MS m/z: 565.5 $[M+H]^+$.

**Example 28**

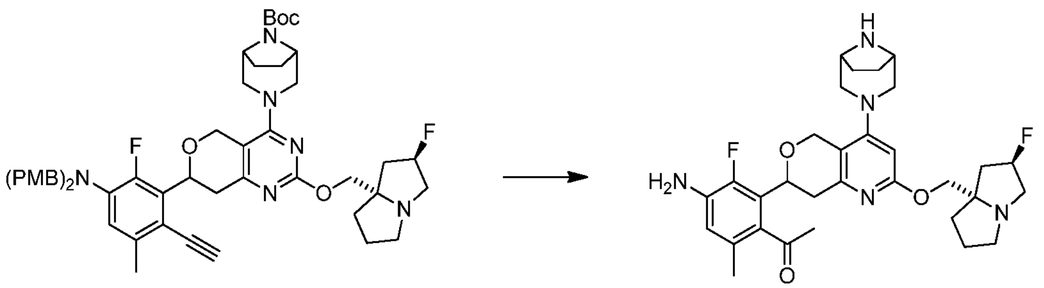

26-1 28

**Step 1: Preparation of compound 28 trifluoroacetate**

To a solution of **26-1** (50 mg, 56.11 μmol) in dichloromethane (0.5 mL) was added trifluoroacetic acid (0.5 mL). The system was stirred at 20 °C for 2 hours. The reaction solution was concentrated. The residue was purified by preparative HPLC (column: Welch Xtimate C18 100*40mm*3μm; mobile phase: [water (0.025% trifluoroacetic acid)-acetonitrile]; (acetonitrile)%: 0%-30%, 8min) to give compound **28** trichloroacetate. $^1H$ NMR (400MHz, CDsOD) δ = 6.69 (d, J =8.4 Hz, 1H), 5.69 - 5.15 (m, 2H), 4.75- 4.71 (m, 2H), 4.60 - 4.36 (m, 4H), 4.22 - 3.96 (m, 3H), 3.95 - 3.65 (m, 5H),

3.53 - 3.42 (m, 1H), 3.21 - 3.11 (m, 1H), 3.07 - 2.97 (m, 1H), 2.75 - 2.55 (m, 2H), 2.45 (s, 3H), 2.43 - 2.25 (m, 4H), 2.20 - 1.96 (m, 7H).

**Example 29**

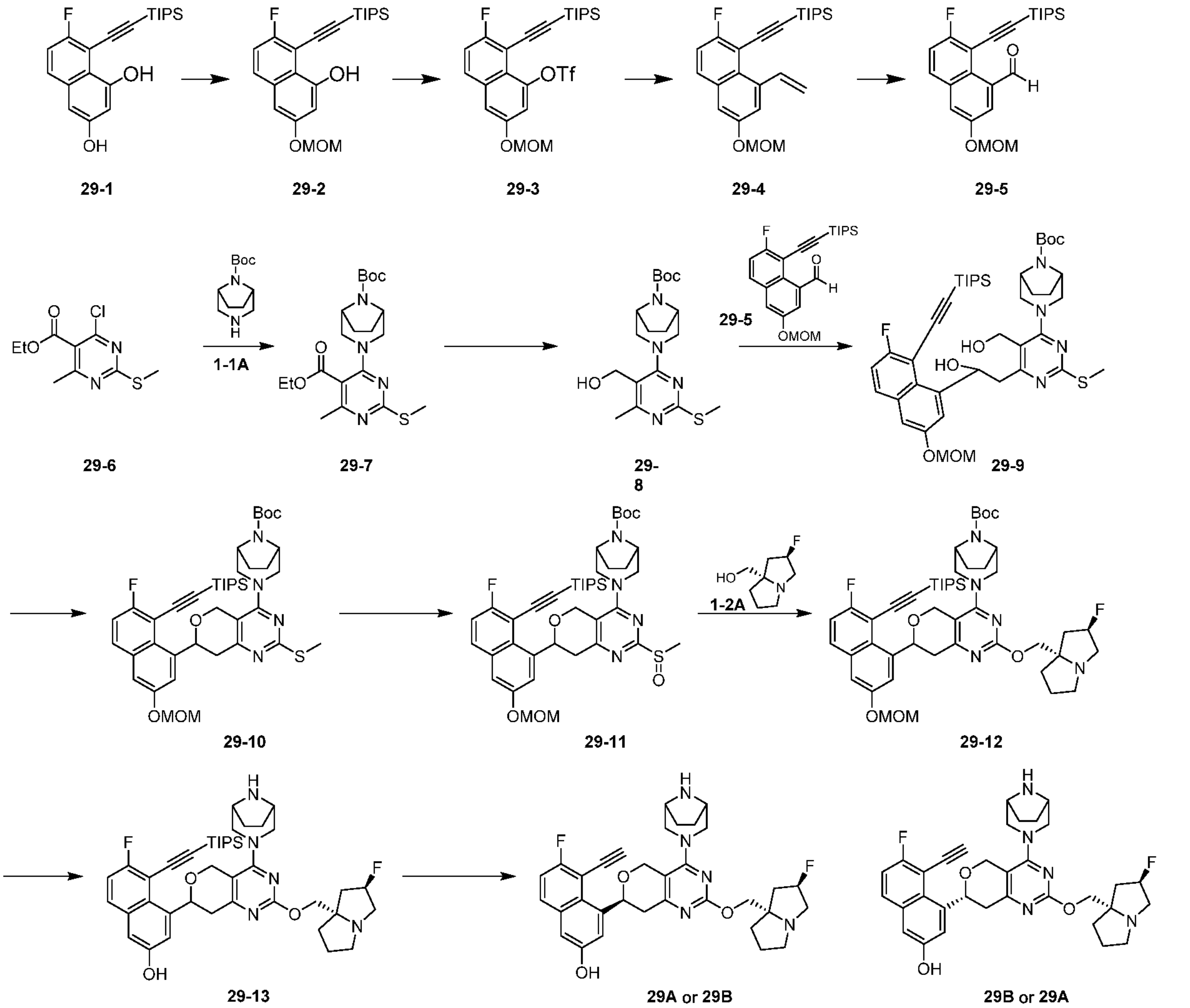


29-1, 29-2, 29-3, 29-4, 29-5, 29-6, 29-7, 29-8, 29-9, 29-10, 29-11, 29-12, 29-13, 29A or 29B, 29B or 29A

**Step 1: Synthesis of intermediate 29-2**

Compound **29-1** (50 g, 139.46 mmol, 1 eq) was added to anhydrous dichloromethane (500 mL), and N,N-diisopropylethylenediamine (54.07 g, 418.39 mmol, 72.87 mL, 3 eq) was added. The mixture was cooled down to 0 °C, and chloromethyl methyl ether (15.59 g, 193.64 mmol, 14.71 mL, 1.39 eq) was added dropwise slowly. The mixture was slowly warmed to 18 °C for 1 hour. The reaction solution was added to 500 mL of ice water. The mixture was extracted with DCM (100 mL*2). The organic phases were combined, and then washed respectively with 500 mL of saturated sodium carbonate, 500 mL of saturated ammonium chloride and 500 mL of half-saturated brine, dried over anhydrous sodium sulfate, and filtered. The filtrate was concentrated. The crude product was purified by column chromatography (ethyl acetate/petroleum ether=0~50%) to give compound **29-2.** MS m/z =403.2[M+H]$^+$.

**Step 2: Synthesis of intermediate 29-3**

Compound **29-2** (36 g, 89.42 mmol, 1 eq) and N,N-diisopropylethylenediamine (34.67 g, 268.27 mmol, 46.73 mL, 3 eq) were added to anhydrous dichloromethane (360 mL). The mixture was cooled down to -40 °C. Trifluoromethanesulfonic anhydride (37.85 g, 134.14 mmol, 22.13 mL, 1.5 eq) was added dropwise. The mixture was reacted for 1 hour. The reaction solution was added to 300 mL of ice water. The layers were separated and extracted. The organic

phase was washed sequentially with 200 mL of saturated sodium bicarbonate solution, 200 mL of saturated ammonium chloride and 200 mL of brine, dried over anhydrous sodium sulfate, and filtered. The filtrate was concentrated. The crude product was purified by column chromatography (ethyl acetate/petroleum ether=0~30%) to give compound **29-3.** $^1$H NMR (400 MHz, $CDCl_3$) $\delta$ = 7.71 (dd, *J* = 5.2, 9.2 Hz, 1H), 7.43 (d, *J* = 2.4 Hz, 1H), 7.36 (d, *J* = 2.0 Hz, 1H), 7.33 (t, *J* = 8.8 Hz, 1H), 5.28 (s, 2H), 3.53 (s, 3H), 1.28 - 1.18 (m, 21H).

**Step 3: Synthesis of intermediate 29-4**

Compound **29-3** (34 g, 63.59 mmol, 1 eq) was added to N,N-dimethylformamide (340 mL), and vinyltributylstannane (42.03 g, 132.55 mmol, 38.56 mL, 2.08 eq) and lithium chloride (10.78 g, 254.38 mmol, 5.21 mL, 4 eq) were added. The atmosphere was replaced with nitrogen three times, and bis(triphenylphosphine)palladium chloride (4.46 g, 6.36 mmol, 0.1 eq) was added under nitrogen. The mixture was reacted at 30 °C for 20 hours. To the reaction solution were added 300 mL of 20% aqueous KF solution and 300 mL of methyl tert-butyl ether. The mixture was stirred for 20 minutes and filtered through a pad of Celite. The filter cake was rinsed with methyl tert-butyl ether (50 mL*4). The aqueous phase was removed. The organic phase was washed with 500 mL of saturated brine, dried over anhydrous sodium sulfate, and filtered. The filtrate was concentrated under reduced pressure. The crude product was purified by column (ethyl acetate/petroleum ether=20%) to give compound **29-4.** MS m/z =413.3$[M+H]^+$.

**Step 4: Synthesis of intermediate 29-5**

Compound **29-4** (20 g, 48.47 mmol, 1 eq) was added to anhydrous tetrahydrofuran (200 mL) and water (50 mL). The mixture was cooled down to 0 °C. Sodium periodate (31.10 g, 145.42 mmol, 8.06 mL, 3 eq) and osmium tetroxide (1.5 g, 5.90 mmol, 306.12 μL, 1.22e-1 eq) were added. The mixture was slowly warmed to 18°C and reacted for 1 hour. The reaction solution was added to 300 mL of 10% sodium thiosulfate solution. The mixture was extracted with ethyl acetate (100 mL*2). The organic phase was washed with 500 mL of saturated brine, dried over anhydrous sodium sulfate, and filtered. The filtrate was concentrated. The crude product was purified by column (ethyl acetate/petroleum ether=20%) to give compound **29-5.** MS m/z =415.3$[M+H]^+$.

**Step 5: Synthesis of intermediate 29-7**

To a solution of compound **29-6** (1 g, 4.05 mmol) and **1-1A** (1.03 g, 4.86 mmol) in dichloromethane was added N,N-diisopropylethylamine (1.05 g, 8.11 mmol). The obtained mixture was stirred at room temperature (25°C) for hr. To the reaction solution were added water (20 mL) and dichloromethane (50 mL). The mixture was stirred for 5 minutes. The aqueous phase was removed. The organic phase was concentrated under reduced pressure. The residue was purified by chromatography purification system (ethyl acetate/petroleum ether=20%) to give compound **29-7.** MS m/z =423.1$[M+H]^+$.

**Step 6: Synthesis of intermediate 29-8**

To a solution of compound **29-7** (0.6 g, 1.42 mmol) in tetrahydrofuran (5 mL) was added in portions lithium aluminum tetrahydride (0.1 g, 2.84 mmol) at 0 °C (in an ice-water bath) under nitrogen. The obtained mixture was naturally warmed to 25 °C and stirred for 2 hours. To the reaction solution were added dropwise sequentially water (0.1 g), 15% aqueous sodium hydroxide solution (0.1 g), and water (0.3 g). The mixture was stirred for 30 minutes, and filtered. The filter cake was washed with tetrahydrofuran (10 mL). The filtrate was concentrated under reduced pressure to give compound **29-8.** MS m/z =381.1$[M+H]^+$.

**Step 7: Synthesis of intermediate 29-9**

To a solution of compound **29-8** (0.5 g, 1.33 mmol) in tetrahydrofuran (10 mL) was added dropwise lithium diisopropylamide (1.51 mL, 3.02 mmol, 2 M) at -65 °C (in a dry ice-ethyl acetate bath) under nitrogen. After the mixture was stirred for 30 minutes, a solution of compound **29-5** (0.5 g, 1.51 mmol) in tetrahydrofuran (5 mL) was added dropwise. The resulting mixture was naturally warmed to 25 °C. To the reaction solution were added 0.1 M aqueous dilute hydrochloric acid solution (15 mL) and ethyl acetate (20 mL). The mixture was stirred for 10 minutes. The aqueous phase was removed. The organic phase was concentrated under reduced pressure. The residue was purified by chromatography purification system (ethyl acetate/petroleum ether=20%) to give compound **29-9.** MS m/z =795.4$[M+H]^+$.

**Step 8: Synthesis of intermediate 29-10**

To a solution of compound **29-9** (0.51 g, 641.44 μmol) in tetrahydrofuran (10 mL) was added dropwise n-butyl lithium (2.5 M, 564.47 μL) at -65 °C (in a dry ice-ethyl acetate bath) under nitrogen. After the mixture was stirred for 30 minutes, a solution of p-toluenesulfonyl chloride (183.43 mg, 962.16 μmol) in tetrahydrofuran (3 mL) was added dropwise. The obtained mixture was naturally warmed to 25 °C and stirred for 2 hours. The reaction was quenched by adding 50 mL of saturated aqueous ammonium chloride solution to the reaction solution, and then 50 milliliters of ethyl acetate was added for extraction. The aqueous phase was removed. The organic phase was concentrated under reduced pressure. The residue was purified by chromatography purification system (ethyl acetate/petroleum ether=30%) to give compound **29-10.** MS m/z =777.4$[M+H]^+$.

**Step 9: Synthesis of intermediate 29-11**

To a solution of compound **29-10** (45 mg, 57.91 μmol) in dichloromethane (2 mL) was added m-chloroperoxybenzoic acid (14.11 mg, 69.49 μmol, 85% content). The obtained mixture was stirred at room temperature (25 °C) for 2 hours. To the reaction solution were added 2 milliliters of aqueous saturated sodium bicarbonate solution, 2 milliliters of aqueous saturated sodium sulfite solution and 5 milliliters of dichloromethane. The mixture was stirred for 5 minutes. The aqueous phase was removed. The organic phase was concentrated under reduced pressure. The residue was purified by preparative TLC (petroleum ether/ethyl acetate=20%) to give compound **29-11.** MS m/z =793.4$[M+H]^+$.

**Step 10: Synthesis of intermediate 29-12**

To a solution of compound **1-2A** (32.12 mg, 201.76 μmol) in tetrahydrofuran (2 mL) was added sodium tert-butoxide (19.39 mg, 201.76 μmol) at 0 °C (in an ice-water bath) under nitrogen. After the mixture was stirred for 30 minutes, compound **29-11** (40 mg, 50.44 μmol) was added. The obtained mixture was naturally warmed to 25 °C and stirred for 2 hours. The reaction solution was adjusted to a pH of about 6 by adding 0.5 M dilute hydrochloric acid. 5 milliliters of ethyl acetate and 2 milliliters of saturated brine were added, and the mixture was stirred for 5 minutes. The aqueous phase was removed. The organic phase was concentrated under reduced pressure to give compound **29-12.** MS m/z =888.5$[M+H]^+$.

**Step 11: Synthesis of intermediate 29-13**

To a solution of compound **29-12** (45 mg, 50.67 μmol) in dichloromethane (1 mL) was added hydrochloric acid-ethyl acetate (4 M, 126.67 μL). The obtained mixture was stirred at room temperature (25°C) for 2 hours. The reaction solution was directly concentrated under reduced pressure to give compound **29-13** trifluoroacetate. MS m/z =744.4$[M+H]^+$.

**Step 12: Synthesis of compound 29A and compound 29B**

To a solution of compound **29-13** (40 mg, trifluoroacetate) in DMF (1 mL) were added cesium fluoride (40.83 mg, 268.82 μmol) and potassium carbonate (22.29 mg, 161.29 μmol). The obtained mixture was stirred at room temperature (25 °C) for 2 hours. The reaction solution was filtered, and washed with 5 milliliters of methanol. The filtrate was concentrated under reduced pressure. The residue was purified by preparative HPLC (column: Phenomenex Luna C18 75*30mm*3μm; mobile phase: [water (0.025% formic acid)-acetonitrile]; acetonitrile%: 10%-40%, 8min), and then subjected to chiral separation (column: DAICEL CHIRALCEL OD (250mm*30mm, 10μm); mobile phase: [0.1% ammonia water methanol]; methanol %: 40%-40%, 12min) to give compound **29A** (Rt=1.386 min) and compound **29B** (Rt=2.079 min). MS m/z =588.3$[M+H]^+$.

**Biological assay data:**

**Assay example 1. Assay of activity of inhibiting $KRAS^{G12D}$**

**1. Purpose**

Compounds that can effectively inhibit the binding of KRAS to GTP were screened out by a TR-FRET method.

**2. Consumable and instrument**

Table 1. Consumable and instrument

| Name | Vendor | Item No. |
|---|---|---|
| HEPES (4-(2-hydroxyethyl)-1-piperazineethanesulfonic acid) pH 7.3 | Thermo Fisher | BP299-500 |
| Sodium chloride | Promega | V4221 |
| EDTA (ethylenediaminetetraacetic acid) | EMD Millipore | 324506 |
| Tween 20 | Bole | 1706531 |
| Magnesium chloride | MP Biomedicine | 191421 |
| Bodipy GDP (guanosine 5'-diphosphate, BODIPY™ FL 2'-(or -3')-O-(N-(2-aminoethyl)urethane), bis(triethylammonium) salt) | Yingjie | G22360 |
| GTP (guanine-5'-triphosphate) | Sigma | G8877 |
| Tb-SA (Terbium-labeled streptavidin) | Yingjie | PV3576 |
| SOS (son of sevenless) protein | | |
| $KRAS^{G12D}$ (Kirsten rat sarcoma viral oncogene) protein | | |
| Compound plate | Labcyte | LP-0200 |
| Assay plate | Perkin Elmer | 6008269 |
| 15-milliliter centrifuge tube | Corning | 430791 |
| 1.5-milliliter centrifuge tube | Axygen | MCT-150-C |
| Dragonfly automatic sampler | TTP | |
| Bravo | Agilent | |
| Echo 550 | Labcyte | |
| Envision | Perkin Elmer | |

**3. Preparation of reagent**

**a. Stock reagents:**

**1) KRAS nucleotide exchange buffer**

20 mL of 1000mM HEPES, 20 mL of 500mM EDTA, 10 mL of 5 M sodium chloride, 0.1 mL of 100% Tween 20, and 949.9 mL of water were weighed and formulated to 1 L of solution. The solution was sterilized by filtration and stored at 4 °C.

**2) KRAS assay buffer**

20 mL of 1000 mM HEPES, 10 mL of 1000mM magnesium chloride, 30 mL of 5 M sodium chloride, 0.05 mL of 100% Tween 20, and 939.95mL of water were weighed and formulated to 1 L of solution. The solution was sterilized by filtration and stored at 4 °C.

**3) KRAS/Bodipy GDP/Tb-SA mixture**

9.5 μL of 95 μM $KRAS^{G12D}$ protein and 440.5 μL of KRAS nucleotide exchange buffer were weighed and mixed. The mixture was incubated at room temperature for 1 hour, and then formulated to 1 L of solution together with 8.4 μL of 17.9 μM Tb-SA, 1.8 μL of 5mM Bodipy GDP and 9539.8 μL of KRAS assay buffer. After mixing, the solution was left to stand at room temperature for 6 hours, and stored at -80 °C.

**b. Assay reagents:**

**1) KRAS kinase solution**

73.3 μL of KRAS/Bodipy GDP/Tb-SA mixture and 2126.7 μL of KRAS assay buffer were weighed and formulated to 2200 μL of solution.

**2) SOS/GTP mixture**

1.59 μL of 166 μM SOS protein, 198 μL of 100 mM GTP and 2000.41 μL of KRAS assay buffer were weighed and formulated to 2200 μL of solution.

**4. Assay process**

1) The concentration of a stock solution of the control compound was 1 mM, and the concentration of a stock solution of compounds to be assayed was 10 mM. 9 μL of the control compound and compounds to be assayed were transfered to a 384-LDV plate;
2) The compounds on the LDV plate were serially diluted 3-fold with Bravo to 10 concentrations;
3) 9 nL of the compounds on the LDV plate were transfered to an assay plate using ECHO;
4) 3 μL of 3 nM Kras/0.5 nM TB-SA/30 nM BodipyGDP mixture and 3 μL of Ras buffer were sequentially added to each well of the assay plate using a Dragonfly automatic sampler, and the assay plate was centrifuged at 1000rpm/min for 1 minute;
5) The assay plate was incubated at room temperature for 1 hour;
6) 3 μL of 120 nM SOS/9 mM GTP mixture was added to each well of the assay plate using a Dragonfly automatic sampler, and the assay plate was centrifuged at 1000rpm/min for 1 minute;
7) The assay plate was incubated at room temperature for 1 hour;
8) The plate was read with Envision and data were recorded;
9) The data were analysed using Excel and Xlfit, and $IC_{50}$ of the compounds to be assayed were calculated.

**5. Assay results**

The results are shown in Table 2.

Table 2 $IC_{50}$ values of compounds on inhibiting $KRAS^{G12D}$ kinase

| Compound No. | $KRAS^{G12D}$ $IC_{50}$ (nM) |
|---|---|
| Compound 1 hydrochloride | 2.5 |
| Compound 2 hydrochloride | 23 |
| Compound 3 hydrochloride | 425 |
| Compound 5 hydrochloride | 12.7 |
| Compound 6 hydrochloride | 30 |
| Compound 7 hydrochloride | 0.1 |
| Compound 8 hydrochloride | 78.3 |
| Compound 9 | 1.1 |
| Compound 10 formate | 3.3 |

Assay conclusion: The compounds of the present disclosure have significant inhibitory effect on $KRAS^{G12D}$ enzyme.

**Assay example 2. Assay of p-ERK inhibition in AGS cells**

**1. Purpose**

Compound that can effectively inhibit p-ERK in AGS cells was screened out by a HTRF method.

**2. Assay process**

1) AGS cells were inoculated in a transparent 96-well cell culture plate, and each well contained 80μL of cell suspension and 10000 cells. The cell plate was inoculated in a carbon dioxide incubator at 37 °C overnight;
2) After completion of the incubation, the cell supernatant was discarded. 80 μL of medium containing 0.02% serum was added to each well. The cell plate was incubated in a carbon dioxide incubator at 37 °C overnight;
3) 2 μL of the compound was weighed and added to 78 μL of the cell medium. After the mixture was mixed thoroughly, 20 μL of the solution of compound was weighed and added to the corresponding well of the cell plate. The cell plate was placed back to the carbon dioxide incubator and incubated for another 3 hours;
4) After completion of the incubation, the cell supernatant was discarded. 50 μL of 1X cell lysate was added to each well. The mixture was incubated with shaking at room temperature for 30 minutes;
5) Phospho-ERK1/2 Eu Cryptate antibody and Phospho-ERK1/2 d2 antibody were diluted 20-fold with detection buffer;
6) 16 μL of cell lysate supernatant was weighed and added into each well of a new 384 white microwell plate, and then 2 μL of the diluted solution of Phospho-ERK1/2 Eu Cryptate antibody and 2 μL of the diluted solution of Phospho-ERK1/2 d2 antibody dilution were added. The mixture was incubated at room temperature for at least 4 hours;
7) After completion of the incubation, HTRF was read with multi-label analyzer: excitation: 320nm, emission: 615nm, 665nm;
8). $IC_{50}$ of the compound to be assayed was calculated.

**3 Assay result**

The result is shown in Table 3.

Table 3 $IC_{50}$ value of the compound on inhibiting p-ERK in AGS cells

| Compound No. | AGS p-ERK $IC_{50}$ (nM) |
|---|---|
| Compound 1 hydrochloride | 45 |

Assay conclusion: The compound of the present disclosure has significant inhibitory effect on p-ERK in AGS cells.

**Assay example 3. Assay of p-ERK inhibition in GP2D cells**

**1. Purpose**

Compounds that can effectively inhibit p-ERK in GP2D cells were screened out by a HTRF method.

**2. Assay process**

1) GP2D cells were inoculated in a transparent 96-well cell culture plate, and each well contained 80μL of cell suspension and 8000 cells. The cell plate was inoculated in a carbon dioxide incubator at 37 °C overnight;
2) 2 μL of the compound was weighed and added to 78 μL of the cell medium. After the mixture was mixed thoroughly, 20 μL of the solution of compound was weighed and added to the corresponding well of the cell plate. The cell plate was placed back to the carbon dioxide incubator and incubated for another 1 hour;
3) After completion of the incubation, the cell supernatant was discarded. 50 μL of 1X cell lysate was added to each well. The mixture was incubated with shaking at room temperature for 30 minutes;

4) Phospho-ERK1/2 Eu Cryptate antibody and Phospho-ERK1/2 d2 antibody were diluted 20-fold with detection buffer;
5) 16 μL of cell lysate supernatant was weighed and added into each well of a new 384 white microwell plate, and then 2 μL of the diluted solution of Phospho-ERK1/2 Eu Cryptate antibody and 2 μL of the diluted solution of Phospho-ERK1/2 d2 antibody dilution were added. The mixture was incubated at room temperature for at least 4 hours;
6) After completion of the incubation, HTRF was read with multi-label analyzer: excitation: 320nm, emission: 615nm, 665nm;
7). $IC_{50}$ of the compounds to be assayed was calculated.

**3 Assay results**

The results are shown in Table 4.

Table 4 $IC_{50}$ values of compounds on inhibiting p-ERK in GP2D cells

| Compound No. | GP2D p-ERK $IC_{50}$ (nM) |
|---|---|
| Compound 1 hydrochloride | 0.8 |
| Compound 7 hydrochloride | 1.1 |
| Compound 8 hydrochloride | 78 |
| Compound 11A hydrochloride | 3.4 |
| Compound 11B hydrochloride | 75.9 |
| Compound 12 formate | 20.9 |
| Compound 14 hydrochloride | 5.2 |
| Compound 15 hydrochloride | 8.4 |
| Compound 16 hydrochloride | 2.9 |
| Compound 17 | 27.1 |
| Compound 18 | 66.4 |
| Compound 19 hydrochloride | 17.5 |
| Compound 20B | 1.3 |
| Compound 21 hydrochloride | 2.6 |
| Compound 22 hydrochloride | 2.7 |
| Compound 23 formate | 23.4 |
| Compound 25 hydrochloride | 96.4 |
| Compound 26 hydrochloride | 18.8 |
| Compound 27 hydrochloride | 118.3 |

Assay conclusion: The compounds of the present disclosure have significant inhibitory effect on p-ERK in GP2D cells.

**Assay example 4. Assay of p-ERK inhibition in PANC0403 cells**

1. Materials of the assay:

PANC0403 cells purchased from Nanjing Kebai; RPMI-1640 medium purchased from Biological Industries; fetal bovine serum purchased from Biosera; and Advanced Phospho-ERK1/2 (THR202/TYR204) KIT purchased from Cisbio.

The composition of Advanced Phospho-ERK1/2(THR202/TYR204) KIT is shown in Table 5.

Table 5

| Ingredient name | Storage temperature |
|---|---|
| Advanced PhosphoERK1/2 Eu Cryptate antibody | ≤-16°C |
| Advanced PhosphoERK1/2 d2 antibody | ≤-16°C |
| Blocking reagent (stock solution 100X) | ≤-16°C |
| Lysis buffer # 1 (stock solution 4X) | ≤-16°C |
| Detection buffer (ready-to-use) | ≤-16°C |

2. Method of the assay:

1) PANC0403 cells were inoculated in a transparent 96-well cell culture plate, and each well contained 80μL of cell suspension and 10000 PANC0403 cells. The cell plate was inoculated in a carbon dioxide incubator at 37 °C overnight;
2) The compound to be assayed was diluted to 2 mM as the first concentration with 100% DMSO, and then serially diluted 5-fold to the eighth concentration with a pipette, i.e., from 2 mM to 25.6 nM. 2 μL of the compound was weighed and added to 78 μL of a cell starvation medium. After the mixture was mixed thoroughly, 20 μL of the solution of compound was weighed and added to the corresponding well of the cell plate. The cell plate was placed back to the carbon dioxide incubator and incubated for another 3 hours; at this time, the concentration of the compound was 10 μM to 0.128 nM, and the concentration of DMSO was 0.5%;
3) After completion of the incubation, the cell supernatant was discarded. 50 μL of cell lysate was added to each well. The mixture was incubated with shaking at room temperature for 30 minutes;
4) Phospho-ERK1/2 Eu Cryptate antibody and Phospho-ERK1/2 d2 antibody were diluted 20-fold with Detection buffer;
5) 16 μL of cell lysate supernatant was weighed and added into each well of a new 384 white microwell plate, and then 2 μL of the diluted solution of Phospho-ERK1/2 Eu Cryptate antibody and 2 μL of the diluted solution of Phospho-ERK1/2 d2 antibody dilution were added. The mixture was incubated at room temperature overnight;
6) After completion of the incubation, HTRF was read with multi-label analyzer: excitation: 320nm, emission: 615nm, 665nm;

3. Data analysis:

The equation **(Sample-Min)/(Max-Min)*100%** was used to convert the raw data into inhibition rate, and the $IC_{50}$ value can be obtained by curve fitting with four parameters ("log(inhibitor) vs. response -- Variable slope" mode in GraphPad Prism).

**Max well:** The reading value of the positive control well was that of 1X lysate.

**Min well:** The reading value of negative control well was that of the cell lysate in 0.5% DMSO cell well.

**4. Assay results**

The result is shown in Table 6.

Table 6 $IC_{50}$ value of the compound on inhibiting p-ERK in PANC0403 cells

| **Compound No.** | **PANC0403 p-ERK $IC_{50}$ (nM)** |
|---|---|
| Compound 29B | 574.2 |

Assay conclusion: The compound of the present disclosure has significant inhibitory effect on p-ERK in PANC0403 cells.

**Assay example 5. Anti-cell proliferation effect of compounds in tumor cell lines AsPC-1 and GP2D**

**Purpose of study**

In this assay, the effect of the compounds on inhibiting cell proliferation was studied by detecting the effect of the compounds on the cell activity in vitro in the tumor cell lines AsPC-1 and GP2D.

**Materials of the assay**

Table 7. Materials of the assay

| Cell line | Tumor type | Growth characteristics | Culture method |
|---|---|---|---|
| AsPC-1 | Pancreatic cancer | Adherent | RPMI 1640 + 10% FBS |
| GP2D | Colon cancer | Adherent | DMEM + 10% FBS + 2 mM L-glutamine |

Ultra Low Cluster-96-well plate (Corning-7007)
Greiner CELLSTAR 96-well plate (# 655090)
Promega CellTiter-Glo 3D Luminescence Cell Viability Assay Kit (Promega-G9683)
2104-10 EnVision Plate Reader, PerkinElmer
RPMI 1640, DMEM, PBS (phosphate buffered saline), FBS (fetal bovine serum), Antibiotic-antimycotic, L-glutamine, DMSO (dimethylsulfoxide)

**Method and steps of the assay**

**Cell culture**

The tumor cell lines were cultured in a 37 °C, 5% $CO_2$ incubator according to the culture conditions indicated in the culture method. Cells were passaged regularly, and cells in logarithmic growth phase were used for plating.

**Cell plating**

Cells were stained with trypan blue and viable cells were counted.
The cell concentration was adjusted to an appropriate concentration.

Table 8. Cell density

| **Cell line** | **Density (per well)** |
|---|---|
| AsPC-1 | 7000 cells |
| GP2D | 8000 cells |

To each well of a ULA culture plate was added 135 μL of cell suspension. To the blank control well was added the same volume of culture medium without cells.
After plating, the ULA culture plate was immediately centrifuged at room temperature at 1000 rpm for 10 minutes. Note: After completion of the centrifugation, subsequent operations must be processed carefully to avoid unnecessary shocks.
The culture plate was incubated in a 37 °C, 5% $CO_2$, 100% relative humidity incubator overnight.

**Formulation of 10X compound working solution and treatment of cells with compound (day 1)**

10X compound working solution (10X working solution in DMSO) was formulated, and then to the ULA culture plate was add 15 μL of 10X compound working solution, and to the vehicle control and blank control was added 15 μL of DMSO-cell culture medium mixture, respectively.
The 96-well cell plate was placed back to the incubator and incubated for 120 hours.
The sphere formation of cells was observed every day until the end of the assay.

**CellTiter-Glo luminescent cell viability detection (Day 5)**

The following steps were performed according to the instructions of the Promega CellTiter-Glo 3D Luminescent Cell Viability Assay Kit (Promega # G9683).

150 μL (equal to the volume of cell culture medium in each well) of CellTiter-Glo 3D reagent was added to each well. The cell plate was wraped by aluminum foil to protect from light.

The culture plate was shaked on an orbital shaker for 5 minutes.

The mixture in wells was mixed thoroughly by pipetting up and down 10 times carefully with pipette, so as to make sure that the spheroids of cells were sufficiently detached before proceeding to the next step.

The solution in the ULA plate was then transferred to a black bottom culture plate (#655090) and left to stand at room temperature for 25 minutes to stabilize the luminescent signal.

The luminescent signal was detected on a 2104 EnVision plate reader.

**Data analysis**

The following formula was used to calculate inhibition rate (IR) of the assay compound: IR (%) = (1 - (RLU of compound - RLU of blank control) / (RLU of vehicle control - RLU of blank control))* 100%. The inhibition rates of different concentrations of compounds were calculated in Excel, and then GraphPad Prism software was used to draw inhibition curves and calculate related parameters, including minimum inhibition rate, maximum inhibition rate, and $IC_{50}$.

**Assay results**

The results are shown in Table 9.

Table 9 $IC_{50}$ value of the compounds on inhibiting $KRAS^{G12D}$ cells

| Compound No. | $KRAS^{G12D}$ AsPC-1 $IC_{50}$ (nM) | $KRAS^{G12D}$ GP2D $IC_{50}$ (nM) |
|---|---|---|
| Compound 1 hydrochloride | 315 | 37 |
| Compound 2 hydrochloride | 1865 | 332 |

Assay conclusion: The compounds of the present disclosure have inhibitory effect on $KRAS^{G12D}$ cell mutation.

**Assay example 6. Assay of plasma protein binding (PPB)**

**Purpose**

The protein binding rates of the compound in the plasma of CD-1 mouse, Sprague-Dawley rat, Beagle dog, cynomolgus monkey and human were determined by equilibrium dialysis method.

**Method of the assay**

The plasmas of the above-mentioned five species were formulated to plasma samples with a compound concentration of 2 μM. The plasma samples were placed in a 96-well rapid equilibrium dialysis device, and dialyzed against phosphate buffered saline at 37±1 °C for 4 h. In this assay, warfarin was used as a control compound. The concentrations of analytes in plasma and dialysis buffer were determined by LC-MS/MS mothed.

**Assay results**

The unbound rate (%) of compound 1 hydrochloride at the assay concentration of 2 μM is shown in Table 10 below.

Table 10. Result of PPB assay

| Compound No. | Unbound PPB H/C/D/R/M |
|---|---|
| Compound **1** hydrochloride | 7.0/6.3/5.2/3.0/2.7 |

The recovery rate (%) of compound 1 in the dialysis device was 82.4-109.5, which met the requirements of this assay for recovery rate and stability.

**Assay results**

The compound of the present disclosure exhibits good free-state concentration in the plasma of the above-mentioned five species at the assay concentration of 2 μM.

**Assay example 7. Pharmacokinetic study of oral and intravenously injected assay compounds in CD-1 mouse**

**Purpose**

To assay the pharmacokinetics of oral and intravenously injected compounds in CD-1 mouse.

**Steps of the assay**

The assay compound was mixed with 5% DMSO+95% (10%HP-β-CD) aqueous solution. The mixture was vortexed and sonicated to prepare a clear solution of 0.5 mg/mL (intravenous) or a clear solution of 3 mg/mL (oral). The clear solution was filtered by a microporous membrane for use. Male SD mice aged 7 to 10 weeks were selected, administered the candidate compound solutions intravenously at a dose of about 2 mg/kg, and administered the candidate compound solutions orally at a dose of about 30 mg/kg. Whole blood was collected for a certain period of time, and prepared to obtain plasma. Drug concentration was analyzed by LC-MS/MS method, and pharmacokinetic parameters were calculated by Phoenix WinNonlin software (Pharsight, USA).

**Assay results**

The results are shown in Tables 11 and 12.

Table 11. Intravenous (IV) PK data

| Assay sample | Compound **1** hydrochloride | Compound **11A** hydrochloride | Compound **20B** |
|---|---|---|---|
| Dosage (mg/kg) | 1.82 | 2.14 | 2.04 |
| $C_0$ (nM) | 1400 | 1593 | 449 |
| $T_{1/2}$ (h) | 23.1 | 15.6 | 11.2 |
| $V_d$ (L/kg) | 148 | 56.2 | 97.2 |
| Cl (mL/Kg/min) | 106 | 62.8 | 138 |
| $AUC_{0\text{-last}}$ (nM.h) | 353 | 690 | 371 |

Table 12. Oral (PO) PK data

| Assay sample | Compound **1** hydrochloride | Compound **11A** hydrochloride | Compound **20B** |
|---|---|---|---|
| Dosage (mg/kg) | 29.3 | 30.3 | 30.3 |
| $C_{max}$ (nM) | 228 | 617 | 2095 |
| $T_{max}$ | 4.5 | 2 | 1.5 |
| $T_{1/2}$ (h) | ND | 8 | 14.4 |
| $AUC_{0\text{-inf}}$ (nM.h) | 1350 | 2403 | 4710 |
| | | | |
| F | 25.5% | 23.2% | 85% |

**Assay conclusion**

The compounds of the present disclosure have good oral bioavailability.

**Assay example 8. Assay on pharmacodynamics in vivo**

Method of the assay:

A Balb/c nude mouse model of subcutaneously xenograft tumor of human colon cancer GP2D cell was established. 0.2 mL (2×$10^6$) of GP2D cells (Matrigel was added, and volume ratio was 1:1) were inoculated subcutaneously on the right back of each mouse. When the average tumor volume reached 149 $mm^3$, the administration was started by group, with 6 mice in each group. On the day of the assay, the animals were administered the corresponding drugs according to the corresponding groups. The first group G1 was set as a negative control group, which was given 5%DMSO+95%(10%HP-β-CD) alone by gavage administration. The second group G2 to the fourth group G4 were given compound 1 hydrochloride, and the dosage and protocol of administration were shown in Table 13.

Table 13 Pharmacodynamic study of the assay compound on transplanted tumor of human diffuse large B lymphoma TMD8 in mouse

| Group | Number of animals | Assay compound | Dosage (mg/kg) | Administration concentration (mg/mL) | Administration volume (mL/kg) | Route and frequency of administration |
|---|---|---|---|---|---|---|
| G1 | 6 | Negative control | (N/A) | (N/A) | (N/A) | PO,BID*20 |
| G2 | 6 | Compound **1** hydrochloride | 3 | 0.3 | 10 | PO,BID*20 |
| G3 | 6 | Compound **1** hydrochloride | 10 | 1 | 10 | PO,BID*20 |
| G4 | 6 | Compound **1** hydrochloride | 30 | 3.0 | 10 | PO,BID*20 |
| Note: PO means oral administration, QD means once daily, BID means once daily. | | | | | | |

During the assay, the body weight of animals and the tumor size were measured twice a week. Meanwhile clinical symptoms of animals were observed and recorded every day. Each administration was referenced to the most recent body weight of animals.

The length (a) and width (b) of a tumor were measured with a digital vernier caliper. The calculation formula of tumor volume (Tumor volume, TV) was: $TV=a\times b^2/2$.

**Assay results:**

Compound **1** hydrochloride has a significant inhibitory effect on the xenograft tumor of human colon cancer GP2D in mouse. After 20 days of administration, the tumor volume inhibition rate TGI (%) of the second group G2 (3 mg/kg, PO, BID) was 19.4% on the 20th day; the tumor volume inhibition rates TGI (%) of the third group G3 (10 mg/kg, PO, BID) and the fourth group G4 (30 mg/kg, PO, BID) were 53.9% and 83.7% on the 20th day, respectively; the detailed results are shown in Table 14.

Table 14 Effects of the assay compound on animal tumor size in xenograft tumor model of human colon cancer GP2D in mouse

| Group | Number of animals | Assay compound | Frequency of administration | Dose mg/kg | Tumor volume inhibition rate TGI (%) |
|---|---|---|---|---|---|
| G1 | 6 | Negative control | PO,BID*20 | NA | N/A |
| G2 | 6 | Compound **1** hydrochloride | PO,BID*20 | 3 | 19.4 |

(continued)

| Group | Number of animals | Assay compound | Frequency of administration | Dose mg/kg | Tumor volume inhibition rate TGI (%) |
|---|---|---|---|---|---|
| G3 | 6 | Compound **1** hydrochloride | PO,BID*20 | 10 | 53.9 |
| G4 | 6 | Compound **1** hydrochloride | PO,BID*20 | 30 | 83.7 |
| Note: N/A means no detection. | | | | | |

**Assay conclusion:** In the aspect of efficacy in vivo, the compound of the present disclosure exhibits good tumor-inhibiting effect in GP2D cell line, and there is an obvious dose-dependence.

## Claims

1. A compound represented by formula (III) or a pharmaceutically acceptable salt thereof,

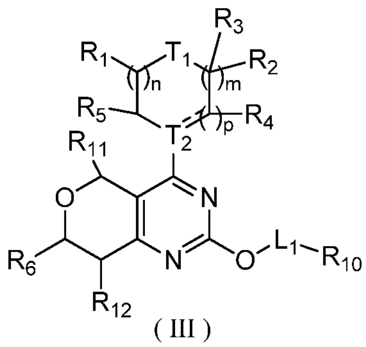

( III )

,

structural moiety

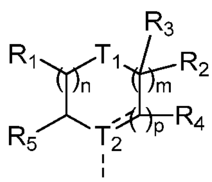

is selected from

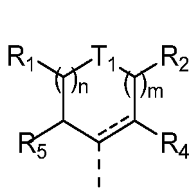

,

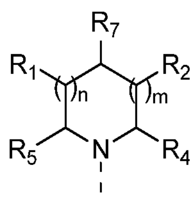

,

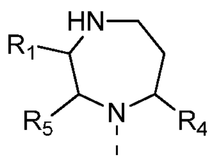, 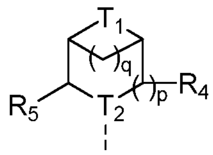, 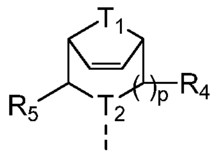, 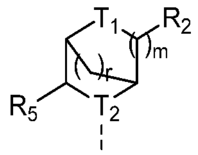, 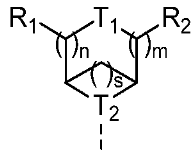,

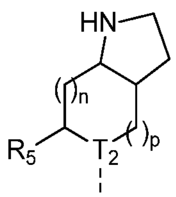, 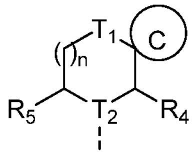 and 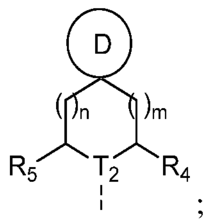;

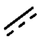

is selected from a single bond and double bond;

$T_1$ is selected from $CR_7R_8$, $NR_9$ and O;

$T_2$ is selected from CH and N;

Li is selected from $-CH_2-$ and a bond;

$R_1$, $R_2$, $R_3$, $R_4$ and $R_5$ are each independently selected from H and $C_{1-3}$ alkyl, wherein the $C_{1-3}$ alkyl is optionally substituted by 1, 2 or 3 $R_a$;

$R_6$ is selected from $C_{6-10}$ aryl and 5-10 membered heteroaryl, wherein the $C_{6-10}$ aryl and 5-10 membered heteroaryl are optionally substituted by 1, 2, 3, 4 or 5 $R_b$;

$R_7$ and $R_8$ are each independently selected from H, $CH_3$ and $NH_2$;

$R_9$ is selected from H and $CH_3$;

$R_{10}$ is selected from 4-8 membered heterocycloalkyl and

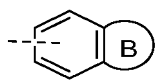,

wherein the 4-8 membered heterocycloalkyl and

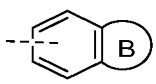

are optionally substituted by 1, 2 or 3 $R_c$;

$R_{11}$ and $R_{12}$ are each independently selected from H, $C_{1-3}$ alkyl and $C_{3-5}$ cycloalkyl, wherein the $C_{1-3}$ alkyl and $C_{3-5}$ cycloalkyl are optionally substituted by 1, 2 or 3 halo;

structural moiety

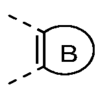

is 5-6 membered heterocycloalkenyl;

structural moiety

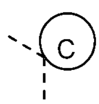

is $C_{3-5}$ cycloalkyl;
structural moiety

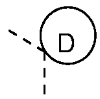

is 4-5 membered heterocycloalkyl;
m is selected from 0, 1 and 2;
n is selected from 0, 1 and 2;
p is selected from 0, 1 and 2;
q is selected from 1, 2 and 3;
r is selected from 1 and 2;
s is selected from 1, 2 and 3;
$R_a$ is each independently selected from F, Cl, Br and I;
$R_b$ is each independently selected from F, Cl, Br, I, OH, $NH_2$, CN, $C_{1-3}$ alkyl, $C_{1-3}$ alkoxy, $C_{2-3}$ alkynyl, $C_{2-3}$ alkenyl, $-C(=O)C_{1-3}$ alkyl and $C_{3-5}$ cycloalkyl, wherein the $C_{1-3}$ alkyl, $C_{1-3}$ alkoxy, $C_{2-3}$ alkynyl, $C_{2-3}$ alkenyl, $-C(=O)C_{1-3}$ alkyl and $C_{3-5}$ cycloalkyl are optionally substituted by 1, 2, 3, 4 or 5 R;
$R_c$ is each independently selected from H, F, Cl, Br, I, OH, CN, $C_{1-3}$ alkyl, $C_{1-3}$ alkoxy and $-C_{1-3}$ alkyl-O-C(=O)-Ci-s alkylamino;
R is each independently selected from F, Cl, Br and I.

2. The compound according to claim 1, or a pharmaceutically acceptable salt thereof, wherein $R_1$, $R_2$, $R_3$, $R_4$ and $R_5$ are each independently selected from H, $CH_3$, $CH_2CH_3$ and $CH(CH_3)_2$, wherein the $CH_3$, $CH_2CH_3$ and $CH(CH_3)_2$ are optionally substituted by 1, 2 or 3 $R_a$.

3. The compound according to claim 1 or 2, or a pharmaceutically acceptable salt thereof, wherein $R_1$, $R_2$, $R_3$, $R_4$ and $R_5$ are each independently selected from H and $CH_3$.

4. The compound according to claim 1, or a pharmaceutically acceptable salt thereof, wherein the structural moiety

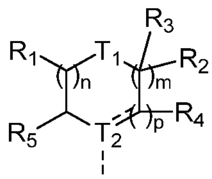

is selected from

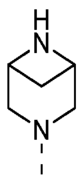 , 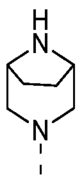 , 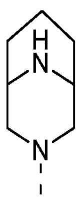 , 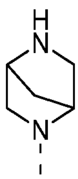 , 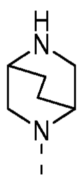 ,

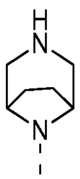 , 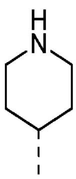 , 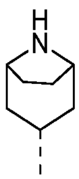 , 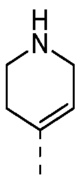 , 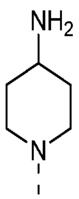 , 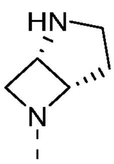 , 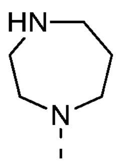 , 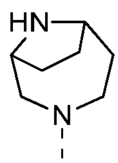 , 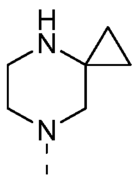 , 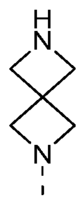 ,

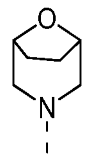 and 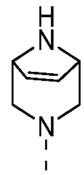 .

5. The compound according to claim 1, or a pharmaceutically acceptable salt thereof, wherein $R_b$ is each independently selected from F, Cl, Br, I, OH, $NH_2$, CN, $CH_3$, $CH_2CH_3$, $OCH_3$, $OCH_2CH_3$, $-CH=CH_2$, $-CH_2-CH=CH_2$, $-C\equiv CH$, $-C(=O)CH_3$ and cyclopropyl, wherein the $CH_3$, $CH_2CH_3$, $OCH_3$, $OCH_2CH_3$, $-CH=CH_2$, $-CH_2-CH=CH_2$, $-C\equiv CH$, $-C(=O)CH_3$ and cyclopropyl are optionally substituted by 1, 2, 3, 4 or 5 R.

6. The compound according to claim 5, or a pharmaceutically acceptable salt thereof, wherein $R_b$ is each independently selected from F, Cl, Br, I, OH, $NH_2$, CN, $CH_3$, $CF_3$, $CH_2CH_3$, $CF_2CF_3$, $-CH=CH_2$, $-C\equiv CH$, $-C(=O)CH_3$ and cyclopropyl.

7. The compound according to any one of claims 1, 5 and 6, or a pharmaceutically acceptable salt thereof, wherein $R_6$ is selected from phenyl, pyridyl, naphthyl, indolyl and indazolyl, wherein the phenyl, pyridyl, naphthyl, indolyl and indazolyl are optionally substituted by 1, 2, 3, 4 or 5 $R_b$.

8. The compound according to claim 7, or a pharmaceutically acceptable salt thereof, wherein $R_6$ is selected from

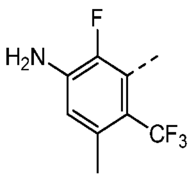 , 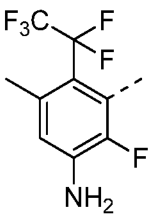 , 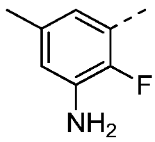 , 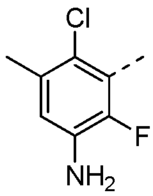 , 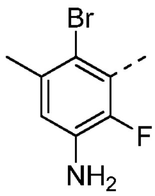 ,

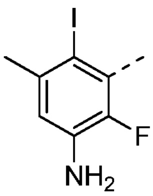 , 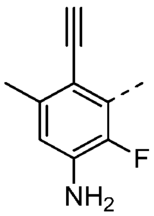 , 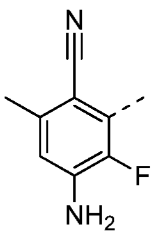 , 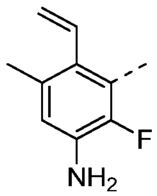 , 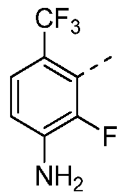 , 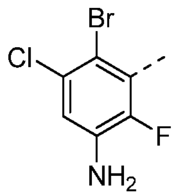 ,

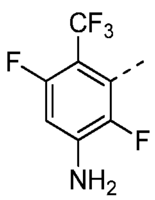 , 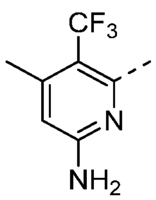 , 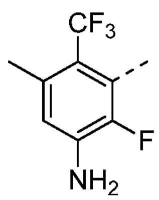 , 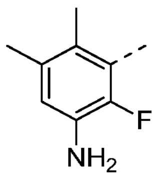 , 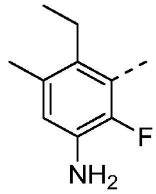 , 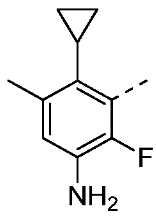 ,

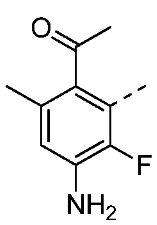 , 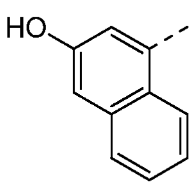 , 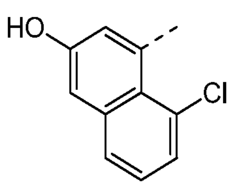 , 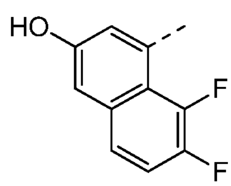 , 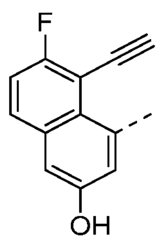 ,

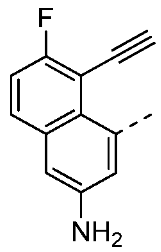

and

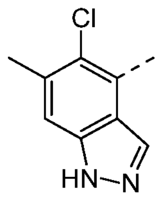

.

9. The compound according to claim 1, or a pharmaceutically acceptable salt thereof, wherein $R_c$ is each independently selected from H, F, Cl, Br, OH, CN, $CH_3$, $CH_2CH_3$, $CH_2CF_3$, $OCH_3$, $OCF_3$ and

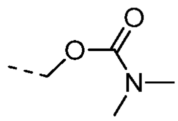

.

10. The compound according to claim 1, or a pharmaceutically acceptable salt thereof, wherein $R_{10}$ is selected from tetrahydropyrrolyl, hexahydro-1H-pyrrolizinyl and 1,2,3,4-tetrahydroisoquinolinyl, wherein the tetrahydropyrrolyl, hexahydro-1H-pyrrolizinyl and 1,2,3,4-tetrahydroisoquinolinyl are optionally substituted by 1, 2 or 3 $R_c$.

11. The compound according to any one of claims 1, 9 and 10, or a pharmaceutically acceptable salt thereof, wherein $R_{10}$ is selected from

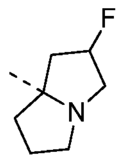

,

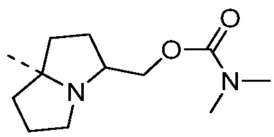

and

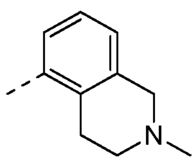

.

12. The compound according to claim 1, or a pharmaceutically acceptable salt thereof, wherein $R_{11}$ and $R_{12}$ are each independently selected from H and $CH_3$.

13. A compound represented by the following formula or a pharmaceutically acceptable salt thereof,

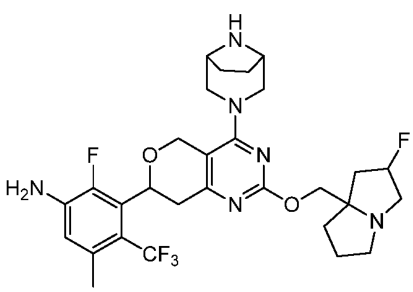

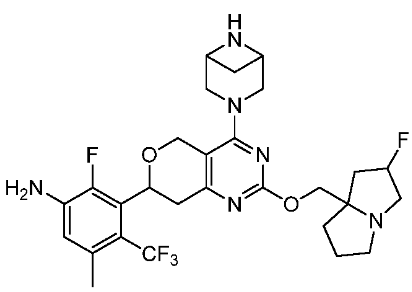

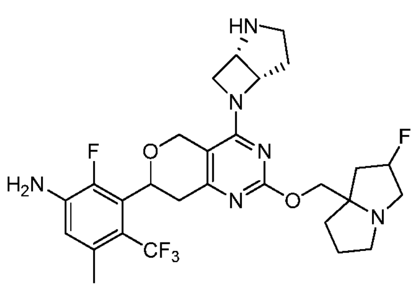

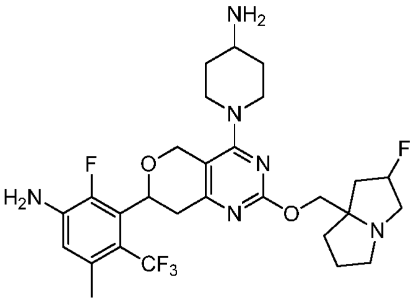
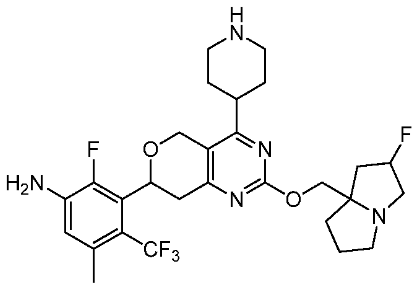
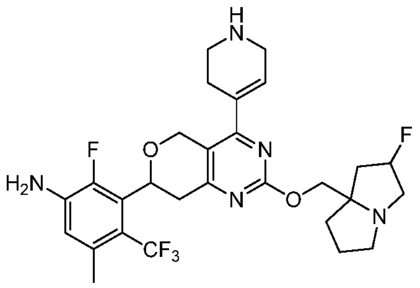

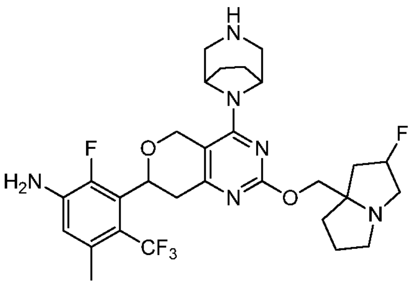
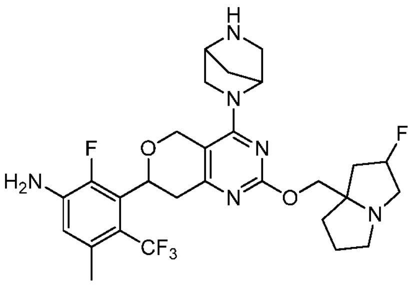
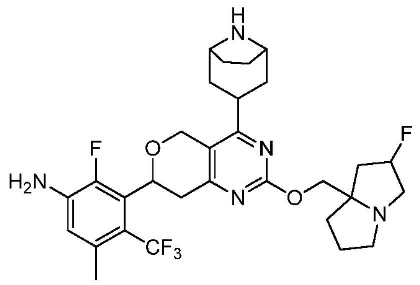

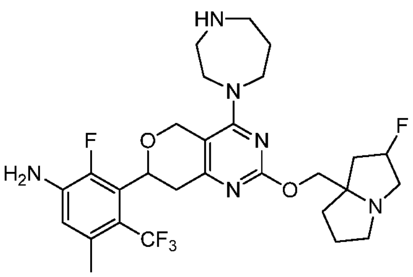
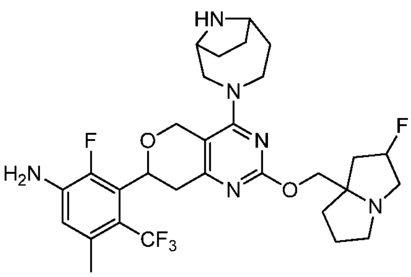
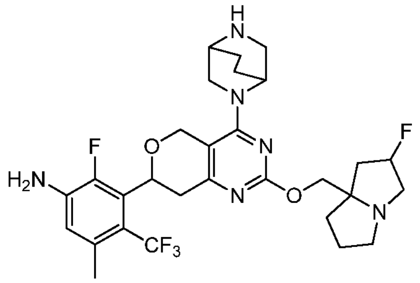

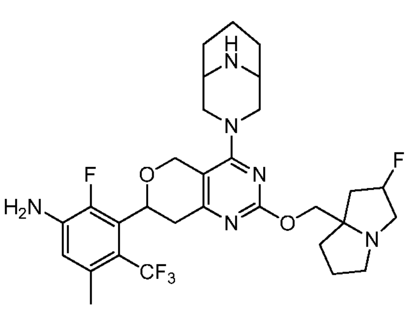
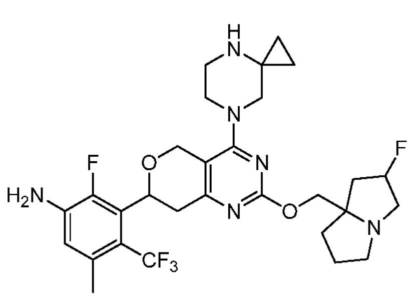
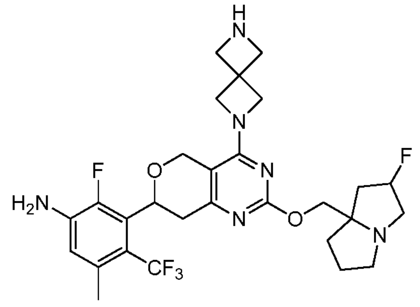

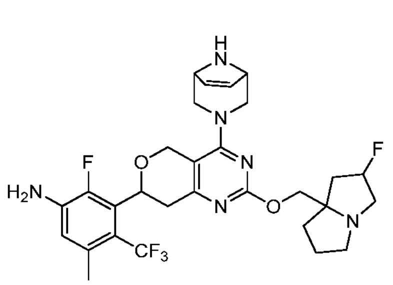
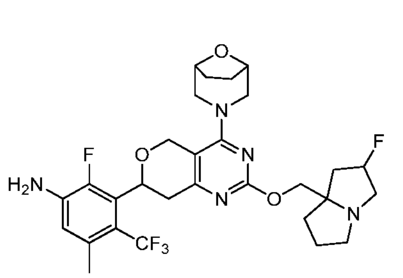
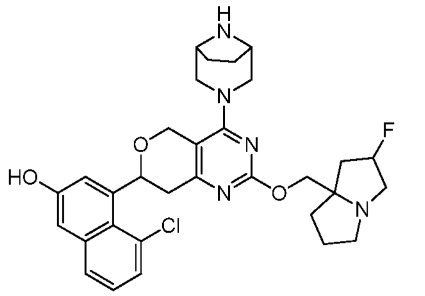

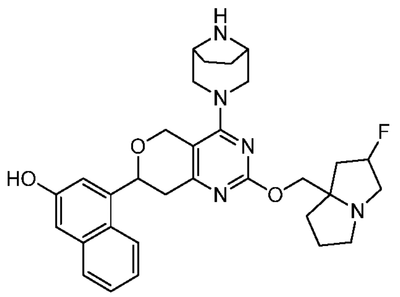
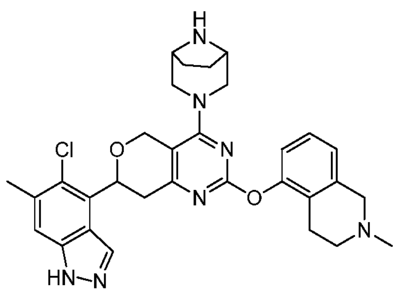
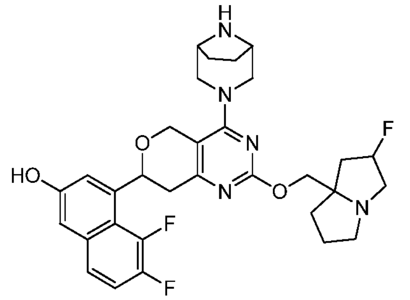
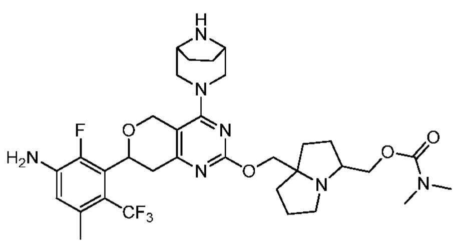
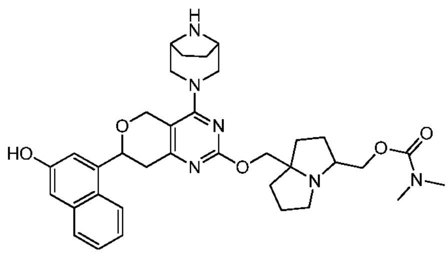
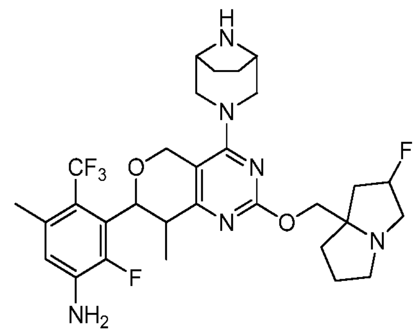
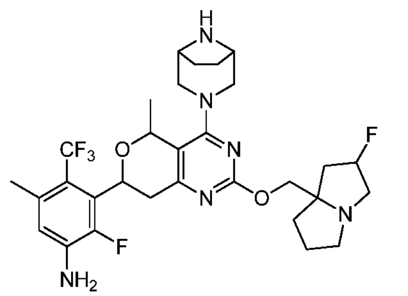
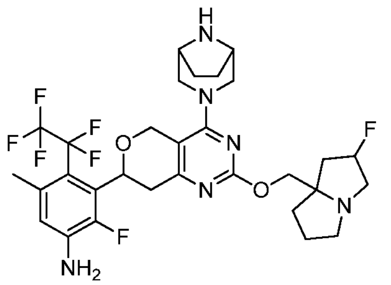
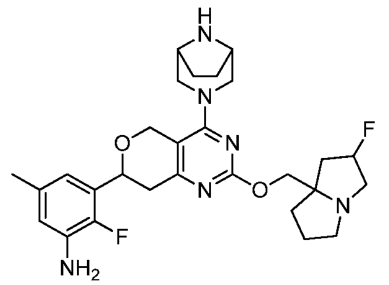
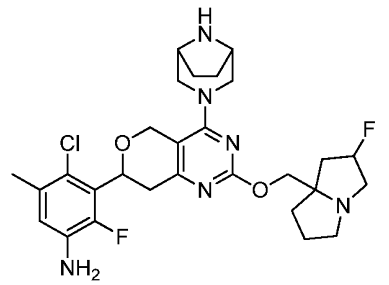
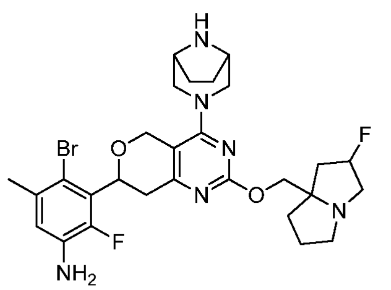
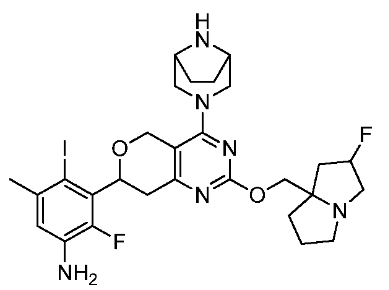
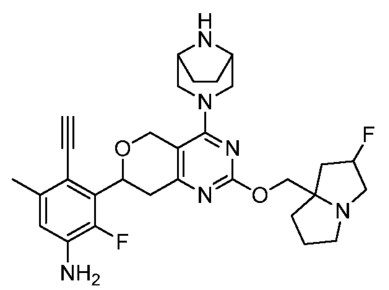

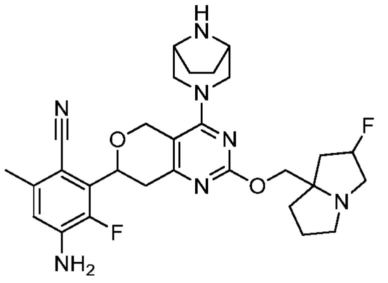
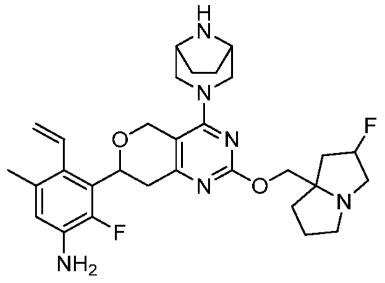
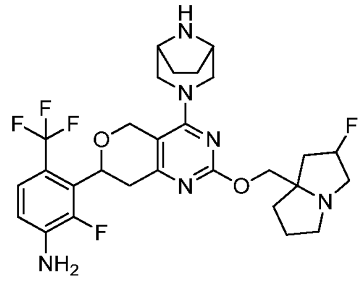

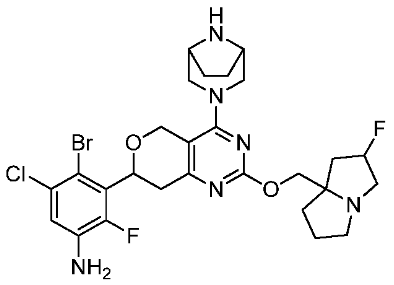
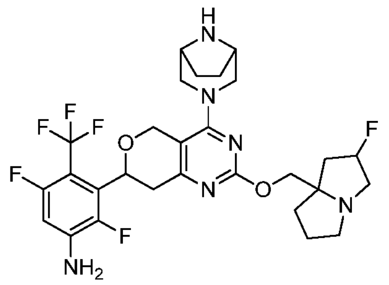
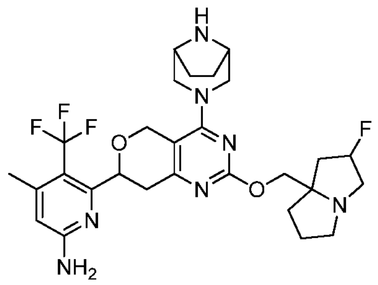

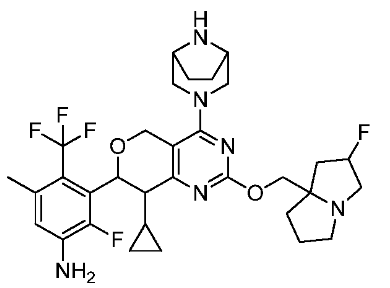
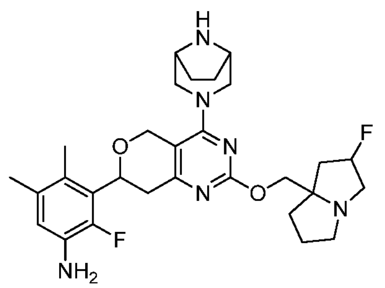
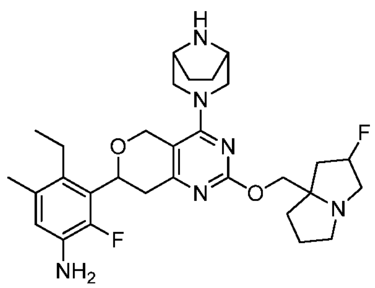

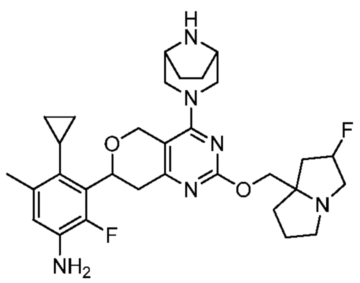
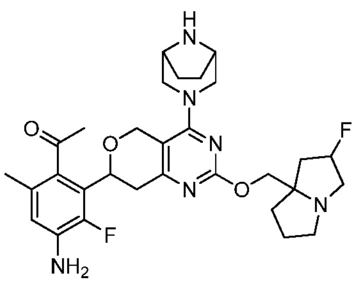
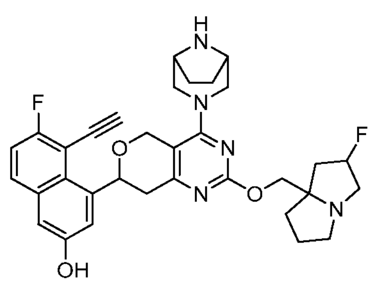
.

**14.** The compound according to claim 13, or a pharmaceutically acceptable salt thereof, wherein the compound is selected from:

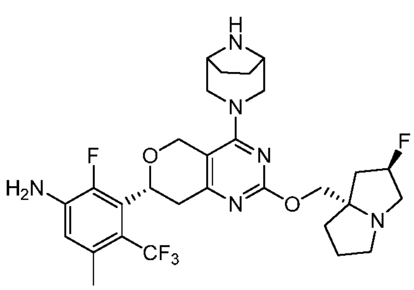
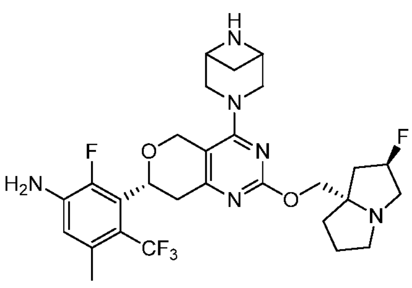
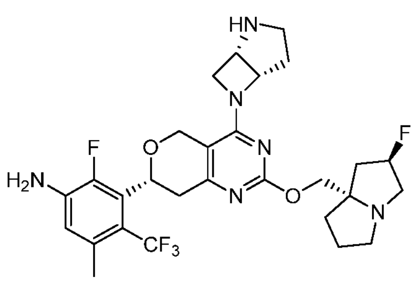

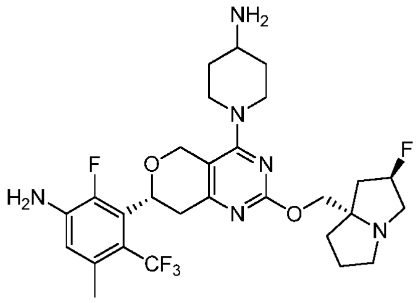

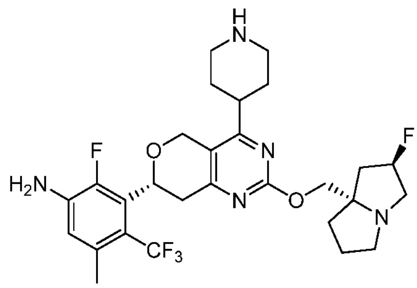

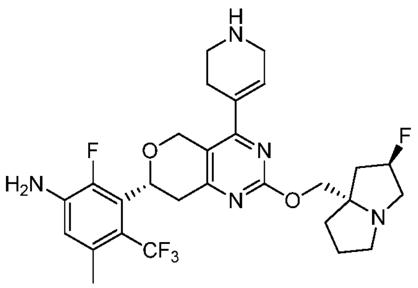

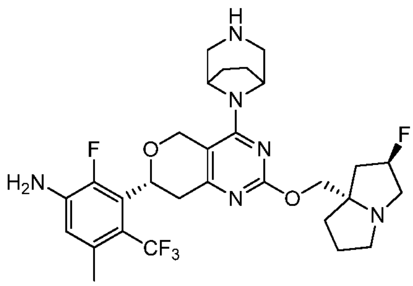

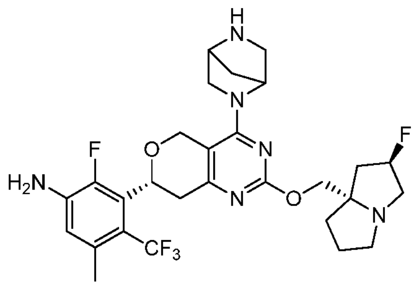

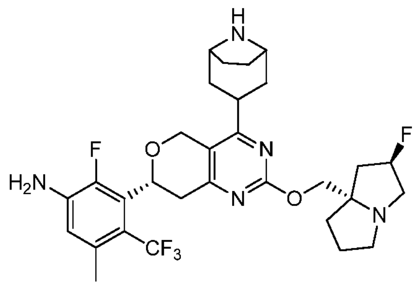

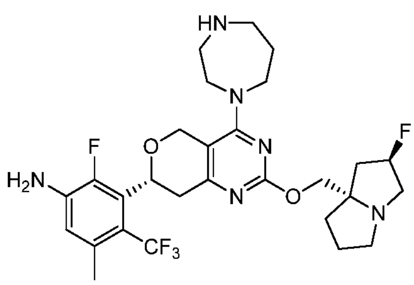

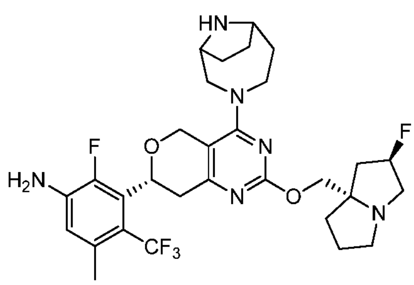

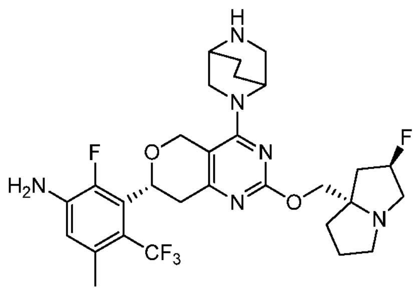

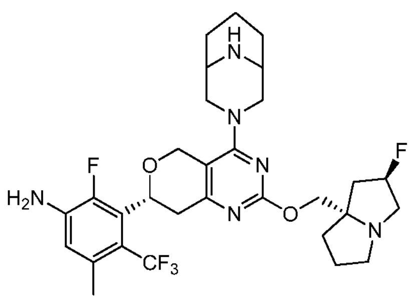

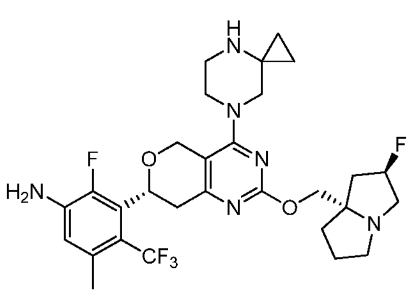

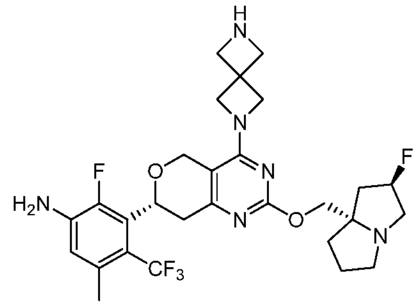

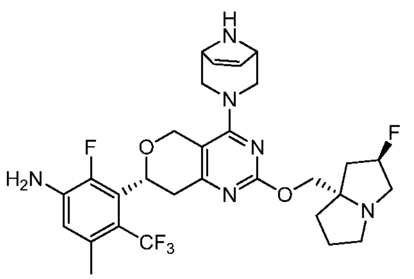

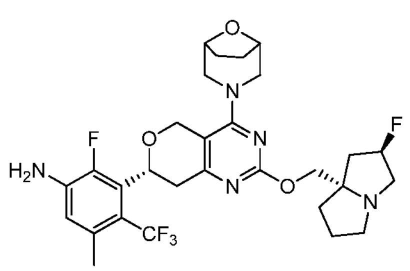

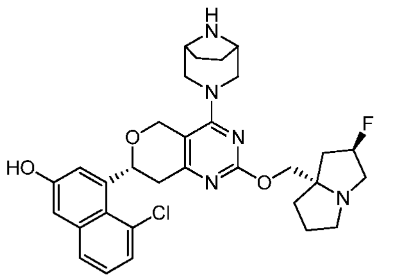

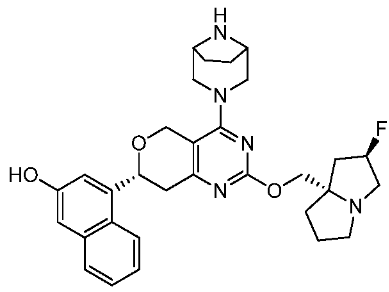
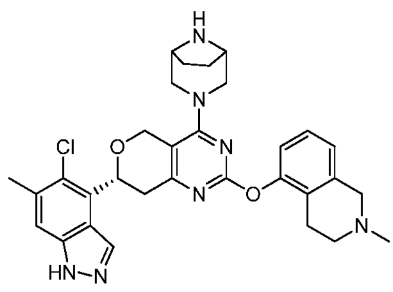
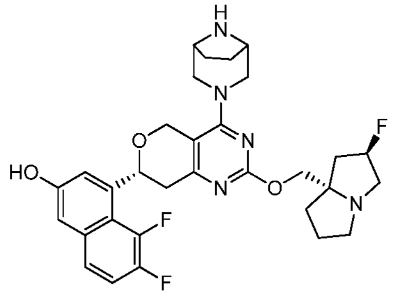

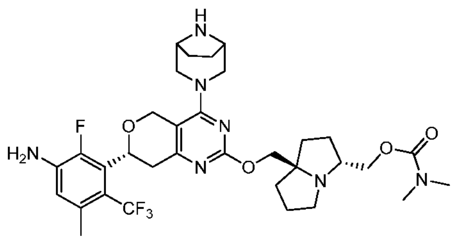
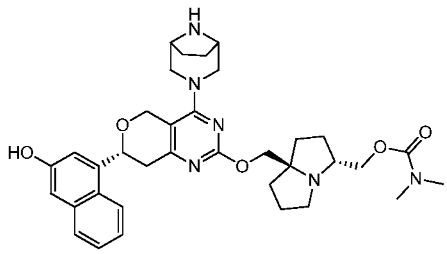

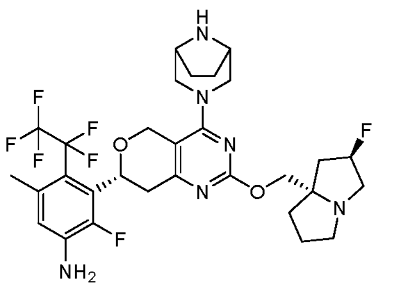
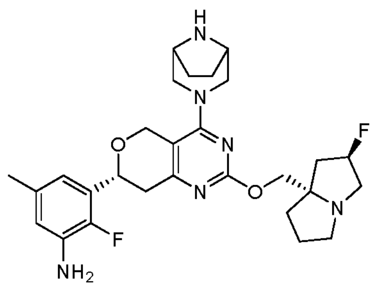

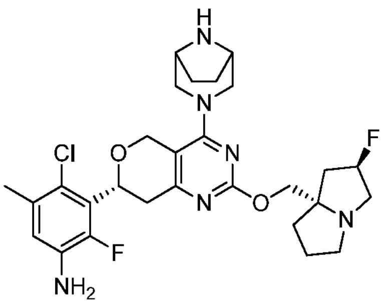
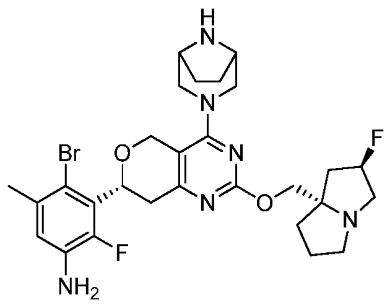
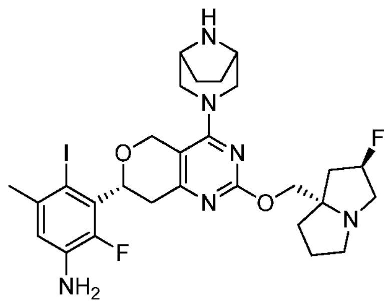

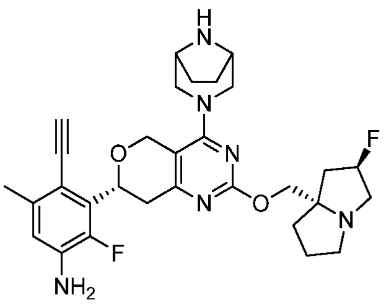
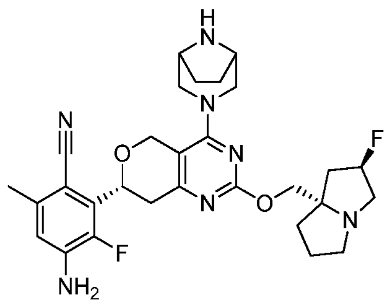
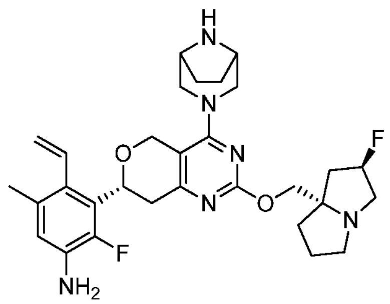

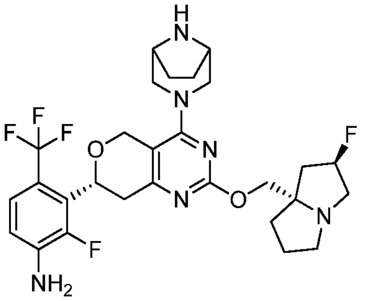
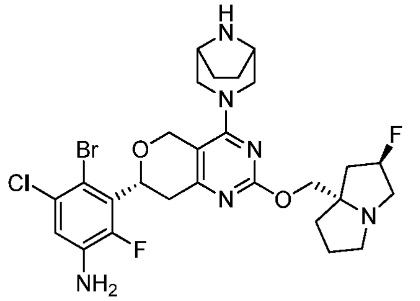
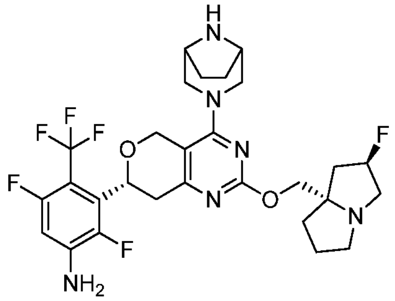

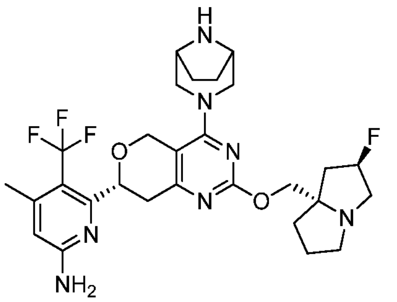
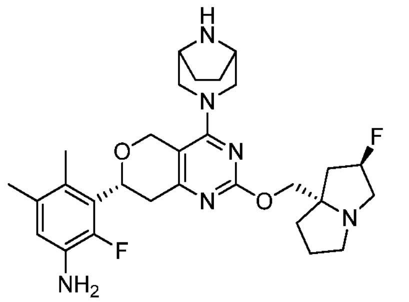
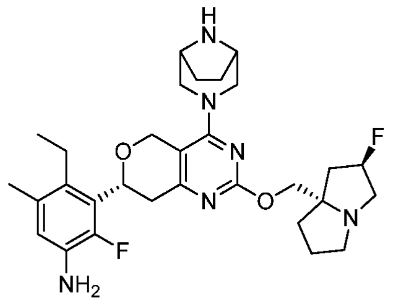

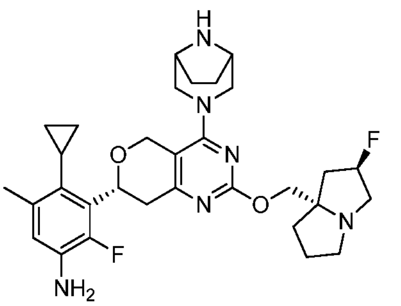
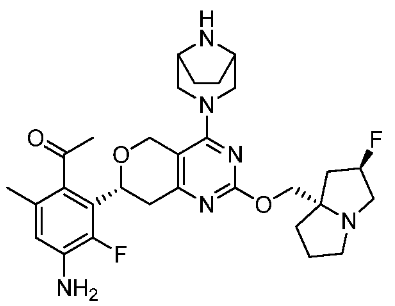
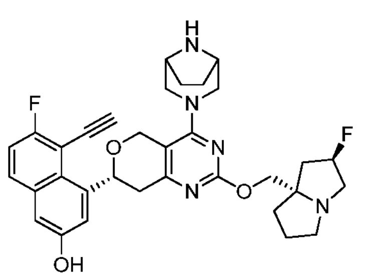

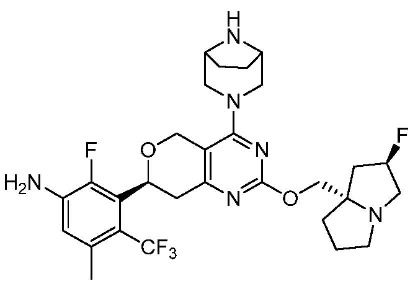
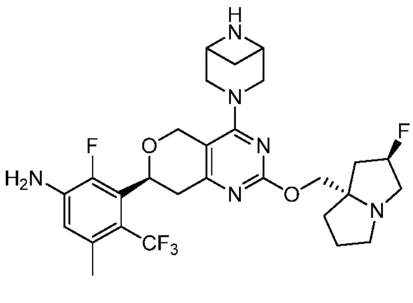
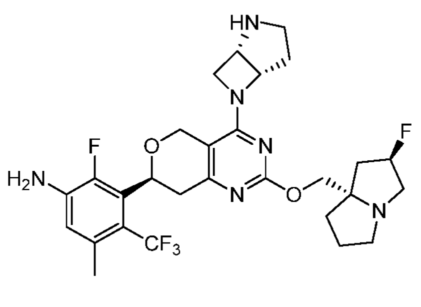

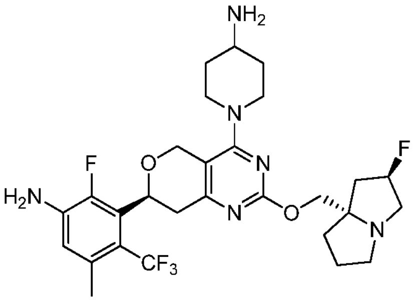
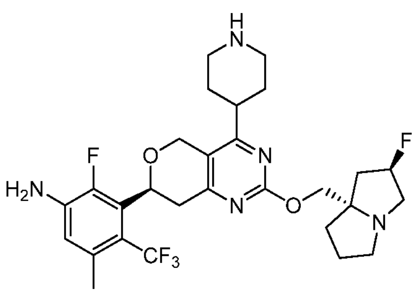
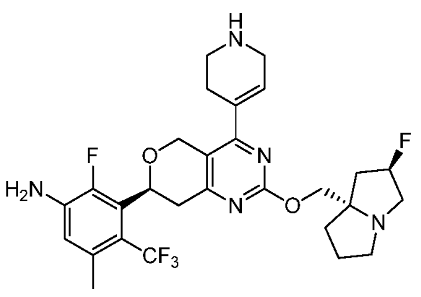

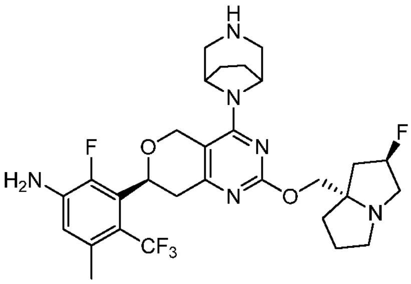
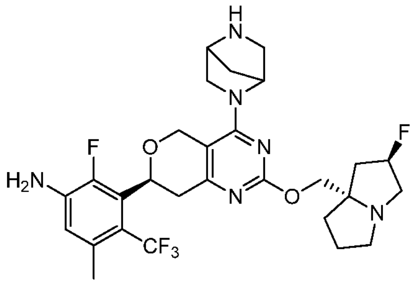
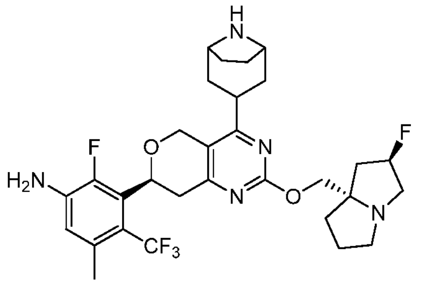

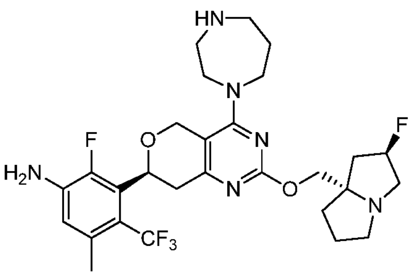
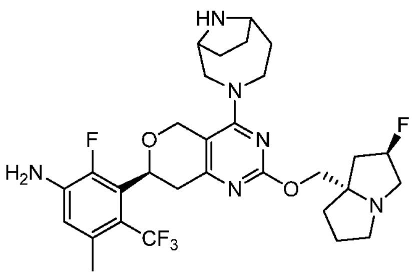
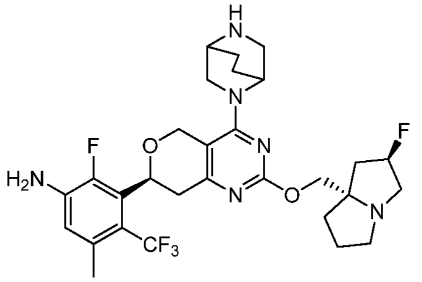

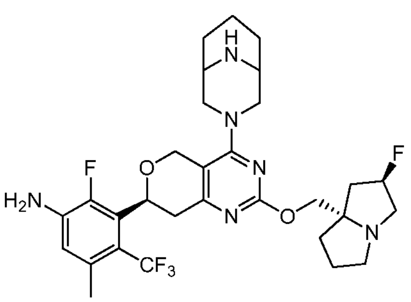
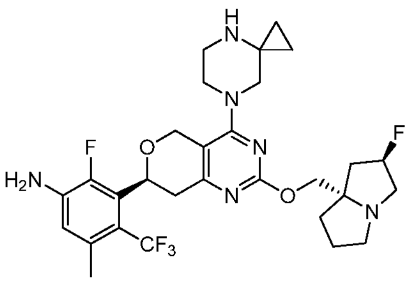
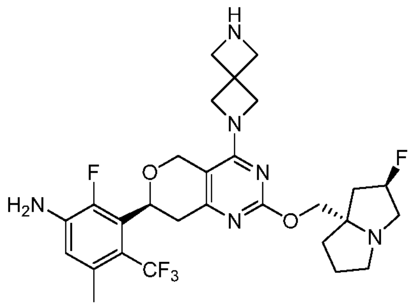

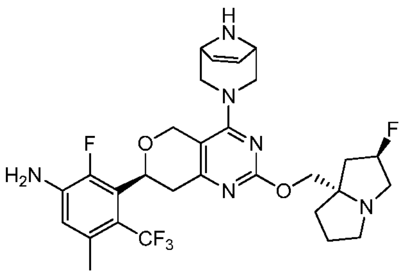
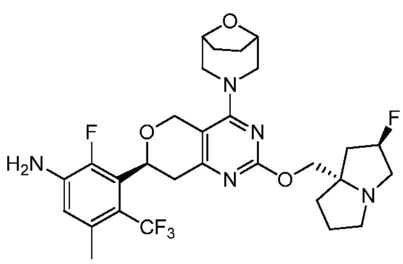
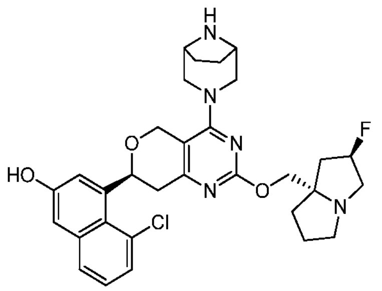

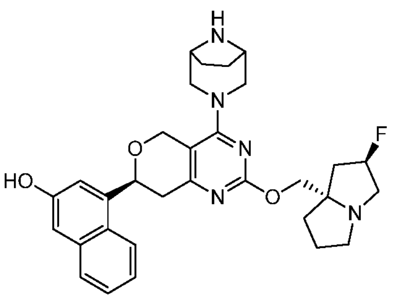
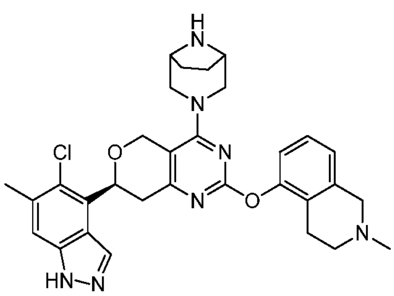
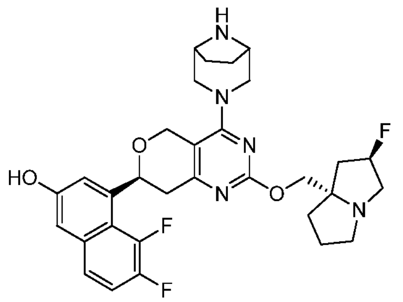

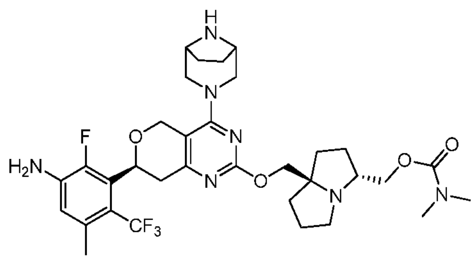

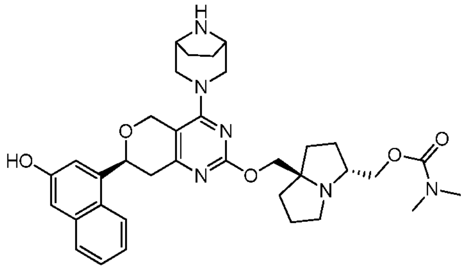

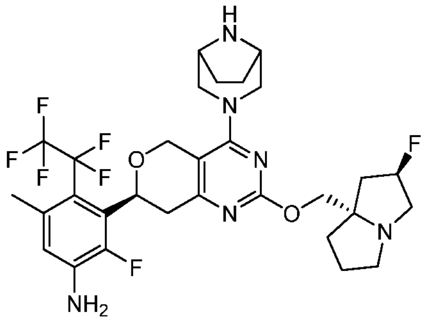

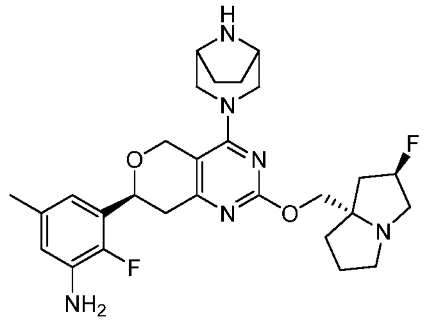

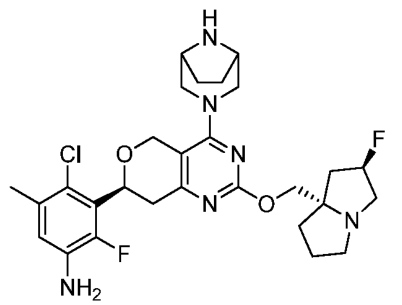

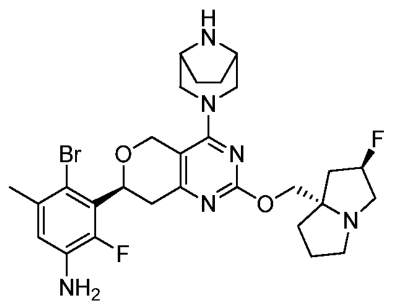

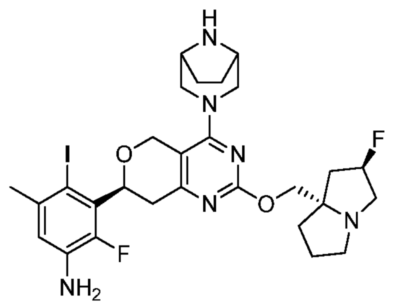

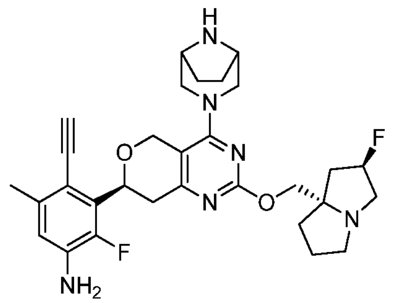

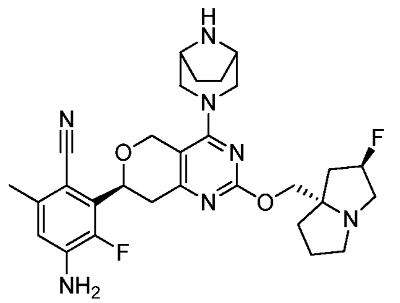

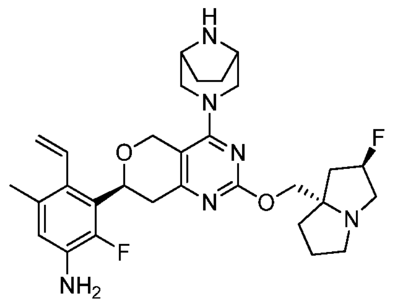

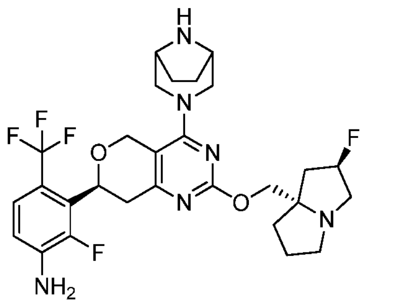

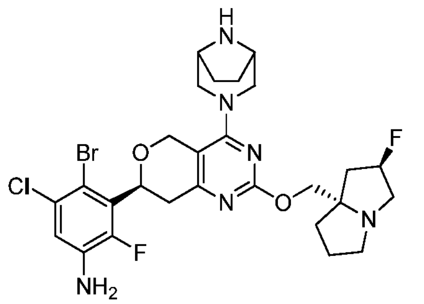

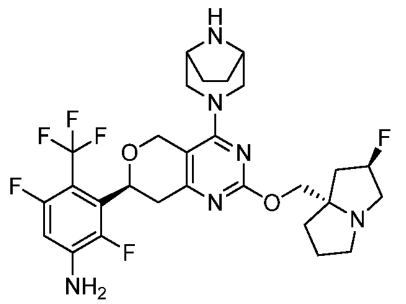

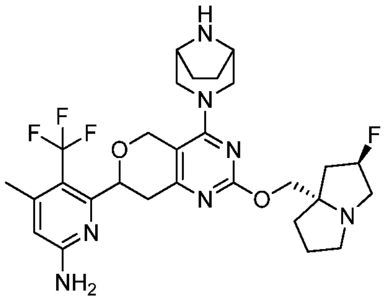
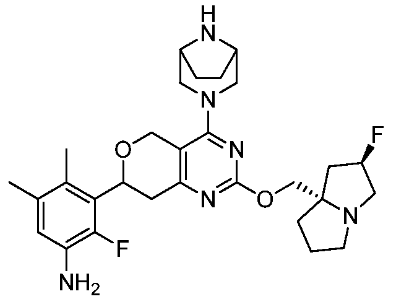
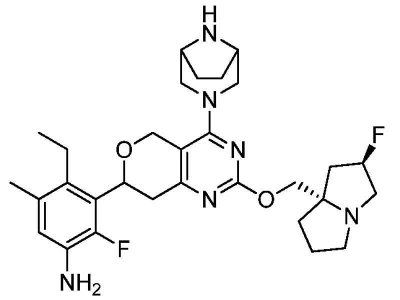
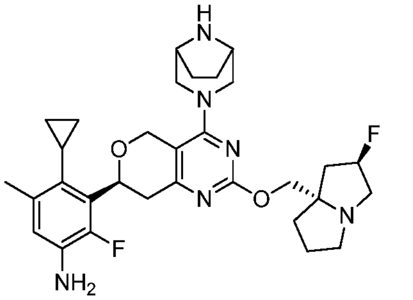
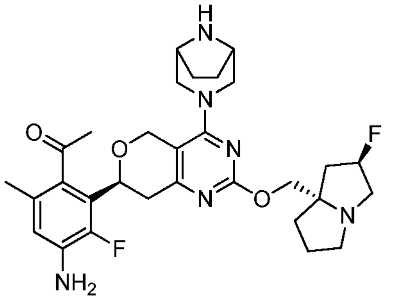
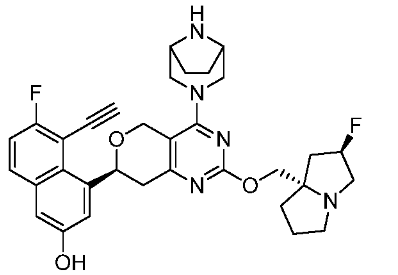
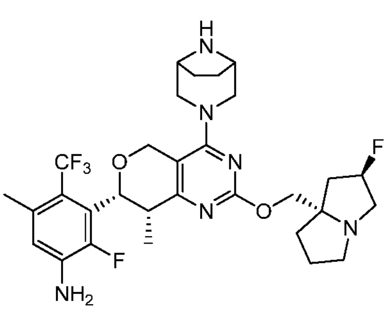
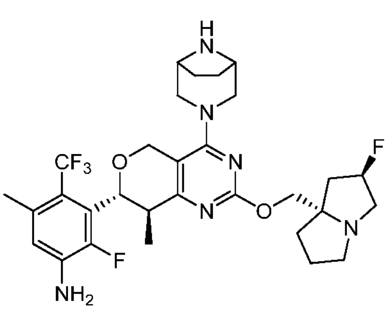
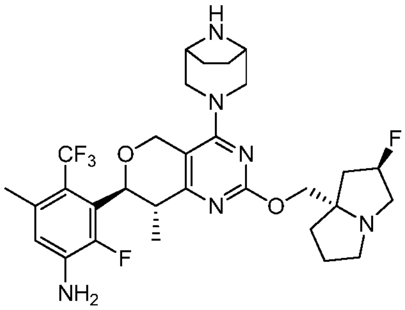
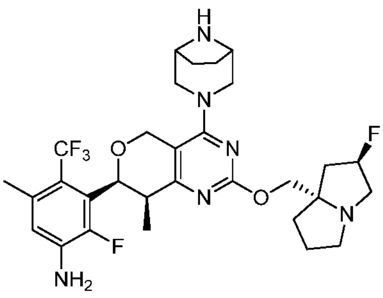
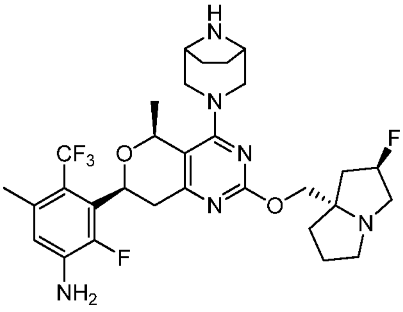
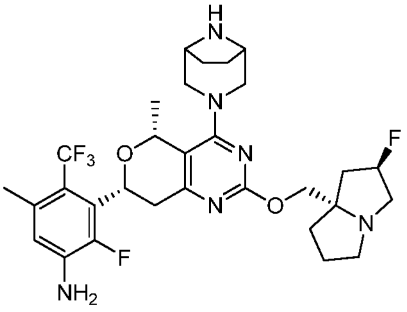
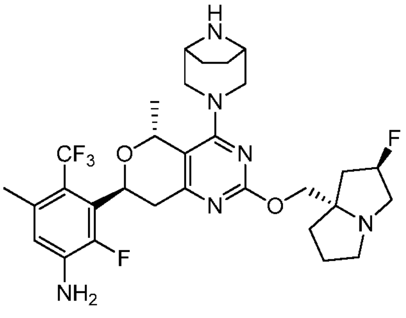
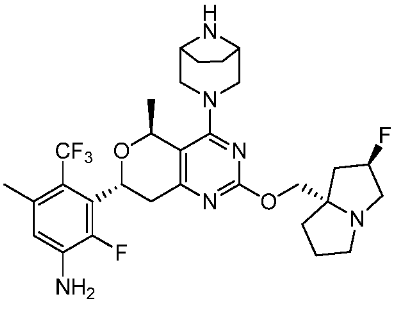
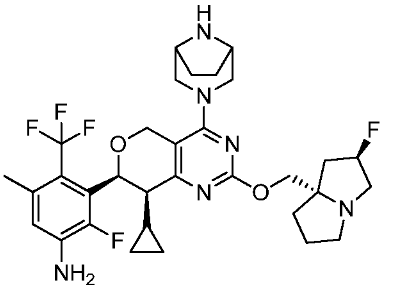

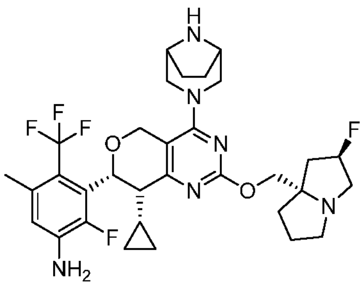
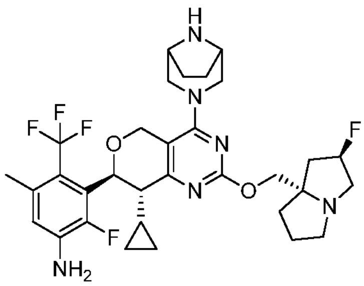
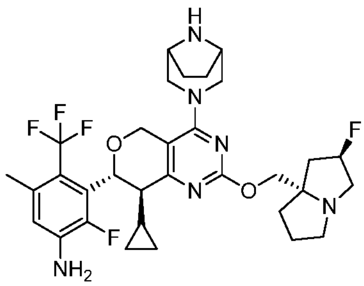
.

**15.** Use of the compound according to any one of claims 1-14, or a pharmaceutically acceptable salt thereof, in the manufacture of a medicament for treating diseases related to $KRAS^{G12D}$ mutation.

**INTERNATIONAL SEARCH REPORT**

International application No. **PCT/CN2022/074390**

**A. CLASSIFICATION OF SUBJECT MATTER**

C07D 403/14(2006.01)i; C07D 403/04(2006.01)i; C07D 491/052(2006.01)i; A61K 31/517(2006.01)i; A61K 31/519(2006.01)i; A61P 35/00(2006.01)i; A61P 35/02(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNABS, CNPAT, WPI, CNTXT, USTXT, EPTXT, WOTXT, ISI web of knowledge, STN: 明德新药, 嘧啶, 吡喃, 稠环, MEDSHINE DISCOVERY, pyrimidine, pyran, fuse+, cyclic, KRAS, structural formula search.

**C. DOCUMENTS CONSIDERED TO BE RELEVANT**

| Category* | Citation of document, with indication, where appropriate, of the relevant passages | Relevant to claim No. |
|---|---|---|
| PX | CN 112390788 A (SUZHOU WENTIAN PHARMACEUTICAL TECHNOLOGY CO., LTD.) 23 February 2021 (2021-02-23) claims 1-25 | 1-15 |
| A | WO 2020239123 A1 (SHANGHAI HANSOH BIOMEDICAL CO., LTD. et al.) 03 December 2020 (2020-12-03) embodiments and claims | 1-15 |
| A | WO 2020035031 A1 (GENENTECH, INC. et al.) 20 February 2020 (2020-02-20) embodiments and claims | 1-15 |
| A | WO 2019099524 A1 (MIRATI THERAPEUTICS, INC. et al.) 23 May 2019 (2019-05-23) embodiments and claims | 1-15 |
| A | WO 2017201161 A1 (MIRATI THERAPEUTICS, INC. et al.) 23 November 2017 (2017-11-23) embodiments and claims | 1-15 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **03 March 2022** | **07 May 2022** |
| Name and mailing address of the ISA/CN<br>**China National Intellectual Property Administration (ISA/CN)**<br>**No. 6, Xitucheng Road, Jimenqiao, Haidian District, Beijing 100088, China** | Authorized officer |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2022/074390**

| Patent document cited in search report | | | Publication date (day/month/year) | Patent family member(s) | | | Publication date (day/month/year) |
|---|---|---|---|---|---|---|---|
| CN | 112390788 | A | 23 February 2021 | None | | | |
| WO | 2020239123 | A1 | 03 December 2020 | None | | | |
| WO | 2020035031 | A1 | 20 February 2020 | AU | 2021218206 | A1 | 16 September 2021 |
| | | | | JP | 2021512135 | A | 13 May 2021 |
| | | | | JP | 6928185 | B2 | 01 September 2021 |
| | | | | CO | 2021003036 | A2 | 19 March 2021 |
| | | | | JP | 2021169491 | A | 28 October 2021 |
| | | | | BR | 112021002772 | A2 | 04 May 2021 |
| | | | | CR | 20210083 | A | 19 April 2021 |
| | | | | IL | 280797 | D0 | 29 April 2021 |
| | | | | EP | 3746436 | A1 | 09 December 2020 |
| | | | | AU | 2019320945 | A1 | 16 July 2020 |
| | | | | AU | 2019320945 | B2 | 03 June 2021 |
| | | | | AU | 2019320945 | C1 | 30 September 2021 |
| | | | | AR | 115978 | A1 | 17 March 2021 |
| | | | | SG | 11202101372 S | A | 30 March 2021 |
| | | | | MA | 51777 | A | 21 April 2021 |
| | | | | PH | 12021500014 | A1 | 13 September 2021 |
| | | | | KR | 20200115549 | A | 07 October 2020 |
| | | | | TW | 202035392 | A | 01 October 2020 |
| | | | | CN | 112119075 | A | 22 December 2020 |
| | | | | CA | 3086867 | A1 | 20 February 2020 |
| | | | | PE | 20211411 | A1 | 02 August 2021 |
| | | | | CL | 2021000387 | A1 | 20 August 2021 |
| WO | 2019099524 | A1 | 23 May 2019 | AU | 2018369759 | A1 | 25 June 2020 |
| | | | | ZA | 202002105 | B | 28 April 2021 |
| | | | | EP | 3710439 | A1 | 23 September 2020 |
| | | | | EP | 3710439 | A4 | 11 August 2021 |
| | | | | SG | 11202004427 T | A | 29 June 2020 |
| | | | | EA | 202091186 | A1 | 01 October 2020 |
| | | | | PH | 12020550622 | A1 | 25 January 2021 |
| | | | | KR | 20200100632 | A | 26 August 2020 |
| | | | | CN | 111989321 | A | 24 November 2020 |
| | | | | IL | 274601 | D0 | 30 June 2020 |
| | | | | CO | 2020007244 | A2 | 30 October 2020 |
| | | | | EP | 3880208 | A1 | 22 September 2021 |
| | | | | BR | 112020009818 | A2 | 03 November 2020 |
| | | | | US | 2019144444 | A1 | 16 May 2019 |
| | | | | US | 10689377 | B2 | 23 June 2020 |
| | | | | JP | 2021502993 | A | 04 February 2021 |
| | | | | US | 2020262837 | A1 | 20 August 2020 |
| | | | | TW | 201938555 | A | 01 October 2019 |
| | | | | CA | 3082579 | A1 | 23 May 2019 |
| | | | | WO | 2020101736 | A1 | 22 May 2020 |
| | | | | CL | 2020001271 | A1 | 18 December 2020 |
| WO | 2017201161 | A1 | 23 November 2017 | CN | 109843856 | A | 04 June 2019 |
| | | | | JP | 2019516718 | A | 20 June 2019 |
| | | | | EP | 3458445 | A1 | 27 March 2019 |
| | | | | EP | 3458445 | A4 | 13 November 2019 |
| | | | | EP | 3458445 | B1 | 17 February 2021 |

Form PCT/ISA/210 (patent family annex) (January 2015)

**INTERNATIONAL SEARCH REPORT**
**Information on patent family members**

International application No.
**PCT/CN2022/074390**

| Patent document cited in search report | Publication date (day/month/year) | Patent family member(s) | Publication date (day/month/year) |
|---|---|---|---|
| | | US 2018072723 A1 | 15 March 2018 |
| | | US 10125134 B2 | 13 November 2018 |
| | | IL 262867 D0 | 31 March 2019 |
| | | IL 262867 A | 01 January 2022 |
| | | US 2021269432 A1 | 02 September 2021 |
| | | SG 11201810171 S A | 28 December 2018 |
| | | CA 3024523 A1 | 23 November 2017 |
| | | AU 2017266911 A1 | 06 December 2018 |
| | | AU 2017266911 B2 | 02 September 2021 |
| | | MX 2018013983 A | 16 August 2019 |
| | | ES 2863873 T3 | 11 October 2021 |
| | | US 2019062330 A1 | 28 February 2019 |
| | | US 10633381 B2 | 28 April 2020 |
| | | KR 20190039475 A | 12 April 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202110139674X
- CN 202110258547
- CN 202110706033
- CN 202210070174X